# EUROPEAN PATENT APPLICATION

(11) **EP 1 944 306 A1**
(43) Date of publication of application: **16.07.2008**
(21) Application number: 07076016.0
(22) Date of filing: 15.09.2004
(51) Int. Cl.: C07D 487/04, A61K 31/435, A61K 31/55, A61P 25/00

(54) **Fused heterocyclic compounds as serotonin receptor modulators**

(30) Priority: 17.09.2003 US 504528 P; 11.03.2004 US 552673 P
(62) Divisional of application: 04816874.4
(71) Applicant: Janssen Pharmaceutica, N.V., 2340 Beerse (BE)
(72) Inventor: Carruthers, Nicholas I., Poway, 5 92065 (US); Chai, Wenying, San Diego California 92130 (US); Deng, Xiaohu, San Diego California 92129 (US); Dvorak, Curt A., San Diego, 5 92126 (US); Kwok, Annette K., Washington, DC 20001 (US); Liang, Jimmy T., San Diego California 92128 (US); Mani, Neelakandha, San Diego California 92129 (US); Rudolph, Dale A., San Deigo, 5 92128 (US); Wong, Victoria D., San Diego California 92126 (US)
(74) Representative: Fisher, Adrian John

(57) **Abstract**

Certain fused pyrrole-containing heterocyclic compounds are serotonin modulators useful in the treatment of serotonin-mediated diseases.

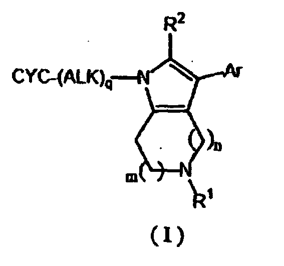

## Description

### Field of the Invention

There is provided by the present invention compounds that are serotonin receptor modulators. More particularly, there is provided by the present invention fused heterocyclic compounds that are serotonin receptor modulators useful for the treatment of disease states mediated by serotonin receptor activity.

### Background of the Invention

Serotonin (5-hydroxytryptamine, 5-HT) is a major neurotransmitter eliciting effects via a multiplicity of receptors. To date, at least fifteen different 5-HT receptors have been identified, largely as the result of cloning cDNA's, and these receptors have been grouped into seven families (5-HT₁ through 5-HT₇) (Hoyer, D. et al. Pharmacol. Biochem. Behav. (2002) 71, 533-554). Fourteen of the fifteen cloned 5-HT receptors are expressed in the brain. 5-HT is implicated in many disease states, particularly conditions of the central nervous system including; depression, anxiety, schizophrenia, eating disorders, obsessive compulsive disorder, learning and memory dysfunction, migraine, chronic pain, sensory perception, motor activity, temperature regulation, nociception, sexual behavior, hormone secretion and cognition. The identification of multiple 5-HT receptors has provided the opportunity to characterize existing therapeutic agents thought to act via the serotonergic system. Consequently, this has led to the realization that many drugs have non-selective properties (Roth, B.L. et al. Neuroscientist (2000) 6(4) 252-262). For example, the antipsychotic drugs, clozapine, chlorpromazine, haloperidol and olanzapine exhibit affinities for multiple serotonin receptors in addition to other families of receptors. Similar behavior has been noted for antidepressants, including imipramine, nortriptaline, fluoxetine and sertraline. Similarly, the anti-migraine agent sumatriptan exhibits high affinity for several serotonin receptors. While the lack of selectivity often contributes to a favorable therapeutic outcome, it can also cause undesirable and dose-limiting side effects (Stahl, S.M. Essential Psychopharmacology, 2nd ed., Cambridge University Press, Cambridge, U.K., 2000). Thus, the inhibition of serotonin and norepinephrine uptake together with 5-HT₂ receptor blockade is responsible for the therapeutic effects of the tricyclic antidepressants. In contrast, their blockade of histamine H₁, muscarinic and alpha-adrenergic receptors can lead to sedation, blurred vision and orthostatic hypertension respectively. Likewise, the atypical antipsychotics, including olanzapine and clozapine, are considered to have positive therapeutic effects attributable to their actions at 5-HT₂, D₂ and 5-HT₇ receptors. Conversely, their side effect liability is due to their affinities at a range of dopaminergic, serotonergic and adrenergic receptors.

More selective ligands therefore have the potential to ameliorate untoward pharmacologies and provide novel therapies. More importantly the ability to obtain compounds with known receptor selectivities affords the prospect to target multiple therapeutic mechanisms and improve clinical responses with a single drug.

### Summary of the Invention

The invention features a compound of formulae (I), (II) and (III): 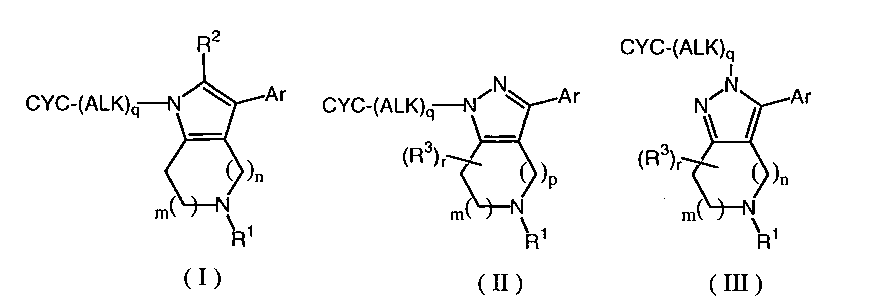 wherein
m is 0, 1 or 2;
n is 1,2 or 3;
p is 1, 2 or 3, with the proviso that where m is 1, p is not 1;
m+n is less than or equal to 4;
m+p is less than or equal to 4;
q is 0 or 1;
r is 0, 1, 2, 3, 4, or 5;
R³ is -C₁₋₄alkyl, allyl, propargyl, or benzyl, each optionally substituted with -C₁₋₃alkyl, -OH, or halo;
Ar is an aryl or heteroaryl ring selected from the group consisting of:
a) phenyl, optionally mono-, di- or tri-substituted with R^{r} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)- or -(CH₂)₁₋₂N(C₁₋₄alkyl)(CH₂)-;
   R^{r} is selected from the group consisting of -OH, -C₁₋₆alkyl, -OC₁₋₆alkyl, -C₂₋₆alkenyl, -OC₃₋₆alkenyl, -C₂₋₆alkynyl, -OC₃₋₆alkynyl, -CN, -NO₂, -N(R^{y})R^{z} (wherein R^{y} and R^{z} are independently selected from H or C₁₋₆alkyl), -(C=O)N(R^{y})R^{z}, -(N-R^{t})COR^{t}, -(N-R^{t})S0₂C₁₋₆alkyl (wherein R^{t} is H or C₁₋₆alkyl), -(C=O)C₁₋₆alkyl, -(S=(O)ₙ)-C₁₋₆alkyl (wherein n is selected from 0, 1 or 2), -SO₂N(R^{y})R^{z}, -SCF₃, halo, -CF₃, -OCF₃, -COOH and -COOC₁₋₆alkyl;
b) phenyl or pyridyl fused at two adjacent carbon ring members to a three membered hydrocarbon moiety to form a fused five membered aromatic ring, which moiety has one carbon atom replaced by >O, >S, >NH or >N(C₁₋₄alkyl) and which moiety has up to one additional carbon atom optionally replaced by -N=, the fused rings optionally mono-, di- or tri-substituted with R^{r};
c) phenyl fused at two adjacent ring members to a four membered hydrocarbon moiety to form a fused six membered aromatic ring, which moiety has one or two carbon atoms replaced by -N=, the fused rings optionally mono-, di- or tri-substituted with R^{r};
d) naphthyl, optionally mono-, di- or tri-substituted with R^{r};
e) a monocyclic aromatic hydrocarbon group having five ring atoms, having a carbon atom which is the point of attachment, having one carbon atom replaced by >O, >S, >NH or >N(C₁₋₄alkyl), having up to one additional carbon atoms optionally replaced by -N=, optionally mono- or di-substituted with R^{r} and optionally benzofused or pyridofused at two adjacent carbon atoms, where the benzofused or pyridofused moiety is optionally mono-, di-, or tri-substituted with R^{r}; and
f) a monocyclic aromatic hydrocarbon group having six ring atoms, having a carbon atom which is the point of attachment, having one or two carbon atoms replaced by -N=, optionally mono- or di-substituted with R^{r} and optionally benzofused or pyridofused at two adjacent carbon atoms, where the benzofused or pyridofused moiety is optionally mono- or di-substituted with R^{r};
g) phenyl or pyridyl, substituted with a substituent selected from the group consisting of phenyl, pyridyl, thiophenyl, oxazolyl and tetrazolyl, where the resultant substituted moiety is optionally further mono-, di- or tri-substituted with R^{r};
ALK is a branched or unbranched C₁₋₈alkylene, C₂₋₈alkenylene, C₂₋₈alkynylene or C₃₋₈cycloalkenylene, optionally mono-, di-, or tri-substituted with a substituent independently selected from the group consisting of: -OH, -OC₁₋₆alkyl, -OC₃₋₆cycloalkyl, -CN, -NO₂, -N(R^{a})R^{b} (wherein R^{a} and R^{b} are independently selected from H, C₁₋₆alkyl or C₂₋₆alkenyl), -(C=O)N(R^{a})R^{b}, -(N-R^{c})COR^{c}, -(N-R^{c})SO₂C₁₋₆alkyl (wherein R^{c} is H or C₁₋₆alkyl), -(C=O)C₁₋₆alkyl, -(S=(O)_{d})-C₁₋₆alkyl (wherein d is selected from 0, 1 or 2), -SO₂N(R^{a})R^{b}, -SCF₃, halo, -CF₃, -OCF₃, -COOH and -COOC₁₋₆alkyl;
CYC is hydrogen or a carbocyclic, heterocyclic, aryl or heteroaryl ring selected from the group consisting of:
i) phenyl, optionally mono-, di- or tri-substituted with R^{q} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)- or -(CH₂)₁₋₂N(C₁₋₄alkyl)(CH₂)-;
   R^{q} is selected from the group consisting of -OH, -C₁₋₆alkyl, -OC₁₋₆alkyl, -C₃₋₆cycloalkyl, -OC₃₋₆cycloalkyl, phenyl, -Ophenyl, benzyl, -Obenzyl, -CN, -NO₂, -N(R^{a})R^{b} (wherein R^{a} and R^{b} are independently selected from H, C₁₋₆alkyl or C₂₋₆alkenyl, or R^{a} and R^{b} may be taken together with the nitrogen of attachment to form an otherwise aliphatic hydrocarbon ring, said ring having 5 to 7 members, optionally having one carbon replaced with >O, =N-, >NH or >N(C₁₋₄alkyl), optionally having one carbon substituted with -OH, and optionally having one or two unsaturated bonds in the ring), -(C=O)N(R^{a})R^{b}, -(N-R^{c})COR^{c}, -(N-R^{c})SO₂C₁₋₆alkyl (wherein R^{c} is H or C₁₋₆alkyl or two R^{c} in the same substituent may be taken together with the amide of attachment to form an otherwise aliphatic hydrocarbon ring, said ring having 4 to 6 members), -N-(SO₂C₁₋₆alkyl)₂, -(C=O)C₁₋₆alkyl, -(S=(O)_{d})-C₁₋₆alkyl (wherein d is selected from 0, 1 or 2), -SO₂N(R^{a})R^{b}, -SCF₃, halo, -CF₃, -OCF₃, -COOH and -COOC₁₋₆alkyl;
ii) phenyl or pyridyl fused at two adjacent carbon ring members to a three membered hydrocarbon moiety to form a fused five membered aromatic ring, which moiety has one carbon atom replaced by >O, >S, >NH or >N(C₁₋₄alkyl) and which moiety has up to one additional carbon atom optionally replaced by -N=, the fused rings optionally mono-, di- or tri-substituted with R^{q};
iii) phenyl fused at two adjacent carbon ring members to a four membered hydrocarbon moiety to form a fused six membered aromatic ring, which moiety has one or two carbon atoms replaced by -N=, the fused rings optionally mono-, di- or tri-substituted with R^{q};
iv) naphthyl, optionally mono-, di- or tri-substituted with R^{q};
v) a monocyclic aromatic hydrocarbon group having five ring atoms, having a carbon atom which is the point of attachment, having one carbon atom replaced by >O, >S, >NH or >N(C₁₋₄alkyl), having up to one additional carbon atoms optionally replaced by -N=, optionally mono- or di-substituted with R^{q} and optionally benzofused or pyridofused at two adjacent carbon atoms, where the benzofused or pyridofused moiety is optionally mono-, di-, or tri-substituted with R^{q};
vi) a monocyclic aromatic hydrocarbon group having six ring atoms, having a carbon atom which is the point of attachment, having one or two carbon atoms replaced by -N=, optionally mono- or di-substituted with R^{q} and optionally benzofused or pyridofused at two adjacent carbon atoms, where the benzofused or pyridofused moiety is optionally mono- or di-substituted with R^{q};
vii) a 3-8 membered non-aromatic carbocyclic or heterocyclic ring said ring having 0, 1 or 2 non-adjacent heteroatom members selected from O, S, -N=, >NH or >NR^{q}, having 0, 1 or 2 unsaturated bonds, having 0, 1 or 2 carbon members which is a carbonyl, optionally having one carbon member which forms a bridge, having 0 to 5 substituents R^{q} and optionally benzofused or pyridofused at two adjacent carbon atoms where the benzofused or pyridofused moiety has 0, 1, 2 or 3 substituents R^{q}; and
viii) a 4-7 membered non-aromatic carbocyclic or heterocyclic ring said ring having 0, 1 or 2 non-adjacent heteroatom members selected from O, S, -N=, >NH or >NR^{q}, having 0, 1 or 2 unsaturated bonds, having 0, 1 or 2 carbon members which is a carbonyl and optionally having one carbon member which forms a bridge, the heterocyclic ring fused at two adjacent carbon atoms forming a saturated bond or an adjacent carbon and nitrogen atom forming a saturated bond to a 4-7 membered carbocyclic or heterocyclic ring, having 0 or 1 possibly additional heteroatom member, not at the ring junction, selected from O, S, -N=, >NH or >NR^{q}, having 0, 1 or 2 unsaturated bonds, having 0, 1 or 2 carbon members which is a carbonyl and the fused rings having 0 to 5 substituents R^{q};
R¹ is selected from the group consisting of H, C₁₋₇alkyl, C₂₋₇alkenyl, C₂₋₇alkynyl, C₃₋₇cycloalkyl, C₃₋₇cycloalkylC₁₋₇alkyl, C₃₋₇cycloalkenyl, C₃₋₇cycloalkenylC₁₋₇alkyl and benzo-fusedC₄₋₇cycloalkyl, each optionally mono-, di-, or tri-substituted with R^{P};
R^{P} is selected from the group consisting of -OH, -OC₁₋₆alkyl, -C₃₋₆cycloalkyl, -OC₃₋₆cycloalkyl, -CN, -NO₂, phenyl, pyridyl, thienyl, furanyl, pyrrolyl, -N(R^{s})R^{u} (wherein R^{s} and R^{u} are independently selected from H or C₁₋₆alkyl, or may be taken together with the nitrogen of attachment to form an otherwise aliphatic hydrocarbon ring, said ring having 5 to 7 members, optionally having one carbon replaced with >O, =N-, >NH or >N(C₁₋₄alkyl) and optionally having one or two unsaturated bonds in the ring), -(C=O)N(R^{s})R^{u}, -(N-R^{v})COR^{v}, -(N-R^{v})SO₂C₁₋₆alkyl (wherein R^{v} is H or C₁₋₆alkyl or two R^{v} in the same substituent may be taken together with the amide of attachment to form an otherwise aliphatic hydrocarbon ring, said ring having 4 to 6 members), -(C=O)C₁₋₆alkyl, -(S=(O)ₙ)-C₁₋₆alkyl (wherein n is selected from 0, 1 or 2), -SO₂N(R^{s})R^{u}, -SCF₃, halo, -CF₃, -OCF₃, -COOH and -COOC₁₋₆alkyl, wherein the foregoing phenyl, pyridyl, thienyl, furanyl and pyrrolyl substituents are optionally mono-, di-, or tri-substituted with a substituent independently selected from the group consisting of: -OH, -C₁₋₆alkyl, -OC₁₋₆alkyl, -CN, -NO₂, -N(R^{a})R^{b} (wherein R^{a} and R^{b} are independently selected from H, C₁₋₆alkyl or C₂₋₆alkenyl), -(C=O)N(R^{a})R^{b}, -(N-R^{c})COR^{c}, -(N-R^{c})SO₂C₁₋₆alkyl (wherein R^{c} is H or C₁₋₆alkyl), -(C=O)C₁₋₆alkyl, -(S=(O)_{d})-C₁₋₆alkyl (wherein d is selected from 0, 1 or 2), -SO₂N(R^{a})R^{b}, -SCF₃, halo, -CF₃, -OCF₃, -COOH and -COOC₁₋₆alkyl;
R² is selected from the group consisting of H, C₁₋₇alkyl, C₂₋₇alkenyl, C₂₋₇alkynyl and C₃₋₇cycloalkyl;
and enantiomers, diastereomers, hydrates, solvates and pharmaceutically acceptable salts, esters and amides thereof.

Similarly, isomeric forms of the compounds of formulae (I), (II), and (III), and of their pharmaceutically acceptable salts, esters, and amides, are encompassed within the present invention, and reference herein to one of such isomeric forms is meant to refer to at least one of such isomeric forms. One of ordinary skill in the art will recognize that compounds according to this invention may exist, for example in a single isomeric form whereas other compounds may exist in the form of a regioisomeric mixture.

The invention also features pharmaceutical compositions containing such compounds and methods of using such compositions in the treatment or prevention of disease states mediated by the serotonin receptors, particularly, 5-HT₇ and/or 5-HT₂ receptor subtypes.

### Detailed Description

Preferably, m is 1 or 2 and most preferably, m is 1.

Preferably, n is 1 or 2.

Preferably, p is 1 or 2.

Preferably, m+n is 2 or 3.

Preferably, m+p is 2 or 3.

Preferably, q is 1.

Preferably, r is 0, 1, or 2.

Preferably, r is 4.

Preferably R³ optionally substituted, is selected from the group consisting of methyl, ethyl, propyl, isopropyl, butyl, allyl, propargyl, and benzyl.

Preferably, R³ is methyl.

Preferably Ar, optionally substituted, is selected from the group consisting of:
a) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
b) 4-, 5-, 6- or 7-benzoxazolyl, 4-, 5-, 6- or 7-benzothiophenyl, 4-, 5-, 6-or 7-benzofuranyl, 4-, 5-, 6- or 7-indolyl, 4-, 5-, 6- or 7-benzthiazolyl, 4-, 5-, 6-or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1 H-pyrrolo[2,3-b]pyridin-4, 5 or 6-yl, 1 H-pyrrolo[3,2-c]pyridin-4, 6 or 7-yl, 1 H-pyrrolo[2,3-c]pyridin-4, 5 or 7-yl, 1 H-pyrrolo[3,2-b]pyridin-5, 6 or 7-yl,
c) 5-, 6-, 7- or 8-isoquinolinyl, 5-, 6-, 7- or 8-quinolinyl, 5-, 6-, 7- or 8-quinoxalinyl, 5-, 6-, 7- or 8-quinazolinyl,
d) naphthyl,
e) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1,2,4-triazolyl, 3-indoxazinyl, 2-benzoxazolyl, 2- or 3-benzothiophenyl, 2- or 3-benzofuranyl, 2- or 3-indolyl, 2-benzthiazolyl, 2-benzimidazolyl, 3-indazolyl,
f) pyridinyl, pyridinyl-N-oxide, pyrazinyl, pyrimidinyl, pyridazinyl, 1-, 3- or 4-isoquinolinyl, 2-, 3- or 4-quinolinyl, 2- or 3-quinoxalinyl, 2- or 4-quinazolinyl, [1,5], [1,6], [1,7], or [1,8]naphthyridin-2-, 3-, or 4-yl, [2,5], [2,6], [2,7], [2,8]naphthyridin-1-, 3-, or 4-yl, and
g) biphenyl, 4-tetrazolylphenyl.

More preferably, Ar, optionally substituted, is selected from the group consisting of phenyl, pyridyl, thiophen-2-yl and thiophen-3-yl.

Specific Ar may be selected from the group consisting of phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 3-nitrophenyl, 4-nitrophenyl, 3-chloro-4-fluorophenyl, 3-fluoro-4-chlorophenyl, benzo[1,3]dioxol-4 or 5-yl, 3-hydroxyphenyl, 4-hydroxyphenyl, 4-hydroxy-2-methylphenyl, 4-hydroxy-3-fluorophenyl, 3,4-dihydroxyphenyl,4-dimethylaminophenyl, 4-carbamoylphenyl, 4-fluoro-3-methylphenyl, furan-2-yl, furan-3-yl, thiophen-2-yl, thiophen-3-yl, 5-chlorothiophen-2-yl, 5-methylthiophen-2-yl, 5-chlorothiophen-3-yl, 5-methylthiophen-3-yl, 4'-chlorobiphenyl, and 4-tetrazolylphenyl.

Preferably, ALK, optionally substituted, is selected from the group consisting of methylene, ethylene, propylene, butylene, tert-butylene, pentylene, 1-ethylpropylene, 2-ethylpropylene, 2-ethylbutylene, isopropylene, but-3-enylene, isobutylene, 3-methylbutylene, allylene, and prop-2-ynylene.

Specific ALK may be selected from the group consisting of methylene, trifluoromethylmethylene, methoxycarbonylmethyl, methylcarbamoylmethyl, ethylene, propylene, 3-methoxycarbonyl propylene, 3-carboxy propylene, butylene, tert-butylene, 4-hydroxybutylene, 4-methoxycarbonyl butylene, 4-carboxy butylene, pentylene, 5-hydroxypentylene, 1-ethylpropylene, 2-ethylpropylene, 2-ethylbutylene, isopropylene, but-3-enylene, isobutylene, 3-methylbutylene, prop-2-ynylene, 2-dimethylaminoethylene, and 2-cyanoethylene.

Preferably CYC, optionally substituted, is hydrogen or is selected from the group consisting of:
i) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
ii) 4-, 5-, 6- or 7-benzoxazolyl, 4-, 5-, 6- or 7-benzothiophenyl, 4-, 5-, 6-or 7-benzofuranyl, 4-, 5-, 6- or 7-indolyl, 4-, 5-, 6- or 7-benzthiazolyl, 4-, 5-, 6-or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1 H-pyrrolo[2,3-b]pyridin-4, 5 or 6-yl, 1 H-pyrrolo[3,2-c]pyridin-4, 6 or 7-yl, 1 H-pyrrolo[2,3-c]pyridin-4, 5 or 7-yl, 1 H-pyrrolo[3,2-b]pyridin-5, 6 or 7-yl,
iii) 5-, 6-, 7- or 8-isoquinolinyl, 5-, 6-, 7- or 8-quinolinyl, 5-, 6-, 7- or 8-quinoxalinyl, 5-, 6-, 7- or 8-quinazolinyl,
iv) naphthyl,
v) furanyl, oxazolyl, isoxazolyl, 1,2,3-oxadiazolyl, 1,2,4-oxadiazolyl, 1,2,5-oxadiazolyl, 1 ,3,4-oxadiazolyl, thiophenyl, thiazolyl, isothiazolyl, pyrrolyl, imidazolyl, pyrazolyl, 1,2,3-triazolyl, 1,2,4-triazolyl, 3-indoxazinyl, 2-benzoxazolyl, 2- or 3-benzothiophenyl, 2- or 3-benzofuranyl, 2- or 3-indolyl, 2-benzthiazolyl, 2-benzimidazolyl, 3-indazolyl,
vi) pyridinyl, pyridinyl-N-oxide, pyrazinyl, pyrimidinyl, pyridazinyl, 1-, 3- or 4-isoquinolinyl, 2-, 3- or 4-quinolinyl, 2- or 3-quinoxalinyl, 2- or 4-quinazolinyl, [1,5], [1 ,6], [1,7], or [1,8]naphthyridin-2-, 3-, or 4-yl, [2,5], [2,6], [2,7], [2,8]naphthyridin-1-, 3-, or 4-yl,
vii) cyclopropyl, cyclobutyl, cyclopentyl, cyclohexyl, cyclohexenyl, cycloheptyl, cyclooctyl, adamantyl, pyrrolinyl, pyrrolidinyl, pyrazolinyl, piperidinyl, homopiperidinyl, azepanyl, tetrahydrofuranyl, tetrahydropyranyl, piperazinyl, morpholinyl, thiomorpholinyl, piperidinonyl, indanyl, dihydroindolyl, oxindolyl, dihydropyrrolopyridinyl, and
viii) bicyclo[4.1.0]heptane, octahydroindolyl, octahydroisoindolinyl, decahydroquinolinyl, decahydroisoquinolinyl, octahydropyrrolopyridinyl, and octahydropyrrolopyrrolidinyl.

More preferably, CYC, optionally substituted, is selected from the group consisting of hydrogen, phenyl, indolyl, benzthiazolyl, isoquinolyl, quinazolinyl, naphthalen-1 or 2-yl, thiophen-2-yl, thiophen-3-yl, furan-2-yl, furan-3-yl, pyridinyl, cyclobutyl, cyclopentyl, cyclohexyl, cycloheptyl, cyclooctyl, piperidin-2,3 or 4-yl, 2-pyrrolin-2, 3, 4 or 5-yl, 3-pyrrolin-2 or 3-yl, 2-pyrazolin-3, 4 or 5-yl, morpholin-2, 3, 5 or 6-yl, thiomorpholin-2, 3, 5 or 6-yl, piperazin-2, 3, 5 or 6-yl, pyrrolidin-2 or 3-yl, homopiperidinyl, adamantanyl, and octahydroindolyl.

Most preferably, CYC, optionally substituted, is selected from the group consisting of hydrogen, phenyl, pyridyl, cyclobutyl, cyclopentyl, cyclohexyl, thiophen-2-yl, thiophen-3-yl, tetrahydropyranyl, furan-2-yl, furan-3-yl and naphthalen-1 or 2-yl.

Specific CYC may be selected from the group consisting of hydrogen, phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 2-cyanophenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 2,6-difluorophenyl, 2,6-dichlorophenyl, 2,6-dimethylphenyl, 2,4,6-trifluorophenyl, 2,4,6-trichlorophenyl, 3,4,5-trimethoxyphenyl, cyclobutyl, cyclohexyl, cyclopentyl, 4-fluoro-3-methylphenyl, 3-nitrophenyl, 4-nitrophenyl, 4-methyl-3-fluorophenyl, 3,4-dimethylphenyl, 4-methoxy-3-fluorophenyl, 4-methoxy-2-methylphenyl, 3-aminophenyl, 4-aminophenyl, 4-carbomethoxyphenyl, 3-methanesulfonylamino-phenyl, 4-methanesulfonylamino-phenyl, 3-dimethanesulfonylamino-phenyl, 4-dimethanesulfonylamino-phenyl, thiophen-2-yl, thiophen-3-yl, 5-chlorothiophen-2-yl, benzo[1,3]dioxol-4 or 5-yl, tetrahydropyran-2,3 or 4-yl, furan-2-yl, furan-3-yl, 5-carboxyethyl-furan-2-yl, naphthalen-1 or 2-yl, 3,4-bisbenzyloxyphenyl, 2-hydroxyphenyl, 3-hydroxyphenyl, 4-hydroxyphenyl, 4-hydroxy-2-methylphenyl, 4-hydroxy-3-fluorophenyl and 3,4-dihydroxyphenyl.

Preferably, R¹ is selected from the group consisting of hydrogen, C₁₋₃alkyl, C₂₋₄alkenyl, C₂₋₄alkynyl, C₃₋₆cycloalkyl, C₃₋₆cycloalkylC₁₋₃alkyl, C₅₋₆cycloalkenyl, benzo-fusedC₅₋₆cycloalkyl, each optionally mono-, di-, or tri-substituted with R^{P}.

More preferably, R¹, optionally R^{p} substituted, is selected from the group consisting of hydrogen, methyl, ethyl, propyl, and isopropyl.

Specific R¹ may be selected from the group consisting of hydrogen, methyl, ethyl, propyl, isopropyl, 3-hydroxypropyl, benzyl, 3,4-dimethoxybenzyl, methoxycarbonylmethyl, carbamoylmethyl, phenethyl, phenpropyl, and hydroxyethyl.

Preferably, R² is hydrogen, C₁₋₃alkyl, C₂₋₄alkenyl, C₂₋₄alkynyl, or C₃₋₆cycloalkyl.

More preferably, R² is hydrogen or methyl.

It is understood that some compounds referred to herein are chiral and/or have geometric isomeric centers, for example E- and Z- isomers. The present invention encompasses all such optical, including stereoisomers and racemic mixtures, diastereomers, and geometric isomers that possess the activity that characterizes the compounds of this invention. In addition, certain compounds referred to herein can exist in solvated as well as unsolvated forms. It is understood that this invention encompasses all such solvated and unsolvated forms that possess the activity that characterizes the compounds of this invention.

Compounds according to the present invention that have been modified to be detectable by some analytic technique are also within the scope of this invention. The compounds of the present invention may be labeled with radioactive elements such as ¹²⁵I, ¹⁸F, ¹¹C, ⁶⁴Cu, and the like for use in imaging or for radioactive treatment of patients. An example of such compounds is an isotopically labeled compound, such as an ¹⁸F isotopically labeled compound that may be used as a probe in detection and/or imaging techniques, such as positron emission tomography (PET) and single-photon emission computed tomography (SPECT). Preferably, compounds of the present invention labeled with ¹⁸F or ¹¹C may be used as a positron emission tomography (PET) molecular probe for studying serotonin-mediated disorders. Another example of such compounds is an isotopically labeled compound, such as a deuterium and/or tritium labeled compound that may be used in reaction kinetic studies. The compounds described herein may be reacted with an appropriate functionalized radioactive reagents using conventional chemistry to provide radiolabeled compounds.

Pharmaceutically acceptable salts, esters, and amides include carboxylate salts (e.g., C₁₋₈alkyl, C₃₋₈cycloalkyl, aryl, C₂₋₁₀heteroaryl, or C₂₋₁₀ non-aromatic heterocyclic), amino addition salts, acid addition salts, esters, and amides that are within a reasonable benefit/risk ratio, pharmacologically effective and suitable for contact with the tissues of patients without undue toxicity, irritation, or allergic response. Representative addition salts for compounds of formula (I) displaying basic functionality include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. Representative addition salts for compounds of formula (I) displaying acidic functionality are those that form non-toxic base salts with such compounds. These salts may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine. See example, S.M. Berge, et al., "Pharmaceutical Salts," J. Pharm. Sci., 1977, 66:1-19, which is incorporated herein by reference.

Representative pharmaceutically acceptable amides of the invention include those derived from ammonia, primary C₁₋₆ alkyl amines and secondary di(C₁₋₆alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C₁₋₃alkyl primary amines, and di(C₁₋₂alkyl)amines. Representative pharmaceutically acceptable esters of the invention include C₁₋₇alkyl, C₅₋₇cycloalkyl, phenyl, and phenyl(C₁₋₆)alkyl esters. Preferred esters include methyl esters.

Preferred compounds, which are fused pyrroles, are selected from the group consisting of:

| EX | CHEMICAL NAME |
|---|---|
| 1 | 1-Benzyl-3-(4-nitro-phenyl)-4,5,6,7-tetrahydro-1*H*-pyrrolo[3,2-c]pyridine; |
| 2 | 1-Benzyl-3-(3-chloro-4-fluoro-phenyl)-4,5,6,7-tetrahydro-1*H*-pyrrolo[3,2-*c*]pyridine; |
| 3 | 4-(1-Benzyl-4,5,6,7-tetrahydro-1*H*-pyrrolo[3,2-*c*]pyridin-3-yl)-phenol; |
| 4 | 1-Benzyl-3-(4-trifluoromethoxy-phenyl)-4,5,6,7-tetrahydro-1*H*-pyrrolo[3,2-c]pyridine; |
| 5 | 1-Benzyl-3-(5-chloro-thiophen-2-yl)-4,5,6,7-tetrahydro-1*H*-pyrrolo[3,2-c]pyridine; |
| 6 | 1-Benzyl-3-thiophen-2-yl-4,5,6,7-tetrahydro-1*H*-pyrrolo[3,2-c]pyridine; |
| 7 | 1-(3-Chloro-benzyl)-3-phenyl-4,5,6,7-tetrahydro-1*H*-pyrrolo[3,2-c]pyridine; |
| 8 | 1-Benzyl-3-(3-fluoro-phenyl)-4,5,6,7-tetrahydro-1*H*-pyrrolo[3,2-c]pyridine; |
| 9 | 3-(4-Chloro-phenyl)-1-(2-fluoro-benzyl)-4,5,6,7-tetrahydro-1*H*-pyrrolo[3,2-*c*]pyridine; |
| 10 | 1-(3-Chloro-benzyl)-3-(4-chloro-phenyl)-4,5,6,7-tetrahydro-1*H*-pyrrolo[3,2-*c*]pyridine; |
| 11 | 1-(2-Chloro-benzyl)-3-phenyl-4,5,6,7-tetrahydro-1*H*-pyrrolo[3,2-c]pyridine; |
| 12 | 1-(4-Chloro-benzyl)-3-(4-chloro-phenyl)-4,5,6,7-tetrahydro-1*H*-pyrrolo[3,2-*c*]pyridine; |
| 13 | 1-Benzyl-3-(2,4-dichloro-phenyl)-4,5,6,7-tetrahydro-1*H*-pyrrolo[3,2-c]pyridine; |
| 14 | 1-(4-Methoxy-benzyl)-3-phenyl-4,5,6,7-tetrahydro-1*H*-pyrrolo[3,2-c]pyridine; |
| 15 | 1-(2-Chloro-benzyl)-3-(4-chloro-phenyl)-4,5,6,7-tetrahydro-1*H* pyrrolo[3,2-*c*]pyridine; |
| 16 | 1-(2,4-Dichloro-benzyl)-3-phenyl-4,5,6,7-tetrahydro-1*H*-pyrrolo[3,2-c]pyridine; |
| 17 | 1-Benzyl-2-methyl-3-phenyl-4,5,6,7-tetrahydro-1*H*-pyrrolo[3,2-*c*]pyridine; |
| 18 | 1-Benzyl-3-p-tolyl-4,5,6,7-tetrahydro-1*H*-pyrrolo[3,2-*c*]pyridine; |
| 19 | 1-Benzyl-3-(3,4-dichloro-phenyl)-4,5,6,7-tetrahydro-1*H*-pyrrolo[3,2-*c*]pyridine; |
| 20 | 3-Benzo[1,3]dioxol-5-yl-1-benzyl-4,5,6,7-tetrahydro-1*H*-pyrrolo[3,2-*c*]pyridine; |
| 21 | 1-Benzyl-3-(4-fluoro-phenyl)-4,5,6,7-tetrahydro-1*H*-pyrrolo[3,2-*c*]pyridine; |
| 22 | 1-Butyl-3-*p*-tolyl-4,5,6,7-tetrahydro-1*H*-pyrrolo[3,2-*c*]pyridine; |
| 23 | 1-Benzyl-3-(4-bromo-phenyl)-4,5,6,7-tetrahydro-1*H*-pyrrolo[3,2-*c*]pyridine; |
| 24 | 1-Benzyl-3-(4-trifluoromethyl-phenyl)-4,5,6,7-tetrahydro-1*H*-pyrrolo[3,2-c]pyridine; |
| 25 | 1-Benzyl-3-(4-chloro-phenyl)-4,5,6,7-tetrahydro-1*H*-pyrrolo[3,2-*c*]pyridine; |
| 26 | 1-Benzyl-3-phenyl-1,4,5,6,7,8-hexahydro-pyrroto[2,3-*d*]azepine; |
| 27 | 1-Benzyl-3-(5-methyl-thiophen-2-yl)-4,5,6,7-tetrahydro-1*H*-pyrrolo[3,2-*c*]pyridine; |
| 28 | 1-Benzyl-3-(4-chloro-phenyl)-1,4,5,6,7,8-hexahydro-pyrrolo[2,3-*d*]azepine; |
| 29 | 1-Benzyl-3-(5-chloro-thiophen-2-yl)-1,4,5,6,7,8-hexahydro-pyrrolo[2,3-*d*]azepine; |
| 30 | 1-(4-Chloro-benzyl)-3-phenyl-4,5,6,7-tetrahydro-1*H*-pyrrolo[3,2-*c*]pyridine; |
| 31 | 1-Benzyl-3-phenyl-4,5,6,7-tetrahydro-1*H*-pyrrolo[3,2-c]pyridine; |
| 32 | 1-Benzyl-3-(3-chloro-phenyl)-1,4,5,6,7,8-hexahydro-pyrrolo[2,3-*d*]azepine; |
| 33 | 1-Benzyl-3-(3-chloro-phenyl)-4,5,6,7-tetrahydro-1*H*-pyrrolo[3,2-*c*]pyridine; |
| 34 | 1-Benzyl-3-(4-methoxy-phenyl)-4,5,6,7-tetrahydro-1*H*-pyrrolo[3,2-*c*]pyridine; |
| 35 | 1-Benzyl-3-(4-chloro-phenyl)-5-ethyl-4,5,6,7-tetrahydro-1*H*-pyrrolo[3,2-*c*]pyridine; |
| 36 | 1-Benzyl-3-(4-chloro-phenyl)-5-isopropyl-4,5,6,7-tetrahydro-1*H*-pyrrolo[3,2-c]pyridine; |
| 37 | 3-[1-Benzyl-3-(4-chloro-phenyl)-1,4,6,7-tetrahydro-pyrrolo[3,2-c]pyridin-5-yl]-propan-1-ol; |
| 38 | 1-Benzyl-3-(4-chloro-phenyl)-5-methyl-4,5,6,7-tetrahydro-1*H*-pyrrolo[3,2-c]pyridine; |
| 39 | 1-Benzyl-3-(3-chloro-phenyl)-5-methyl-4,5,6,7-tetrahydro-1*H*-pyrrolo[3,2-*c*]pyridine; |
| 40 | 1-Benzyl-3-(3-chloro-4-fluoro-phenyl)-5-methyl-4,5,6,7-tetrahydro-1*H*-pyrrolo[3,2-c]pyridine; |
| 41 | 1,5-Dibenzyl-3-phenyl-4,5,6,7-tetrahydro-1*H*-pyrrolo[3,2-*c*]pyridine; and |
| 42 | 1-Benzyl-5-isopropyl-3-phenyl-4,5,6,7-tetrahydro-1*H*-pyrrolo[3,2-*c*]pyridine. |

Preferred compounds, which are fused 1-substituted pyrazoles, are selected from the group consisting of:

| EX | CHEMICAL NAME |
|---|---|
| 43 | 1-Benzyl-3-(4-trifluoromethyl-phenyl)-1,4,5,6,7,8-hexahydro-1,2,6-triaza-azulene; |
| 44 | 1-Benzyl-3-phenyl-1,4,5,6,7,8-hexahydro-1,2,6-triaza-azulene; |
| 45 | 1-Benzyl-3-(2-fluoro-phenyl)-1,4,5,6,7,8-hexahydro-1,2,6-triaza-azulene; |
| 46 | 1-Benzyl-3-(3-fluoro-phenyl)-1,4,5,6,7,8-hexahydro-1,2,6-triaza-azulene; |
| 47 | 1-Benzyl-3-(4-fluoro-phenyl)-1,4,5,6,7,8-hexahydro-1,2,6-triaza-azulene; |
| 48 | 1-Benzyl-3-(2,3-difluoro-phenyl)-1,4,5,6,7,8-hexahydro-1,2,6-triaza-azulene; |
| 49 | 1-Benzyl-3-(3,4-dichloro-phenyl)-1,4,5,6,7,8-hexahydro-1,2,6-triaza-azulene; |
| 50 | 1-[4-(1-Benzyl-1,4,5,6,7,8-hexahydro-1,2,6-triaza-azulen-3-yl)-phenyl]-ethanone; |
| 51 | 1-Benzyl-3-(4-trifluoromethoxy-phenyl)-1,4,5,6,7,8-hexahydro-1,2,6-triaza-azulene; |
| 52 | 1-Benzyl-3-(3-chloro-phenyl)-1,4,5,6,7,8-hexahydro-1,2,6-triaza-azulene; |
| 53 | 3-(1-Benzyl-1,4,5,6,7,8-hexahydro-1,2,6-triaza-azulen-3-yl)-benzonitrile; |
| 54 | 4-(1-Benzyl-1,4,5,6,7,8-hexahydro-1,2,6-triaza-azulen-3-yl)-benzonitrile; |
| 55 | 1-(4-Chloro-benzyl)-3-phenyl-1,4,5,6,7,8-hexahydro-1,2,6-triaza-azulene; |
| 56 | 1-(4-Chloro-benzyl)-3-(4-chloro-phenyl)-1,4,5,6,7,8-hexahydro-1,2,6-triaza-azulene; |
| 57 | 1-Benzyl-3-phenyl-6-propyl-1,4,5,6,7,8-hexahydro-1,2,6-triaza-azulene; |
| 58 | 1-Benzyl-6-isopropyl-3-phenyl-1,4,5,6,7,8-hexahydro-1,2,6-triaza-azulene; |
| 59 | 1-Benzyl-3-(4-chloro-phenyl)-1,4,5,6,7,8-hexahydro-1,2,6-triaza-azulene; |
| 60 | 1-Benzyl-3-(4-chloro-phenyl)-1,4,5,6,7,8-hexahydro-1,2,5-triaza-azulene; |
| 61 | 3-(4-Chloro-phenyl)-1-methyl-1,4,5,6,7,8-hexahydro-1,2,6-triaza-azulene; |
| 63 | 3-(4-Chloro-phenyl)-1-ethyl-1,4,5,6,7,8-hexahydro-1 ,2,6-triaza-azulene; |
| 65 | 3-(4-Chloro-phenyl)-1-propyl-1,4,5,6,7,8-hexahydro-1,2,6-triaza-azulene; |
| 67 | 1-Butyl-3-(4-chloro-phenyt)-1,4,5,6,7,8-hexahydro-1,2,6-triaza-azulene; |
| 69 | 3-(4-Chloro-phenyl)-1-(2-cyclohexyl-ethyl)-1,4,5,6,7,8-hexahydro-1,2,6-triaza-azulene; |
| 71 | 3-(4-Chloro-phenyl)-1-phenethyl-1,4,5,6,7,8-hexahydro-1,2,6-triaza-azulene; |
| 73 | 3-(4-Chloro-phenyl)-1-(4-fluoro-3-methyl-benzyl)-1,4,5,6,7,8-hexahydro-1,2,6-triaza-azulene; |
| 74 | 3-(4-Chloro-phenyl)-1-(3-methyl-benzyl)-1,4,5,6,7,8-hexahydro-1,2,6-triaza-azulene; |
| 75 | 3-(4-Chloro-phenyl)-1-(4-fluoro-benzyl)-1,4,5,6,7,8-hexahydro-1,2,6-triaza-azulene; |
| 76 | 3-(4-Chloro-phenyl)-1-(3-fluoro-benzyl)-1,4,5,6,7,8-hexahydro-1,2,6-triaza-azulene; |
| 77 | 3-(4-Chloro-phenyl)-1-(4-methyl-benzyl)-1,4,5,6,7,8-hexahydro-1,2,6-triaza-azulene; |
| 78 | 3-(4-Chloro-phenyl)-1-(3,4-difluoro-benzyl)-1,4,5,6,7,8-hexahydro-1,2,6-triaza-azulene; |
| 79 | 3-(4-Chloro-phenyl)-1-(3-nitro-benzyl)-1,4,5,6,7,8-hexahydro-1,2,6-triaza-azulene; |
| 80 | 3-(4-Chloro-phenyl)-1-(3-fluoro-4-methyl-benzyl)-1,4,5,6,7,8-hexahydro-1,2,6-triaza-azulene; |
| 81 | 3-(4-Chloro-phenyl)-1-(3,4-dimethyl-benzyl)-1,4,5,6,7,8-hexahydro-1,2,6-triaza-azulene; |
| 85 | 5-[3-(4-Chloro-phenyl)-5,6,7,8-tetrahydro-4*H*-1,2,6-triaza-azulen-1-yl]-pentanoic acid methyl ester; |
| 86 | 5-[3-(4-Chloro-phenyl)-5,6,7,8-tetrahydro-4*H*-1,2,6-triaza-azulen-1-yl]-pentanoic acid; |
| 87 | 5-[3-(4-Chloro-phenyl)-5,6,7,8-tetrahydro-4*H*-1,2,6-triaza-azulen-1-yl]-pentan-1-ol; |
| 88 | 4-[3-(4-Chloro-phenyl)-5,6,7,8-tetrahydro-4*H*-1,2,6-triaza-azulen-1-yl]-butyric acid methyl ester; |
| 91 | 4-[3-(4-Chloro-phenyl)-5,6,7,8-tetrahydro-4*H*-1,2,6-triaza-azulen-1-yl]-butyric acid; |
| 93 | 4-[3-(4-Chloro-phenyl)-5,6,7,8-tetrahydro-4*H*-1,2,6-triaza-azulen-1-yl]-butan-1-ol; |
| 96 | 3-(4-Chloro-phenyl)-1-(3-fluoro-4-methoxy-benzyl)-1,4,5,6,7,8-hexahydro-1,2,6-triaza-azulene; |
| 98 | 3-(4-Chloro-phenyl)-1-(4-nitro-benzyl)-1,4,5,6,7,8-hexahydro-1 ,2,6-triaza-azulene; |
| 99 | 4-(3-Phenyl-5,6,7,8-tetrahydro-4*H*-1,2,6-triaza-azulen-1-ylmethyl)-phenylamine; |
| 100 | *N*-[4-(3-Phenyl-5,6,7,8-tetrahydro-4*H*-1,2,6-triaza-azulen-1-ylmethyl)-phenyl]-methanesulfonamide; |
| 101 | *N,N*-[4-(3-phenyl-5,6,7,8-tetrahydro-4*H*-1,2,6-triaza-azulen-1-ylmethyl)-phenyl]-dimethanesulfonamide; |
| 102 | 1-Benzyl-3-p-totyl-1,4,5,6,7,8-hexahydro-1,2,6-triaza-azutene; |
| 103 | 3-(4-Chloro-phenyl)-1-thiophen-2-ylmethyl-1,4,5,6,7,8-hexahydro-1,2,6-triaza-azulene; |
| 104 | 1-Benzyl-3-thiophen-2-yl-1,4,5,6,7,8-hexahydro-1,2,6-triaza-azulene; |
| 105 | 3-(4-Chloro-phenyl)-1-(3-methoxy-benzyl)-1,4,5,6,7,8-hexahydro-1,2,6-triaza-azulene; |
| 106 | 3-(4-Chloro-phenyl)-1-(2-fluoro-benzyl)-1,4,5,6,7,8-hexahydro-1,2,6-triaza-azulene; |
| 107 | 3-(4-Chloro-phenyl)-1-(2-methyl-benzyl)-1,4,5,6,7,8-hexahydro-1,2,6-triaza-azulene; |
| 108 | 3-(4-Chloro-phenyl)-1-(2,4-difluoro-benzyl)-1,4,5,6,7,8-hexahydro-1,2,6-triaza-azulene; |
| 109 | 3-(4-Chloro-phenyl)-1-(2-methoxy-benzyl)-1,4,5,6,7,8-hexahydro-1,2,6-triaza-azulene; |
| 110 | 1-(2-Chloro-benzyl)-3-(4-chloro-phenyl)-1,4,5,6,7,8-hexahydro-1,2,6-triaza-azulene; |
| 111 | 1-But-3-enyl-3-(4-chloro-phenyl)-1,4,5,6,7,8-hexahydro-1,2,6-triaza-azulene; |
| 112 | 1-(2-Bromo-benzyl)-3-(4-chloro-phenyl)-1,4,5,6,7,8-hexahydro-1,2,6-triaza-azulene; |
| 113 | 1-(4-Bromo-benzyl)-3-(4-chloro-phenyl)-1,4,5,6,7,8-hexahydro-1,2,6-triaza-azulene; |
| 114 | 3-(4-Chloro-phenyl)-1-(2-ethyl-butyl)-1,4,5,6,7,8-hexahydro-1,2,6-triaza-azulene; |
| 115 | 3-(4-Chloro-phenyl)-1-(5-chloro-thiophen-2-ylmethyl)-1,4,5,6,7,8-hexahydro-1,2,6-triaza-azulene; |
| 116 | 1-(3-Bromo-benzyl)-3-(4-chloro-phenyl)-1,4,5,6,7,8-hexahydro-1,2,6-triaza-azutene; |
| 117 | 3-(4-Chloro-phenyl)-1-cyclohexylmethyl-1,4,5,6,7,8-hexahydro-1,2,6-triaza-azulene; |
| 118 | 3-(4-Chloro-phenyl)-1-isobutyl-1,4,5,6,7,8-hexahydro-1,2,6-triaza-azulene; |
| 119 | 1-Benzo[1,3]dioxol-5-ylmethyl-3-(4-chloro-phenyl)-1,4,5,6,7,8-hexahydro-1,2,6-triaza-azulene; |
| 120 | 3-(4-Chloro-phenyl)-1-(tetrahydro-pyran-4-ylmethyl)-1,4,5,6,7,8-hexahydro-1,2,6-triaza-azulene; |
| 121 | 3-(4-Chloro-phenyl)-1-(2,6-difluoro-benzyl)-1,4,5,6,7,8-hexahydro-1,2,6-triaza-azulene; |
| 123 | 3-(4-Chloro-phenyl)-1-(4-methoxy-benzyl)-1,4,5,6,7,8-hexahydro-1,2,6-triaza-azulene; |
| 124 | 3-(4-Chloro-phenyl)-1-(3-methyl-butyl)-1,4,5,6,7,8-hexahydro-1,2,6-triaza-azulene; |
| 125 | 3-(4-Chloro-phenyl)-1-(2-trifluoromethyl-benzyl)-1,4,5,6,7,8-hexahydro-1,2,6-triaza-azulene |
| 128 | 3-(4-Chloro-phenyl)-1-(4-methoxy-2-methyl-benzyl)-1,4,5,6,7,8-hexahydro-1,2,6-triaza-azulene; |
| 134 | 3-(4-Chloro-phenyl)-l -prop-2-ynyl-1,4,5,6,7,8-hexahydro-1,2,6-triaza-azulene; |
| 135 | 3-(4-Chloro-phenyl)-1-pentafluorophenylmethyl-1,4,5,6,7,8-hexahydro-1,2,6-triaza-azulene; |
| 137 | 3-(4-Chloro-phenyl)-1-(2,4,6-trifluoro-benzyl)-1,4,5,6,7,8-hexahydro-1,2,6-triaza-azulene; |
| 138 | 2-[3-(4-Chloro-phenyl)-5,6,7,8-tetrahydro-4*H*-1,2,6-triaza-azulen-1-ylmethyl]-benzonitrile; |
| 142 | 3-(4-Chloro-phenyl)-1-naphthalen-2-ylmethyl-1,4,5,6,7,8-hexahydro-1,2,6-triaza-azulene; |
| 144 | 5-[3-(4-Chloro-phenyl)-5,6,7,8-tetrahydro-4*H*-1,2,6-triaza-azulen-1-ylmethyl]-furan-2-carboxylic acid ethyl ester; |
| 145 | 3-(4-Chloro-phenyl)-1-naphthalen-1-ylmethyl-1,4,5,6,7,8-hexahydro-1,2,6-triaza-azulene; |
| 147 | [3-(4-Chloro-phenyl)-5,6,7,8-tetrahydro-4*H*-1,2,6-triaza-azulen-1-yl]-acetic acid methyl ester; |
| 148 | 2-[3-(4-Chloro-phenyl)-5,6,7,8-tetrahydro-4*H*-1,2,6-triaza-azulen-1-yl]-*N*-methyl-acetamide; |
| 150 | 3-(4-Chloro-phenyl)-1-(3,4,5-trimethoxy-benzyl)-1,4,5,6,7,8-hexahydro-1,2,6-triaza-azulene; |
| 152 | 3-(4-Chloro-phenyl)-1-(2,6-dimethyl-benzyl)-1,4,5,6,7,8-hexahydro-1,2,6-triaza-azulene; |
| 154 | 1-(3,4-Bis-benzyloxy-benzyl)-3-(4-chloro-phenyl)-1,4,5,6,7,8-hexahydro-1,2,6-triaza-azulene; |
| 156 | 3-[3-(4-Chloro-phenyl)-5,6,7,8-tetrahydro-4*H*-1,2,6-triaza-azulen-1-ylmethyl]-phenol; |
| 157 | 4-[3-(4-Chloro-phenyl)-5,6,7,8-tetrahydro-4*H*-1,2,6-triaza-azulen-1-ylmethyl]-phenol; |
| 158 | 4-[3-(4-Chloro-phenyl)-5,6,7,8-tetrahydro-4*H*-1,2,6-triaza-azulen-1-ylmethyl]-3-methyl-phenol; |
| 159 | 4-[3-(4-Chloro-phenyl)-5,6,7,8-tetrahydro-4*H*-1,2,6-triaza-azulen-1-ylmethyl]-benzene-1,2-diol; |
| 160 | 4-[3-(4-Chloro-phenyl)-5,6,7,8-tetrahydro-4*H*-1,2,6-triaza-azulen-1-ylmethyl]-2-fluoro-phenol; |
| 162 | 2-[3-(4-Chloro-phenyl)-5,6,7,8-tetrahydro-4*H*-1,2,6-triaza-azulen-1-ylmethyl]-phenol; |
| 165 | 1-Benzyl-3-(4-chloro-phenyl)-6-methyl-1,4,5,6,7,8-hexahydro-1,2,6-triaza-azulene; |
| 166 | 1-Benzyl-3-(4-chloro-phenyl)-6-ethyl-1,4,5,6,7,8-hexahydro-1,2,6-triaza-azulene; |
| 167 | 3-(4-Chloro-phenyl)-6-(3,4-dimethoxy-benzyl)-1,4,5,6,7,8-hexahydro-1,2,6-triaza-azulene; |
| 168 | 1-Butyl-3-(4-chloro-phenyl)-6-(3,4-dimethoxy-benzyl)-1,4,5,6,7,8-hexahydro-1,2,6-triaza-azulene; |
| 169 | 1-Benzyl-3-(4-chloro-phenyl)-6-(3,4-dimethoxy-benzyl)-1,4,5,6,7,8-hexahydro-1,2,6-triaza-azulene; |
| 170 | [1-Benzyl-3-(4-chloro-phenyl)-4,5,7,8-tetrahydro-1*H*-1,2,6-triaza-azulen-6-yl]-acetic acid methyl ester; |
| 171 | 2-[1-Benzyl-3-(4-chloro-phenyl)-4,5,7,8-tetrahydro-1*H*-1,2,6-triaza-azulen-6-yl]-ethanol; |
| 172 | 3-(4-Chloro-phenyl)-1-phenyl-1,4,5,6,7,8-hexahydro-1,2,6-triaza-azulene; |
| 173 | 3-(4-Chloro-phenyl)-1-(2-methyl-benzyl)-4,5,6,7,8,9-hexahydro-1*H*-1,2,6-triaza-cyclopentacyclooctene; |
| 174 | 3-(4-Chloro-phenyl)-1-(2-methyl-benzyl)-4,5,6,7,8,9-hexahydro-1*H-*1,2,7-triaza-cyclopentacyclooctene; |
| 175 | 3-(4-Chloro-phenyl)-1-(2-methyl-benzyl)-4,5,6,7-tetrahydro-1*H*-pyrazolo[3,4-c]pyridine; |
| 230 | {4-[3-(4-Chloro-phenyl)-5,6,7,8-tetrahydro-4H-1,2,6-triaza-azulen-1-ylmethyl]-phenyl}-methyl-amine; |
| 237 | 3-(4-Chloro-phenyl)-1-cyclobutyl-1,4,5,6,7,8-hexahydro-1,2,6-triaza-azulene; |
| 239 | 3-(4-Chloro-phenyl)-1-cyclohexyl-1,4,5,6,7,8-hexahydro-1,2,6-triaza-azulene; |
| 254 | 3-(4-Chloro-phenyl)-1-cycloheptyl-1,4,5,6,7,8-hexahydro-1,2,6-triaza-azulene; |
| 255 | 3-(4-Chloro-phenyl)-1-cyclooctyl-1,4,5,6,7,8-hexahydro-1,2,6-triaza-azulene; |
| 273 | 1-Benzyl-3-(4-chloro-phenyl)-1,4,5,6,7,8-hexahydro-1,2,6-triaza-azulene citrate salt; |
| 316 | 3-(4-Chloro-phenyl)-1-pyridin-4-ylmethyl-1,4,5,6,7,8-hexahydro-1,2,6-triaza-azulene; |
| 317 | 3-(4-Chloro-phenyl)-1-pyridin-2-ylmethyl-1,4,5,6,7,8-hexahydro-1,2,6-triaza-azulene; |
| 319 | 3-(4-Chloro-phenyl)-1-pyridin-3-ylmethyl-1,4,5,6,7,8-hexahydro-1,2,6-triaza-azulene; |
| 320 | 4-[3-(4-Chloro-phenyl)-5,6,7,8-tetrahydro-4H-1,2,6-triaza-azulen-1-ylmethyl]-benzoic acid methyl ester; |
| 321 | 3-(4-Chloro-phenyl)-1-(tetrahydro-pyran-4-yl)-1,4,5,6,7,8-hexahydro-1,2,6-triaza-azulene; |
| 322 | 3-(4-Chloro-phenyl)-1-(4-methyl-cyclohexyl)-1,4,5,6,7,8-hexahydro-1,2,6-triaza-azulene; |
| 323 | {2-[3-(4-Chloro-phenyl)-5,6,7,8-tetrahydro-4H-1,2,6-triaza-azulen-1-yl]-ethyl}-dimethyl-amine. |
| 324 | 3-(4-Chloro-phenyl)-1-(1-oxy-pyridin-2-ylmethyl)-1,4,5,6,7,8-hexahydro-1,2,6-triaza-azulene; |
| 325 | 2-[1-Benzyl-3-(4-chloro-phenyl)-4,5,7,8-tetrahydro-1H-1,2,6-triaza-azulen-6-yl]-acetamide; |
| 326 | 3-[3-(4-Chloro-phenyl)-5,6,7,8-tetrahydro-4H-1,2,6-triaza-azulen-1-yl]-propionitrile. |
| 332 | 1-(4-Chloro-benzyl)-3-(4-chloro-phenyl)-1,4,5,6,7,8-hexahydro-1,2,5-triaza-azulene; |
| 333 | 3-(4-Chloro-phenyl)-1-(4-methyl-benzyl)-1,4,5,6,7,8-hexahydro-1,2,5-triaza-azulene; |
| 334 | 3-(4-Chloro-phenyl)-1-(3,4-difluoro-benzyl)-1,4,5,6,7,8-hexahydro-1,2,5-triaza-azulene; |
| 335 | 3-(4-Chloro-phenyl)-1-(3-methyl-benzyl)-1,4,5,6,7,8-hexahydro-1,2,5-triaza-azulene; |
| 336 | 3-(4-Chloro-phenyl)-1-(3-fluoro-4-methyl-benzyl)-1,4,5,6,7,8-hexahydro-1,2,5-triaza-azulene; and |
| 337 | 3-(4-Chloro-phenyl)-1-(4-fluoro-3-methyl-benzyl)-1,4,5,6,7,8-hexahydro-1,2,5-triaza-azulene. |

Preferred compounds, which are fused 2-substituted pyrazoles, are selected from the group consisting of:

| EX | CHEMICAL NAME |
|---|---|
| 62 | 3-(4-Chloro-phenyl)-2-methyl-2,4,5,6,7,8-hexahydro-1,2,6-triaza-azulene; |
| 64 | 3-(4-Chloro-phenyl)-2-ethyl-2,4,5,6,7,8-hexahydro-1,2,6-triaza-azulene; |
| 66 | 3-(4-Chloro-phenyl)-2-propyl-2,4,5,6,7,8-hexahydro-1,2,6-triaza-azulene; |
| 68 | 2-Butyl-3-(4-chloro-phenyl)-2,4,5,6,7,8-hexahydro-1,2,6-triaza-azulene; |
| 70 | 3-(4-Chloro-phenyl)-2-(2-cyclohexyl-ethyl)-2,4,5,6,7,8-hexahydro-1,2,6-triaza-azulene; |
| 72 | 3-(4-Chloro-phenyl)-2-phenethyl-2,4,5,6,7,8-hexahydro-1,2,6-triaza-azulene; |
| 82 | 5-[3-(4-Chloro-phenyl)-5,6,7,8-tetrahydro-4*H*-1,2,6-triaza-azulen-2-yl]-pentanoic acid methyl ester; |
| 83 | 5-[3-(4-Chloro-phenyl)-5,6,7,8-tetrahydro-4*H*-1,2,6-triaza-azulen-2-yl]-pentanoic acid; |
| 84 | 5-[3-(4-Chloro-phenyl)-5,6,7,8-tetrahydro-4*H*-1,2,6-triaza-azulen-2-yl]-pentan-1-ol; |
| 89 | 4-[3-(4-Chloro-phenyl)-5,6,7,8-tetrahydro-4*H*-1,2,6-triaza-azulen-2-yl]-butyric acid methyl ester; |
| 90 | 4-[3-(4-Chloro-phenyl)-5,6,7,8-tetrahydro-4*H*-1,2,6-triaza-azulen-2-yl]-butyric acid; |
| 92 | 4-[3-(4-Chloro-phenyl)-5,6,7,8-tetrahydro-4*H*-1,2,6-triaza-azulen-2-yl]-butan-1-ol; |
| 94 | 3-(4-Chloro-phenyl)-2-(3,4-difluoro-benzyl)-2,4,5,6,7,8-hexahydro-1,2,6-triaza-azulene; |
| 95 | 3-(4-Chloro-phenyl)-2-(4-methyl-benzyl)-2,4,5,6,7,8-hexahydro-1,2,6-triaza-azulene; |
| 97 | 3-(4-Chloro-phenyl)-2-(3-fluoro-4-methoxy-benzyl)-2,4,5,6,7,8-hexahydro-1,2,6-triaza-azulene; |
| 122 | 3-(4-Chloro-phenyl)-2-cyclohexylmethyl-2,4,5,6,7,8-hexahydro-1,2,6-triaza-azulene; |
| 126 | 3-(4-Chloro-phenyl)-2-(2-methyl-benzyl)-2,4,5,6,7,8-hexahydro-1,2,6-triaza-azulene; |
| 127 | 2-Benzyl-3-(4-chloro-phenyl)-2,4,5,6,7,8-hexahydro-1,2,6-triaza-azulene; |
| 129 | 3-(4-Chloro-phenyl)-2-(2,4-difluoro-benzyl)-2,4,5,6,7,8-hexahydro-1,2,6-triaza-azulene; |
| 130 | 5-[3-(4-Chloro-phenyl)-5,6,7,8-tetrahydro-4*H*-1,2,6-triaza-azulen-2-ylmethyl]-furan-2-carboxylic acid ethyl ester; |
| 131 | 3-(4-Chloro-phenyl)-2-isobutyl-2,4,5,6,7,8-hexahydro-1,2,6-triaza-azulene; |
| 132 | 3-(4-Chloro-phenyl)-2-(2-methoxy-benzyl)-2,4,5,6,7,8-hexahydro-1,2,6-triaza-azulene; |
| 133 | 2-Benzyl-3-phenyl-2,4,5,6,7,8-hexahydro-1,2,6-triaza-azulene; |
| 136 | 3-(4-Chloro-phenyl)-2-thiophen-2-ylmethyl-2,4,5,6,7,8-hexahydro-1,2,6-triaza-azulene; |
| 139 | 3-(4-Chloro-phenyl)-2-(5-chloro-thiophen-2-ylmethyl)-2,4,5,6,7,8-hexahydro-1,2,6-triaza-azulene; |
| 140 | 3-(4-Chloro-phenyl)-2-(2,6-difluoro-benzyl)-2,4,5,6,7,8-hexahydro-1,2,6-triaza-azulene; |
| 141 | 3-(4-Chloro-phenyl)-2-(2-trifluoromethyl-benzyl)-2,4,5,6,7,8-hexahydro-1,2,6-triaza-azulene; |
| 143 | 3-(4-Chloro-phenyl)-2-(2-ethyl-butyl)-2,4,5,6,7,8-hexahydro-1,2,6-triaza-azulene; |
| 146 | 2-Benzo[1,3]dioxol-5-ylmethyl-3-(4-chloro-phenyl)-2,4,5,6,7,8-hexahydro-1,2,6-triaza-azulene; |
| 149 | 3-(4-Chloro-phenyl)-2-pentafluorophenylmethyl-2,4,5,6,7,8-hexahydro-1,2,6-triaza-azulene; |
| 151 | 3-(4-Chloro-phenyl)-2-naphthalen-1-ylmethyl-2,4,5,6,7,8-hexahydro-1,2,6-triaza-azulene; |
| 153 | 3-(4-Chloro-phenyl)-2-(3,4,5-trimethoxy-benzyl)-2,4,5,6,7,8-hexahydro-1,2,6-triaza-azulene; |
| 155 | 2-(3,4-Bis-benzyloxy-benzyl)-3-(4-chloro-phenyl)-2,4,5,6,7,8-hexahydro-1,2,6-triaza-azulene; |
| 161 | 4-[3-(4-Chloro-phenyl)-5,6,7,8-tetrahydro-4*H*-1,2,6-triaza-azulen-2-ylmethyl]-2-fluoro-phenol; |
| 163 | 4-[3-(4-Chloro-phenyl)-5,6,7,8-tetrahydro-4*H*-1,2,6-triaza-azulen-2-ylmethyl]-3-methyl-phenol; |
| 164 | 2-[3-(4-Chloro-phenyl)-5,6,7,8-tetrahydro-4*H*-1,2,6-triaza-azulen-2-ylmethyl]-phenol; |
| 176 | 2,3-Diphenyl-2,4,5,6,7,8-hexahydro-1,2,6-triaza-azulene; |
| 177 | 2-Cyclohexyl-3-phenyl-2,4,5,6,7,8-hexahydro-1,2,6-triaza-azulene; |
| 178 | 3-(4-Chloro-phenyl)-2-cyclohexyl-2,4,5,6,7,8-hexahydro-1,2,6-triaza-azulene; |
| 179 | 2-Cyclohexyl-3-(4-trifluoromethyl-phenyl)-2,4,5,6,7,8-hexahydro-1,2,6-triaza-azulene; |
| 180 | 2-Cyclopentyl-3-phenyl-2,4,5,6,7,8-hexahydro-1,2,6-triaza-azulene; |
| 181 | 3-(4-Chloro-phenyl)-2-cyclopentyl-2,4,5,6,7,8-hexahydro-1,2,6-triaza-azulene; |
| 182 | 2-Cyclopentyl-3-(4-fluoro-phenyl)-2,4,5,6,7,8-hexahydro-1,2,6-triaza-azulene; |
| 183 | 2-(1-Ethyl-propyl)-3-(3-fluoro-phenyl)-2,4,5,6,7,8-hexahydro-1,2,6-triaza-azulene; |
| 184 | 2-(1-Ethyl-propyl)-3-(4-fluoro-phenyl)-2,4,5,6,7,8-hexahydro-1,2,6-triaza-azulene; |
| 185 | 2-(1-Ethyl-propyl)-3-thiophen-3-yl-2,4,5,6,7,8-hexahydro-1,2,6-triaza-azulene; |
| 186 | 2-(1-Ethyl-propyl)-3-phenyl-2,4,5,6,7,8-hexahydro-1,2,6-triaza-azulene; |
| 187 | 3-(4-Chloro-phenyl)-2-(2,2,2-trifluoro-ethyl)-2,4,5,6,7,8-hexahydro-1,2,6-triaza-azulene; |
| 188 | 2-(2,2,2-Trifluoro-ethyl)-3-(4-trifluoromethyl-phenyl)-2,4,5,6,7,8-hexahydro-1,2,6-triaza-azulene; |
| 189 | 2-Isopropyl-3-phenyl-2,4,5,6,7,8-hexahydro-1,2,6-triaza-azulene; |
| 190 | 3-(4-Fluoro-phenyl)-2-isopropyl-2,4,5,6,7,8-hexahydro-1,2,6-triaza-azulene; |
| 191 | 2-(1-Ethyl-propyl)-3-thiophen-2-yl-2,4,5,6,7,8-hexahydro-1,2,6-triaza-azulene; |
| 192 | 2-Cyclopentyl-3-thiophen-3-yl-2,4,5,6,7,8-hexahydro-1,2,6-triaza-azulene; |
| 193 | 2-Ethyl-3-phenyl-2,4,5,6,7,8-hexahydro-1,2,6-triaza-azulene; |
| 194 | 2-Ethyl-3-(4-fluoro-phenyl)-2,4,5,6,7,8-hexahydro-1,2,6-triaza-azulene; |
| 195 | 2-Ethyl-3-thiophen-2-yl-2,4,5,6,7,8-hexahydro-1,2,6-triaza-azulene; |
| 196 | 2-(3-Chloro-phenyl)-3-phenyl-2,4,5,6,7,8-hexahydro-1,2,6-triaza-azulene; |
| 197 | 2-(3-Fluoro-phenyl)-3-phenyl-2,4,5,6,7,8-hexahydro-1,2,6-triaza-azulene; |
| 198 | 2-(2-Chloro-phenyl)-3-phenyl-2,4,5,6,7,8-hexahydro-1,2,6-triaza-azulene; |
| 199 | 2-Phenyl-3-thiophen-2-yl-2,4,5;6,7,8-hexahydro-1,2,6-triaza-azulene; |
| 200 | 3-(4-Fluoro-phenyl)-2-phenyl-2,4,5,6,7,8-hexahydro-1,2,6-triaza-azulene; |
| 201 | 3-(4-Chloro-phenyl)-2-phenyl-2,4,5,6,7,8-hexahydro-1,2,6-triaza-azulene; |
| 202 | 3-(3-Chloro-phenyl)-2-phenyl-2,4,5,6,7,8-hexahydro-1,2,6-triaza-azulene; |
| 203 | 2-Phenyl-3-*p*-tolyl-2,4,5,6,7,8-hexahydro-1,2,6-triaza-azulene; |
| 204 | 2,3-Diphenyl-4,5,6,7-tetrahydro-2*H*-pyrazolo[4,3-*c*]pyridine; |
| 205 | 3-Phenyl-2-(3-trifluoromethyl-phenyl)-2,4,5,6,7,8-hexahydro-1,2,6-triaza-azulene; |
| 206 | 3-(4-Methoxy-phenyl)-2-phenyl-2,4,5,6,7,8-hexahydro-1,2,6-triaza-azulene; |
| 207 | 2-(4-Chloro-phenyl)-3-phenyl-2,4,5,6,7,8-hexahydro-1,2,6-triaza-azulene; |
| 208 | 6-Methyl-2,3-diphenyl-2,4,5,6,7,8-hexahydro-1,2,6-triaza-azulene; |
| 209 | 2-Isopropyl-3-p-tolyl-2,4,5,6,7,8-hexahydro-1,2,6-triaza-azulene; |
| 210 | 3-(4-Ethyl-phenyl)-2-isopropyl-2,4,5,6,7,8-hexahydro-1,2,6-triaza-azulene; |
| 211 | 3-(4-Chloro-phenyl)-2-isopropyl-2,4,5,6,7,8-hexahydro-1,2,6-triaza-azulene; |
| 212 | 4-(2-Isopropyl-2,4,5,6,7,8-hexahydro-1,2,6-triaza-azulen-3-yl)-benzonitrile; |
| 213 | 2-Isopropyl-3-(4-trifluoromethyl-phenyl)-2,4,5,6,7,8-hexahydro-1,2,6-triaza-azulene; |
| 214 | 2-Ethyl-3-p-tolyl-2,4,5,6,7,8-hexahydro-1,2,6-triaza-azulene; |
| 215 | 2-tert-Butyl-3-phenyl-2,4,5,6,7,8-hexahydro-1,2,6-triaza-azulene; |
| 216 | 2-tert-Butyl-3-(4-fluoro-phenyl)-2,4,5,6,7,8-hexahydro-1,2,6-triaza-azulene; |
| 217 | 2-Cyclopentyl-3-p-tolyl-2,4,5,6,7,8-hexahydro-1,2,6-triaza-azulene; |
| 218 | 2-Cyclopentyl-3-(4-trifluoromethyl-phenyl)-2,4,5,6,7,8-hexahydro-1,2,6-triaza-azulene; |
| 219 | 3-(3-Chloro-phenyl)-2-cyclopentyl-2,4,5,6,7,8-hexahydro-1,2,6-triaza-azulene; |
| 220 | 2-Cyclopentyl-3-(4-methoxy-phenyl)-2,4,5,6,7,8-hexahydro-1,2,6-triaza-azulene; |
| 221 | 2-(3,3-Dimethyl-cyclopentyl)-3-phenyl-2,4,5,6,7,8-hexahydro-1,2,6-triaza-azulene; |
| 222 | 2-(3,3-Dimethyl-cyclopentyl)-3-(4-fluoro-phenyl-2,4,5,6,7,8-hexahydro-1,2,6-triaza-azulene; |
| 223 | 3-(4-Chloro-phenyl)-2-(3,3-dimethyl-cyclopentyl)-2,4,5,6,7,8-hexahydro-1,2,6-triaza-azulene; |
| 224 | 2-Cyclohexyl-3-(4-fluoro-phenyl)-2,4,5,6,7,8-hexahydro-1,2,6-triaza-azulene; |
| 225 | 2-Cyclohexyl-3-(3,4-difluoro-phenyl)-2,4,5,6,7,8-hexahydro-1,2,6-triaza-azulene; |
| 226 | 2-Cyclohexyl-3-p-tolyl-2,4,5,6,7,8-hexahydro-1,2,6-triaza-azulene; |
| 227 | 2-Cyclohexyl-3-(4-methoxy-phenyl)-2,4,5,6,7,8-hexahydro-1,2,6-triaza-azulene; |
| 228 | 4-(2-Cyclohexyl-2,4,5,6,7,8-hexahydro-1,2,6-triaza-azulen-3-yl)-benzonitrile; |
| 229 | 3-(3-Chloro-phenyl)-2-cyclohexyl-2,4,5,6,7,8-hexahydro-1,2,6-triaza-azulene; |
| 231 | 3-(4-Fluoro-phenyl)-2-isopropyl-4,5,6,7-tetrahydro-2H-pyrazolo[4,3-c]pyridine; |
| 232 | 2-Cyclopentyl-3-furan-3-yl-2,4,5,6,7,8-hexahydro-1,2,6-triaza-azulene; |
| 233 | 2-Cyclopentyl-3-thiophen-2-yl-2,4,5,6,7,8-hexahydro-1,2,6-triaza-azulene; |
| 234 | 2-tert-Butyl-3-thiophen-3-yl-2,4,5,6,7,8-hexahydro-1,2,6-triaza-azulene; |
| 235 | 2-tert-Butyl-3-furan-3-yl-2,4,5,6,7,8-hexahydro-1,2,6-triaza-azulene; |
| 236 | 2-Cyclopentyl-3-(3,4-difluoro-phenyl)-2,4,5,6,7,8-hexahydro-1,2,6-triaza-azulene; |
| 238 | 3-(4-Chloro-phenyl)-2-cyclobutyl-2,4,5,6,7,8-hexahydro-1,2,6-triaza-azulene; |
| 240 | 2-tert-Butyl-3-thiophen-2-yl-2,4,5,6,7,8-hexahydro-1,2,6-triaza-azulene; |
| 241 | 3-(3-Chloro-4-fluoro-phenyl)-2-cyclopentyl-2,4,5,6,7,8-hexahydro-1,2,6-triaza-azulene; |
| 242 | 2-Isopropyl-3-(4-methoxy-phenyl)-2,4,5,6,7,8-hexahydro-1,2,6-triaza-azulene; |
| 243 | 2-Isopropyl-3-(4-trifluoromethoxy-phenyl)-2,4,5,6,7,8-hexahydro-1,2,6-triaza-azulene; |
| 244 | 2-Isopropyl-3-(4-isopropyl-phenyl)-2,4,5,6,7,8-hexahydro-1,2,6-triaza-azulene; |
| 245 | 3-(4-tert-Butyl-phenyl)-2-isopropyl-2,4,5,6,7,8-hexahydro-1,2,6-triaza-azulene; |
| 246 | 2-Isopropyl-3-m-tolyl-2,4,5,6,7,8-hexahydro-1,2,6-triaza-azulene; |
| 247 | 2-Isopropyl-3-o-tolyl-2,4,5,6,7,8-hexahydro-1,2,6-triaza-azulene; |
| 248 | 3-(3,4-Dichloro-phenyl)-2-isopropyl-2,4,5,6,7,8-hexahydro-1,2,6-triaza-azulene; |
| 249 | 2-Benzyl-3-(4-fluoro-phenyl)-2,4,5,6,7,8-hexahydro-1,2,6-triaza-azulene; |
| 250 | 2-Isopropyl-3-thiophen-2-yl-2,4,5,6,7,8-hexahydro-1,2,6-triaza-azulene; |
| 251 | 3-(2-Chloro-phenyl)-2-isopropyl-2,4,5,6,7,8-hexahydro-1,2,6-triaza-azulene; |
| 252 | 1-[4-(2-Isopropyl-2,4,5,6,7,8-hexahydro-1,2,6-triaza-azulen-3-yl)-phenyl]-ethanone; |
| 253 | 2-Isopropyl-3-(4-nitro-phenyl)-2,4,5,6,7,8-hexahydro-1,2,6-triaza-azulene; |
| 256 | 2-Benzyl-3-(4-chloro-phenyl)-2,4,5,6,7,8-hexahydro-1,2,5-triaza-azulene; |
| 257 | 2-Ethyl-3-(4-ethyl-phenyl)-2,4,5,6,7,8-hexahydro-1,2,6-triaza-azulene; |
| 258 | 4-(2-Ethyl-2,4,5,6,7,8-hexahydro-1,2,6-triaza-azulen-3-yl)-benzonitrile; |
| 259 | 3-(4-Fluoro-phenyl)-2-isopropyl-6-methyl-2,4,5,6,7,8-hexahydro-1,2,6-triaza-azulene; |
| 260 | 3-(4-Fluoro-phenyl)-2,6-diisopropyl-2,4,5,6,7,8-hexahydro-1,2,6-triaza-azulene; |
| 261 | 2-Ethyl-3-(4-isopropyl-phenyl)-2,4,5,6,7,8-hexahydro-1,2,6-triaza-azulene; |
| 262 | 2-Ethyl-3-(4-methoxy-phenyl)-2,4,5,6,7,8-hexahydro-1,2,6-triaza-azulene; |
| 263 | 2-Ethyl-3-(4-trifluoromethyl-phenyl)-2,4,5,6,7,8-hexahydro-1,2,6-triaza-azulene; |
| 264 | 2-Ethyl-3-o-tolyl-2,4,5,6,7,8-hexahydro-1,2,6-triaza-azulene; |
| 265 | 3-(2-Chloro-phenyl)-2-ethyl-2,4,5,6,7,8-hexahydro-1,2,6-triaza-azulene; |
| 266 | 2-Ethyl-3-(2-fluoro-phenyl)-2,4,5,6,7,8-hexahydro-1,2,6-triaza-azulene; |
| 267 | 3-(2,4-Dichloro-phenyl)-2-isopropyl-2,4,5,6,7,8-hexahydro-1,2,6-triaza-azulene; |
| 268 | [4-(2-Ethyl-2,4,5,6,7,8-hexahydro-1,2,6-triaza-azulen-3-yl)-phenyl]-dimethyl-amine; |
| 269 | 6-Benzyl-3-(4-fluoro-phenyl)-2-isopropyl-2,4,5,6,7,8-hexahydro-1,2,6-triaza-azulene; |
| 270 | 3-(4-Fluoro-phenyl)-2-isopropyl-6-(3-phenyl-propyl)-2,4,5,6,7,8-hexahydro-1,2,6-triaza-azulene; |
| 271 | 3-(4-Fluoro-phenyl)-2-isopropyl-6-phenethyl-2,4,5,6,7,8-hexahydro-1,2,6-triaza-azulene; and |
| 272 | 3-(4-Fluoro-phenyl)-2-isopropyl-4,5,7,8-tetrahydro-2H-1,2,6-triaza-azulene-6-carboxylic acid tert-butyl ester. |
| 274 | 3-(4'-Chloro-biphenyl-4-yl)-2-(2,2,2-trifluoro-ethyl)-2,4,5,6,7,8-hexahydro-1,2,6-triaza-azulene; |
| 275 | 3-(4'-Chloro-biphenyl-4-yl)-2-cyclopentyl-2,4,5,6,7,8-hexahydro-1,2,6-triaza-azulene; |
| 276 | 2-Cyclobutyl-3-phenyl-2,4,5,6,7,8-hexahydro-1,2,6-triaza-azulene; |
| 277 | 2-Cyclobutyl-3-(4-fluoro-phenyl)-2,4,5,6,7,8-hexahydro-1,2,6-triaza-azulene; |
| 278 | 2-Cyclobutyl-3-p-tolyl-2,4,5,6,7,8-hexahydro-1,2,6-triaza-azulene; |
| 279 | 2-Cyclobutyl-3-(4-trifluoromethyl-phenyl)-2,4,5,6,7,8-hexahydro-1,2,6-triaza-azulene; |
| 280 | 4-(2-Cyclobutyl-2,4,5,6,7,8-hexahydro-1,2,6-triaza-azulen-3-yl)-benzonitrile |
| 281 | 2-Cyclopropyl-3-phenyl-2,4,5,6,7,8-hexahydro-1,2,6-triaza-azulene; |
| 282 | 2-Cyclopropyl-3-(4-fluoro-phenyl)-2,4,5,6,7,8-hexahydro-1,2,6-triaza-azulene; |
| 283 | 2-(1-Ethyt-propyl)-3-(4-fluoro-3-methyl-phenyl)-2,4,5.,6,7,8-hexahydro-1,2,6-triaza-azulene; |
| 284 | 2-Cyclopropyl-3-p-tolyl-2,4,5,6,7,8-hexahydro-1,2,6-triaza-azulene; |
| 285 | 2-Cyclopropyl-3-thiophen-3-yl-2,4,5,6,7,8-hexahydro-1,2,6-triaza-azulene; |
| 286 | 4-(2-Cyclopropyl-2,4,5,6,7,8-hexahydro-1,2,6-triaza-azulen-3-yl)-benzonitrile; |
| 287 | 6-Benzyl-2-isopropyl-3-phenyl-4,5,6,7-tetrahydro-2H-pyrazolo[3,4-c]pyridine; |
| 288 | 2-lsopropyl-3-phenyl-4,5,6,7-tetrahydro-2H-pyrazolo[3,4-c]pyridine; |
| 289 | 6-Benzyl-2-isopropyl-3-thiophen-3-yl-4,5,6,7-tetrahydro-2H-pyrazolo[3,4-c]pyridine; |
| 290 | 6-Benzyl-2-isopropyl-3-p-tolyl-4,5,6,7-tetrahydro-2H-pyrazolo[3,4-c]pyridine. |
| 291 | 6-Benzyl-3-(4-fluoro-phenyl)-2-isopropyl-4,5,6,7-tetrahydro-2H-pyrazolo[3,4-c]pyridine; |
| 292 | 3-(4-Fluoro-phenyl)-2-isopropyl-4,5,6,7-tetrahydro-2H-pyrazolo[3,4-c]pyridine; |
| 293 | 2-Isopropyl-3-p-tolyl-4,5,6,7-tetrahydro-2H-pyrazolo[3,4-c]pyridine; |
| 294 | 2-Cyclopentyl-3-(4-fluoro-phenyl)-5,5,7,7-tetramethyl-2,4,5,6,7,8-hexahydro-1,2,6-triaza-azulene; |
| 295 | 2-Cyclopentyl-5,5,7,7-tetramethyl-3-phenyl-2,4,5,6,7,8-hexahydro-1,2,6-triaza-azulene; |
| 296 | 2-Isopropyl-5,5,7,7-tetramethyl-3-phenyl-2,4,5,6,7,8-hexahydro-1,2,6-triaza-azulene; |
| 297 | 3-(4-Fluoro-phenyl)-2-isopropyl-5,5,7,7-tetramethyl-2,4,5,6,7,8-hexahydro-1,2,6-triaza-azulene; |
| 298 | 2-sec-Butyl-3-phenyl-2,4,5,6,7,8-hexahydro-1,2,6-triaza-azulene; |
| 299 | 2-*sec*-Butyl-3-(4-fluoro-phenyl)-2,4,5,6,7,8-hexahydro-1,2,6-triaza-azulene; |
| 300 | 2-sec-Butyl-3-p-tolyl-2,4,5,6,7,8-hexahydro-1,2,6-triaza-azulene; |
| 301 | 2-*sec*-Butyl-3-(4-trifluoromethyl-phenyl)-2,4,5,6,7,8-hexahydro-1,2,6-triaza-azulene; |
| 302 | 2-Cyclopentyl-3-(4-fluoro-phenyl)-6-methyl-2,4,5,6,7,8-hexahydro-1,2,6-triaza-azulene; |
| 303 | 4-(2-Isopropyl-2,4,5,6,7,8-hexahydro-1,2,6-triaza-azulen-3-yl)-benzamide; |
| 304 | 2-Isopropyl-3-[4-(1H-tetrazol-5-yl)-phenyl]-2,4,5,6,7,8-hexahydro-1,2,6-triaza-azulene; |
| 305 | 6-Benzyl-3-(4-fluoro-phenyl)-2-isopropyl-8-methyl-2,4,5,6,7,8-hexahydro-1,2,6-triaza-azulene; |
| 306 | 3-(4-Fluoro-phenyl)-2-isopropyl-8-methyl-2,4,5,6,7,8-hexahydro-1,2,6-triaza-azulene; |
| 307 | 3-(4-Fluoro-phenyl)-2-isopropyl-4-methyl-2,4,5,6,7,8-hexahydro-1,2,6-triaza-azulene; |
| 308 | 2-Cyclopentyl-3-(4-fluoro-phenyl)-7-methyl-2,4,5,6,7,8-hexahydro-1,2,6-triaza-azulene; |
| 309 | 2-Cyclopentyl-3-(4-fluoro-phenyl)-5-methyl-2,4,5,6,7,8-hexahydro-1,2,6-triaza-azulene; |
| 310 | 2-Cyclopentyl-7-methyl-3-p-tolyl-2,4,5,6,7,8-hexahydro-1,2,6-triaza-azulene; |
| 311 | 2-Isopropyl-7-methyl-3-phenyl-2,4,5,6,7,8-hexahydro-1,2,6-triaza-azulene; |
| 312 | 2-Isopropyl-5-methyl-3-phenyl-2,4,5,6,7,8-hexahydro-1,2,6-triaza-azulene; |
| 313 | 3-(4-Fluoro-phenyl)-2-isopropyl-7-methyl-2,4,5,6,7,8-hexahydro-1,2,6-triaza-azulene; |
| 314 | 3-(4-Fluoro-phenyl)-2-isopropyl-5-methyl-2,4,5,6,7,8-hexahydro-1,2,6-triaza-azulene; |
| 315 | 2-Isopropyl-7-methyl-3-p-tolyl-2,4,5,6,7,8-hexahydro-1,2,6-triaza-azulene; |
| 318 | 3-(4-Chloro-phenyl)-2-pyridin-2-ylmethyl-1,4,5,6,7,8-hexahydro-1,2,6-triaza-azulene; |
| 327 | 3-[3-(4-Chloro-phenyl)-5,6,7,8-tetrahydro-4H-1,2,6-triaza-azulen-2-yl]-propionitrile; |
| 328 | 3-(4-Chloro-phenyl)-2-cycloheptyl-2,4,5,6,7,8-hexahydro-1,2,6-triaza-azulene; |
| 329 | 3-(4-Chloro-phenyl)-2-cyclooctyl-2,4,5,6,7,8-hexahydro-1,2,6-triaza-azulene; |
| 330 | 3-(4-Chloro-phenyl)-2-(4-methyl-cyclohexyl)-2,4,5,6,7,8-hexahydro-1,2,6-triaza-azulene; |
| 331 | 2-Benzyl-3-(4-chloro-phenyl)-2,4,5,6-tetrahydro-pyrrolo[3,4-c]pyrazole; and |
| 338 | 3-(4-Fluoro-phenyl)-2-isopropyl-5,7-dimethyl-2,4,5,6,7,8-hexahydro-1,2,6-triaza-azulene. |

In another embodiment of the present invention, preferred compounds are selected from the group consisting of:

| EX | CHEMICAL NAME |
|---|---|
| 59 | 1 -Benzyl-3-(4-chloro-phenyl)-1,4,5,6,7,8-hexahydro-1,2,6-triaza-azulene; |
| 74 | 3-(4-Chloro-phenyl)-1-(3-methyl-benzyl)-1,4,5,6,7,8-hexahydro-1,2,6-triaza-azulene; |
| 75 | 3-(4-Chloro-phenyl)-1-(4-fluoro-benzyl)-1,4,5,6,7,8-hexahydro-1,2,6-triaza-azulene; |
| 76 | 3-(4-Chloro-phenyl)-1-(3-fluoro-benzyl)-1,4,5,6,7,8-hexahydro-1,2,6-triaza-azulene; |
| 103 | 3-(4-Chloro-phenyl)-1-thiophen-2-ylmethyl-1,4,5,6,7,8-hexahydro-1,2,6-triaza-azulene; |
| 104 | 1-Benzyl-3-thiophen-2-yl-1,4,5,6,7,8-hexahydro-1,2,6-triaza-azulene; |
| 108 | 3-(4-Chloro-phenyl)-1-(2,4-difluoro-benzyl)-1,4,5,6,7,8-hexahydro-1,2,6-triaza-azulene; |
| 160 | 4-[3-(4-Chloro-phenyl)-5,6,7,8-tetrahydro-4H-1,2,6-triaza-azulen-1-ylmethyl]-2-fluoro-phenol; |
| 165 | 1-Benzyl-3-(4-chloro-phenyl)-6-methyl-1,4,5,6,7,8-hexahydro-1,2,6-triaza-azulene; |
| 166 | 1-Benzyl-3-(4-chloro-phenyl)-6-ethyl-1,4,5,6,7,8-hexahydro-1,2,6-triaza-azulene; |
| 214 | 2-Ethyl-3-p-tolyl-2,4,5,6,7,8-hexahydro-1,2,6-triaza-azulene; |
| 257 | 2-Ethyl-3-(4-ethyl-phenyl)-2,4,5,6,7,8-hexahydro-1,2,6-triaza-azulene; and |
| 273 | 1-Benzyl-3-(4-chtoro-phenyl)-1,4,5,6,7,8-hexahydro-1,2,6-triaza-azulene citrate salt. |

In still another embodiment of the present invention, preferred compounds are selected from the group consisting of:

| EX | CHEMICAL NAME |
|---|---|
| 131 | 3-(4-Chloro-phenyl)-2-isobutyl-2,4,5,6,7,8-hexahydro-1,2,6-triaza-azulene; |
| 133 | 2-Benzyl-3-phenyl-2,4,5,6,7,8-hexahydro-1,2,6-triaza-azulene; |
| 177 | 2-Cyclohexyl-3-phenyl-2,4,5,6,7,8-hexahydro-1,2,6-triaza-azulene; |
| 178 | 3-(4-Chloro-phenyl)-2-cyclohexyl-2,4,5,6,7,8-hexahydro-1,2,6-triaza-azulene; |
| 181 | 3-(4-Chloro-phenyl)-2-cyclopentyl-2,4,5,6,7,8-hexahydro-1,2,6-triaza-azulene; |
| 182 | 2-Cyclopentyl-3-(4-fluoro-phenyl)-2,4,5,6,7,8-hexahydro-1,2,6-triaza-azulene; |
| 183 | 2-(1-Ethyl-propyl)-3-(3-fluoro-phenyl)-2,4,5,6,7,8-hexahydro-1,2,6-triaza-azulene; |
| 184 | 2-(1-Ethyl-propyl)-3-(4-fluoro-phenyl)-2,4,5,6,7,8-hexahydro-1,2,6-triaza-azulene; |
| 186 | 2-(1-Ethyl-propyl)-3-phenyl-2,4,5,6,7,8-hexahydro-1,2,6-triaza-azulene; |
| 191 | 2-(1-Ethyl-propyl)-3-thiophen-2-yl-2,4,5,6,7,8-hexahydro-1,2,6-triaza-azulene; |
| 215 | 2-tert-Butyl-3-phenyl-2,4,5,6,7,8-hexahydro-1,2,6-triaza-azulene; |
| 216 | 2-tert-Butyl-3-(4-fluoro-phenyl)-2,4,5,6,7,8-hexahydro-1,2,6-triaza-azulene; |
| 217 | 2-Cyclopentyl-3-p-tolyl-2,4,5,6,7,8-hexahydro-1,2,6-triaza-azulene; |
| 218 | 2-Cyclopentyl-3-(4-trifluoromethyl-phenyl)-2,4,5,6,7,8-hexahydro-1,2,6-triaza-azulene; |
| 220 | 2-Cyclopentyl-3-(4-methoxy-phenyl)-2,4,5,6,7,8-hexahydro-1,2,6-triaza-azulene; |
| 236 | 2-Cyclopentyl-3-(3,4-difluoro-phenyl)-2,4,5,6,7,8-hexahydro-1,2,6-triaza-azulene; |
| 238 | 3-(4-Chloro-phenyl)-2-cyclobutyl-2,4,5,6,7,8-hexahydro-1,2,6-triaza-azulene; |
| 241 | 3-(3-Chloro-4-fluoro-phenyl)-2-cyclopentyl-2,4,5,6,7,8-hexahydro-1,2,6-triaza-azulene; |
| 242 | 2-Isopropyl-3-(4-methoxy-phenyl)-2,4,5,6,7,8-hexahydro-1,2,6-triaza-azulene; |
| 277 | 2-Cyclobutyl-3-(4-fluoro-phenyl)-2,4,5,6,7,8-hexahydro-1,2,6-triaza-azulene; |
| 278 | 2-Cyclobutyl-3-p-tolyl-2,4,5,6,7,8-hexahydro-1,2,6-triaza-azulene; |
| 279 | 2-Cyclobutyl-3-(4-trifluoromethyl-phenyl)-2,4,5,6,7,8-hexahydro-1,2,6-triaza-azulene; |
| 284 | 2-Cyclopropyl-3-p-tolyl-2,4,5,6,7,8-hexahydro-1,2,6-triaza-azulene; |
| 300 | 2-sec-Butyl-3-p-tolyl-2,4,5,6,7,8-hexahydro-1,2,6-triaza-azulene; |
| 302 | 2-Cyclopentyl-3-(4-fluoro-phenyl)-6-methyl-2,4,5,6,7,8-hexahydro-1,2,6-triaza-azulene; |
| 306 | 3-(4-Fluoro-phenyl)-2-isopropyl-8-methyl-2,4,5,6,7,8-hexahydro-1,2,6-triaza-azulene; and |
| 310 | 2-Cyclopentyl-7-methyl-3-p-tolyl-2,4,5,6,7,8-hexahydro-1,2,6-triaza-azulene. |

In yet another embodiment of the present invention preferred compounds are selected from the group consisting of:

| EX | CHEMICAL NAME |
|---|---|
| 47 | 1-Benzyl-3-(4-fluoro-phenyl)-1,4,5,6,7,8-hexahydro-1,2,6-triaza-azulene; |
| 64 | 3-(4-Chloro-phenyl)-2-ethyl-2,4,5,6,7,8-hexahydro-1,2,6-triaza-azulene; |
| 118 | 3-(4-Chloro-phenyl)-1 -isobutyl-1,4,5,6,7,8-hexahydro-1,2,6-triaza-azulene; |
| 180 | 2-Cyclopentyl-3-phenyl-2,4,5,6,7,8-hexahydro-1,2,6-triaza-azulene; |
| 190 | 3-(4-Fluoro-phenyl)-2-isopropyl-2,4,5,6,7,8-hexahydro-1,2,6-triaza-azulene; |
| 192 | 2-Cyclopentyl-3-thiophen-3-yl-2,4,5,6,7,8-hexahydro-1,2,6-triaza-azulene; |
| 209 | 2-Isopropyl-3-p-tolyl-2,4,5,6,7,8-hexahydro-1,2,6-triaza-azulene; |
| 210 | 3-(4-Ethyl-phenyl)-2-isopropyl-2,4,5,6,7,8-hexahydro-1,2,6-triaza-azulene; |
| 211 | 3-(4-Chloro-phenyl)-2-isopropyl-2,4,5,6,7,8-hexahydro-1,2,6-triaza-azulene; |
| 212 | 4-(2-Isopropyl-2,4,5,6,7,8-hexahydro-1,2,6-triaza-azulen-3-yl)-benzonitrile; |
| 213 | 2-lsopropyl-3-(4-trifluoromethyl-phenyl)-2,4,5,6,7,8-hexahydro-1,2,6-triaza-azulene; |
| 232 | 2-Cyclopentyl-3-furan-3-yl-2,4,5,6,7,8-hexahydro-1,2,6-triaza-azulene; |
| 233 | 2-Cyclopentyl-3-thiophen-2-yl-2,4,5,6,7,8-hexahydro-1,2,6-triaza-azulene; |
| 284 | 2-Cyclopropyl-3-p-tolyl-2,4,5,6,7,8-hexahydro-1,2,6-triaza-azulene; |
| 300 | 2-sec-Butyl-3-p-tolyl-2,4,5,6,7,8-hexahydro-1,2,6-triaza-azulene; and |
| 315 | 2-Isopropyl-7-methyl-3-p-tolyl-2,4,5,6,7,8-hexahydro-1,2,6-triaza-azulene. |

The features and advantages of the invention are apparent to one of ordinary skill in the art. Based on this disclosure, including the summary, detailed description, background, examples, and claims, one of ordinary skill in the art will be able to make modifications and adaptations to various conditions and usages. Publications described herein are incorporated by reference in their entirety.

The fused heterocyclic compounds of formulas (I), (II), and (III) may be prepared by a number of reaction schemes. Access to compounds of formula (I) is described in Scheme 1. Preparation of compounds of formula (II) is described in Schemes 2, 3, 5, and 6. Synthesis of compounds of formula (III) is shown in Schemes 3 and 4. Persons skilled in the art will recognize that certain compounds are more advantageously produced by one scheme as compared to the other.

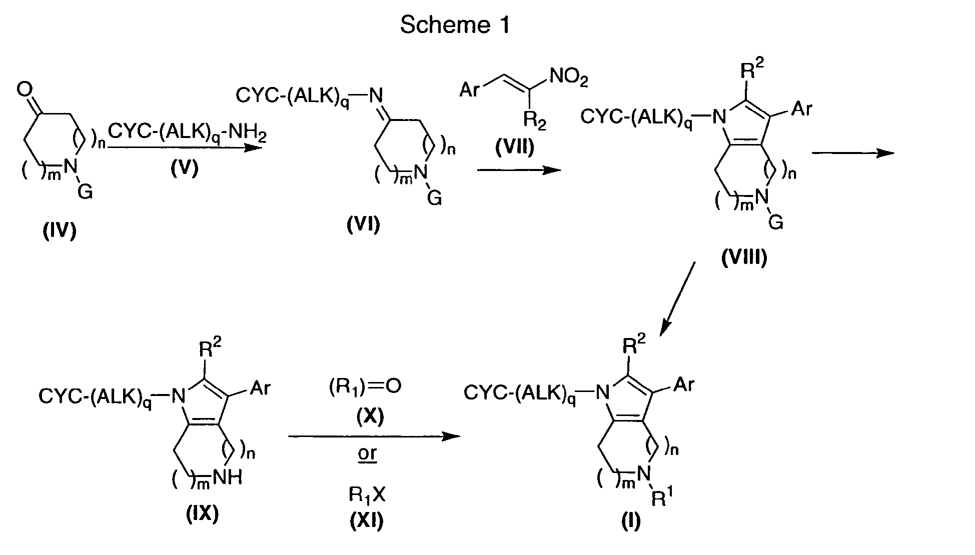

Referring to Scheme 1, compounds of formula (I) may be prepared from compounds of formula (IV). The amine moiety in compounds of formula (IV) can be suitably protected, shown by substituent G, as an alkyl or benzyl amine, amide, carbamate or other groups such as those described in "Protecting Groups In Organic Synthesis", 3rd ed.; T.W. Greene and P.G.M. Wuts, John Wiley & Sons, 1999 (G is -C₁₋₆alkyl, -COOC₁₋₆alkyl, -(C=O)C₁₋₆alkyl, or benzyl unsubstituted or substituted with -OC₁₋₆alkyl or -C₁₋₆alkyl). A preferred protecting group would be the t-butyl carbamate (Boc) group. The carbonyl functional group of compound (IV) can be treated with a primary amine of type (V), in a suitable solvent like THF, toluene, benzene, methanol or ethanol at temperatures between 20 and 110 °C with removal of water by either Dean-Stark apparatus or by the addition of a dehydrating agent such as SiO₂, MgSO₄, CuSO₄, Ti(O-iPr)₄ or 4 Å molecular sieves to form the corresponding imines of type (VI). Preferred solvents are toluene and ethanol with preferred dehydrating agents being SiO₂ and 4 Å molecular sieves. One skilled in the art would recognize that the imines of type (VI) might exist as more than one tautomeric form. Compounds of type (VI) can then be treated with a nitro olefin of type (VII) to give pyrrole compounds of formula (VIII). One skilled in the art would recognize that imines of formula (VI), existing as more that one enamine tautomer, would give rise to regioisomers upon treatment with a nitro olefin of type (VII) depending on the structure of the compound of formula (IV). The protecting group on the nitrogen can either be removed using generally accepted methods or, depending on the type of group involved, can be converted directly to compounds of formula (I). More specifically, a group such as a t-butyl carbamate can be removed with an acid like trifluoroacetic acid or hydrochloric acid and the like in a solvent such as CH₂Cl₂, ethanol or methanol to afford compounds of formula (IX). It will be generally recognized that compounds of formula (IX) represent a subset of compounds of formula (I) wherein R¹ is equal to H. Compounds of formula (IX) and (I) may be converted to their corresponding salts using methods known to those skilled in the art.

Compounds such as (I) can be prepared from compounds of type (IX) using conventional synthetic methods such as alkylation or reductive amination. Thus, treatment of compounds of formula (IX) with a compound of formula (X) containing a carbonyl group in the presence of a reductant such as NaBH₄, NaBH₃CN, NaBH(OAc)₃ or hydrogen gas in the presence of a catalyst in a solvent such as CH₂Cl₂, DCE, THF, ethanol, methanol or similar will afford compounds of formula (I). One skilled in the art will recognize that the addition of acid to decrease the pH of the reaction mixture to less than pH 7 may be required. Examples of acids may include AcOH, Ti(O-iPr)₄, trifluoroacetic acid or hydrochloric acid and the like. In addition, compounds such as (IX) can be treated with an alkylating agent of type (XI). For example, treatment with an alkyl chloride, bromide, iodide, mesylate or tosylate (wherein X is Cl, Br, I, OMs, OTs, or the like) in solvent such as DMF, DMA, THF or ethanol and in the presence of a base like NaHCO₃, Na₂CO₃, K₂CO₃ or Cs₂CO₃ will give compounds of formula (I).

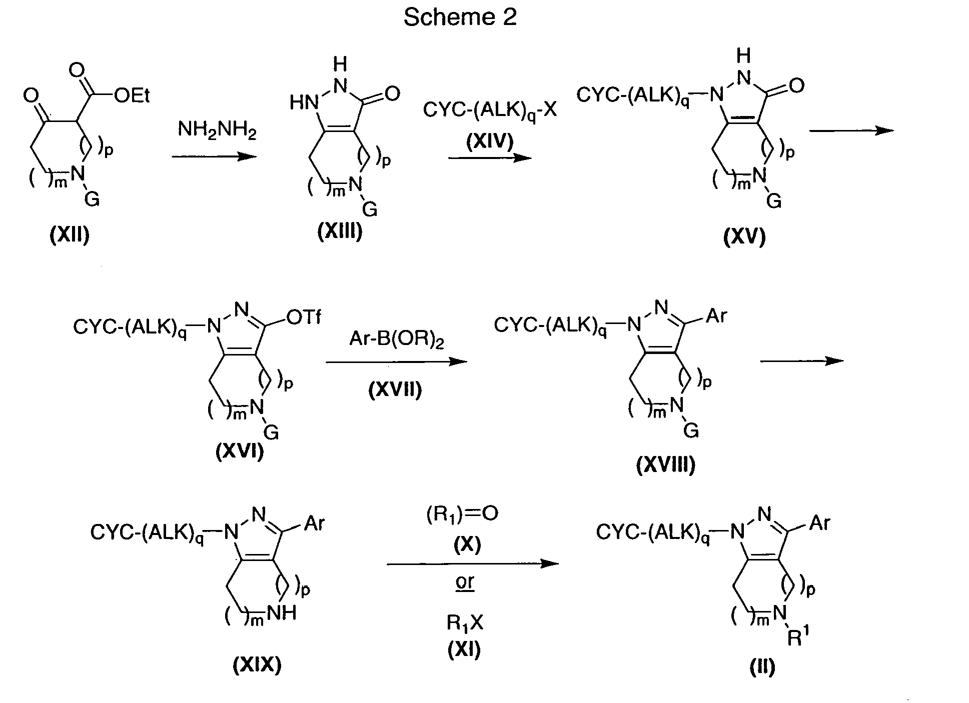

Referring to Scheme 2, compounds of formula (II) can be prepared from compounds of formula (XII). As in Scheme 1, the amine moiety in compounds of formula (XII) can be suitably protected, shown by substituent G, as an alkyl or benzyl amine, amide, carbamate or other groups such as those described in "Protecting Groups In Organic Synthesis", 3rd ed.; T.W. Greene and P.G.M. Wuts, John Wiley & Sons, 1999. A preferred protecting group would be the t-butyl carbamate (Boc) group. The condensation of hydrazine with compounds of formula (XII) in a solvent like methanol, ethanol, isopropanol or t-butyl alcohol at temperatures from 20 to 80 °C will form compounds of type (XIII). One skilled it the art will recognize that compounds of formula (XIII) may exist in more that one resonance form. More specifically, compounds of formula (XIII) are tautomeric with the corresponding 3-hydroxypyrazoles. Compounds such as (XIII) can be treated with an alkylating agent such as formula (XIV) to afford compounds of type (XV). For example, treatment with an alkyl or benzyl chloride, bromide, iodide, mesylate or tosylate (wherein X is Cl, Br, I, OMs, OTs, or the like) in DMF, DMA, THF or ethanol in the presence of a base like NaHCO₃, Na₂CO₃, K₂CO₃, NaH, potassium tert-butoxide, or Cs₂CO₃ will afford compounds of formula (XV). One skilled in the art will recognize that the alkylation of compounds of formula (XIII) may give rise to regioisomers. Compounds of type (XV) can be converted into a precursor for transition metal-catalyzed cross-coupling reactions, such as Stille, Suzuki, Negishi or other such coupling reactions known to one skilled in the art. For example, treatment with POCl₃, PCl₃, PCl₅, PBr₃ or POBr₃ can afford the corresponding 3-halopyrazoles. A preferred method would involve treatment with a triflating agent such as trifluoromethanesulfonic anhydride or N-phenyltrifluoromethanesulfonimide in DCE, CH₂Cl₂, THF or the like in the presence of a base like pyridine, triethylamine or diisopropylethylamine to provide pyrazole triflates of formula (XVI). Treatment of triflates of formula (XVI) with an organoboron compound of formula (XVII) in the presence of a catalyst like Pd(PPh₃)₄, PdCl₂(PPh₃)₂, PdCl₂(P*o*-tol₃)₂, PdCl₂(dppe) or PdCl₂(dppf) in a solvent such as THF, 1,4-dioxane, DMA, DMF, DME, toluene, toluene/ethanol, or toluene/H₂O mixtures, in the presence of a base such as Na₂CO₃, K₂CO₃, C_{S2}CO₃, K₃PO₄, KF, CsF, KOAc or the like will afford compounds of formula (XVIII). Preferred catalysts are Pd(PPh₃)₄ and PdCl₂(dppf), with or without additives such as dppf and catalytic Bu₄NBr. Preferred solvents include THF, 1,4-dioxane, toluene, and toluene/H₂O mixtures with preferred bases being Na₂CO₃, K₂CO₃, Cs₂CO₃, and K₃PO₄. The protecting group on the nitrogen of compounds of formula (XVIII) may be removed using generally accepted methods, which one skilled in the art would recognize. More specifically, a group such as a t-butyl carbamate can be removed with an acid like trifluoroacetic acid or hydrochloric acid and the like in a solvent such as CH₂Cl₂, ethanol or methanol to afford compounds of formula (XIX). Compounds of formula (XIX) or (II) may be converted to their corresponding salts using methods known to those skilled in the art. For example, amines of formula (XIX) can be treated with citric acid in a solvent such as methanol to provide the corresponding citrate salt. It will be generally recognized that compounds of formula (XIX) represent a subset of compounds of formula (II) wherein R¹ is equal to H.

Compounds such as (II) can be prepared from compounds of type (XIX) using conventional synthetic methods such as alkylation or reductive amination. Thus, treatment of compounds of formula (XIX) with a compound of formula (X) containing a carbonyl group in the presence of a reductant such as NaBH₄, NaBH₃CN, NaBH(OAc)₃ or hydrogen gas in the presence of a catalyst in a solvent such as CH₂CI₂, DCE, THF, ethanol, methanol or similar will afford compounds of formula (II). One skilled in the art will recognize that the addition of acid to decrease the pH of the reaction mixture to less than pH 7 may be required. Examples of acids may include AcOH, Ti(O-iPr)₄, trifluoroacetic acid or hydrochloric acid and the like. In addition, compounds such as (XIX) can be treated with an alkylating agent of type (XI). For example, treatment with an alkyl chloride, bromide, iodide, mesylate or tosylate (wherein X is CI, Br, I, OMs, OTs, or the like) in solvent such as DMF, DMA, THF or ethanol and in the presence of a base like NaHCO₃, Na₂CO₃, K₂CO₃ or Cs₂CO₃ will give compounds of formula (II).

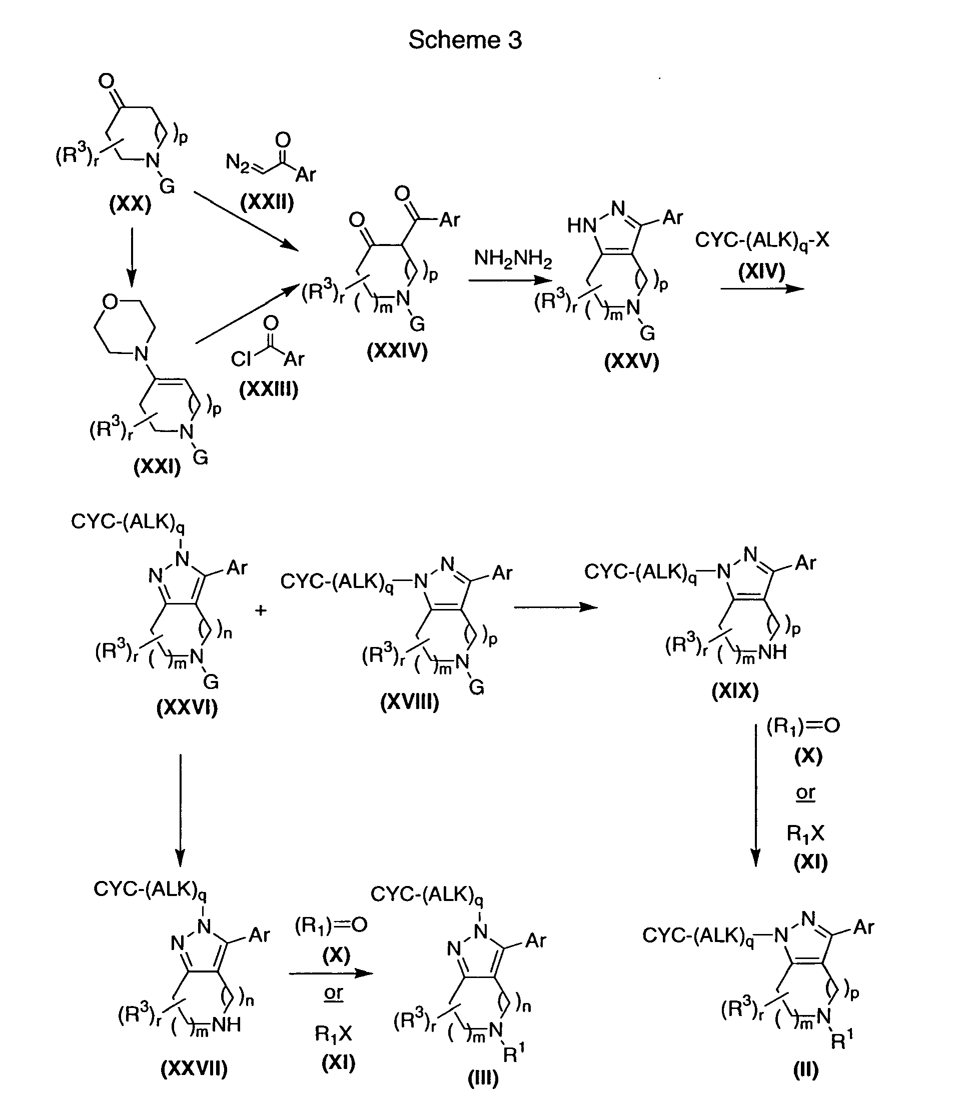

Referring to Scheme 3, compounds of formula (II), (III), (XXVII), and (XXVIII) can be prepared as described. The amine moiety in compounds of formula (XX) can be suitably protected, shown by substituent G, as an alkyl or benzyl amine, amide, carbamate or other groups such as those described in "Protecting Groups In Organic Synthesis", 3rd ed.; T.W. Greene and P.G.M. Wuts, John Wiley & Sons, 1999. A preferred protecting group would be the t-butyl carbamate (Boc) group. The carbonyl functional group of compound (XX) can be treated with a saturated secondary amine, such as morpholine, in a suitable solvent like toluene or benzene at temperatures between 20 and 110 °C with removal of water by a Dean-Stark apparatus with or without an acid catalyst such as TsOH, will afford the corresponding enamines of type (XXI). One skilled in the art would recognize that enamines of type (XXI) might exist as more that one enamine regioisomer depending on the structure of the compound of formula (XX). Treatment of enamines (XXI) with a benzoyl chloride will afford the diketone compounds of formula (XXIV). Additionally, the carbonyl functional group of compound (XX) can be treated with a diazoketone in the presence of a Lewis acid, such as BF₃, to give the diketone compounds (XXIV) directly. The condensation of hydrazine with compounds of formula (XXIV) in a solvent like methanol, ethanol, isopropanol or t-butyl alcohol at temperatures from 20 to 80 °C will form pyrazole compounds of type (XXV). Compounds such as (XXV) can be treated with an alkylating agent of formula (XIV). For example, treatment with an alkyl or benzyl chloride, bromide, iodide, mesylate or tosylate (wherein X is Cl, Br, I, OMs, OTs or the like) in DMF, DMA, THF or ethanol in the presence of a base like NaHCO₃, Na₂CO₃, NaH, potassium tert-butoxide, K₂CO₃ or Cs₂CO₃ will afford a mixture of compounds of formula (XXVI) and (XVIII). One skilled in art would recognize that a mixture of compounds of formula (XXVI) and (XVIII) may be separated by chromatographic or crystallization techniques. The protecting group on the nitrogen may be removed using generally accepted methods, which one skilled in the art would recognize. More specifically, a group such as a t-butyl carbamate can be removed from compounds of formula (XXVI) and (XVIII) with an acid like trifluoroacetic acid or hydrochloric acid and the like in a solvent such as CH₂Cl₂, ethanol or methanol to afford compounds of formula (XXVII) and (XIX) respectively. Compounds of formula (XXVII), (XIX), (II), or (III) may be converted to their corresponding salts using methods known to those skilled in the art. It will be generally recognized that compounds of formula (XXVII) and (XIX) represent subsets of compounds of formula (III) and (II) respectively, wherein R¹ is equal to H.

Compounds such as (II) and (III) can be prepared from compounds of formula (XIX) and (XXVII) respectively, using conventional synthetic methods such as alkylation or reductive amination. Thus, treatment of compounds of formula (XIX) with a compound of formula (X) containing a carbonyl group in the presence of a reductant such as NaBH₄, NaBH₃CN, NaBH(OAc)₃ or hydrogen gas in the presence of a catalyst in a solvent such as CH₂Cl₂, DCE, THF, ethanol, methanol or similar will afford compounds of formula (II). One skilled in the art will recognize that the addition of acid to decrease the pH of the reaction mixture to less than pH 7 may be required. Examples of acids may include AcOH, Ti(O-iPr)₄, trifluoroacetic acid or hydrochloric acid and the like. In addition, compounds such as (XIX) can be treated with an alkylating agent of type (XI). For example, treatment with an alkyl chloride, bromide, iodide, mesylate or tosylate (wherein X is Cl, Br, I, OMs, OTs, or the like) in solvent such as DMF, DMA, THF or ethanol in the presence of a base like NaHCO₃, Na₂CO₃, K₂CO₃ or Cs₂CO₃ will give compounds of formula (II).

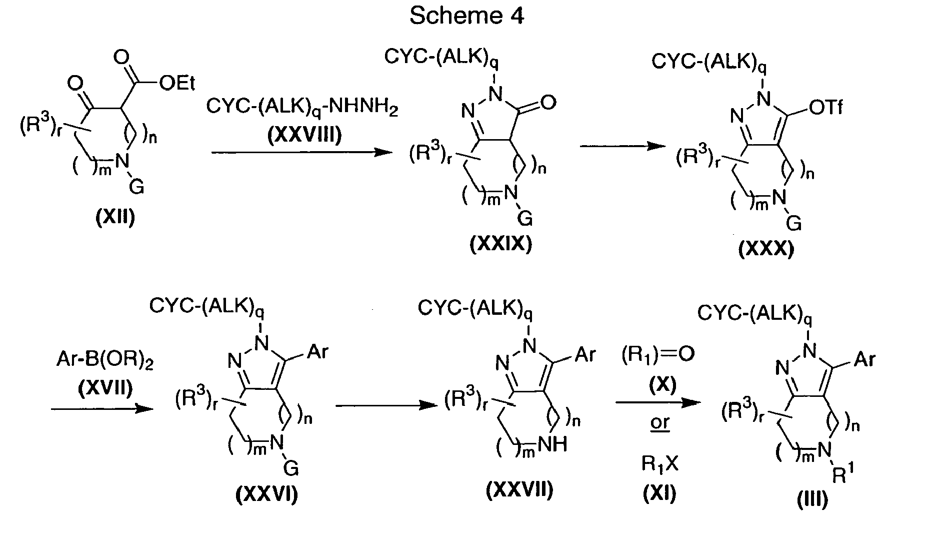

Referring to Scheme 4, compounds of formula (III) can be prepared as outlined. The amine moiety in compounds of formula (XII) can be suitably protected, shown by substituent G, as an alkyl or benzyl amine, amide, carbamate or other groups such as those described in "Protecting Groups In Organic Synthesis", 3rd ed.; T.W. Greene and P.G.M. Wuts, John Wiley & Sons, 1999. The condensation of an alkyl or aryl hydrazine of type (XXVIII), or the salt thereof, with compounds of formula (XII) in a solvent like methanol, ethanol, isopropanol or t-butyl alcohol at temperatures from 20 to 80 °C with or without a base such as NaHCO₃, Na₂CO₃, K₂CO₃, Cs₂CO₃, triethylamine or diisopropylethylamine will afford compounds of formula (XXIX). Preferred solvents are ethanol and t-butyl alcohol with preferred bases being triethylamine and diisopropylethylamine. Compounds of formula (XXIX) can be converted into a precursor for transition metal-catalyzed cross-coupling reactions, such as Stille, Suzuki, Negishi or other such coupling reactions known to one skilled in the art. For example, treatment with POCl₃, PCl₃, PCl₅, PBr₃ or POBr₃ can afford the corresponding 3-halopyrazoles. A preferred method would involve treatment with a triflating agent such as trifluoromethanesulfonic anhydride or N-phenyltrifluoromethanesulfonimide in DCE, CH₂Cl₂, THF or the like in the presence of a base like pyridine, triethylamine or diisopropylethylamine to provide pyrazole triflates of formula (XXX). Treatment of triflates of formula (XXX) with an organoboron compound of formula (XVII) in the presence of a catalyst like Pd(PPh₃)₄, PdCl₂(PPh₃)₂, PdCl₂(P*o*-tol₃)₂, PdCl₂(dppe) or PdCl₂(dppf) in a solvent such as THF, 1,4-dioxane, DMA, DMF, DME, toluene, toluene/ethanol, or toluene/H₂O mixtures, in the presence of a base such as Na₂CO₃, K₂CO₃, Cs₂CO₃, K₃PO₄, KF, CsF, KOAc or the like will afford compounds of formula (XXVI). Preferred catalysts are Pd(PPh₃)₄ and PdCl₂(dppf), with or without additives such as dppf and catalytic Bu₄NBr. Preferred solvents are THF, 1,4-dioxane, toluene, and toluene/H₂O mixtures with preferred bases being Na₂CO₃, K₂CO₃, Cs₂CO₃, and K₃PO₄. The protecting group on the nitrogen of compounds of formula (XXVI) may be removed using generally accepted methods, which one skilled in the art would recognize. More specifically, a group such as a t-butyl carbamate can be removed with an acid like trifluoroacetic acid or hydrochloric acid and the like in a solvent such as CH₂Cl₂, ethanol or methanol to afford compounds of formula (XXVII). Compounds of formula (XXVII) or (III) may be converted to their corresponding salts using methods known to those skilled in the art. It will be generally recognized that compounds of formula (XXVII) represent a subset of compounds of formula (III) wherein R¹ is equal to H.

Compounds such as (III) can be prepared from compounds of type (XXVII) using conventional synthetic methods such as alkylation or reductive amination. Thus, treatment of compounds of formula (XXVII) with a compound of formula (X) containing a carbonyl group in the presence of a reductant such as NaBH₄, NaBH₃CN, NaBH(OAc)₃ or hydrogen gas in the presence of a catalyst in a solvent such as CH₂Cl₂, DCE, THF, ethanol, methanol or similar will afford compounds of formula (III). One skilled in the art will recognize that the addition of acid to decrease the pH of the reaction mixture to less than pH 7 may be required. Examples of acids may include AcOH, Ti(O-iPr)₄, trifluoroacetic acid or hydrochloric acid and the like. In addition, compounds such as (XXVII) can be treated with an alkylating agent of type (XI). For example, treatment with an alkyl chloride, bromide, iodide, mesylate or tosylate (wherein X is Cl, Br, I, OMs, OTs, or the like) in solvent such as DMF, DMA, THF or ethanol in the presence of a base like NaHCO₃, Na₂CO₃, K₂CO₃ or Cs₂CO₃ will afford compounds of formula (III).

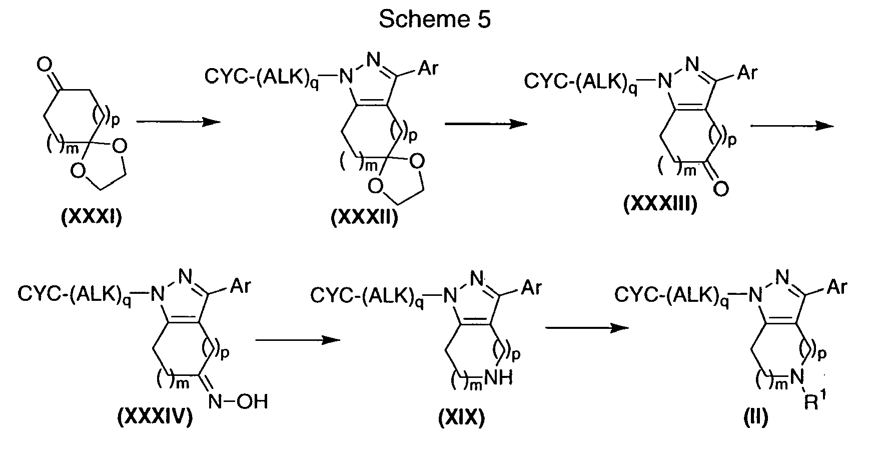

Referring to Scheme 5, in an alternative embodiment, compounds of formula (II) may be prepared from a ketone of formula (XXXI). A ketone of formula (XXXI) may be converted to the pyrazole of formula (XXXII) according to the procedure shown in Scheme 3 for the conversion of a compound of formula (XX) to a compound of formula (XVIII). A compound of formula (XXXIII) may be prepared from a compound of formula (XXXII) upon treatment with aqueous acid. For example, treatment of a compound of formula (XXXII) with HCl in aqueous THF at elevated temperatures will afford compounds of formula (XXXIII). A ketone of formula (XXXIII) may be converted to an oxime of formula (XXXIV) by treatment with hydroxylamine, preferably upon treatment with hydroxylamine in pyridine. Compounds of formula (XXXIV) may exist as a single isomer or mixture of stereoisomers. Treatment of an oxime of formula (XXXIV) with a hydride reducing agent can afford compounds of formula (XIX). In a preferred embodiment, the reducing agent is diisobutylaluminum hydride in CH₂Cl₂. Conversion of compounds of formula (XIX) to compounds of formula (II) can be effected using the methods described in Scheme 3.

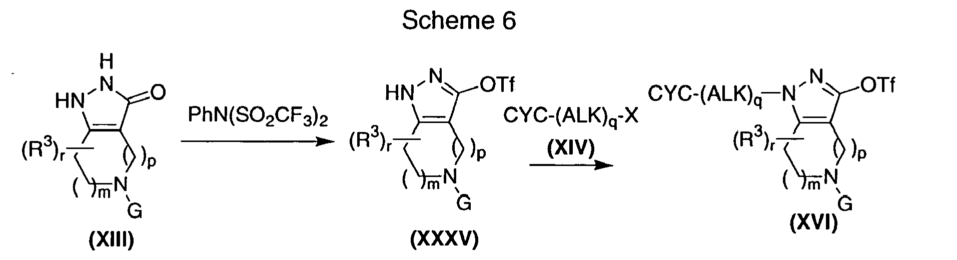

Referring to Scheme 6, in an alternative embodiment, compounds of formula (XIX) may also be prepared as outlined. The amine moiety in compounds of formula (XIII) can be suitably protected, shown by substituent G, as an alkyl or benzyl amine, amide, carbamate or other groups such as those described in "Protecting Groups In Organic Synthesis", 3rd ed.; T.W. Greene and P.G.M. Wuts, John Wiley & Sons, 1999. Preferably, the sequence outlined in Scheme 6 may be employed for compounds where p = 1, m = 2, and G = t-butyl carbamoyl. Treatment of pyrazolones of formula (XIII) with a triflating agent such as N-phenyltrifluoromethanesulfonimide or trifluoromethanesulfonic anhydride in pyridine or another non-nucleophilic amine base gives pyrazole triflates of formula (XXXV). Compounds such as (XXXV) can be treated with an alkylating agent of formula (XIV). For example, treatment with an alkyl or benzyl chloride, bromide, iodide, mesylate or tosylate (wherein X is Cl, Br, I, OMs, OTs or the like) in DMF, DMA, THF or ethanol in the presence of a base like NaHCO₃, Na₂CO₃, NaH, K₂CO₃, C_{S2}CO₃, or potassium tert-butoxide will afford compounds of formula (XVI). Preferably, alkylation is affected using alkylating agents such as benzyl bromide in the presence of a suitable base such as potassium tert-butoxide. Pyrazoles of formula (XVI) can be carried forward as described in Scheme 2 to provide compounds of formula (XIX) and (II).

The compounds of the present invention are serotonin receptor modulators, and as such, the compounds are useful in the treatment of serotonin-mediated disease states. Particularly, the compounds may be used in the treatment or prevention of CNS disorders, such as sleep disorders, depression/anxiety, generalized anxiety disorder, schizophrenia, bipolar disorders, psychotic disorders, obsessive-compulsive disorder, mood disorders, post-traumatic stress and other stress-related disorders, migraine, pain, eating disorders, obesity, sexual dysfunction, metabolic disturbances, hormonal imbalance, alcohol abuse, addictive disorders, nausea, inflammation, centrally mediated hypertension, sleep/wake disturbances, jetlag, and circadian rhythm abnormalities. The compounds may also be used in the treatment and prevention of hypotension, peripheral vascular disorders, cardiovascular shock, renal disorders, gastric motility, diarrhea, spastic colon, irritable bowel disorders, ischemias, septic shock, urinary incontinence, and other disorders related to the gastrointestinal and vascular systems. In addition, compounds of the present invention may be used in the treatment or prevention of a range of ocular disorders including glaucoma, optic neuritis, diabetic retinopathy, retinal edema, and age-related macular degeneration.

The compounds of the present invention are 5-HT₇ modulators and many are 5-HT₇ antagonists. As such, the compounds are useful in the treatment of 5-HT₇-mediated disease states. Where the compounds possess substantial 5-HT₇ antagonist activity, they may be particularly useful in the treatment or prevention of depression/anxiety, sleep/wake disturbances, jetlag, migraine, urinary incontinence, gastric motility, and irritable bowel disorders.

Many of the compounds of the present invention are 5-HT₂ modulators and many are 5-HT₂ antagonists. As such, the compounds are useful in the treatment of 5-HT₂-mediated diseases and conditions. Where the compounds possess substantial 5-HT₂ antagonist activity, they may be particularly useful in the treatment or prevention of depression/anxiety, generalized anxiety disorder, schizophrenia, bipolar disorders, psychotic disorders, obsessive-compulsive disorder, mood disorders, post-traumatic stress disorders, sleep disturbances, sexual dysfunction, eating disorders, migraine, addictive disorders, and peripheral vascular disorders.

It is anticipated that the compounds of the invention can be administered by oral or parenteral routes, including intravenous, intramuscular, intraperitoneal, subcutaneous, rectal and topical administration, and inhalation. For oral administration, the compounds of the invention will generally be provided in the form of tablets or capsules or as an aqueous solution or suspension. Tablets for oral use may include the active ingredient mixed with pharmaceutically acceptable excipients such as inert diluents, disintegrating agents, binding agents, lubricating agents, sweetening agents, flavoring agents, coloring agents and preservatives. Suitable inert diluents include sodium and calcium carbonate, sodium and calcium phosphate and lactose. Cornstarch and alginic acid are suitable disintegrating agents. Binding agents may include starch and gelatin. The lubricating agent, if present, will generally be magnesium stearate, stearic acid or talc. If desired, the tablets may be coated with a material such as glyceryl monostearate or glyceryl distearate, to delay absorption in the gastrointestinal tract. Capsules for oral use include hard gelatin capsules in which the active ingredient is mixed with a solid diluent and soft gelatin capsules wherein the active ingredient is mixed with water or an oil such as peanut oil, liquid paraffin or olive oil. For intramuscular, intraperitoneal, subcutaneous and intravenous use, the compounds of the invention will generally be provided in sterile aqueous solutions or suspensions, buffered to an appropriate pH and isotonicity. Suitable aqueous vehicles include Ringer's solution and isotonic sodium chloride. Aqueous suspensions according to the invention may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

Effective doses of the compounds of the present invention may be ascertained by conventional methods. The specific dosage level required for any particular patient will depend on a number of factors, including severity of the condition being treated, the route of administration and the weight of the patient. In general, however, it is anticipated that the daily dose (whether administered as a single dose or as divided doses) will be in the range 0.01 to 1000 mg per day, more usually from 1 to 500 mg per day, and most usually from 10 to 200 mg per day. Expressed as dosage per unit body weight, a typical dose will be expected to be between 0.0001 mg/kg and 15 mg/kg, especially between 0.01 mg/kg and 7 mg/kg, and most especially between 0.15 mg/kg and 2.5 mg/kg.

### EXAMPLES

In order to illustrate the invention, the following examples are included. These examples do not limit the invention. They are only meant to suggest a method of practicing the invention. Those skilled in the art may find other methods of practicing the invention, which are obvious to them. However, those methods are deemed to be within the scope of this invention.

### Protocol for Preparative Reversed-Phase HPLC

Gilson®
Column: YMC-Pack ODS-A, 5 µm, 75x30 mm
Flow rate: 25 mL/min
Detection: λ = 220 & 254 nm

| Gradient (acetonitrile/water, 0.05% trifluoroacetic acid) | | |
|---|---|---|
| 1) | 0.0 min | 15% acetonitrile/85% water |
| 2) | 20.0 min | 99% acetonitrile/1% water |

### Protocol for HPLC (Reversed-Phase)

### Method A:

Hewlett Packard Series 1100
Column: Agilent ZORBAX® Bonus RP, 5 µm, 4.6x250 mm
Flow rate: 1 mL/min
Detection: λ = 220 & 254 nm

| Gradient (acetonitrile/water, 0.05% trifluoroacetic acid) | | |
|---|---|---|
| 1) | 0.0 min | 1% acetonitrile/99% water |
| 2) | 20.0 min | 99% acetonitrile/1% water |

### Method B:

Hewlett Packard HPLC
Column: Agilent ZORBAX® Eclipse XDB-C8, 5 µm, 4.6x150 mm
Flow rate: 1 mL/min
Detection: λ = 220 & 254 nm

| Gradient (acetonitrile/water, 0.05% trifluoroacetic acid) | | |
|---|---|---|
| 1) | 0.0 min | 1 % acetonitrile/99% water |
| 2) | 8.0 min | 99% acetonitrile/1 % water |
| 3) | 12.0 min | 99% acetonitrile/1 % water |

### Protocol for Preparative SFC

Thar Technologies®
Column: Chiracel AD, 10 µm, 250x20 mm
Flow rate: 37gm/min
Detection: λ = 220 & 254 nm
Mobile phase: Isocratic 30% IPA/ 70% CO₂
Pressure: 150 Bar
Temperature: 35 °C

### Protocol for Analytical SFC

Jasco®
Column: Chiracel AD, 10 µm, 250x4.6 mm
Flow rate: 1 gm/min
Detection: λ = 220 & 254 nm
Mobile phase: Isocratic 30% IPA/ 70% CO₂
Pressure: 150 Bar
Temperature: 35°C

Mass spectra were obtained on an Agilent series 1100 MSD using electrospray ionization (ESI) in either positive or negative modes as indicated.

Thin-layer chromatography was performed using Merck silica gel 60 F₂₅₄ 2.5 cm x 7.5 cm 250 µm or 5.0 cm x 10.0 cm 250 µm pre-coated silica gel plates. Preparative thin-layer chromatography was performed using EM Science silica gel 60 F₂₅₄ 20 cm x 20 cm 0.5 mm pre-coated plates with a 20 cm x 4 cm concentrating zone.

NMR spectra were obtained on either a Bruker model DPX400 (400 MHz), DPX500 (500 MHz), or DPX600 (600 MHz) spectrometer. The format of the ¹H NMR data below is: chemical shift in ppm down field of the tetramethylsilane reference (multiplicity, coupling constant *J* in Hz, integration).

### Example 1

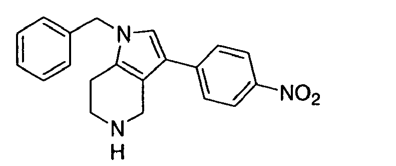

1-Benzyl-3-(4-nitro-phenyl)-4,5,6,7-tetrahydro-1*H*-pyrrolo[3,2-c]pyridine. Step A. 1-Benzyl-3-(4-nitro-phenyl)-1,4,6,7-tetrahydro-pyrrolo[3,2-*c*]pyridine-5-carboxylic acid *tert*-butyl ester. To a stirred solution of 4-oxo-piperidine-1-carboxylic acid *tert*-butyl ester (0.69 g) in toluene (5 mL) was added 378 µL of benzylamine. The mixture was stirred for 10 min and then 0.70 g of silica gel (SiO₂) was added. After stirring at RT for 8 h, 0.77 g of 1-nitro-4-(2-nitro-vinyl)-benzene in toluene (5 mL) was added and the mixture was stirred for 14 h at RT. The mixture was then filtered through diatomaceous earth and the filtrate was concentrated *in vacuo.* Chromatography on SiO₂ (8 to 20% EtOAc/hexanes) afforded 0.48 g of the desired compound. MS (ESI): exact mass calculated for C₂₅H₂₇N₃O₄, 433.20; found, *m*/*z*434.2 [M+H]⁺, 456.2 [M+Na]⁺.

Step B. To a stirred solution of 0.20 g of the above compound in a 10:1 mixture of CH₂Cl₂/MeOH (6 mL) was added 1.9 mL of 1.0 M HCl in Et₂O. After stirring for 12 h at RT, a white solid had formed, which was collected by filtration to afford 0.11 g of the title compound. MS (ESI): exact mass calculated for C₂₀H₁₉N₃O₂, 333.15; found, *m*/*z* 334.2 [M+H]⁺. ¹H NMR (500 MHz, CD₃OD): 8.26-8.21 (m, 2H), 7.59-7.55 (m, 2H), 7.42 (s, 1H), 7.36 (t, *J*= 7.4 Hz, 2H), 7.30 (t, *J* = 7.4 Hz, 1 Hz), 7.20 (d, *J =* 7.4 Hz, 2H), 5.19 (s, 2H), 4.44 (s, 2H), 3.53 (t, *J* = 6.3 Hz, 2H), 2.89 (t, *J =* 6.3 Hz, 2H).

Examples 2-25 were prepared according to the procedure described in Example 1, with alterations as noted.

### Example 2

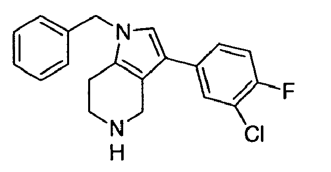

1-Benzyl-3-(3-chloro-4-fluoro-phenyl)-4,5,6,7-tetrahydro-1*H*-pyrrolo[3,2-c]pyridine.
The title compound (0.18 g) was prepared from 0.54 g of 4-oxo-piperidine-1-carboxylic acid *tert*-butyl ester, 293 µL of benzylamine, and 0.62 g of 2-chloro-1-fluoro-4-(2-nitro-vinyl)-benzene. MS (ESI): exact mass calculated for C₂₀H₁₈CIFN₂, 340.11; found, *m*/*z*341.1 [M+H]⁺, 343.2 [M+H]⁺. ¹H NMR (500 MHz, CD₃OD): 7.45-7.43 (m, 1 H), 7.36-7.16 (m, 8H), 5.15 (s, 2H), 4.35 (s, 2H), 3.50 (t, J = 6.3 Hz, 2H), 2.85 (t, J = 6.3 Hz, 2H).

### Example 3

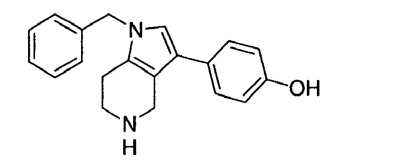

4-(1-Benzyl-4,5,6,7-tetrahydro-1*H*-pyrrolo[3,2-c]pyridin-3-yl)-phenol.
The title compound (0.09 g) was prepared from 1.22 g of 4-oxo-piperidine-1-carboxylic acid *tert*-butyl ester, 856 µL of benzylamine, and 1.29 g of 4-(2-nitrovinyl)-phenol, which was added in EtOH (12 mL). MS (ESI): exact mass calculated for C₂₀H₂₀N₂O, 304.16; found, *m*/*z*305.2 [M+H]⁺. ¹H NMR (500 MHz, CD₃OD): 7.35-7.32 (m, 2H), 7.29-7.25 (m, 2H), 7.17-7.14 (m, 4H), 6.98 (s, 1 H), 6.80-6.77 (m, 2H), 5.12 (s, 2H), 4.31 (s, 2H), 3.49 (t, J = 6.3 Hz, 2H), 2.85 (t, J = 6.3 Hz, 2H).

### Example 4

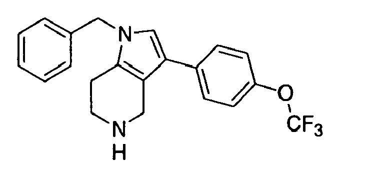

1-Benzyl-3-(4-trifluoromethoxy-phenyl)-4,5,6,7-tetrahydro-1*H*-pyrrolo[3,2-c]pyridine.
The title compound (0.28 g) was prepared from 0.50 g of 4-oxo-piperidine-1-carboxylic acid *tert*-butyl ester, 274 µL of benzylamine, and 0.59 g of 1-trifluoromethoxy-4-(2-nitro-vinyl)-benzene using CH₂Cl₂ as the solvent. MS (ESI): exact mass calculated for C₂₁H₁₉F₃N₂O, 372.14; found, *m*/*z* 373.2 [M+H]⁺. ¹H NMR (400 MHz, CD₃OD): 7.44-7.41 (m, 2H), 7.37-7.26 (m, 5H), 7.19- 7.17 (m, 3H), 5.15 (s, 2H), 4.37 (s, 2H), 3.51 (t, J = 6.3 Hz, 2H), 2.87 (t, J = 6.3 Hz, 2H).

### Example 5

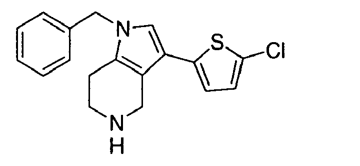

1-Benzyl-3-(5-chloro-thiophen-2-yl)-4,5,6,7-tetrahydro-1*H*-pyrrolo[3,2-c]pyridine.
The title compound (82.3 mg) was prepared from 0.56 g of 4-oxo-piperidine-1-carboxylic acid *tert*-butyl ester, 300 µL of benzylamine, and 0.53 g of 2-chloro-5-(2-nitro-vinyl)-thiophene. MS (ESI): exact mass calculated for C₁₈H₁₇ClN₂S, 328.08; found, *m*/*z*329.1 [M+H]⁺. ¹H NMR (500 MHz, CD₃OD): 7.36-7.33 (m, 2H), 7.30-7.27 (m, 1 H), 7.17-7.15 (m, 2H), 7.12 (s, 1 H), 6.89 (d, *J* = 3.8 Hz, 1 H), 6.73 (d, J=3.8 Hz, 1 H), 5.12 (s, 2H), 4.31 (s, 2H), 3.48 (t, J = 6.3 Hz, 2H), 2.84 (t, J = 6.3 Hz, 2H).

### Example 6

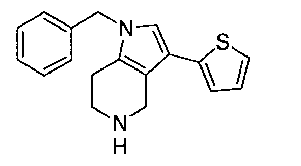

1-Benzyl-3-thiophen-2-yl-4,5,6,7-tetrahydro-1 *H*-pyrrolo[3,2-*c*]pyridine. The title compound (136.8 mg) was prepared from 0.53 g of 4-oxo-piperidine-1-carboxylic acid *tert*-butyl ester, 300 µL of benzylamine, and 0.41 g of 2-(2-nitrovinyl)-thiophene. MS (ESI): exact mass calculated for C₁₈Hₗ₈N₂S, 294.12; found, *m*/*z*295.2 [M+H]⁺. ¹H NMR (500 MHz, CD₃OD): 7.36-7.32 (m, 2H), 7.30-7.26 (m, 1 H), 7.22 (dd, J = 5.2, 1.1 Hz, 1 H), 7.18-7.15 (m, 2H), 7.12 (s, 1 H), 7.02 (dd, J = 5.2, 3.6 Hz, 1 H), 6.94 (dd, J = 3.6, 1.1 Hz, 1 H), 5.13 (s, 2H), 4.34 (s, 2H), 3.49 (t, J = 6.3 Hz, 2H), 2.85 (t, J = 6.3 Hz, 2H).

### Example 7

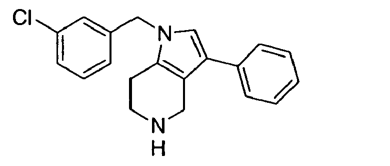

1-(3-Chloro-benzyl)-3-phenyl-4,5,6,7-tetrahydro-1 *H*-pyrrolo[3,2-c]pyridine. The title compound (159.0 mg) was prepared from 0.55 g of 4-oxo-piperidine-1-carboxylic acid *tert*-butyl ester, 334 µL of 3-chlorobenzylamine, and 0.40 g of (2-nitro-vinyl)-benzene and without SiO₂. MS (ESI): exact mass calculated for C₂₀H₁₉ClN₂, 322.12; found, *m*/*z*323.2 [M+H]⁺. ¹H NMR (500 MHz, CD₃OD): 7.38-7.32 (m, 5H), 7.31-7.28 (m, 1 H), 7.23-7.19 (m, 1 H), 7.17-7.16 (m, 1 H), 7.13 (s, 1 H), 7.12-7.10 (m, 1 H), 5.16 (s, 2H), 4.37 (s, 2H), 3.52 (t, J = 6.3 Hz, 2H), 2.86 (t, J = 6.3 Hz, 2H).

### Example 8

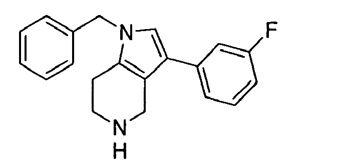

1-Benzyl-3-(3-fluoro-phenyl)-4,5,6,7-tetrahydro-1 *H*-pyrrolo[3,2-*c*]pyridine. The title compound (282.6 mg) was prepared from 0.61 g of 4-oxo-piperidine-1-carboxylic acid *tert*-butyl ester, 330 µL of benzylamine, and 0.50 g of (2-nitrovinyl)-3-fluorobenzene, using EtOH as the solvent and without SiO₂. MS (ESI): exact mass calculated for C₂₀H₁₉FN₂, 306.15; found, *m*/*z*307.2 [M+H]⁺. ¹H NMR (500 MHz, CD₃OD): 7.38-7.32 (m, 3H), 7.30-7.26 (m, 1 H), 7.20-7.14 (m, 4H), 7.10-7.06 (m, 1 H), 6.95-6.90 (m, 1 H), 5.15 (s, 2H), 4.36 (s, 2H), 3.50 (t, J = 6.3 Hz, 2H), 2.86 (t, J = 6.3 Hz, 2H).

### Example 9

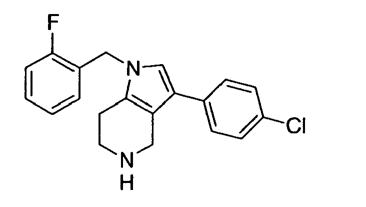

3-(4-Chloro-phenyl)-1-(2-fluoro-benzyl)-4,5,6,7-tetrahydro-1*H*-pyrrolo[3,2-c]pyridine.
The title compound (129.2 mg) was prepared from 0.49 g of 4-oxo-piperidine-1-carboxylic acid *tert*-butyl ester, 286 µL of 2-fluorobenzylamine, and 0.46 g of (2-nitro-vinyl)-4-chlorobenzene, replacing SiO₂ with crushed 4Å molecular sieves. MS (ESI): exact mass calculated for C₂₀H₁₈ClFN₂, 340.11; found, *m*/*z* 341.1 [M+H] ⁺. ¹H NMR (500 MHz, CD₃OD): 7.38-7.30 (m, 5H), 7.18-7.12 (m, 3H), 7.10-7.06 (m, 1 H), 5.20 (s, 2H), 4.35-4.34 (m, 2H), 3.54 (t, J = 6.3 Hz, 2H), 2.94 (t, J = 6.3 Hz, 2H).

### Example 10

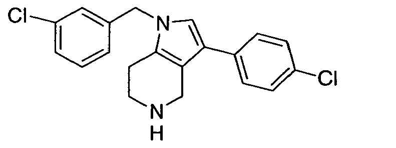

1-(3-Chloro-benzyl)-3-(4-chloro-phenyl)-4,5,6,7-tetrahydro-1*H*-pyrrolo[3,2-*c*]pyridine.
The title compound (212.8 mg) was prepared from 0.55 g of 4-oxo-piperidine-1-carboxylic acid *tert*-butyl ester, 340 µL of 3-chlorobenzylamine, and 0.51 g of (2-nitro-vinyl)-4-chlorobenzene, replacing SiO₂ with crushed 4Å molecular sieves. MS (ESI): exact mass calculated for C₂₀H₁₈Cl₂N₂, 356.08; found, *m*/*z* 357.1 [M+H]⁺. ¹H NMR (500 MHz, CD₃OD): 7.38-7.28 (m, 6H), 7.18-7.15 (m, 2H), 7.12-7.09 (m, 1 H), 5.16 (s, 2H), 4.36 (s, 2H), 3.52 (t, J = 6.3 Hz, 2H), 2.86 (t, J = 6.3 Hz, 2H).

### Example 11

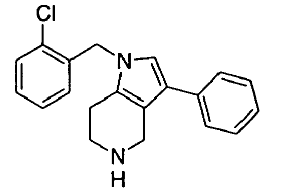

1-(2-Chloro-benzyl)-3-phenyl-4,5,6,7-tetrahydro-1*H*-pyrrolo[3,2-*c*]pyridine.
The title compound (113.8 mg) was prepared from 0.55 g of 4-oxo-piperidine-1-carboxylic acid *tert*-butyl ester, 334 µL of 2-chlorobenzylamine, 0.40 g of (2-nitro-vinyl)-benzene, and without SiO₂. MS (ESI): exact mass calculated for C₂₀H₁₉ClN₂, 322.12; found, *m*/*z*323.2 [M+H]⁺. ¹H NMR (500 MHz, CD₃OD): 7.46 (m, 1 H), 7.37-7.26 (m, 6H), 7.22-7.19 (m, 1 H), 7.07 (s, 1 H), 6.85-6.82 (m, 1 H), 5.25 (s, 2H), 4.39 (s, 2H), 3.54 (t, J = 6.3 Hz, 2H), 2.88 (t, J = 6.3 Hz, 2H).

### Example 12

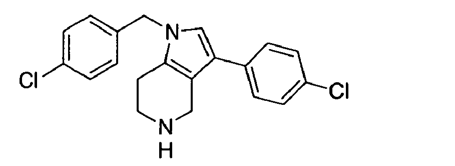

1-(4-Chloro-benzyl)-3-(4-chloro-phenyl)-4,5,6,7-tetrahydro-1 *H*-pyrrolo[3,2-c]pyridine.
The title compound (260.2 mg) was prepared from 0.55 g of 4-oxo-piperidine-1-carboxylic acid *tert-*butyl ester, 340 µL of 4-chlorobenzylamine, and 0.51 g of (2-nitro-vinyl)-4-chlorobenzene. MS (ESI): exact mass calculated for C₂₀H₁₈Cl₂N₂, 356.08; found, *m*/*z* 357.1 [M+H]⁺. ¹H NMR (500 MHz, CD₃OD): 7.37-7.31 (m, 6H), 7.17-7.13 (m, 3H), 5.15 (s, 2H), 4.35 (s, 2H), 3.51 (t, J = 6.3 Hz, 2H), 2.85 (t, J = 6.3 Hz, 2H).

### Example 13

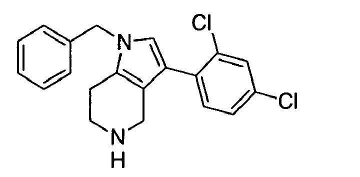

1-Benzyl-3-(2,4-dichloro-phenyl)-4,5,6,7-tetrahydro-1*H*-pyrrolo[3,2-*c*]pyridine.
The title compound (454.6 mg) was prepared from 0.52 g of 4-oxo-piperidine-1-carboxylic acid *tert*-butyl ester, 280 µL of benzylamine, and 0.57 g of 2,4-dichloro-1-(2-nitro-vinyl)-benzene, using a 5:1 EtOH/toluene mixture as the solvent. MS (ESI): exact mass calculated for C₂₀H₁₈Cl₂N₂, 356.08; found, *m*/*z* 357.1 [M+H]⁺. ¹H NMR (500 MHz, CD₃OD): 7.53 (d, *J*=2.2 Hz, 1H), 7.36-7.32 (m, 3H), 7.30-7.28 (m, 2H), 7.20-7.17 (m, 2H), 7.04 (s, 1 H), 5.16 (s, 2H), 4.13 (s, 2H), 3.50 (t, J = 6.3 Hz, 2H), 2.88 (t, J = 6.3 Hz, 2H).

### Example 14

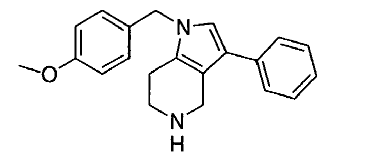

1-(4-Methoxy-benzyl)-3-phenyl-4,5,6,7-tetrahydro-1 *H*-pyrrolo[3,2-*c*]pyridine.
The title compound (0.19 g) was prepared from 1.51 g of 4-oxo-piperidine-1-carboxylic acid *tert*-butyl ester, 1.0 mL of 4-methoxybenzylamine, and 1.13 g of (2-nitro-vinyl)-benzene, using EtOH as the solvent and omitting SiO₂. MS (ESI): exact mass calculated for C₂₁H₂₂N₂O, 318.17; found, *m*/*z*319.2 [M+H]⁺. ¹H NMR (500 MHz, CD₃OD): 7.39-7.30 (m, 4H), 7.20-7.15 (m, 1 H), 7.14-7.11 (m, 2H), 7.08 (s, 1 H), 6.90-6.87 (m, 2H), 5.05 (s, 2H), 4.34 (s, 2H), 3.50 (t, *J*= 6.3 Hz, 2H), 2.88 (t, J = 6.3 Hz, 2H).

### Example 15

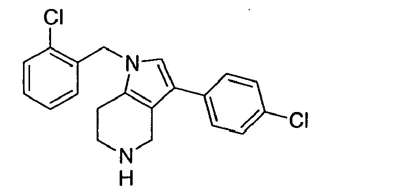

1-(2-Chloro-benzyl)-3-(4-chloro-phenyl)-4,5,6,7-tetrahydro-1*H*-pyrrolo[3,2-c]pyridine.
The title compound (149.9 mg) was prepared from 0.50 g of 4-oxo-piperidine-1-carboxylic acid *tert*-butyl ester, 304 µL of 2-chlorobenzylamine, and 0.46 g of (2-nitro-vinyl)-4-chlorobenzene, replacing SiO₂ with crushed 4Å molecular sieves. MS (ESI): exact mass calculated for C₂₀H₁₈Cl₂N₂, 356.08; found, *m*/*z* 357.1 [M+H]⁺. ¹H NMR (500 MHz, CD₃OD): 7.58-7.56 (m, 1 H), 7.47-7.45 (m, 1H), 7.37-7.26 (m, 5H), 7.10 (s, 1H), 6.85-6.82 (m, 1H), 5.25 (s, 2H), 4.38 (s, 2H), 3.53 (t, J = 6.3 Hz, 2H), 2.88 (t, J = 6.3 Hz, 2H).

### Example 16

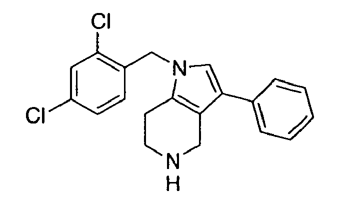

1-(2,4-Dichloro-benzyl)-3-phenyl-4,5,6,7-tetrahydro-1 *H*-pyrrolo[3,2-*c*]pyridine. The title compound (0.43 g) was prepared from 0.55 g of 4-oxo-piperidine-1-carboxylic acid *tert*-butyl ester, 370 µL of 2,4-dichlorobenzylamine, and 0.41 g of (2-nitro-vinyl)-benzene, using EtOH as the solvent. MS (ESI): exact mass calculated for C₂₀H₁₈Cl₂N₂, 356.08; found, m/z 357.1 [M+H]⁺. ¹H NMR (500 MHz, CD₃OD): 7.55-7.50 (m, 2H), 7.37-7.29 (m, 4H), 7.22-7.18 (m, 1 H), 7.07 (s, 1 H), 6.77 (d, J = 8.5 Hz, 1 H), 5.23 (s, 2H), 4.38 (s, 2H), 3.54 (t, J = 6.3 Hz, 2H), 2.86 (t, J = 6.3 Hz, 2H).

### Example 17

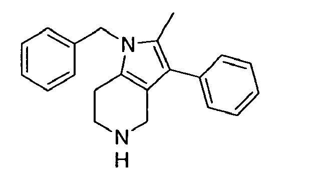

1-Benzyl-2-methyl-3-phenyl-4,5,6,7-tetrahydro-1 *H*-pyrrolo[3,2-*c*]pyridine. The title compound (89.4 mg) was prepared from 0.51 g of 4-oxo-piperidine-1-carboxylic acid *tert*-butyl ester, 272 µL of benzylamine, and 0.41 g of (2-nitro-propenyl)-benzene. MS (ESI): exact mass calculated for C₂₁H₂₂N₂, 302.18; found, m/z303.2 [M+H]⁺. ¹H NMR (400 MHz, CD₃OD): 7.41-7.36 (m, 2H), 7.35-7.30 (m, 2H), 7.28-7.21 (m, 4H), 7.05-7.01 (m, 2H), 5.16 (s, 2H), 4.18 (s, 2H), 3.52 (t, J = 6.3 Hz, 2H), 2.89 (t, J = 6.3 Hz, 2H).

### Example 18

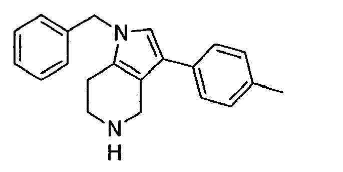

1-Benzyl-3-*p*-tolyl-4,5,6,7-tetrahydro-1*H*-pyrrolo[3,2-*c*]pyridine.
The title compound (89.7 mg) was prepared from 0.51 g of 4-oxo-piperidine-1-carboxylic acid *tert*-butyl ester, 272 µL of benzylamine, and 0.41 g of 1-methyl-4-(2-nitro-vinyl)-benzene. MS (ESI): exact mass calculated for C₂₁H₂₂N₂, 302.18; found, *m*/*z* 303.2 [M+H]⁺. ¹H NMR (500 MHz, CD₃OD): 7.35-7.31 (m, 2H), 7.29-7.25 (m, 1 H), 7.23-7.20 (m, 2H), 7.18-7.14 (m, 4H), 7.06 (s, 1 H), 5.13 (s, 2H), 4.33 (s, 2H), 3.49 (t, J = 6.3 Hz, 2H), 2.86 (t, J = 6.3 Hz, 2H).

### Example 19

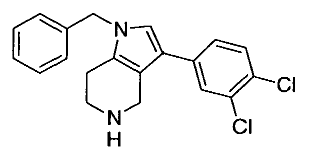

1-Benzyl-3-(3,4-dichloro-phenyl)-4,5,6,7-tetrahydro-1*H*-pyrrolo[3,2-c]pyridine.
The title compound (228.2 mg) was prepared from 0.49 g of 4-oxo-piperidine-1-carboxylic acid *tert*-butyl ester, 268 µL of benzylamine, and 0.55 g of 1,2-dichloro-4-(2-nitro-vinyl)-benzene. MS (ESI): exact mass calculated for C₂₀H₁₈Cl₂N₂, 356.08; found, *m*/*z*357.1 [M+H]⁺. ¹H NMR (400 MHz, CD₃OD): 7.51-7.47 (m, 2H), 7.36-7.32 (m, 2H), 7.32-7.25 (m, 2H), 7.22 (s, 1 H), 7.19-7.15 (m, 2H), 5.15 (s, 2H), 4.35 (s, 2H), 3.50 (t, J = 6.3 Hz, 2H), 2.85 (t, J = 6.3 Hz, 2H).

### Example 20

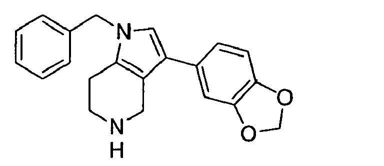

3-Benzo[1,3]dioxol-5-yl-1-benzyl-4,5,6,7-tetrahydro-1*H*-pyrrolo[3,2-c]pyridine.
The title compound (306.0 mg) was prepared from 0.49 g of 4-oxo-piperidine-1-carboxylic acid *tert*-butyl ester, 268 µL of benzylamine, and 0.48 g of 5-(2-nitrovinyl)-benzo[1,3]dioxole. MS (ESI): exact mass calculated for C₂₁H₂₀N₂O₂, 332.15; found, *m*/*z* 333.2 [M+H]⁺. ¹H NMR (400 MHz, CD₃OD): 7.36-7.30 (m, 2H), 7.29-7.24 (m, 1 H), 7.18-7.14 (m, 2H), 7.01 (s, 1 H), 6.85-6.75 (m, 3H), 5.93 (s, 2H), 5.12 (s, 2H), 4.31 (s, 2H), 3.49 (t, J = 6.3 Hz, 2H), 2.85 (t, J = 6.3 Hz, 2H).

### Example 21

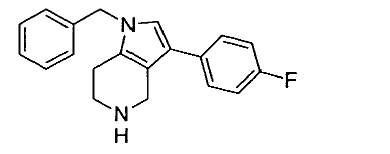

1-Benzyl-3-(4-fluoro-phenyl)-4,5,6,7-tetrahydro-1*H*-pyrrolo[3,2-*c*]pyridine.
The title compound (706.2 mg) was prepared from 1.31 g of 4-oxo-piperidine-1-carboxylic acid *tert*-butyl ester, 700 µL of benzylamine, and 1.10 g of 1-fluoro-4-(2-nitro-vinyl)-benzene. MS (ESI): exact mass calculated for C₂₀H₁₉FN₂, 306.15; found, *m*/*z* 307.2 [M+H]⁺. ¹H NMR (500 MHz, CD₃OD): 7.36-7.25 (m, 5H), 7.18-7.15 (m, 2H), 7.11-7.05 (m, 3H), 5.13 (s, 2H), 4.33 (s, 2H), 3.50 (t, J = 6.3 Hz, 2H), 2.86 (t, J = 6.3 Hz, 2H).

### Example 22

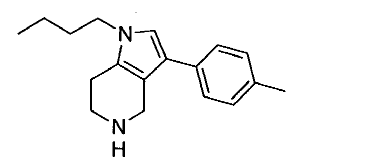

1-Butyl-3-p-tolyl-4,5,6,7-tetrahydro-1 *H*-pyrrolo[3,2-c]pyridine.
The title compound (292.8 mg) was prepared from 0.56 g of 4-oxo-piperidine-1-carboxylic acid *tert*-butyl ester, 260 µL of butylamine, and 0.45 g of 1-methyl-4-(2-nitro-vinyl)-benzene. MS (ESI): exact mass calculated for C₁₈H₂₄N₂, 268.19; found, *m*/*z* 269.2 [M+H]⁺. ¹H NMR (500 MHz, CD₃OD): 7.20-7.14 (m, 4H), 6.93 (s, 1 H), 4.32 (s, 2H), 3.88 (t, J = 7.1 Hz, 2H), 3.56 (t, J = 6.0 Hz, 2H), 3.00 (t, *J* = 6.0 Hz, 2H), 2.32 (s, 3H), 1.77-1.70 (m, 2H), 1.41-1.33 (m, 2H), 0.97 (t, J = 7.4 Hz, 3H).

### Example 23

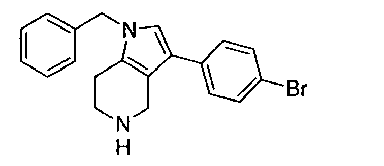

1-Benzyl-3-(4-bromo-phenyl)-4,5,6,7-tetrahydro-1 *H*-pyrrolo[3,2-*c*]pyridine.
The title compound (0.38 g) was prepared from 0.66 g of 4-oxo-piperidine-1-carboxylic acid *tert*-butyl ester, 300 µL of benzylamine, and 0.63 g of 1-bromo-4-(2-nitro-vinyl)-benzene. MS (ESI): exact mass calculated for C₂₀H₁₉BrN₂, 366.07; found, *m*/*z* 367.1 [M+H]⁺. ¹H NMR (500 MHz, CD₃OD): 7.51-7.48 (m, 2H), 7.36-7.32 (m, 2H), 7.30-7.25 (m, 3H), 7.19-7.16 (m, 2H), 5.14 (s, 2H), 4.35 (s, 2H), 3.50 (t, J = 6.3 Hz, 2H), 2.87 (t, J = 6.3 Hz, 2H).

### Example 24

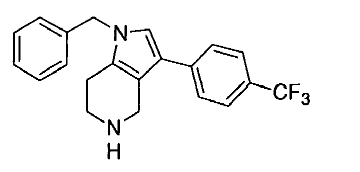

1-Benzyl-3-(4-trifluoromethyl-phenyl)-4,5,6,7-tetrahydro-1*H*-pyrrolo[3,2-c]pyridine.
The title compound (0.23 g) was prepared from 0.50 g of 4-oxo-piperidine-1-carboxylic acid *tert*-butyl ester, 274 µL of benzylamine, and 0.55 g of 1-trifluoromethyl-4-(2-nitro-vinyl)-benzene, using acetonitrile as the solvent. MS (ESI): exact mass calculated for C₂₁H₁₉F₃N₂, 356.16; m/zfound, 357.2 [M+H]⁺. ¹H NMR (500 MHz, CD₃OD): 7.65-7.63 (m, 2H), 7.54-7.52 (m, 2H), 7.36-7.27 (m, 4H), 7.20-7.18 (m, 2H), 5.17 (s, 2H), 4.40 (s, 2H), 3.52 (t, J= 6.3 Hz, 2H), 2.88 (t, J = 6.3 Hz, 2H).

### Example 25

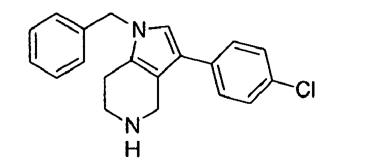

1-Benzyl-3-(4-chloro-phenyl)-4,5,6,7-tetrahydro-1*H*-pyrrolo[3,2-*c*]pyridine.
The title compound (1.19 g) was prepared from 1.55 g of 4-oxo-piperidine-1-carboxylic acid *tert-*butyl ester, 850 µL of benzylamine, and 1.43 g of 1-chloro-4-(2-nitro-vinyl)-benzene, using a 1:1 mixture of EtOH/toluene as the solvent. MS (ESI): exact mass calculated for C₂₀H₂₀Cl₂N₂, 322.12; m/z found, 323.2 [M+H]⁺, 325.2 [M+H]⁺. ¹H NMR (400 MHz, CD₃OD): 7.37-7.26 (m, 7H), 7.18-7.16 (m, 3H), 5.15 (s, 2H), 4.35 (s, 2H), 3.50 (t, J = 6.3 Hz, 2H), 2.87 (t, J = 6.3 Hz,2H).

### Example 26

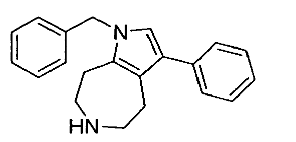

1-Benzyl-3-phenyl-1,4,5,6,7,8-hexahydro-pyrrolo[2,3-*d*]azepine.
Step A. 1-Benzyl-3-phenyl-4,5,7,8-tetrahydro-1*H*-pyrrolo[2,3-*d*]azepine-6-carboxylic acid *tert*-butyl ester. A solution of the compound (0.53 g) from Example 59, Step B, and 272 µL of benzylamine in benzene (10 mL) was heated at reflux for 24 h using a Dean-Stark apparatus. The solvent was removed, the crude material was dissolved in toluene (10 mL), and 0.38 g of (2-nitro-vinyl)-benzene was added. The mixture was stirred for 24 h at RT and concentrated *in vacuo.* Chromatography on SiO₂ (1 to 20% EtOAc/hexanes) afforded 108.0 mg of the desired compound. MS (ESI): exact mass calculated for C₂₆H₃₀N₂O₂, 402.53; found, *m*/*z* 403.2 [M+H]⁺.
Step B. To a stirred solution of the compound from Step A (108.0 mg) in CH₂Cl₂ (5 mL) was added TFA (1 mL). The mixture was stirred at RT for 12 h and then concentrated *in vacuo.* The residue was partitioned between CH₂Cl₂ (10 mL) and 1 M NaOH (10 mL). The layers were separated and the aqueous layer was extracted with CH₂Cl₂ (2x10 mL). The combined organic layers were concentrated. Chromatography on SiO₂ (5% 2 M NH₃ in MeOH/CH₂Cl₂) gave 66.5 mg of the title compound. MS (ESI): exact mass calculated for C₂₁H₂₂N₂, 302.41; found, *m*/*z* 303.2 [M+H]⁺. ¹H NMR (500 MHz, CDCl₃): 7.42-7.22 (m, 8H), 7.08 (m, 2H), 6.67 (s, 1 H), 5.08 (s, 2H), 3.06-2.91 (m, 4H), 2.90-2.82 (m, 2H), 2.77-2.68 (m, 2H), 2.25 (br s, 1 H).

### Example 27

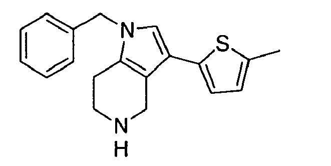

1-Benzyl-3-(5-methyl-thiophen-2-yl)-4,5,6,7-tetrahydro-1*H*-pyrrolo[3,2-c]pyridine.
Step A. 1-Benzyl-3-(5-methyl-thiophen-2-yl)-1,4,6,7-tetrahydro-pyrrolo[3,2-*c*]pyridine-5-carboxylic acid *tert*-butyl ester. A mixture of 4-oxo-piperidine-1-carboxylic acid *tert*-butyl ester (0.54 g) and 300 µL of benzylamine in toluene (10 mL) was heated at reflux for 6 h using a Dean-Stark apparatus. The solution was cooled to RT and 0.47 g of 2-methyl-5-(2-nitro-vinyl)-thiophene was added. The mixture was stirred for 16 h at RT and then was concentrated *in vacuo.* The residue was chromatographed on SiO₂ (1 to 30% EtOAc/hexanes) to afford 281.9 mg of the desired compound. TLC (SiO₂, 33% EtOAc/hexanes): R_{f} = 0.54.
Step B. To a stirred solution of the compound from Step A (281.9 mg) in EtOH (10 mL) was added HCl (1 M in Et₂O, 5 mL). The resulting mixture was stirred at RT for 24 h and concentrated *in vacuo.* The residue was then partitioned between CH₂Cl₂ (10 mL) and 1 M NaOH (10 mL). The layers were separated and the aqueous layer was extracted with CH₂Cl₂ (2 x 10 mL). The combined organic layers were concentrated. Chromatography on SiO₂ (CH₂Cl₂ to 5% 2 M NH₃ in MeOH/CH₂Cl₂) gave 59.0 mg of the title compound. MS (ESI): exact mass calculated for C₁₉H₂₀N₂S, 308.13; found, *m*/*z* 309.2 [M+H]⁺. ¹H NMR (500 MHz, CDCl₃): 7.35-7.25 (m, 3H), 7.09-7.06 (m, 2H), 6.76 (s, 1 H), 6.65-6.62 (m, 2H), 4.96 (s, 2H), 4.03 (s, 2H), 3.14 (t, J = 5.8 Hz, 2H), 2.50 (t, J = 5.8 Hz, 2H), 2.45 (s, 3H).

### Example 28

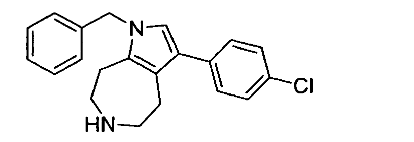

1-Benzyl-3-(4-chloro-phenyl)-1,4,5,6,7,8-hexahydro-pyrrolo[2,3-*d*]azepine.
Step A. 1-Benzyl-3-(4-chloro-phenyl)-4,5,7,8-tetrahydro-1*H*-pyrrolo[2,3-*d*]azepine-6-carboxylic acid *tert*-butyl ester. The desired compound (54.2 mg) was prepared from the compound of Example 59, Step B (0.56 g), 280 µL of benzylamine, and 0.49 g of 1-chloro-4-(2-nitro-vinyl)-benzene as in Example 1, Step A. MS (ESI): exact mass calculated for C₂₆H₂₉ClN₂O₂, 436.19; found, *m*/*z* 437.2 [M+H]⁺.
Step B. The above compound (54.2 mg) was converted to the title compound (19.2 mg) as in Example 27, Step B. MS (ESI): exact mass calculated for C₂₁H₂₁ClN₂, 336.14; found, *m*/*z* 337.1 [M+H]⁺. ¹H NMR (500 MHz, CDCl₃): 7.34-7.24 (m, 7H), 7.04 (d, *J* = 7.1 Hz, 2H), 6.62 (s, 1H), 5.05 (s, 2H), 3.03-3.00 (m, 2H), 2.97-2.94 (m, 2H), 2.83-2.80 (m, 2H), 2.74-2.71 (m, 2H).

### Example 29

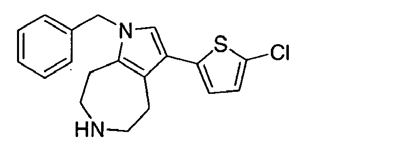

1-Benzyl-3-(5-chloro-thiophen-2-yl)-1,4,5,6,7,8-hexahydro-pyrrolo[2,3-*d*]azepine.
Step A. 1-Benzyl-3-(5-chloro-thiophen-2-yl)-4,5,7,8-tetrahydro-1*H*-pyrrolo[2,3-*d*]azepine-6-carboxylic acid *tert*-butyl ester. The desired compound (124.5 mg) was prepared from the compound of Example 59, Step B (0.55 g), 280 µL of benzylamine, and 0.49 g of 2-chloro-5-(2-nitro-vinyl)-thiophene as in Example 1, Step A. MS (ESI): exact mass calculated for C₂₄H₂₇ClN₂O₂S, 442.15; found, *m*/*z* 443.2 [M+H]⁺.
Step B. The above compound (124.5 mg) was converted to the title compound (30.7 mg) as in Example 27, Step B. MS (ESI): exact mass calculated for C₁₉H₁₉ClN₂S, 342.10; found, *m*/*z* 343.1 [M+H]⁺. ¹H NMR (500 MHz, CDCl₃): 7.35-7.31 (m, 2H), 7.29-7.25 (m, 1 H), 7.04-7.00 (m, 2H), 6.82 (d, J = 3.8 Hz, 1 H), 6.66 (s, 1 H), 6.64 (d, J = 3.8 Hz, 1 H), 5.02 (s, 2H), 3.06-3.03 (m, 2H), 2.97-2.93 (m, 2H), 2.88-2.84 (m, 2H), 2.72-2.68 (m, 2H).

### Example 30

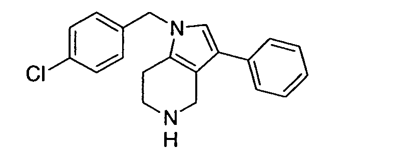

1-(4-Chloro-benzyl)-3-phenyl-4,5,6,7-tetrahydro-1*H*-pyrrolo[3,2-c]pyridine.
Step A. 1-(4-Chloro-benzyl)-3-phenyl-1,4,6,7-tetrahydro-pyrrolo[3,2-*c*]pyridine-5-carboxylic acid *tert*-butyl ester. The desired compound (405.6 mg) was prepared from 0.53 g of 4-oxo-piperidine-1-carboxylic acid *tert*-butyl ester, 334 µL of 2-chlorobenzylamine, and 0.34 g of (2-nitro-vinyl)-benzene as in Example 1, Step A. MS (ESI): exact mass calculated for C₂₅H₂₇ClN₂O₂, 422.18; found, *m*/*z* 423.2 [M+H]⁺.
Step B. The above compound (405.6 mg) was converted to the title compound (206.7 mg) as in Example 27, Step B, using MeOH as the solvent. The desired product was then treated with malic acid (75.0 mg) in EtOAc. The solids were collected by filtration to give the corresponding maleate salt. MS (ESI): exact mass calculated for C₂₀H₁₉CIN₂, 322.12; found, *m*/*z* 323.2 [M+H]⁺. ¹H NMR (500 MHz, CD₃OD): 7.37-7.32 (m, 6H), 7.22-7.18 (m, 1 H), 7.16-7.13 (m, 2H), 7.12 (s, 1H), 6.24 (s, 2H), 5.14 (s, 2H), 4.35 (s, 2H), 3.51 (t, *J =* 6.3 Hz, 2H), 2.84 (t, J = 6.3 Hz, 2H).

### Example 31

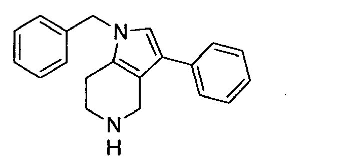

1-Benzyl-3-phenyl-4,5,6,7-tetrahydro-1 *H*-pyrrolo[3,2-*c*]pyridine.
Step A. 1-Benzyl-3-phenyl-1,4.6,7-tetrahydro-pyrrolof3.2-clpyridine-5-carboxylic acid tert-butyl ester. The desired compound (380.7 mg) was prepared from 0.51 g of 4-oxo-piperidine-1-carboxylic acid *tert*-butyl ester, 280 µL of benzylamine, and 0.39 g of (2-nitro-vinyl)-benzene as in Example 1, Step A. MS (ESI): exact mass calculated for C₂₅H₂₈N₂O₂, 388.22; found, m/z 389.2 [M+H]⁺.
Step B. The above compound (0.37 g) was converted to the title compound (234.7 mg) as in Example 26, Step B. MS (ESI): exact mass calculated for C₂₀H₂₀N₂, 288.16; found, *m*/*z* 289.2 [M+H]⁺. ¹H NMR (500 MHz, CDCl₃): 7.27-7.23 (m, 6H), 7.22-7.17 (m, 1H), 7.12-7.07 (m, 1H), 7.03-6.99 (m, 2H), 6.77 (s, 1 H), 4.93 (s, 2H), 3.98 (s, 2H), 3.07 (t, J = 5.8 Hz, 2H), 2.48 (t, J = 5.8 Hz, 2H).

### Example 32

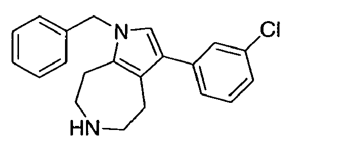

1-Benzyl-3-(3-chloro-phenyl)-1,4,5,6,7,8-hexahydro-pyrrolo[2,3-*d*]azepine.
To a solution of the compound from Example 59, Step B (0.51 g) in toluene (5 mL) was added 280 µL of benzylamine and 0.8 mL of Ti(OiPr)₄. The resulting mixture was stirred for 3 h at RT. 1-Chloro-3-(2-nitro-vinyl)-benzene (0.46 g) was then added in one portion and stirring was continued for an additional 16 h at RT. The mixture was poured into water and filtered through diatomaceous earth. The aqueous filtrate was extracted with EtOAc (3 x 20 mL) and the combined organic layers were concentrated *in vacuo.* Chromatography on SiO₂ (1 to 35% EtOAc/hexanes) afforded 106.7 mg of 1-benzyl-3-(3-chlorophenyl)-4,5,7,8-tetrahydro-1*H*-pyrrolo[2,3-*d*]azepine-6-carboxylic acid *tert*-butyl ester. This compound was then converted to the title compound (19.1 mg) as in Example 27, Step B, using 10:1 CH₂Cl₂/MeOH as the solvent. MS (ESI): exact mass calculated for C₂₁H₂₁CIN₂, 336.14; found, *m*/*z* 337.2 [M+H]⁺. ¹H NMR (500 MHz, CDCl₃): 7.36-7.30 (m, 3H), 7.29-7.25 (m, 2H), 7.23-7.17 (m, 2H), 7.05-7.02 (m, 2H), 6.64 (s, 1 H), 5.05 (s, 2H), 3.01-2.98 (m, 2H), 2.94-2.91 (m, 2H), 2.82-2.97 (m, 2H), 2.71-2.68 (m, 2H).

### Example 33

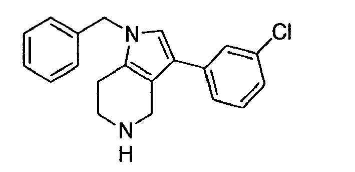

1-Benzyl-3-(3-chloro-phenyl)-4,5,6,7-tetrahydro-1 *H*-pyrrolo[3,2-*c*]pyridine.
The title compound (193.3 mg) was prepared from 0.50 g of 4-oxo-piperidine-1-carboxylic acid *tert*-butyl ester, 260 µL of benzylamine, and 0.45 g of 1-chloro-3-(2-nitro-vinyl)-benzene as in Example 9. MS (ESI): exact mass calculated for C₂₀H₁₉ClN₂, 322.12; found, *m*/*z* 323.2 [M+H]⁺. ¹H NMR (500 MHz, CDCl₃): 7.36-7.20 (m, 6H), 7.14-7.07 (m, 3H), 6.81 (s, 1 H), 5.01 (s, 2H), 4.04 (s, 2H), 3.14 (t, J= 5.8 Hz, 2H), 2.51 (t, J= 5.8 Hz, 2H).

### Example 34

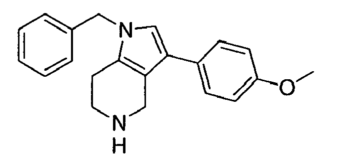

1-Benzyl-3-(4-methoxy-phenyl)-4,5,6,7-tetrahydro-1*H*-pyrrolo[3,2-*c*]pyridine.
Step A. 1-Benzyl-3-(4-methoxy-phenyl)-1,4,6,7-tetrahydro-pyrrolo[3,2-c]pyridine-5-carboxylic acid *tert*-butyl ester. To a solution of 0.50 g of 4-oxo-piperidine-1-carboxylic acid *tert*-butyl ester and 260 µL of benzylamine in toluene (5 mL) was added 0.48 g of MgSO₄ and 16.7 mg of Bu₂SnCl₂. After 1 h, 0.45 g of 1-methoxy-4-(2-nitro-vinyl)-benzene was added and the mixture was stirred for 16 h at RT. The mixture was then diluted with water (80 mL) and extracted with EtOAc (3 x 15 mL) and the combined organic layers were concentrated *in vacuo.* Chromatography on SiO₂ (1 to 20% EtOAc/hexanes) afforded 0.38 g of the desired compound. MS (ESI): exact mass calculated for C₂₆H₃₀N₂O₃, 418.23; found, *m*/*z* 419.2 [M+H]⁺.
Step B. The above compound (0.47 g) was converted to the title compound (275.2 mg) as in Example 26, Step B. MS (ESI): exact mass calculated for C₂₁H₂₂N₂O, 318.17; found, *m*/*z* 319.2 [M+H]⁺. ¹H NMR (400 MHz, CDCI₃): 7.35-7.24 (m, 5H), 7.12-7.08 (m, 2H), 6.90-6.86 (m, 2H), 6.74 (s, 1 H), 4.99 (s, 2H), 4.04 (s, 2H), 3.81 (s, 3H), 3.16 (t, J = 5.8 Hz, 2H), 2.53 (t, J = 5.8 Hz, 2H).

### Example 35

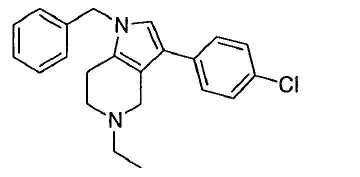

1-Benzyl-3-(4-chloro-phenyl)-5-ethyl-4,5,6,7-tetrahydro-1 *H*-pyrrolo[3,2-c]pyridine.
To a solution of 1-benzyl-3-(4-chloro-phenyl)-4,5,6,7-tetrahydro-1*H*-pyrrolo[3,2-c]pyridine (Example 25; 0.11 g) in 1,2-dichloroethane (5 mL) was added 18 µL of acetic acid, 26 µL of acetaldehyde, and 0.10 g of NaBH(OAc)₃. The mixture was stirred at RT for 15 h. The mixture was diluted with CH₂Cl₂ and washed with satd. aq. NaHCO₃ (2x). The combined organic layers were dried over Na₂SO₄, filtered, and concentrated *in vacuo.* Chromatography on SiO₂ (1% 2 M NH₃ in MeOH/CH₂Cl₂) afforded 0.02 g of the title compound. The product was dissolved in Et₂O and treated with excess 1.0 M HCl in Et₂O to afford 0.02 g of the corresponding HCl salt. MS (ESI): exact mass calculated for C₂₂H₂₃CIN₂, 350.15; found, *m*/*z* 351.2 [M+H]⁺, 353.2 [M+H]⁺. ¹H NMR (500 MHz, CD₃OD): 7.37-7.27 (m, 7H), 7.19-7.17 (m, 3H), 5.17-5.13 (m, 2H), 4.48-4.37 (m, 2H), 3.85-3.76 (m, 2H), 3.45-3.23 (m, 2H), 3.00-2.84 (m, 2H), 1.38 (t, J= 7.1 Hz, 3H).

### Example 36

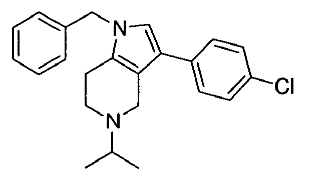

1-Benzyl-3-(4-chloro-phenyl)-5-isopropyl-4,5,6,7-tetrahydro-1 *H*-pyrrolo[3,2-*c*]pyridine.
The title compound (0.1 g) was prepared from 1-benzyl-3-(4-chloro-phenyl)-4,5,6,7-tetrahydro- 1*H*-pyrrolo[3,2-*c*]pyridine (Example 25; 0.10 g) and 32 µL of acetone as in Example 35. MS (ESI): exact mass calculated for C₂₃H₂₅ClN₂, 364.17; found, *m*/*z*365.2 [M+H]⁺, 367.2 [M+H]⁺. ¹H NMR (500 MHz, CD₃OD): 7.37-7.27 (m, 7H), 7.21-7.17 (m, 3H), 5.15 (d, J = 5.2 Hz, 2H), 4.58-4.54 (m, 1 H), 4.28-4.25 (m, 1 H), 3.78-3.65 (m, 2H), 3.45-3.35 (m, 1 H), 3.03-2.85 (m, 2H), 1.42 (t, J = 6.6 Hz, 6H).

### Example 37

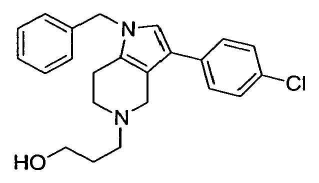

3-[1-Benzyl-3-(4-chloro-phenyl)-1,4,6,7-tetrahydro-pyrrolo[3,2-c]pyridin-5-yl]-propan-1-ol.
To a solution of 1-benzyl-3-(4-chloro-phenyl)-4,5,6,7-tetrahydro-1 *H*-pyrrolo[3,2-*c*]pyridine (Example 25; 0.51 g) in DMF (14 mL) was added 1.39 g of Cs₂CO₃ and 142 µL of 3-bromo-1-propanol. The mixture was stirred at RT for 12 h and then was diluted with water. The aqueous layer was extracted with Et₂O and the combined organic layers were dried over Na₂SO₄, filtered, and concentrated *in vacuo.* Chromatography on SiO₂ (2% 2 M NH₃ in MeOH/CH₂Cl₂) afforded 0.15 g of the title compound. The product was dissolved in Et₂O and treated with excess 1.0 M HCl in Et₂O to afford 0.16 g the corresponding HCl salt. MS (ESI): exact mass calculated for C₂₃H₂₅ClN₂O, 380.17; found, *m*/*z* 381.2 [M+H]⁺, 383.2 [M+H]⁺. ¹H NMR (500 MHz, CD₃OD): 7.37-7.27 (m, 7H), 7.19-7.17 (m, 3H), 5.15 (s, 2H), 4.47 (br s, 2H), 3.93-3.25 (m, 6H), 2.94-2.93 (m, 2H), 2.01-1.96 (m, 2H).

### Example 38

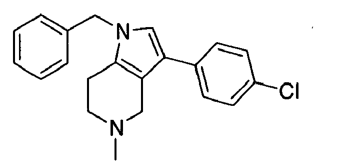

1-Benzyl-3-(4-chloro-phenyl)-5-methyl-4,5,6,7-tetrahydro-1*H*-pyrrolo[3,2-c]pyridine.
Step A. 1-Benzyl-3-(4-chloro-phenyl)-1,4,6,7-tetrahydro-pyrrolo[3,2-*c*]pyridine-5-carboxylic acid ethyl ester. To a stirred solution of 3.0 g of 4-oxo-piperidine-1-carboxylic acid ethyl ester in benzene (35 mL) was added 1.91 mL of benzylamine. The mixture was heated at reflux for 24 h using a Dean-Stark apparatus. The solvent was removed to give a pale yellow oil. A portion of the crude product (0.50 g) was dissolved in toluene (4 mL) and 0.35 g of 1-chloro-4-(2-nitro-vinyl)-benzene was added, followed by 0.7 g of 4Å molecular sieves. The resulting mixture was stirred for 12 h at RT. The mixture was then filtered through diatomaceous earth and the filtrate was washed with satd. aq. NH₄Cl (3x). The combined organic extracts were dried over Na₂SO₄, filtered, and concentrated *in vacuo.* Chromatography on SiO₂ (8% EtOAc/hexanes) afforded 0.25 g the title compound. TLC (SiO₂, 25% EtOAc/hexanes): R_{f} = 0.34. MS (ESI): exact mass calculated for C₂₃H₂₃ClN₂O₂, 394.14; found, *m*/*z* 395.2 [M+H]⁺, 397.2 [M+H]⁺, 417.1 [M+Na]⁺.
Step B. To a stirred solution of the above compound (0.25 g) in toluene (20 mL) was added 571 µL of sodium bis(2-methoxyethoxy)aluminum hydride (Red-Al, 1.5 M in toluene). The mixture was stirred for 48 h at RT and then was quenched by the addition of satd. aq. potassium sodium tartrate. The organic layer was separated and dried over Na₂SO₄, filtered, and concentrated *in vacuo* to give 0.16 g of the title compound. TLC (SiO₂, 10% MeOH/EtOAc): R_{f} =0.14. MS (ESI): exact mass calculated for C₂₁H₂₁ClN₂, 336.14; found, *m*/*z* 337.2 [M+H]⁺, 339.2 [M+H]⁺. ¹H NMR (500 MHz, CDCl₃): 7.31-7.24 (m, 7H), 7.07-7.06 (m, 2H), 6.76 (s, 1 H), 4.98 (s, 2H), 3.56 (s, 2H), 2.72 (t, J = 6.3 Hz, 2H), 2.60 (t, J = 6.3 Hz, 2H).

### Example 39

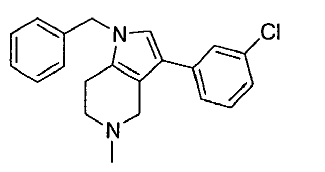

1-Benzyl-3-(3-chloro-phenyl)-5-methyl-4,5,6,7-tetrahydro-1*H*-pyrrolo[3,2-c]pyridine.
The title compound (0.34 g) was prepared from 3.0 g of 4-oxo-piperidine-1-carboxylic acid ethyl ester, 1.91 mL of benzylamine, and 1.2 g of 1-chloro-3-(2-nitro-vinyl)-benzene as in Example 38. TLC (SiO₂, 2% NH₃ in MeOH/EtOAc): R_{f} = 0.25. MS (ESI): exact mass calculated for C₂₁H₂₁ClN₂, 336.14; found, m/z 337.2 [M+H]⁺, 339.2 [M+H]⁺. ¹H NMR (500 MHz, CD₃OD): 7.36-7.32 (m, 4H), 7.30-7.25 (m, 2H), 7.21-7.16 (m, 4H), 5.15 (s, 2H), 4.41 (s, 2H), 3.53 (t, J= 6.3 Hz, 2H), 2.98 (s, 3H), 2.91 (t, J = 6.3 Hz, 2H), 2.82-2.70 (m, 4H).

### Example 40

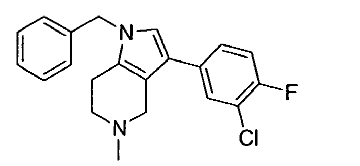

1-Benzyl-3-(3-chloro-4-fluoro-phenyl)-5-methyl-4,5,6,7-tetrahydro-1 H pyrrolo[3,2-c]pyridine.
The title compound (0.03 g) was prepared from 1-benzyl-3-(3-chloro-4-fluorophenyl)-4,5,6,7-tetrahydro-1 *H*-pyrrolo[3,2-*c*]pyridine (Example 2) and paraformaldehyde as in Example 35. The product was then dissolved in 1/1 EtOAc/CH₂Cl₂ and treated with 0.03 g (0.15 mmol) of citric acid to afford 0.05 g of the corresponding citrate salt. MS (ESI): exact mass calculated for C₂₁H₂₀ClFN₂, 354.13; found, *m*/*z* 355.1 [M+H]⁺, 357.2 [M+H]⁺. ¹H NMR (500 MHz, CD₃OD): 7.44-7.22 (m, 1 H), 7.34 (t, J = 7.7 Hz, 2H), 7.30-7.26 (m, 2H), 7.22 (t, J= 9.1 Hz, 1 H), 7.19-7.13 (m, 3H), 5.14 (s, 2H), 4.36 (s, 2H), 3.50 (t, J = 5.8 Hz, 2H), 2.96 (s, 3H), 2.89 (t, J = 5.8, 2H), 2.82-2.71 (m, 4H).

### Example 41

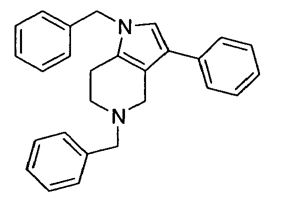

1,5-Dibenzyl-3-phenyl-4,5,6,7-tetrahydro-1*H*-pyrrolo[3,2-*c*]pyridine.
To a stirred solution of 0.46 mL of 1-benzyl-piperidin-4-one in absolute EtOH (5 mL) was added 0.27 mL of benzylamine. After 3 h, the solvent was removed *in vacuo.* The residue was diluted with absolute EtOH (5 mL) and 0.37 g of (2-nitro-vinyl)-benzene was added in one portion. The mixture was stirred at RT for 16 h, filtered through diatomaceous earth, and the filtrate was concentrated. Chromatography on SiO₂ (1 to 20% EtOAc/hexanes) afforded 0.48 g of the title compound. MS (ESI): exact mass calculated for C₂₇H₂₆N₂, 378.21; found, m/z 379.2 [M+H] ⁺. ¹H NMR (500 MHz, CDCl₃): 7.40-7.22 (m, 12H), 7.18-7.10 (m, 3H), 6.80 (s, 1 H), 4.99 (s, 2H), 3.78 (br m, 2H), 3.75 (s, 2H), 2.79-2.74 (m, 2H), 2.59-2.55 (m, 2H).

### Example 42

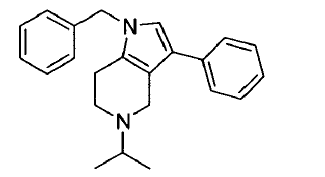

1-Benzyl-5-isopropyl-3-phenyl-4,5,6,7-tetrahydro-1*H*-pyrrolo[3,2-*c*]pyridine.
The title compound (111.0 mg) was prepared from 372 µL of 1-isopropyl-piperidin-4-one, 270 µL of benzylamine, and 0.38 g of (2-nitro-vinyl)-benzene as in Example 41. The product was diluted with EtOAc and malic acid (39.0 mg) was added. The solids that formed were collected by filtration to give the title compound as a maleate salt. MS (ESI): exact mass calculated for C₂₃H₂₆N₂, 330.21; found, m/z 331.2 [M+H]⁺. ¹H NMR (500 MHz, CD₃OD): 7.38-7.33 (m, 6H), 7.30-7.27 (m, 1 H), 7.23-7.19 (m, 3H), 7.14 (s, 1 H), 6.25 (s, 2H), 5.15 (s, 2H), 3.74-3.66 (m, 1 H), 2.96-2.91 (br m, 2H), 1.41 (d, J = 6.6 Hz, 6H).

### Example 43

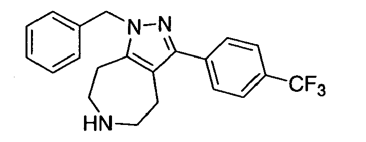

1-Benzyl-3-(4-trifluoromethyl-phenyl)-1,4,5,6,7,8-hexahydro-1,2,6-triazaazulene.
Step A. 3-Oxo-2,3,4,5,7,8-hexahydro-1*H*-1,2,6-triaza-azulene-6-carboxylic acid *tert*-butyl ester. To a solution of 5-oxo-azepane-1,4-dicarboxylic acid 1-*tert*-butyl ester 4-ethyl ester (Example 59, Step A; 8.29 g) in 80 mL of EtOH was added 1.5 mL of hydrazine hydrate. The solution was heated at reflux for 2 days and then was cooled to RT. The solvent volume was reduced to ca. 20 mL and the resulting solution was stored at -15 °C for 16 h. Water was added and the solids were collected by filtration, washed with water, and dried to give 4.99 g of the desired compound as a white crystalline solid. MS (ESI): exact mass calculated for C₁₂H₁₉N₃O₃, 253.14; found, *m*/*z* 254.1 [M+H]⁺.
Step B. 1-Benzyl-3-oxo-2,3,4,5,7,8-hexahydro-1 *H*-1,2,6-triaza-azulene-6-carboxylic acid *tert*-butyl ester. To a stirred solution of 1.16 g the compound from step A in 15 mL of DMF was added 1.80 g of Cs₂CO₃. The suspension was stirred at RT for 20 min. Benzyl bromide (0.6 mL) was added and the mixture was stirred at RT for an additional 12 h. The mixture was diluted with water and extracted with Et₂O. The combined organic layers were washed with water, brine, dried over Na₂SO₄, and concentrated to afford 1.77 g of a colorless semi-solid. Chromatography on SiO₂ (15 to 50% EtOAc/hexanes) over 1 h gave 1.21 g of the desired compound as a mixture of mono-benzylated isomers. TLC (SiO₂, 50% EtOAc/hexanes): R_{f} = 0.34. MS (ESI): exact mass calculated for C₁₉H₂₅N₃O₃, 343.19; found, *m*/*z* 344.2 [M+H]⁺, 366.2 [M+Na]⁺.
Step C. 1-Benzyl-3-trifluoromethanesulfonyloxy-4,5,7,8-tetrahydro-1*H*-1,2,6-triaza-azulene-6-carboxylic acid *tert*-butyl ester. To a stirred solution of the above mixture of regioisomers (1.21 g) in 35 mL of CH₂Cl₂ was added 1.93 mL of *i*-Pr₂NEt and 1.58 g of *N*-phenyltrifluoromethane-sulfonimide. The mixture was heated at reflux for 12 h and then was cooled and concentrated *in vacuo.* Chromatography on SiO₂ (5 to 20% EtOAc/hexanes) afforded 0.63 g of the desired compound. TLC (SiO₂, 25% EtOAc/hexanes): R_{f} = 0.37. MS (ESI): exact mass calculated for C₂₀H₂₄F₃N₃O₅S, 475.14; found, *m*/*z* 476.2 [M+H]⁺. Also, 0.68 g of the undesired mono-benzylated 3-benzyloxy-4,5,7,8-tetrahydro-1 *H*-1,2,6-triaza-azulene-6-carboxylic acid *tert*-butyl ester was obtained.
Step D. 1-Benzyl-3-(4-trifluoromethyl-phenyl)-4,5,7,8-tetrahydro-1*H*-1,2,6-triaza-azulene-6-carboxylic acid *tert*-butyl ester. To a solution of the compound from step C (0.17 g) in 5 mL of THF was added 0.12 g of K₃PO₄, 0.08 g of 4-trifluoromethylphenylboronic acid and 0.03 g of PdCl₂dppf. The mixture was heated at reflux for 12 h. The mixture was cooled, filtered through diatomaceous earth, and concentrated *in vacuo.* Chromatography on SiO₂ (5 to 40% EtOAc/hexanes) afforded 0.05 g of the desired compound. TLC (SiO₂, 25% EtOAc/hexanes): R_{f} = 0.49.
Step E. 1-Benzyl-3-(4-trifluoromethyl-phenyl)-1,4,5,6,7,8-hexahydro-1,2,6-triaza-azulene. To a stirred solution of the compound from step D (0.05 g) in 2 mL of CH₂Cl₂ was added 2.0 mL of TFA. The mixture was stirred at RT for 2 h and concentrated *in vacuo.* The crude product was re-dissolved in CH₂Cl₂ and treated with Dowex^{®} 550A resin. After stirring for 2 h, the mixture was filtered and concentrated *in vacuo* to afford 0.04 g of the title compound. The product was dissolved in Et₂O and treated with excess 1.0 M HCl in Et₂O for 30 min.
   The solvent was removed *in vacuo* to afford 0.05 g of the corresponding HCl salt. MS (ESI): exact mass calculated for C₂₁H₂₀F₃N₃, 371.16; found, m/z 372.2 [M+H]⁺. ¹H NMR (500 MHz, CD₃OD): 7.78-7.74 (m, 4H), 7.37-7.28 (m, 3H), 7.20 (t, J = 6.9 Hz, 2H), 5.46 (br s, 2H), 4.65 (br s, 1 H), 3.40- 3.37 (m, 3H), 3.17- 3.10 (m, 4H).

The title compounds of Examples 44 - 53 were prepared according to the general procedure indicated by Example 43, Steps D and E, unless otherwise noted.

### Example 44

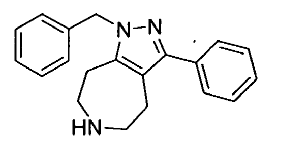

1-Benzyl-3-phenyl-1,4,5,6,7,8-hexahydro-1,2,6-triaza-azulene.
The title compound (0.07 g) was prepared from the compound of Example 43, Step C (0.16 g), and 0.05 g of phenylboronic acid. MS (ESI): exact mass calculated for C₂₀H₂₁N₃, 303.17; found, *m*/*z* 304.2 [M+H]⁺. ¹H NMR (500 MHz, CD₃OD): 7.56-7.54 (m, 2H), 7.51-7.43 (m, 3H), 7.39-7.36 (m, 2H), 7.33-7.29 (m, 1 H), 7.21 (d, J = 6.9 Hz, 2H), 5.50 (s, 2H), 3.43-3.38 (m, 4H), 3.22-3.20 (m, 2H), 3.12-3.10 (m,2H).

### Example 45

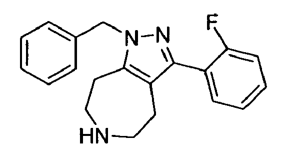

1-Benzyl-3-(2-fluoro-phenyl)-1,4,5,6,7,8-hexahydro-1,2,6-triaza-azulene.
The title compound (0.06 g) was prepared from the compound of Example 43, Step C (0.31 g), and 0.10 g of 2-fluorophenylboronic acid, using 1,4-dioxane as the solvent. MS (ESI): exact mass calculated for C₂₀H₂₀FN₃, 321.16; found, *m*/*z* 322.2 [M+H]⁺. ¹H NMR (500 MHz, CD₃OD): 7.52-7.46 (m, 2H), 7.38-7.18 (m, 7H), 5.46 (s, 2H), 3.38-3.33 (m, 4H), 3.17-3.15 (m, 2H), 2.91-2.89 (m, 2H).

### Example 46

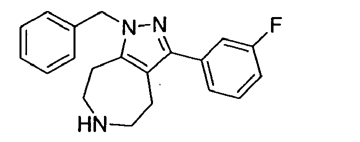

1-Benzyl-3-(3-fluoro-phenyl)-1,4,5,6,7,8-hexahydro-1,2,6-triaza-azulene.
The title compound (0.07 g) was prepared from the compound of Example 43, Step C (0.30 g), and 0.10 g of 3-fluorophenylboronic acid, using 1,4-dioxane as the solvent. MS (ESI): exact mass calculated for C₂₀H₂₀FN₃, 321.16; found, *m*/*z* 322.2 [M+H]⁺. ¹H NMR (400 MHz, CD₃OD): 7.52-7.47 (m, 1H), 7.38-7.27 (m, 5H), 7.20-7.13 (m, 3H), 5.47 (s, 2H), 3.43-3.34 (m, 4H), 3.23-3.06 (m, 4H).

### Example 47

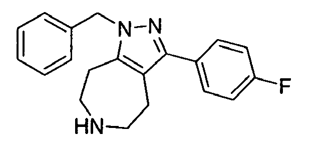

1-Benzyl-3-(4-fluoro-phenyl)-1,4,5,6,7,8-hexahydro-1,2,6-triaza-azulene.
The title compound (0.07 g) was prepared from the compound of Example 43, Step C (0.22 g), and 0.20 g of 4-fluorophenylboronic acid, adding 9.1 mg of dppf and using 1,4-dioxane as the solvent. MS (ESI): exact mass calculated for C₂₀H₂₀FN₃, 321.16; found, *m*/*z* 322.2 [M+H]⁺. ¹H NMR (500 MHz, CDCl₃): 7.54-7.51 (m, 2H), 7.33-7.30 (m, 2H), 7.28-7.24 (m, 1 H), 7.12-7.07 (m, 4H), 5.35 (s, 2H), 2.98-2.95 (m, 2H), 2.94-2.92 (m, 2H), 2.80-2.77 (m, 2H), 2.76-2.74 (m, 2H).

### Example 48

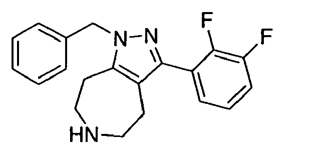

1-Benzyl-3-(2,3-difluoro-phenyl)-1,4,5,6,7,8-hexahydro-1,2,6-triaza-azulene.
The title compound (0.05 g) was prepared from the compound of Example 43, Step C (0.30 g), and 0.11 g of 3,4-difluorophenylboronic acid, using 1,4-dioxane as the solvent. MS (ESI): exact mass calculated for C₂₀H₁₉F₂N₃, 339.15; found, *m*/*z* 340.2 [M+H]⁺. ¹H NMR (500 MHz, CD₃OD): 7.37-7.26 (m, 6H), 7.21-7.18 (m, 2H), 5.46 (s, 2H), 3.38-3.34 (m, 4H), 3.18-3.16 (m, 2H), 2.92-2.90 (m, 2H).

### Example 49

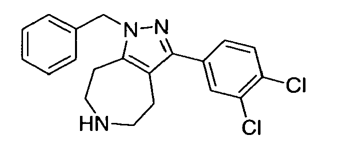

1-Benzyl-3-(3,4-dichloro-phenyl)-1,4,5,6,7,8-hexahydro-1,2,6-triaza-azulene.
The title compound (0.02 g) was prepared from the compound of Example 43, Step C (0.17 g), and 0.08 g of 3,4-dichlorophenylboronic acid. MS (ESI): exact mass calculated for C₂₀H₁₉Cl₂N₃, 371.10; found, m/z 372.1 [M+H]⁺, 374.1 [M+H]⁺, 376.1 [M+H]⁺. ¹H NMR (500 MHz, CD₃OD): 7.78-7.74 (m, 4H), 7.37-7.28 (m, 3H), 7.21-7.18 (m, 2H), 5.46-5.45 (m, 2H), 3.40-3.30 (m, 4H), 3.17-3.10 (m, 4H).

### Example 50

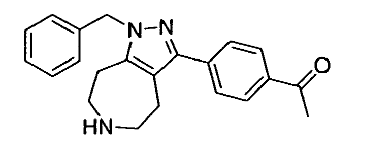

1-[4-(1-Benzyl-1,4,5,6,7,8-hexahydro-1,2,6-triaza-azulen-3-yl)-phenyl]-ethanone.
The title compound (0.02 g) was prepared from the compound of Example 43, Step C (0.20 g), and 0.08 g of 4-acetylphenylboronic acid, using 1,4-dioxane as the solvent. MS (ESI): exact mass calculated for C₂₂H₂₃N₃O, 345.18; found, m/z346.2 [M+H]⁺. ¹H NMR (500 MHz, CD₃OD): 8.10-8.09 (m, 2H), 7.71-7.70 (m, 2H), 7.38-7.19 (m, 5H), 5.48 (s, 2H), 3.40 (br s, 4H), 3.28-3.10 (m, 4H), 2.64 (s, 3H).

### Example 51

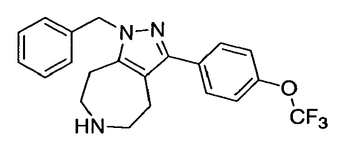

1-Benzyl-3-(4-trifluoromethoxy-phenyl)-1,4,5,6,7,8-hexahydro-1,2,6-triaza-azulene.
The title compound (0.03 g) was prepared from the compound of Example 43, Step C (0.26 g), and 0.13 g of 4-trifluoromethoxyphenylboronic acid, using 1,4-dioxane as the solvent. MS (ESI): exact mass calculated for C₂₁H₂₀F₃N₃O, 387.16; found, *m*/*z* 388.1 [M+H]⁺. ¹H NMR (500 MHz, CD₃OD): 7.67-7.63 (m, 2H), 7.39-7.28 (m, 5H), 7.20-7.17 (m, 2H), 5.44 (s, 2H), 3.39-3.36 (m, 2H), 3.32-3.30 (m, 2H), 3.15-3.06 (m, 4H).

### Example 52

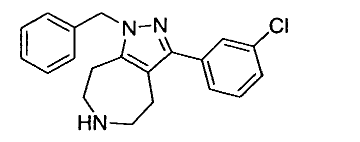

1-Benzyl-3-(3-chloro-phenyl)-1,4,5,6,7,8-hexahydro-1,2,6-triaza-azulene.
The title compound (0.04 g) was prepared from the compound of Example 43, Step C (0.20 g), and 0.07 g of 3-chlorophenylboronic acid, using 1,4-dioxane as the solvent. MS (ESI): exact mass calculated for C₂₀H₂₀CIN₃, 337.13; found, m/z 338.1 [M+H]⁺, 340.1 [M+H]⁺. ¹H NMR (400 MHz, CD₃OD): 7.56 (br s, 1 H), 7.47-7.29 (m, 6H), 7.29-7.19 (m, 2H), 5.44 (s, 2H), 3.38-3.36 (m, 2H), 3.32-3.30 (m, 2H), 3.19-3.06 (m, 4H).

### Example 53

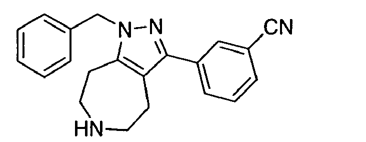

3-(1-Benzyl-1,4,5,6,7,8-hexahydro-1,2,6-triaza-azulen-3-yl)-benzonitrile.
The title compound (0.04 g) was prepared from the compound of Example 43, Step C (0.30 g), and 0.10 g of 3-cyanophenylboronic acid, using 1,4-dioxane as the solvent. MS (ESI): exact mass calculated for C₂₁H₂₀N₄, 328.17; found, *m*/*z* 329.2 [M+H]⁺. ¹H NMR (500 MHz, CD₃OD): 7.91-7.86 (m, 2H), 7.76-7.75 (m, 1 H), 7.65 (t, J = 7.7 Hz, 1 H), 7.37-7.20 (m, 3H), 7.19-7.18 (m, 2H), 5.45 (s, 2H), 3.39-3.37 (m, 4H), 3.17-3.08 (m, 4H).

### Example 54

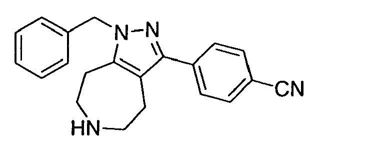

4-(1-Benzyl-1,4,5,6,7,8-hexahydro-1,2,6-triaza-azulen-3-yl)-benzonitrile.
To a solution of 1-benzyl-3-trifluoromethanesulfonyloxy-4,5,7,8-tetrahydro-1*H-*1,2,6-triaza-azulene-6-carboxylic acid *tert*-butyl ester (Example 43, Step C; 0.30 g) in 9 mL of 1,4-dioxane was added 0.20 g of K₃PO₄, 0.10 g of 4-cyanophenylboronic acid, and 0.05 g of PdCl₂dppf. The mixture was heated at reflux for 72 h. The mixture was filtered through diatomaceous earth and concentrated *in vacuo* to give 0.33 g of an orange solid. Chromatography on SiO₂ (5 to 30% EtOAc/hexanes) afforded 0.21 g of a 7:1 mixture of 1-benzyl-3-(4-cyano-phenyl)-4,5,7,8-tetrahydro-1*H*-1,2,6-triaza-azulene-6-carboxylic acid *tert-*butyl ester and a side product, 1-benzyl-4,5,7,8-tetrahydro-1 *H*-1,2,6-triaza-azulene-6-carboxylic acid *tert*-butyl ester. The mixture of compounds (0.21 g) was dissolved in 7 mL CH₂Cl₂ and 7 mL of TFA was added. The mixture was stirred at RT for 1 h and concentrated *in vacuo.* The crude product was dissolved in CH₂Cl₂ and treated with Dowex^{®} 550A resin. After stirring for 2 h, the mixture was filtered and concentrated *in vacuo.* Chromatography using a C₁₈ reverse phase column afforded 0.14 g of the title compound as its TFA salt. MS (ESI): exact mass calculated for C₂₁H₂₀N₄, 328.17; found, *m*/*z* 329.1 [M+H]⁺, 351.1 [M+Na]⁺. ¹H NMR (500 MHz, CD₃OD): 7.83-7.81 (m, 2H), 7.76-7.74 (m, 2H), 7.36-7.28 (m, 3H), 7.19-7.17 (m, 2H), 5.46 (s, 2H), 3.39-3.37 (m, 4H), 3.15-3.09 (m, 4H).

### Example 55

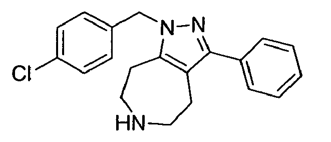

1-(4-Chloro-benzyl)-3-phenyl-1,4,5,6,7,8-hexahydro-1,2,6-triaza-azulene.
Step A. 1-(4-Chloro-benzyl)-3-phenyl-4,5,7,8-tetrahydro-1*H*-1,2,6-triaza-azulene-6-carboxylic acid *tert*-butyl ester. To a solution of 0.13 g of 1-(4-chloro-benzyl)-3-trifluoromethanesulfonyl-4,5,7,8-tetrahydro-1*H*-1,2,6-triaza-azulene-6-carboxylic acid *tert*-butyl ester, prepared as in Example 43, Steps A through C, in 3 mL of THF was added 300 µL of water, 0.11 g of K₂CO₃, 44.9 mg of phenylboronic acid, and 20.0 mg of PdCl₂dppf. The mixture was heated at 100 °C for 18 h. The mixture was filtered through diatomaceous earth and concentrated *in vacuo.* Chromatography on SiO₂ (hexanes to 45% EtOAc/hexanes) afforded 16.1 mg of the desired compound. MS (ESI): exact mass calculated for C₂₅H₂₈ClN₃O₂, 437.19; found, *m*/*z* 438.2 [M+H]⁺.
Step B. The above compound (16.1 mg) was converted to the title compound (7.1 mg) as in Example 26, Step B. MS (ESI): exact mass calculated for C₂₀H₂₀ClN₃, 337.13; found, *m*/*z* 338.1 [M+H]⁺. ¹H NMR (500 MHz, CDCI₃): 7.57-7.54 (m, 2H), 7.44-7.40 (m, 2H), 7.35-7.31 (m, 1 H), 7.30-7.26 (m, 3H), 7.04 (d, J = 8.5 Hz, 2H), 5.32 (s, 2H), 2.99-2.93 (m, 4H), 2.85-2.82 (m, 2H), 2.77-2.73 (m, 2H), 1.97 (br s, 1 H).

### Example 56

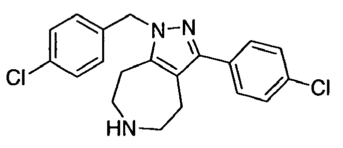

1-(4-Chloro-benzyl)-3-(4-chloro-phenyl)-1,4,5,6,7,8-hexahydro-1,2,6-triaza-azulene.
Step A. 1-(4-Chloro-benzyl)-3-(4-chloro-phenyl)-4,5,7,8-tetrahydro-1*H*-1,2,6-triaza-azulene-6-carboxylic acid *tert*-butyl ester. To a solution of 142.7 mg of 1-(4-chloro-benzyl)-3-trifluoromethanesulfonyl-4,5,7,8-tetrahydro-1*H*-1,2,6-triaza-azulene-6-carboxylic acid *tert*-butyl ester, prepared as in Example 43, Steps A through C, in 3 mL of THF was added 0.17 g of K₃PO₄, 54.9 mg of 4-chlorophenylboronic acid, and 22.1 mg of PdCl₂dppf. The mixture was heated at reflux for 48 h. The mixture was filtered through diatomaceous earth, rinsing with toluene, and the filtrate was concentrated *in vacuo.* Chromatography on SiO₂ (hexanes to 75% EtOAc/hexanes) afforded 6.7 mg of the desired compound. MS (ESI): exact mass calculated for C₂₅H₂₇Cl₂N₃O₂, 471.15; found, *m*/*z* 472.1 [M+H]⁺.
Step B. The above compound (6.7 mg) was converted to the title compound (5.0 mg) as in Example 26, Step B. MS (ESI): exact mass calculated for C₂₀H₁₉Cl₂N₃, 371.10; found, *m*/*z* 372.1 [M+H]⁺. ¹H NMR (500 MHz, CDCl₃): 7.48 (d, J = 8.5 Hz, 2H), 7.38 (d, J = 8.5 Hz, 2H), 7.29 (d, J = 8.5 Hz, 2H), 7.03 (d, J = 8.5 Hz, 2H), 5.30 (s, 2H), 3.02-2.96 (m, 4H), 2.84-2.80 (m, 2H), 2.79-2.76 (m, 2H).

### Example 57

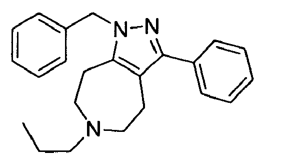

1-Benzyl-3-phenyl-6-propyl-1,4,5,6,7,8-hexahydro-1,2,6-triaza-azulene.
The title compound (0.05 g) was prepared from 1-benzyl-3-phenyl-1,4,5,6,7,8-hexahydro-1,2,6-triaza-azulene (Example 44, 0.09 g) and 23 µL of propionaldehyde as in Example 35. MS (ESI): exact mass calculated for C₂₃H₂₇N₃, 345.22; found, *m*/*z* 346.3 [M+H]⁺. ¹H NMR (400 MHz, CD₃OD): 7.56-7.54 (m, 2H), 7.47-7.28 (m, 6H), 7.21-7.19 (m, 2H), 5.44 (s, 2H), 3.70-3.66 (m, 2H), 3.41-3.07 (m, 8H), 1.86-1.76 (m, 2H), 1.03 (t, *J*= 7.3 Hz, 3H).

### Example 58

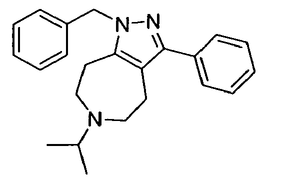

1-Benzyl-6-isopropyl-3-phenyl-1,4,5,6,7,8-hexahydro-1,2,6-triaza-azulene.
The title compound (0.03 g) was prepared from 1-benzyl-3-phenyl-1,4,5,6,7,8-hexahydro-1,2,6-triaza-azulene (Example 44, 0.07 g) and 22 µL of acetone as in Example 35. MS (ESI): exact mass calculated for C₂₃H₂₇N₃, 345.22; found, m/z346.3 [M+H]⁺. ¹H NMR (500 MHz, CD₃OD): 7.57-7.52 (m, 2H), 7.50-7.27 (m, 6H), 7.22-7.20 (m, 2H), 5.47 (s, 2H), 3.77-3.61 (m, 3H), 3.29-3.28 (m, 3H), 3.24-3.08 (m, 3H), 1.40 (d, J = 6.0 Hz, 6H).

### Example 59

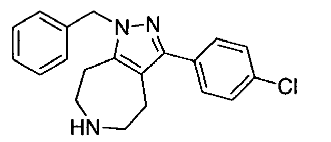

1-Benzyl-3-(4-chloro-phenyl)-1,4,5,6,7,8-hexahydro-1,2,6-triaza-azulene.
Step A. 5-Oxo-azepane-1,4-dicarboxylic acid 1-*tert*-butyl ester 4-ethyl ester. A solution of 4-oxo-piperidine-1-carboxylic acid *tert*-butyl ester (35 mmol, 7.0 g) in anhydrous Et₂O (50 mL) was stirred in a 200 mL 3-neck flask equipped with two addition funnels. The solution was cooled to -25 °C. Ethyl diazoacetate (46.5 mmol, 4.89 mL) in anhydrous Et₂O (10 mL) and BF₃•OEt₂ (36.7 mmol, 4.65 mL) in anhydrous Et₂O (10 mL) were simultaneously but independently added to the solution over 90 min. The mixture was stirred for an additional 1 h and was slowly warmed to RT. Then, 30% aq. K₂CO₃ was added dropwise to the mixture until gas evolution ceased. The organic layer was separated, dried over Na₂SO₄, and concentrated. The residue was purified via chromatography (SiO₂, 5 to 20% EtOAc/hexanes) to yield the desired compound (7.5 g).
Step B. 4-Oxo-azepane-1-carboxylic acid *tert*-butyl ester. To a solution of the product of Step A in 1,4-dioxane (50 mL) was added 1 N NaOH (40.83 mmol, 40.83 mL). The mixture was allowed to stir at rt overnight. The solution was then acidified to pH 4-5 with 3 N HCl. The mixture was extracted with Et₂O followed by CH₂Cl₂ until TLC showed no product remaining in the aqueous layer. The combined organic layers were dried over Na₂SO₄ and concentrated *in vacuo* to yield the desired compound (7.46 g). MS (ESI): exact mass calculated for C₁₁H₁₉NO₃, 213.14; found, *m*/*z* 236.2 [M+Na]⁺.
Step C. 3-(4-Chloro-phenyl)-4,5,7,8-tetrahydro-1*H*-1,2,6-triaza-azulene-6-carboxylic acid *tert*-butyl ester. *p*-Toluenesulfonic acid (0.033 mg, 0.18 mmol) and morpholine (3.4 mL, 38 mmol) were added to a solution of the product of step B (7.46 g, 35.0 mmol) in benzene (15 mL). The reaction mixture was heated at reflux for 20 h using a Dean-Stark trap. The reaction mixture was cooled to RT and concentrated *in vacuo* to afford the intermediate enamine, which was used without further purification. To a 0 °C solution of the enamine in CH₂Cl₂ (30 mL) was added triethylamine (27.5 mmol, 3.80 mL) followed by a solution of 4-chlorobenzoyl chloride (27.5 mmol, 3.50 mL) in CH₂Cl₂ (10 mL). The reaction mixture was allowed to warm to RT and was stirred for 16 h. The mixture was poured over water and the layers were separated. The organic layer was dried over Na₂SO₄ and concentrated. The resulting oil was diluted with EtOH (120 mL), cooled to 0 °C, and treated with hydrazine (75 mmol, 2.4 mL). The reaction mixture was allowed to warm to RT and was stirred for 16 h. The mixture was concentrated and the residue was purified by SFC purification to yield the desired compound (1.2 g). MS (ESI): exact mass calculated for C₁₈H₂₂ClN₃O₂, 347.14; found, m/z 346.0 [M-H]⁻. ¹H NMR (500 MHz, CD₃OD): 7.40-7.35 (m, 4H), 3.62-3.59 (m, 2H), 3.54-3.51 (m, 2H), 2.96-2.93 (m, 2H), 2.81-2.77 (m, 2H), 1.20 (s, 9H). The reaction sequence also yielded 3-(4-chloro-phenyl)-4,6,7,8-tetrahydro-1*H*-1,2,5-triaza-azulene-5-carboxylic acid *tert-*butyl ester (1.5 g). MS (ESI): exact mass calculated for C₁₈H₂₂ClN₃O₂, 347.14; found, *m*/*z* 346.0 [M-H]⁻. ¹H NMR (500 MHz, CD₃OD): 7.65. (d, *J*= 8.2 Hz, 1 H), 7.47-7.41 (m, 3H), 4.67-4.45 (m, 2H), 3.71-3.65 (m, 2H), 2.90-2.89 (m, 2H), 1.90-1.87 (m, 2H), 1.18 (s, 9H).
Step D. 1-Benzyl-3-(4-chloro-phenyl)-1,4,5,6,7,8-hexahydro-1,2,6-triaza-azulene-6-carboxylic acid *tert*-butyl ester. To a 0 °C solution of the product from Step C (0.10 g, 0.29 mmol) in DMF (2 mL) was added NaH (60% dispersion in oil, 92 mg, 2.3 mmol). The solution was allowed to warm to RT over 1 h, and, benzyl chloride (2.3 mmol) was then added. The reaction mixture was stirred for 16 h and then concentrated. The residue was diluted with water and extracted with CH₂Cl₂. The organic layer was washed with brine, dried over Na₂SO₄, and concentrated. The crude product was purified via SiO₂ chromatography to give the desired ester, which was carried directly into the next step. Also obtained was 2-benzyl-3-(4-chloro-phenyl)-4,5,7,8-tetrahydro-2*H*-1,2,6-triaza-azulene-6-carboxylic acid *tert*-butyl ester. MS (ESI): exact mass calculated for C₂₅H₂₈ClN₃O₂, 437.19; found, *m*/*z* 438.4 [M+H]⁺. ¹H NMR (500 MHz, CDCl₃): 7.50-7.48 (m, 2H), 7.40-7.38, (m, 2H), 7.33-7.26 (m, 3H), 7.13-7.11 (m, 2H), 5.33 (s, 2H), 3.55-3.51 (m, 4H), 2.86-2.77 (m, 4H), 1.47 (s, 9H).
Step E. The product from Step D was dissolved in 9:1 CH₂Cl₂/MeOH (4 mL). An excess of 1 N HCI in Et₂O was added and the resulting mixture was stirred for 2 h. The progress of the reaction was monitored by MS until no more starting material was evident. The reaction mixture was concentrated to obtain the desired product (51 mg). MS (ESI): exact mass calculated for C₂₀H₂₀ClN₃, 337.13; found, *m*/*z* 338.2 [M+H]⁺. ¹H NMR (500 MHz, CD₃OD): 7.56-7.53 (m, 2H), 7.51-7.48 (m, 2H), 7.38-7.29 (m, 3H), 7.20-7.19 (m, 2H), 5.48 (s, 2H), 3.42-3.37 (m, 4H), 3.20-3.18 (m, 2H), 3.10-3.08 (m, 2H).

An alternative method as outlined in Scheme 5 is shown below:
Step F. 3-(4-Chloro-phenyl)-1,4,6,7-tetrahydro-indazol-5-[1,3]dioxolane. The desired compound (5.0 g) was prepared from 5.0 g of 1,4-dioxa-spiro[4.5]decan-8-one, 4.5 mL of 4-chloro-benzoyl chloride and 3.0 mL of hydrazine according to the procedure outlined in Step C above. ¹H NMR (500 MHz, CDCl₃): 7.53-7.50 (m, 2H), 7.36-7.33 (m, 2H), 4.02 (s, 4H), 2.91 (s, 2H), 2.89 (t, J = 6.6 Hz, 2H), 2.01 (t, J = 6.6 Hz, 2H).
Step G. 1-Benzyl-3-(4-chloro-phenyl)-1,4,6,7-tetrahydro-indazol-5-[1,3]dioxolane. The desired compound (3.93 g) was prepared from 4.0 g of the compound from step F as outlined in Step D, using benzyl bromide (1.9 mL) in place of benzyl chloride and K₂CO₃ (6.1 g) in place of NaH. ¹H NMR (500 MHz, CDCl₃): 7.67-7.64 (m, 2H), 7.39-7.27 (m, 5H), 7.21-7.18 (m, 2H), 5.29 (s, 2H), 4.05-3.98 (m, 4H), 2.95 (s, 2H), 2.71 (t, J = 6.6 Hz, 2H), 1.98 (t, J = 6.6 Hz, 2H).
Step H. 1-Benzyl-3-(4-chloro-phenyl)-1,4,6,7-tetrahydro-indazol-5-one oxime. A solution of 3.87 g of the compound from step G in 80 mL of THF with 5 mL of 1 M HCl was heated at reflux for 16 h. The volatiles were removed *in vacuo* and water was added (300 mL). The mixture was adjusted to pH 9 by the addition of 1 M NaOH and then was extracted with CH₂Cl₂. The combined extracts were washed with brine and the solvent was removed *in vacuo* to provide 1-benzyl-3-(4-chloro-phenyl)-1,4,6,7-tetrahydro-indazol-5-one. This product (3.13 g) was treated with hydroxylamine hydrochloride (3.0 g) in 20 mL of pyridine. The reaction mixture was stirred at RT for 14 h then was diluted with water (300 mL), and stirred an additional hour. The mixture was filtered on paper and the solids were washed with EtOAc and dried *in vacuo* to afford 2.48 g of the desired compound. ¹H NMR (500 MHz, acetone-d₆): 10.24 (s, 1 H), 7.30-7.26 (m, 2H), 7.06-7.02 (m, 2H), 6.91-9.87 (m, 2H), 6.85-6.81 (m, 1 H), 6.77-6.73 (m, 2H), 4.87 (s, 2H), 3.21 (s, 2H), 2.31 (t, J = 6.6 Hz, 2H), 2.09 (t, J = 6.6 Hz, 2H).
Step I. 1-Benzyl-3-(4-chloro-phenyl)-1,4,5,6,7,8-hexahydro-1,2,6-triaza-azulene. A solution of the compound from step H (78.2 mg) in 15 mL of CH₂Cl₂ was cooled to 0 °C and diisobutylaluminum hydride (1.5 M in toluene, 0.75 mL) was added. The mixture was allowed to warm to RT and was stirred for 12 h. Water (0.2 mL) and NaF (0.40 g) were added and the mixture was stirred for 1 h. The mixture was filtered through diatomaceous earth and the filtrate was concentrated to afford 66.7 mg of a mixture of the title compound and 1-benzyl-3-(4-chloro-phenyl)-1,4,5,6,7,8-hexahydro-1,2,5-triaza-azulene.
   MS (ESI): exact mass calculated for C₂₀H₂₀ClN₃, 337.13; found, *m*/*z* 338.0 [M+H]⁺.

Example 60 through 102 were prepared using the procedures described in Example 59, Steps D and E, unless otherwise noted.

### Example 60

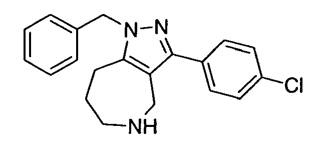

1-Benzyl-3-(4-chloro-phenyl)-1,4,5,6,7,8-hexahydro-1,2,5-triaza-azulene.
The title compound (0.068 g) was prepared as in Example 59, Steps D and E, starting with 3-(4-chloro-phenyl)-4,6,7,8-tetrahydro-1*H*-1,2,5-triaza-azulene-5-carboxylic acid *tert*-butyl ester (0.1 g), the isomer from Example 59, Step C. The reaction sequence also yielded 2-benzyl-3-(4-chloro-phenyl)-2,6,7,8-tetrahydro-4H-1,2,5-triaza-azulene-5-carboxylic acid tert-butyl ester. MS (ESI): exact mass calculated for C₂₀H₂₀CIN₃, 337.13; found, *m*/*z* 338.2 [M+H]⁺. ¹H NMR (500 MHz, CD₃OD): 7.51-7.30 (m, 4H), 7.37-7.29 (m, 3H), 7.29-7.21 (m, 3H), 5.45 (s, 2H), 4.32 (s, 2H), 3.53-3.50 (m, 2H), 3.06-3.03 (m, 2H), 2.04-1.99 (m, 2H).

### Example 61

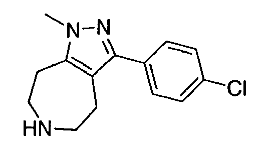

3-(4-Chloro-phenyl)-1-methyl-1,4,5,6,7,8-hexahydro-1,2,6-triaza-azulene.
The title compound (0.028 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.1 g) using methyl iodide (0.21 mL) in place of benzyl chloride. The reaction sequence also yielded 3-(4-chloro-phenyl)-2-methyl-4,5,7,8-tetrahydro-2*H*-1,2,6-triaza-azulene-6-carboxylic acid *tert-*butyl ester in the alkylation step. MS (ESI): exact mass calculated for C₁₄H₁₆CIN₃, 261.10; found, *m*/*z* 262.1 [M+H]⁺. ¹H NMR (500 MHz, CD₃OD): 7.69-7.65 (m, 4H), 4.07 (s, 3H), 3.69-3.67 (m, 2H), 3.58-3.56 (m, 2H), 3.44-3.42 (m, 2H), 3.05-3.04 (m, 2H).

### Example 62

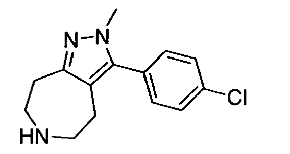

3-(4-Chloro-phenyl)-2-methyl-2,4,5,6,7,8-hexahydro-1,2,6-triaza-azulene.
The title compound (0.011 g) was prepared from 3-(4-chloro-phenyl)-2-methyl-4,5,7,8-tetrahydro-2*H*-1,2,6-triaza-azulene-6-carboxylic acid *tert*-butyl ester (Example 61) according to Example 59, Step E. MS (ESI): exact mass calculated for C₁₄H₁₆ClN₃, 261.10; found, *m*/*z* 262.1 [M+H]⁺. ¹H NMR (500 MHz, CD₃OD): 7.48-7.45 (m, 2H), 7.28-7.26 (m, 2H), 3.60 (s, 3H), 3.31-3.29 (m, 2H), 3.21 (m, 2H), 3.04-3.02 (m, 2H), 2.72-2.70 (m, 2H).

### Example 63

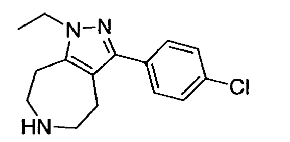

3-(4-Chloro-phenyl)-1-ethyl-1,4,5,6,7,8-hexahydro-1,2,6-triaza-azulene.
The title compound (0.035 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.1 g) using ethyl iodide (0.27 mL) in place of benzyl chloride. The reaction sequence also yielded 3-(4-chloro-phenyl)-2-ethyl-4,5,7,8-tetrahydro-2*H*-1,2,6-triaza-azulene-6-carboxylic acid *tert*-butyl ester in the alkylation step. MS (ESI): exact mass calculated for C₁₅H₁₈ClN₃, 275.12; found, m/z 276.1 [M+H]⁺. ¹H NMR (500 MHz, CD₃OD): 7.39-7.34 (m, 4H), 7.11 (q, *J* = 7.3 Hz, 2H), 3.38-3.36 (m, 2H), 3.27-3.24 (m, 2H), 3.15-3.13 (m, 2H), 2.94-2.92 (m, 2H), 1.30 (t, J = 7.3 Hz, 3H).

### Example 64

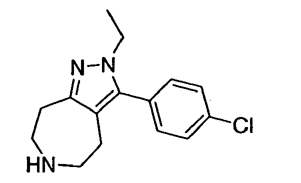

3-(4-Chloro-phenyl)-2-ethyl-2,4,5,6,7,8-hexahydro-1,2,6-triaza-azulene.
The title compound (0.021 g) was prepared from 3-(4-chloro-phenyl)-2-ethyl-4,5,7,8-tetrahydro-2H 1,2,6-triaza-azulene-6-carboxylic acid *tert-*butyl ester (Example 63) according to Example 59, Step E. MS (ESI): exact mass calculated for C₁₅H₁₈CIN₃, 275.12; found, m/z 276.2 [M+H]⁺. ¹H NMR (500 MHz, CD₃OD): 7.46 (d, *J* = 8.6 Hz, 2H), 7.24 (d, *J* = 8.6 Hz, 2H), 3.90 (q, *J* = 7.2 Hz, 2H), 3.31-3.29 (m, 2H), 3.20-3.19 (m, 2H), 3.06-3.04 (m, 2H), 2.69-2.67 (m, 2H), 1.17 (t, J = 7.2 Hz, 3H).

### Example 65

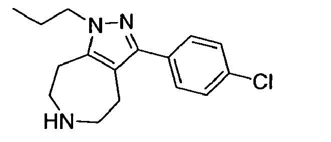

3-(4-Chloro-phenyl)-1-propyl-1,4,5,6,7,8-hexahydro-1,2,6-triaza-azulene.
The title compound (0.031 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.1 g) using 1-iodopropane (0.33 mL) in place of benzyl chloride. The reaction sequence also yielded 3-(4-chloro-phenyl)-2-propyl-4,5,7,8-tetrahydro-2H-1,2,6-triaza-azulene-6-carboxylic acid *tert*-butyl ester in the alkylation step. MS (ESI): exact mass calculated for C₁₆H₂₀ClN₃, 289.13; found, *m*/*z* 290.2 [M+H]⁺. ¹H NMR (500 MHz, CD₃OD): 7.39-7.35 (m, 4H), 4.03 (t, *J* = 7.2 Hz, 2H), 3.37-3.35 (m, 2H), 3.27-3.24 (m, 2H), 3.15-3.13 (m, 2H), 2.95-2.93 (m, 2H), 1.76-1.69 (m, 2H), 0.84 (t, *J* = 7.4 Hz, 3H).

### Example 66

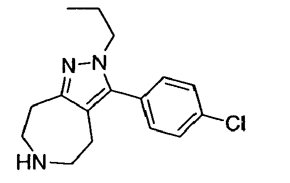

3-(4-Chloro-phenyl)-2-propyl-2,4,5,6,7,8-hexahydro-1,2,6-triaza-azulene.
The title compound (0.016 g) was prepared from 3-(4-chloro-phenyl)-2-propyl-4,5,7,8-tetrahydro-2*H*-1,2,6-triaza-azulene-6-carboxylic acid *tert*-butyl ester (Example 65) according to Example 59, Step E. MS (ESI): exact mass calculated for C₁₆H₂₀ClN₃, 289.13; found, *m*/*z* 290.2 [M+H]⁺. ¹H NMR (500 MHz, CD₃OD): 7.45 (d, *J* = 8.5 Hz, 2H), 7.23 (d, *J* = 8.5 Hz, 2H), 3.83 (d, *J* = 7.2 Hz, 2H), 3.30-3.28 (m, 2H), 3.20-3.19 (m, 2H), 3.05-3.03 (m, 2H), 2.68-2.66 (m, 2H), 1.62-1.18 (m, 2H), 0.65 (t, *J* = 7.4 Hz, 3H).

### Example 67

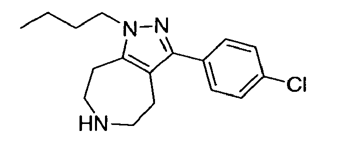

1-Butyl-3-(4-chloro-phenyl)-1,4,5,6,7,8-hexahydro-1,2,6-triaza-azulene.
The title compound (0.033 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.1 g) using 1-iodobutane (0.038 mL) in place of benzyl chloride. The reaction sequence also yielded 2-butyl-3-(4-chloro-phenyl)-4,5,7,8-tetrahydro-2*H*-1,2,6-triaza-azulene-6-carboxylic acid *tert*-butyl ester in the alkylation step. MS (ESI): exact mass calculated for C₁₇H₂₂CIN₃, 303.15; found, *m*/*z* 304.2 [M+H]⁺. ¹H NMR (500 MHz, CD₃OD): 7.39-7.34 (m, 4H), 4.07 (t, *J* = 7.2 Hz, 2H), 3.37-3.35 (m, 2H), 3.27-3.25 (m, 2H), 3.14-3.12 (m, 2H), 2.95-2.92 (m, 2H), 1.69-1.66 (m, 2H), 1.22-1.20 (m, 2H), 0.86 (t, *J* = 7.4 Hz, 3H).

### Example 68

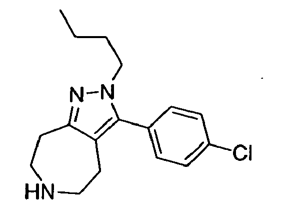

2-Butyl-3-(4-chloro-phenyl)-2,4,5,6,7,8-hexahydro-1,2,6-triaza-azulene.
The title compound (0.018 g) was prepared from 2-butyl-3-(4-chloro-phenyl)-4,5,7,8-tetrahydro-2*H*-1,2,6-triaza-azulene-6-carboxylic acid *tert*-butyl ester (Example 67) according to Example 59, Step E. MS (ESI): exact mass calculated for C₁₇H₂₂ClN₃ 303.15; found, *m*/*z* 304.2 [M+H]⁺. ¹H NMR (500 MHz, CD₃OD): 7.46 (d, *J* = 8.4 Hz, 2H), 7.25 (d, *J* = 8.4 Hz, 2H), 3.91-3.88 (m, 2H), 3.32-3.30 (m, 2H), 3.21-3.19 (m, 2H), 3.07-3.05 (m, 2H), 2.70-2.68 (m, 2H), 1.58-1.52 (m, 2H), 1.06-1.03 (m, 2H), 0.68 (t, *J* = 7.4 Hz, 3H).

### Example 69

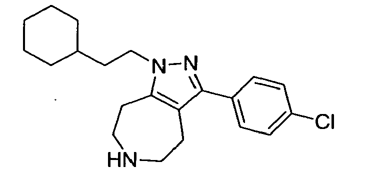

3-(4-Chloro-phenyl)-1-(2-cyclohexyl-ethyl)-1,4,5,6,7,8-hexahydro-1,2,6-triaza-azulene.
The title compound (0.056 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.1 g) using 1-bromo-2-cyclohexylethane (0. 053 mL) in place of benzyl chloride. The reaction sequence also yielded 3-(4-chloro-phenyl)-2-(2-cyclohexyl-ethyl)-4,5,7,8-tetrahydro-2*H*-1,2,6-triaza-azulene-6-carboxylic acid *tert*-butyl ester in the alkylation step. MS (ESI): exact mass calculated for C₂₁H₂₈ClN₃, 357.20; found, *m*/*z* 358.2 [M+H]⁺. ¹H NMR (500 MHz, CD₃OD): 7.46-7.34 (m, 4H), 4.08 (t, *J* = 7.6 Hz, 2H), 3.37-3.35 (m, 2H), 3.27-3.25 (m, 2H), 3.13-3.11 (m, 2H), 2.94-2.92 (m, 2H), 1.68-1.20 (m, 7H), 1.18-1.05 (m, 4H), 0.93-0.86 (m, 2H).

### Example 70

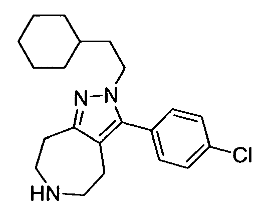

3-(4-Chloro-phenyl)-2-(2-cyclohexyl-ethyl)-2,4,5,6,7,8-hexahydro-1,2,6-triaza-azulene.
The title compound (0.026 g) was prepared from 3-(4-chloro-phenyl)-2-(2-cyclohexyl-ethyl)-4,5,7,8-tetrahydro-2H-1,2,6-triaza-azulene-6-carboxylic acid *tert*-butyl ester (Example 69) according to Example 59, Step E. MS (ESI): exact mass calculated for C₂₁H₂₈ClN₃, 357.20; found, *m*/*z* 358.2 [M+H]⁺. ¹H NMR (500 MHz, CD₃OD): 7.46 (d, *J* = 8.5 Hz, 2H), 7.23 (d, *J* = 8.5 Hz, 2H), 3.90 (t, *J* = 7.3 Hz, 2H), 3.30-3.28 (m, 2H), 3.20-3.19 (m, 2H), 3.05-3.03 (m, 2H), 2.69-2.67 (m, 2H), 1.48-1.41 (m, 5H), 1.36-1.33 (m, 2H), 1.03-1.00 (m, 3H), 0.95-0.89 (m, 1 H), 0.81-0.67 (m, 2H).

### Example 71

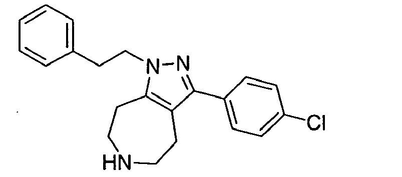

3-(4-Chloro-phenyl)-1-phenethyl-1,4,5,6,7,8-hexahydro-1,2,6-triaza-azulene.
The title compound (0.048 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.1 g) using (2-chloroethyl)benzene (0.045 mL) in place of benzyl chloride. The reaction sequence also yielded 3-(4-chloro-phenyl)-2-phenethyl-4,5,7,8-tetrahydro-2*H*-1,2,6-triaza-azulene-6-carboxylic acid *tert*-butyl ester in the alkylation step. MS (ESI): exact mass calculated for C₂₁H₂₂ClN₃, 351.15; found, *m*/*z* 352.2 [M+H]⁺. ¹H NMR (500 MHz, CD₃OD): 7.52-7.47 (m, 4H), 7.28-7.21 (m, 3H), 7.03-7.01 (m, 2H), 4.39 (t, *J* = 6.4 Hz, 2H), 3.20-3.18 (m, 2H), 3.13-3.10 (m, 2H), 2.95-2.93 (m, 2H), 2.91-2.89 (m, 2H), 2.69-2.67 (m, 2H).

### Example 72

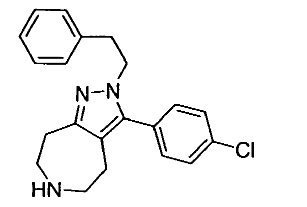

3-(4-Chloro-phenyl)-2-phenethyl-2,4,5,6,7,8-hexahydro-1,2,6-triaza-azulene.
The title compound (0.020 g) was prepared from 3-(4-chloro-phenyl)-2-phenethyl-4,5,7,8-tetrahydro-2*H*-1,2,6-triaza-azulene-6-carboxylic acid *tert-*butyl ester (Example 71) according to Example 59, Step E. MS (ESI): exact mass calculated for C₂₁H₂₂ClN₃, 351.15; found, *m*/*z* 352.2 [M+H]⁺. ¹H NMR (500 MHz, CD₃OD): 7.38-7.36 (m, 2H), 7.19-7.14 (m, 3H), 6.83-6.79 (m, 4H), 4.14 (t, *J* = 6.7 Hz, 2H), 3.41-3.39 (m, 2H), 3.26-3.24 (m, 2H), 3.19-3.17 (m, 2H), 3.01-2.98 (m, 2H), 2.69-2.67 (m, 2H).

### Example 73

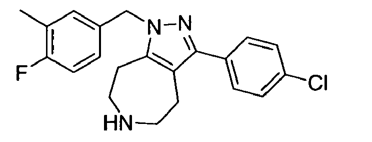

3-(4-Chloro-phenyl)-1-(4-fluoro-3-methyl-benzyl)-1,4,5,6,7,8-hexahydro-1,2,6-triaza-azulene.
The title compound (0.002 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.1 g) using 4-fluoro-3-methylbenzyl bromide (0.09 g) in place of benzyl chloride. MS (ESI): exact mass calculated for C₂₁H₂₁ClFN₃, 369.14; found, *m*/*z* 370.1 [M+H]⁺. ¹H NMR (500 MHz, CD₃OD): 7.49-7.47 (m, 2H), 7.45-7.42 (m, 2H), 7.07-7.01 (m, 1 H), 7.00-6.95 (m, 1 H), 6.95-6.90 (m, 1 H), 5.31 (s, 2H), 2.97-2.91 (m, 4H), 2.89-2.84 (m, 2H), 2.82-2.77 (m, 2H), 2.22 (s, 3H).

### Example 74

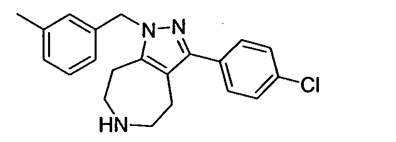

3-(4-Chloro-phenyl)-1-(3-methyl-benzyl)-1,4,5,6,7,8-hexahydro-1,2,6-triaza-azulene.
The title compound (0.004 g) was prepared from 3-(4-chloro-phenyl)-4,5,7,8-tetrahydro-1 *H*-1,2,6-triaza-azulene-6-carboxylic acid *tert-*butyl ester (Example 59, Step C; 0.1 g) using 3-methylbenzyl chloride (0.6 mL) in place of benzyl chloride. MS (ESI): exact mass calculated for C₂₁H₂₂ClN₃, 351.15; found, *m*/*z* 352.2 [M+H]⁺. ¹H NMR (500 MHz, CD₃OD): 7.31-7.26 (m, 2H), 7.26-7.21 (m, 2H), 6.99 (t, J = 7.5 Hz, 1 H), 6.88 (d, J = 7.1 Hz, 1 H), 6.76 (s, 1 H), 6.67 (d, J = 7.1 Hz, 1 H), 5.13 (s, 2H), 2.79-2.73 (m, 4H), 2.69-2.65 (m, 2H), 2.63-2.60 (m, 2H), 2.09 (s, 3H).

### Example 75

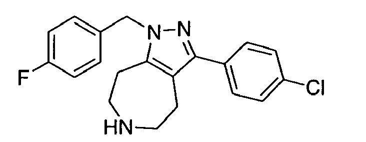

3-(4-Chloro-phenyl)-1-(4-fluoro-benzyl)-1,4,5,6,7,8-hexahydro-1,2,6-triaza-azulene.
The title compound (0.003 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.1 g) using 4-fluorobenzyl chloride (0.5 mL) in place of benzyl chloride. MS (ESI): exact mass calculated for C₂₀H₁₉ClFN₃, 355.13; found, *m*/*z*356.2 [M+H]⁺. ¹H NMR (500 MHz, CD₃OD): 7.40-7.37 (m, 2H), 7.35-7.32 (m, 2H), 7.08-7.04 (m, 2H), 6.98-6.94 (m, 2H), 5.26 (s, 2H), 2.89-2.86 (m, 4H), 2.80-2.78 (m, 2H), 2.73-2.70 (m, 2H).

### Example 76

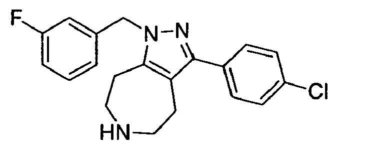

3-(4-Chloro-phenyl)-1-(3-fluoro-benzyl)-1,4,5,6,7,8-hexahydro-1,2,6-triaza-azulene.
The title compound (0.01 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.1 g) using 3-fluorobenzyl chloride (0.5 mL) in place of benzyl chloride. MS (ESI): exact mass calculated for C₂₀H₁₉ClFN₃, 355.13; found, *m*/*z*356.1 [M+H]⁺. ¹H NMR (500 MHz, CD₃OD): 7.42-7.37 (m, 2H), 7.35-7.32 (m, 2H), 7.28-7.21 (m, 1 H), 6.93-6.88 (m, 1 H), 6.84 (d, J = 7.7 Hz, 1 H), 6.75-6.71 (m, 1 H), 5.29 (s, 2H), 2.89-2.85 (m, 4H), 2.79-2.76 (m, 2H), 2.74-2.70 (m, 2H).

### Example 77

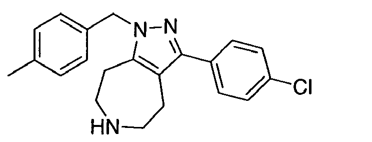

3-(4-Chloro-phenyl)-1-(4-methyl-benzyl)-1,4,5,6,7,8-hexahydro-1,2,6-triaza-azulene.
The title compound (0.013 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.74 g) using 4-methylbenzyl chloride (0.45 g) in place of benzyl chloride. The reaction sequence also yielded 3-(4-chloro-phenyl)-2-(4-methylbenzyl)-4,5,7,8-tetrahydro-2*H*-1,2,6-triaza-azulene-6-carboxylic acid *tert*-butyl ester in the alkylation step. MS (ESI): exact mass calculated for C₂₁H₂₂ClN₃, 351.15; found, *m*/*z* 352.2 [M+H]⁺. ¹H NMR (500 MHz, CD₃OD): 7.39-7.36 (m, 2H), 7.34-7.31 (m, 2H), 7.03 (d, J = 8.0 Hz, 2H), 6.90 (d, J = 8.0 Hz, 2H), 5.21 (s, 2H), 2.83-2.67 (m, 4H), 2.75-2.72 (m, 2H), 2.69-2.67 (m, 2H), 2.20 (s, 3H).

### Example 78

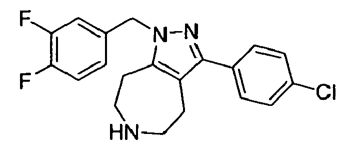

3-(4-Chloro-phenyl)-1-(3,4-difluoro-benzyl)-1,4,5,6,7,8-hexahydro-1,2,6-triaza-azulene.
The title compound (0.002 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.07 g) using 3,4-difluorobenzyl bromide (0.4 mL) in place of benzyl chloride. The reaction sequence also yielded 3-(4-chloro-phenyl)-2-(3,4-difluoro-benzyl)-4,5,7,8-tetrahydro-2*H*-1,2,6-triaza-azulene-6-carboxylic acid *tert*-butyl ester in the alkylation step. MS (ESI): exact mass calculated for C₂₀H₁₈CIF₂N₃, 373.12; found, *m*/*z*374.1 [M+H]⁺. ¹H NMR (500 MHz, CD₃OD): 7.41-7.38 (m, 2H), 7.36-7.33 (m, 2H), 7.22-7.20 (m, 1 H), 7.07-7.02 (m, 1 H), 6.96-6.92 (m, 1H), 5.26 (s, 2H), 3.06-3.02 (m, 4H), 2.94-2.91 (m, 2H), 2.87-2.84 (m, 2H).

### Example 79

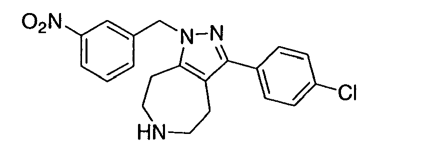

3-(4-Chloro-phenyl)-1-(3-nitro-benzyl)-1,4,5,6,7,8-hexahydro-1,2,6-triaza-azulene.
The title compound (0.005 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.1 g) using 3-nitrobenzyl bromide (0.09 g) in place of benzyl chloride and Cs₂CO₃ (0.2 g) in place of NaH. MS (ESI): exact mass calculated for C₂₀H₁₉CIN₄O₂, 382.12; found, *m*/*z* 383.1 [M+H]⁺. ¹H NMR (500 MHz, CD₃OD): 8.07-8.05 (m, 1 H), 7.91-7.87 (m, 1 H), 7.50 (t, J = 7.9 Hz, 1 H), 7.44-7.38 (m, 3H), 7.36-7.33 (m, 2H), 5.41 (s, 2H), 2.88-2.85 (m, 4H), 2.82-2.79 (m, 2H),2.73-2.71 (m, 2H).

### Example 80

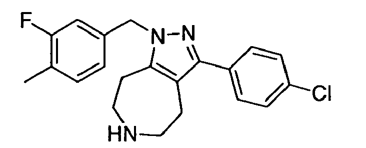

3-(4-Chloro-phenyl)-1-(3-fluoro-4-methyl-benzyl)-1,4,5,6,7,8-hexahydro-1,2,6-triaza-azulene.
The title compound (0.003 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.1 g) using 3-fluoro-4-methylbenzyl bromide (0.9 g) in place of benzyl chloride. MS (ESI): exact mass calculated for C₂₁H₂₁ClFN₃, 369.14; found, *m*/*z* 370.1 [M+H]⁺. ¹H NMR (500 MHz, CD₃OD): 7.50-7.47 (m, 2H), 7.44-7.42 (m, 2H), 7.19 (t, J = 7.6 Hz, 1 H), 6.84-6.81 (m, 1 H), 6.78-6.75 (m, 1H), 5.33 (s, 2H), 2.95-2.92 (m, 4H), 2.87-2.84 (m, 2H), 2.81-2.78 (m, 2H), 2.22 (s, 3H).

### Example 81

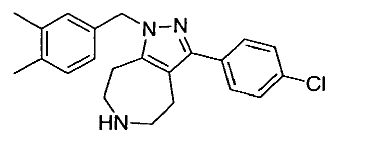

3-(4-Chloro-phenyl)-1-(3,4-dimethyl-benzyl)-1,4,5,6,7,8-hexahydro-1,2,6-triaza-azulene.
The title compound (0.003 g) was prepared from 3-(4-chloro-phenyl)-4,5,7,8-tetrahydro-1 *H*-1,2,6-triaza-azulene-6-carboxylic acid *tert*-butyl ester (Example 59, Step C; 0.1 g) using 3,4-dimethylbenzyl chloride (0.6 mL) in place of benzyl chloride. MS (ESI): exact mass calculated for C₂₂H₂₄CIN₃, 365.17; found, *m*/*z* 366.2 [M+H]⁺. ¹H NMR (500 MHz, CD₃OD): 7.40-7.38 (m, 2H), 7.35-7.32 (m, 2H), 6.98-6.96 (m, 1 H), 6.90-6.86 (m, 1 H), 6.72-6.69 (m, 1 H), 5.30 (s, 1 H), 5.19 (s, 1 H), 2.90-2.87 (m, 2H), 2.86-2.82 (m, 2H), 2.77-2.73 (m, 2H), 2.72-2.68 (m, 2H), 2.21 (s, 3H), 2.17 (s, 3H).

### Example 82

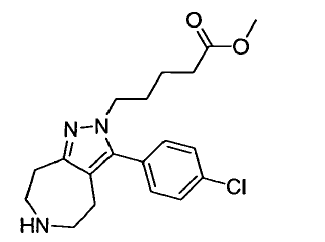

5-[3-(4-Chloro-phenyl)-5,6,7,8-tetrahydro-4*H*-1,2,6-triaza-azulen-2-yl]-pentanoic acid methyl ester.
The title compound (0.0042 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.15 g) using methyl 5-chlorovalerate (0.90 mL) in place of benzyl chloride. The reaction sequence also yielded 3-(4-chloro-phenyl)-1-(4-methoxycarbonyl-butyl)-4,5,7,8-tetrahydro-1*H*-1,2,6-triaza-azulene-6-carboxylic acid *tert*-butyl ester in the alkylation step. MS (ESI): exact mass calculated for C₁₉H₂₄ClN₃O₂, 361.16; found, *m*/*z* 362.2 [M+H]⁺. ¹H NMR (500 MHz, CD₃OD): 7.54-7.51 (m, 2H), 7.31-7.29 (m, 2H), 3.95 (t, *J*= 7.0 Hz, 2H), 3.60 (s, 3H), 3.03-3.01 (m, 2H), 2.93-2.91 (m, 4H), 2.56-2.53 (m, 2H), 2.16 (t, J= 7.4 Hz, 2H), 1.67-1.62 (m, 2H), 1.41-1.38 (m, 2H).

### Example 83

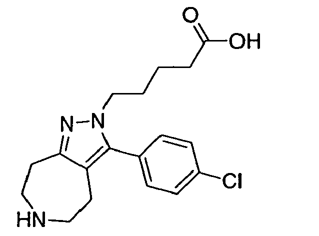

5-[3-(4-Chloro-phenyl)-5,6,7,8-tetrahydro-4*H*-1,2,6-triaza-azulen-2-yl]-pentanoic acid.
5-[3-(4-Chloro-phenyl)-5 ,6,7,8-tetrahydro-4*H*-1 ,2,6-triaza-azulen-2-yl]-pentanoic acid methyl ester (Example 82, 0.009 g) was dissolved in 1 mL of 9:1 THF/MeOH and treated with 2 mL of 1 M NaOH. After stirring at RT for 5 h, the solvent was removed *in vacuo.* The aqueous residue was acidified with 1 mL of 1 N HCl and the mixture was extracted with EtOAc (3x). The combined organic layers were dried over Na₂SO₄, concentrated, and dried on a vacuum line. The residue was then dissolved in 1 mL of 9:1 CH₂Cl₂/MeOH and treated with 3 mL of 1 N HCl in Et₂O. After 4 h, the volatiles were removed *in vacuo.* The crude oil was purified by preparative TLC (9:1 CH₂Cl₂/2 M NH₃ in MeOH) to afford 0.002 g of the title compound. MS (ESI): exact mass calculated for C,₁₈H₂₂CIN₃O₂, 347.14; found, *m*/*z* 348.1 [M+H]⁺. ¹H NMR (500 MHz, CD₃OD): 7.55-7.53 (m, 4H), 7.35-7.32 (m, 2H), 3.98 (t, J = 7.0 Hz, 2H), 3.38-3.35 (m, 2H), 3.28-3.26 (m, 2H), 3.13-3.10 (m, 2H), 2.77-2.74 (m, 2H), 2.09-2.06 (m, 2H), 1.71-1.66 (m, 2H), 1.41-1.37 (m, 2H).

### Example 84

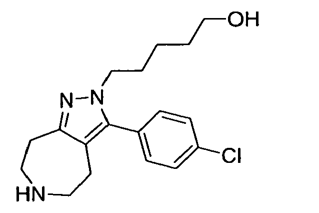

5-[3-(4-Chloro-phenyl)-5,6,7,8-tetrahydro-4*H*-1,2,6-triaza-azulen-2-yl]-pentan-1-ol.
5-[3-(4-Chloro-phenyl)-5,6,7,8-tetrahydro-4*H*-1,2,6-triaza-azulen-2-yl]-pentanoic acid methyl ester (Example 82, 0.009 g) was dissolved in 9:1 Et₂O/CH₂Cl₂ (3 mL) and the solution was added slowly to a stirred suspension of lithium aluminum hydride (2 mg) in 5 mL of anhydrous Et₂O. After stirring at RT for 6 h, the reaction was quenched with 2 mL of water. The mixture was treated with 2 mL of 1 N NaOH, followed by another 2 mL of water. The mixture was then filtered through diatomaceous earth. The organic layer was separated, dried over MgSO₄, and concentrated. After further drying via vacuum line, the resulting oil was dissolved in 2 mL of 9:1 CH₂Cl₂/MeOH and treated with 3 mL of 1 N HCl in Et₂O. After 4 h, the volatiles were removed *in vacuo.* The crude oil was purified by preparative TLC (9:1 CH₂Cl₂/2 M NH₃ in MeOH) to afford 0.001 g of the title compound as a colorless oil. MS (ESI): exact mass calculated for C₁₈H₂₄ClN₃O, 333.16; found, *m*/*z* 334.2 [M+H]⁺. ¹H NMR (500 MHz, CD₃OD): 7.54-7.52 (m, 2H), 7.32-7.30 (m, 2H), 3.95 (t, *J* = 7.1 Hz, 2H), 3.44 (t, J = 6.6 Hz, 2H), 3.13-3.09 (m, 2H), 3.03-3.00 (m, 2H), 2.98-2.95 (m, 2H), 2.61-2.58 (m, 2H), 1.70-1.64 (m, 2H), 1.40-1.35 (m, 2H), 1.20-1.16 (m, 2H).

### Example 85

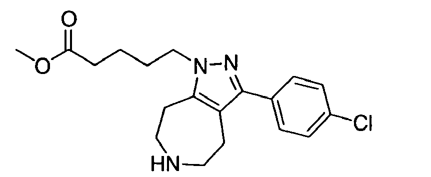

5-[3-(4-Chloro-phenyl)-5,6,7,8-tetrahydro-4*H*-1,2,6-triaza-azulen-1-yl]-pentanoic acid methyl ester.
The title compound (0.0051 g) was prepared from 3-(4-chloro-phenyl)-1-(4-methoxycarbonyl-butyl)-4,5,7,8-tetrahydro-1*H*-1,2,6-triaza-azulene-6-carboxylic acid *tert*-butyl ester (Example 82) according to Example 59, Step E. MS (ESI): exact mass calculated for C₁₉H₂₄CIN₃O₂, 361.16; found, m/z 362.2 [M+H]⁺. ¹H NMR (500 MHz, CD₃OD): 7.45-7.40 (m, 4H), 4.13 (t, *J* = 7.0 Hz, 2H), 3.63 (s, 3H), 3.04-3.03 (m, 2H), 2.97-2.95 (m, 4H), 2.79-2.76 (m, 2H), 2.34 (t, J = 7.4 Hz, 2H), 1.81-1.77 (m, 2H), 1.61-1.58 (m, 2H).

### Example 86

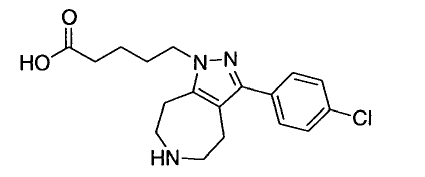

5-[3-(4-Chloro-phenyl)-5,6,7,8-tetrahydro-4*H*-1,2,6-triaza-azulen-1-yl]-pentanoic acid.
5-[3-(4-Chloro-phenyl)-5,6,7,8-tetrahydro-4*H*-1,2,6-triaza-azulen-1-yl]-pentanoic acid methyl ester (Example 85, 0.014 g) was hydrolyzed and de-protected as in Example 83 to afford the title compound (0.0014 g). MS (ESI): exact mass calculated for C₁₈H₂₂ClN₃O₂, 347.14; found, *m*/*z* 348.0 [M+H]⁺. ¹H NMR (500 MHz, CD₃OD): 7.49-7.43 (m, 4H), 4.18 (t, *J* = 7.0 Hz, 2H), 3.45-3.43 (m, 2H), 3.25-3.22 (m, 2H), 3.17-3.16 (m, 2H), 3.04-3.01 (m, 2H), 2.28-2.24 (m, 2H), 1.84-1.82 (m, 2H), 1.60-1.57 (m, 2H).

### Example 87

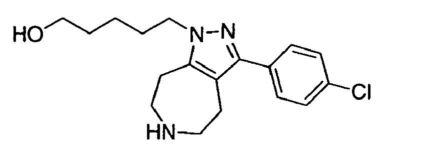

5-[3-(4-Chloro-phenyl)-5,6,7,8-tetrahydro-4H-1,2,6-triaza-azulen-1-yl]-pentan-1-ol.
5-[3-(4-Chloro-phenyl)-5,6,7,8-tetrahydro-4H-1,2,6-triaza-azulen-1 -yl]-pentanoic acid methyl ester (Example 85, 0.015 g) was reduced as in Example 84 to afford the title compound (0.0063 g) as a colorless oil. MS (ESI): exact mass calculated for C₁₈H₂₄ClN₃O, 333.16; found, *m*/*z* 334.1 [M+H]⁺. ¹H NMR (500 MHz, CD₃OD): 7.45-7.40 (m, 4H), 4.13 (t, *J*= 7.2 Hz, 2H), 3.53 (t, *J*= 6.4 Hz, 2H), 3.04-3.01 (m, 2H), 2.97-2.92 (m, 4H), 2.78-2.75 (m, 2H), 1.82-1.75 (m, 2H), 1.57-1.51 (m, 2H), 1.41-1.34 (m, 2H).

### Example 88

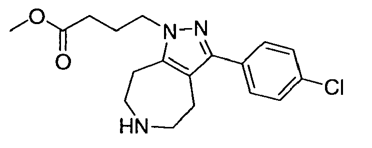

4-[3-(4-Chloro-phenyl)-5,6,7,8-tetrahydro-4*H*-1,2,6-triaza-azulen-1-yl]-butyric acid methyl ester.
The title compound (0.003 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.1 g) using methyl 4-chlorobutyrate (0.8 mL) in place of benzyl chloride. The reaction sequence also yielded 3-(4-chloro-phenyl)-2-(3-methoxycarbonyl-propyl)-4,5,7,8-tetrahydro-2H-1,2,6-triaza-azulene-6-carboxylic acid *tert*-butyl ester in the alkylation step. MS (ESI): exact mass calculated for C₁₈H₂₂ClN₃O₂, 347.14; found, *m*/*z* 348.1 [M+H]⁺. ¹H NMR (500 MHz, CD₃OD): 7.37-7.31 (m, 4H), 4.07 (t, J = 6.6 Hz, 2H), 3.52 (s, 3H), 2.97-2.94 (m, 2H), 2.88-2.84 (m, 4H), 2.70-2.66 (m, 2H), 2.25 (t, J = 6.6 Hz, 2H), 1.98-1.95 (m, 2H).

### Example 89

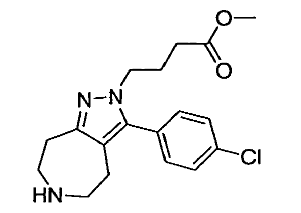

4-[3-(4-Chloro-phenyl)-5,6,7,8-tetrahydro-4*H*-1,2,6-triaza-azulen-2-yl]-butyric acid methyl ester.
The title compound (0.003 g) was prepared from 3-(4-chloro-phenyl)-2-(3-methoxycarbonyl-propyl)-4,5,7,8-tetrahydro-2*H*-1,2,6-triaza-azulene-6-carboxylic acid *tert*-butyl ester (Example 88) according to Example 59, Step E. MS (ESI): exact mass calculated for C₁₈H₂₂ClN₃O₂, 347.14; found, *m*/*z* 348.2 [M+H]⁺. ¹H NMR (400 MHz, CD₃OD): 7.45-7.42 (m, 2H), 7.23-7.20 (m, 2H), 3.91 (t, J = 6.9 Hz, 2H), 3.44 (s, 3H), 3.04-3.00 (m, 2H), 2.93-2.86 (m, 4H), 2.52-2.49 (m, 2H), 2.07 (t, J = 7.0 Hz, 2H), 1.88-1.80 (m, 2H).

### Example 90

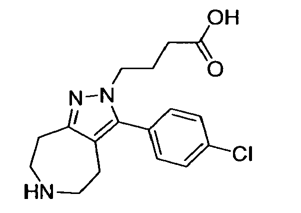

4-[3-(4-Chloro-phenyl)-5,6,7,8-tetrahydro-4*H*-1,2,6-triaza-azulen-2-yl]-butyric acid.
4-[3-(4-Chloro-phenyl)-5,6,7,8-tetrahydro-4H-1 ,2,6-triaza-azulen-2-yl]-butyric acid methyl ester (Example 89, 0.006 g) was hydrolyzed as in Example 83 to afford the title compound (0.005 g). MS (ESI): exact mass calculated for C₁₇H₂₀ClN₃O₂, 333.12; found, *m*/*z* 334.1 [M+H]⁺. ¹H NMR (400 MHz, CD₃OD): 7.55-7.52 (m, 2H), 7.35-7.33 (m, 2H), 3.98 (t, J = 6.8 Hz, 2H), 3.12-3.09 (m, 2H), 3.03-2.96 (m, 4H), 2.62-2.59 (m, 2H), 2.03-1.97 (m, 4H).

### Example 91

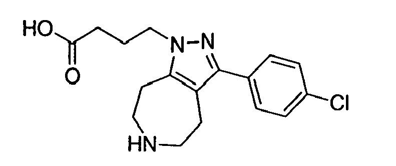

4-[3-(4-Chloro-phenyl)-5,6,7,8-tetrahydro-4H-1,2,6-triaza-azulen-1 -yl]-butyric acid.
4-[3-(4-Chloro-phenyl)-5,6,7,8-tetrahydro-4*H*-1,2,6-triaza-azulen-1-yl]-butyric acid methyl ester (Example 88, 0.009 g) was hydrolyzed as in Example 83 to afford the title compound (0.003 g). MS (ESI): exact mass calculated for C₁₇H₂₀ClN₃O₂, 333.12; found, *m*/*z* 334.1[M+H]⁺, *m*/*z* 332.0 [M-H]⁻. ¹H NMR (400 MHz, CD₃OD): 7.46-7.44 (m, 4H), 4.17 (t, J = 7.1 Hz, 2H), 3.11-3.08 (m, 2H), 3.05-2.98 (m, 4H), 2.83-2.79 (m, 2H), 2.18-2.15 (m, 2H), 2.07-2.03 (m, 2H).

### Example 92

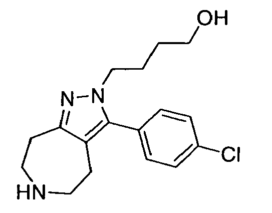

4-[3-(4-Chloro-phenyl)-5,6,7,8-tetrahydro-4*H*-1,2,6-triaza-azulen-2-yl]-butan-1-ol.
4-[3-(4-Chloro-phenyl)-5,6,7,8-tetrahydro-4*H*-1,2,6-triaza-azulen-2-yl]-butyric acid methyl ester (Example 89, 0.006 g) was reduced as in Example 84 to afford the title compound as a white solid (0.001 g). MS (ESI): exact mass calculated for C₁₇H₂₂ClN₃O, 319.15; found, *m*/*z* 320.2 [M+H]⁺. ¹H NMR (400 MHz, CD₃OD): 7.45-7.41 (m, 2H), 7.22-7.20 (m, 2H), 3.89-3.84 (m, 2H), 3.32-3.29 (m, 2H), 2.94-2.91 (m, 2H), 2.85-2.81 (m, 4H), 2.47-2.43 (m, 2H), 1.63-1.58 (m, 2H), 1.24-1.17 (m, 2H).

### Example 93

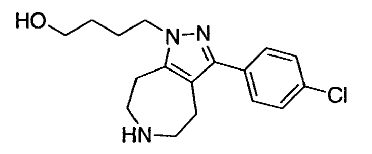

4-[3-(4-Chloro-phenyl)-5,6,7,8-tetrahydro-4*H*-1,2,6-triaza-azulen-1-yl]-butan-1-ol.
4-[3-(4-Chloro-phenyl)-5,6,7,8-tetrahydro-4*H*-1,2,6-triaza-azulen-1-yl]-butyric acid methyl ester (Example 88, 0.02g) was reduced and de-protected as in Example 84 to afford the title compound as a white solid (0.007 g). MS (ESI): exact mass calculated for C₁₇H₂₂ClN₃O, 319.15; found, *m*/*z* 320.2 [M+H]⁺. ¹H NMR (400 MHz, CD₃OD): 7.36-7.31 (m, 4H), 4.05 (t, J= 7.2 Hz, 2H), 3.45 (t, J = 6.4 Hz, 2H), 2.96-2.94 (m, 2H), 2.89-2.84 (m, 4H), 2.70-2.66 (m, 2H), 1.77-1.68 (m, 2H), 1.46-1.39 (m, 2H).

### Example 94

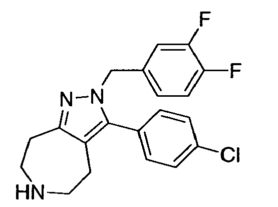

3-(4-Chloro-phenyl)-2-(3,4-difluoro-benzyl)-2,4,5,6,7,8-hexahydro-1,2,6-triaza-azulene.
The title compound (0.001 g) was prepared from 3-(4-chloro-phenyl)-2-(3,4-difluoro-benzyl)-4,5,7,8-tetrahydro-2*H*-1,2,6-triaza-azulene-6-carboxylic acid *tert*-butyl ester (Example 78) according to Example 59, Step E. MS (ESI): exact mass calculated for C₂₀H₁₈ClF₂N₃, 373.12; found, *m*/*z*374.1 [M+H]⁺. ¹H NMR (500 MHz, CD₃OD): 7.41-7.36 (m, 2H), 7.15-7.10 (m, 2H), 7.07-7.01 (m, 1H), 6.77-6.72 (m, 1H), 6.65-6.62 (m, 1H), 5.06 (s, 2H), 3.17-3.15 (m, 2H), 09-3.05 (m, 2H), 2.99-2.97 (m, 2H), 2.62-2.59 (m, 2H).

### Example 95

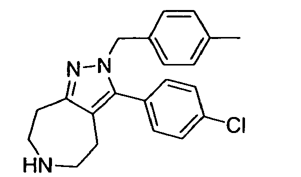

3-(4-Chloro-phenyl)-2-(4-methyl-benzyl)-2,4,5,6,7,8-hexahydro-1,2,6-triaza-azulene.
The title compound (0.005 g) was prepared from 3-(4-chloro-phenyl)-2-(4-methyl-benzyl)-4,5,7,8-tetrahydro-2*H*-1,2,6-triaza-azulene-6-carboxylic acid *tert*-butyl ester (Example 77) according to Example 59, Step E. MS (ESI): exact mass calculated for C₂₁H₂₂ClN₃, 351.15; found, *m*/*z* 352.2 [M+H]⁺. ¹H NMR (500 MHz, CD₃OD): 7.36-7.33 (m, 2H), 7.10-7.01 (m, 2H), 6.95 (d, J= 7.8 Hz, 2H), 6.69 (d, J = 8.0 Hz, 2H), 5.01 (s, 2H), 2.92-2.90 (m, 2H), 2.83-2.80 (m, 4H), 2.46-2.43 (m, 2H), 2.16 (s, 3H).

### Example 96

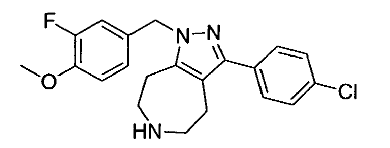

3-(4-Chloro-phenyl)-1-(3-fluoro-4-methoxy-benzyl)-1,4,5,6,7,8-hexahydro-1,2,6-triaza-azulene.
The title compound (0.021 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.35 g) using 3-fluoro-4-methoxybenzyl bromide (0.25 g) in place of benzyl chloride. The reaction sequence also provided 3-(4-chloro-phenyl)-2-(3-fluoro-4-methoxy-benzyl)-4,5,7,8-tetrahydro-2*H*-1,2,6-triaza-azulene-6-carboxylic acid *tert*-butyl ester in the alkylation step. MS (ESI): exact mass calculated for C₂₁H₂₁ClFN₃O, 385.14; found, *m*/*z* 386.1 [M+H]⁺. ¹H NMR (500 MHz, CD₃OD): 7.50-7.47 (m, 2H), 7.47-7.42 (m, 2H), 7.06-7.02 (m, 1 H), 6.90-6.86 (m, 2H), 5.29 (s, 2H), 3.84 (s, 3H), 3.35-3.29 (m, 2H), 2.94-2.92 (m, 4H), 2.88-2.85 (m, 2H), 2.80-2.77 (m, 2H). ¹³C NMR (125 MHz, CD₃OD): 154.2, 152.2, 149.1, 148.1, 143.5, 134.2, 132.9, 131.1, 131.0, 130.5, 129.1, 123.3, 118.7, 115.0, 114.8, 114.4, 56.2, 52.4, 49.9, 28.8, 27.3.

### Example 97

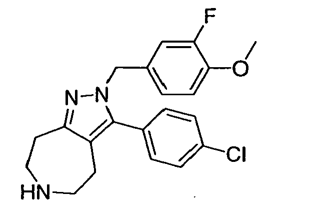

3-(4-Chloro-phenyl)-2-(3-fluoro-4-methoxy-benzyl)-2,4,5,6,7,8-hexahydro-1,2,6-triaza-azulene.
The title compound (0.017 g) was prepared from 3-(4-chloro-phenyl)-2-(3-fluoro-4-methoxy-benzyl)-4,5,7,8-tetrahydro-2*H*-1,2,6-triaza-azulene-6-carboxylic acid *tert*-butyl ester (Example 96) according to Example 59, Step E. MS (ESI): exact mass calculated for C₂₁H₂₁ClFN₃O, 385.14; found, *m*/*z* 386.0 [M+H]⁺. ¹H NMR (500 MHz, CD₃OD): 7.48-7.45 (m, 2H), 7.21-7.18 (m, 2H), 6.95-6.92 (m, 1 H), 6.67-6.63 (m, 2H), 5.08 (s, 2H), 3.81 (s, 3H), 3.31-3.30 (m, 2H), 3.01-2.98 (m, 2H), 2.94-2.88 (m, 4H), 2.55-2.52 (m, 2H). ¹³C NMR (125 MHz, CD₃OD): 154.9, 153.8, 153.0, 148.9, 142.5, 136.6, 133.0, 132.1, 130.5, 130.0, 129.4, 124.3, 120.7, 116.0, 115.8, 115.1, 57.1, 53.3, 51.3, 32.7, 28.0.

### Example 98

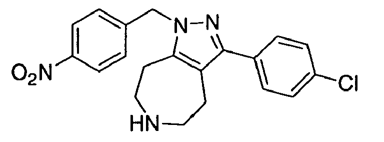

3-(4-Chloro-phenyl)-1-(4-nitro-benzyl)-1,4,5,6,7,8-hexahydro-1,2,6-triaza-azulene.
The title compound (0.004 g) was prepared from 3-(4-chloro-phenyl)-4,5,7,8-tetrahydro-1*H*-1,2,6-triaza-azulene-6-carboxylic acid *tert*-butyl ester (Example 59, Step C; 0.3 g) using 4-nitrobenzyl bromide (0.3 g) in place of benzyl chloride. MS (ESI): exact mass calculated for C₂₀H₁₉CIN₄O₂, 382.12; found, *m*/*z* 383.1 [M+H]⁺. ¹H NMR (400 MHz, CD₃OD): 8.23-8.19 (m, 2H), 7.51-7.47 (m, 2H), 7.45-7.47 (m, 2H), 7.32 (d, J = 8.6 Hz, 2H), 5.52 (s, 2H), 2.98-2.95 (m, 4H), 2.89-2.85 (m, 2H), 2.83-2.79 (m, 2H).

### Example 99

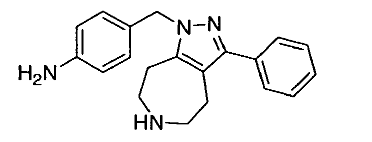

4-(3-Phenyl-5,6,7,8-tetrahydro-4H-1,2,6-triaza-azulen-1 -ylmethyl)-phenylamine. 3-(4-Chloro-phenyl)-1-(4-nitro-benzyl)-4,5,7,8-tetrahydro-1*H-*1,2,6-triaza-azulene-6-carboxylic acid *tert*-butyl ester (Example 98, 70 mg) was dissolved in 25 mL of anhydrous EtOH and treated with 10% palladium on carbon (20 mg).
The mixture was subjected to hydrogen for 4 h at 30 psi. The mixture was filtered through diatomaceous earth. The filtrate was concentrated and dried via vacuum line to afford 55 mg of 1-(4-amino-benzyl)-3-phenyl-4,5,7,8-tetrahydro-1*H*-1,2,6-triaza-azulene-6-carboxylic acid *tert*-butyl ester. The intermediate aniline was then dissolved in 1 mL of MeOH and treated with 5 mL of 1 N HCI in Et₂O. After 6 h, the volatiles were removed *in vacuo.* The resulting yellow semi-solid was purified by preparative TLC (9:1 CH₂Cl₂/2 M NH₃ in MeOH) to afford 0.007 g of the title compound as a light yellow solid. MS (ESI): exact mass calculated for C₂₀H₂₂N₄, 318.18; found, *m*/*z* 319.2 [M+H]⁺. ¹H NMR (500 MHz, CD₃OD): 7.50-7.47 (m, 2H), 7.44-7.41 (m, 2H), 7.37-7.34 (m, 1 H), 6.94-6.90 (m, 2H), 6.68-6.65 (m, 2H), 5.23 (s, 2H), 3.11-3.06 (m, 4H), 2.99-2.96 (m, 2H), 2.91-2.88 (m, 2H).

### Example 100

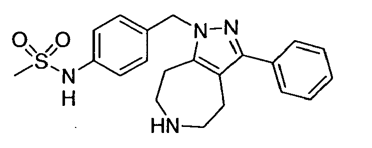

*N*-[4-(3-Phenyl-5,6,7,8-tetrahydro-4H-1,2,6-triaza-azulen-1-ylmethyl)-phenyl]-methanesulfonamide.
To a solution of 0.022 g of 1-(4-amino-benzyl)-3-phenyl-4,5,7,8-tetrahydro-1 *H-*1,2,6-triaza-azulene-6-carboxylic acid *tert*-butyl ester (Example 99) in DMF (1 mL) was added 1 equivalent of triethylamine. After 5 min, 1 equivalent of methanesulfonyl chloride was added and the mixture was stirred overnight. The reaction was quenched with water and extracted with EtOAc (3x). The combined organic layers were dried over Na₂SO₄ and concentrated. The resulting oil was purified by preparative TLC (50% EtOAc/hexanes) to give 1-(4-methanesulfonylamino-benzyl)-3-phenyl-4,5,7,8-tetrahydro-1*H*-1,2,6-triaza-azulene-6-carboxylic acid *tert*-butyl ester. This mono-mesylate was then dissolved in 9:1 CH₂Cl₂/MeOH (2 mL) and treated with 3 mL of 1 N HCl in Et₂O. After 6 h, the volatiles were removed *in vacuo.* The resulting oil was purified by preparative TLC (9:1 CH₂Cl₂/2 M NH₃ in MeOH) to afford 0.004 g of the title compound as a white solid. MS (ESI): exact mass calculated for C₂₁H₂₄N₄O₂S, 396.16; found, *m*/*z* 397.1 [M+H]⁺. ¹H NMR (500 MHz, CD₃OD): 7.50-7.47 (m, 2H), 7.42 (t, J = 7.7 Hz, 2H), 7.37-7.34 (m, 1 H), 7.22-7.20 (m, 2H), 7.13-7.10 (m, 2H), 5.34 (s, 2H), 2.97-2.93 (m, 4H), 2.92 (s, 3H), 2.90-2.87 (m, 2H), 2.82-2.78 (m, 2H).

### Example 101

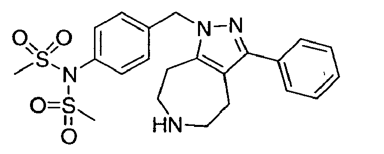

*N*,*N*-[4-(3-phenyl-5,6,7,8-tetrahydro-4*H*-1,2,6-triaza-azulen-1-ylmethyl)-phenyl]-dimethanesulfonamide.
1-(4-Amino-benzyl)-3-phenyl-4,5,7,8-tetrahydro-1*H*-1,2,6-triaza-azulene-6-carboxylic acid *tert*-butyl ester (Example 99, 0.05 mmol) was dissolved in 1 mL of DMF and treated with 1 equivalent of triethylamine. After 5 min, 1.5 equivalents of methanesulfonyl chloride were added and the mixture was stirred overnight. The reaction was quenched with water and extracted with EtOAc (3x). The combined organic layers were dried over Na₂SO₄ and concentrated. The resulting oil was purified by preparative TLC (50% EtOAc/hexanes) to provide 1-(4-dimethanesulfonylamino-benzyl)-3-phenyl-4,5,7,8-tetrahydro-1*H*-1,2,6-triaza-azulene-6-carboxylic acid *tert*-butyl ester. The intermediate was then dissolved in 2 mL of 9:1 CH₂Cl₂/MeOH and treated with 3 mL of 1 N HCl in Et₂O. After 6 h, the volatiles were removed *in vacuo.* The crude oil was purified by preparative TLC (9:1 CH₂Cl₂/2 M NH₃ in MeOH) to afford 0.006 g of the title compound as an off-white solid. MS (ESI): exact mass calculated for C₂₂H₂₆N₄O₄S₂, 474.14; found, *m*/*z* 475.1 [M+H]⁺. ¹H NMR (500 MHz, CD₃OD): 7.50-7.47 (m, 2H), 7.44-7.40 (m, 2H), 7.37-7.35 (m, 1 H), 7.22-7.20 (m, 2H), 7.13-7.11 (m, 2H), 5.34 (s, 2H), 2.97-2.94 (m, 4H), 2.92 (s, 6H), 2.89-2.87 (m, 2H), 2.82-2.80 (m, 2H).

### Example 102

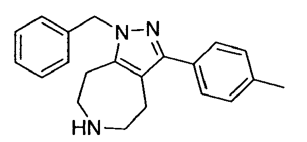

1-Benzyl-3-*p*-tolyl-1,4,5,6,7,8-hexahydro-1,2,6-triaza-azulene.
The title compound (0.2 g) was prepared from 4-oxo-azepane-1-carboxylic acid *tert*-butyl ester (Example 59, Step B; 10 mmol) as in Example 59, Steps C through E, using 4-methyl-benzoyl chloride (11 mmol) in place of 4-chloro-benzoyl chloride. MS (ESI): exact mass calculated for C₂₁H₂₃N₃, 317.19; found, *m*/*z* 318.2 [M+H]⁺. ¹H NMR (500 MHz, CD₃OD): 7.34-7.33 (m, 2H), 7.29-7.26 (m, 2H), 7.24-7.21 (m, 3H), 7.10-7.09 (m, 2H), 5.40 (s, 2H), 3.33-3.27 (m, 4H), 3.11-3.09 (m, 2H), 3.00-2.98 (m, 2H), 2.30 (s, 3H).

### Example 103

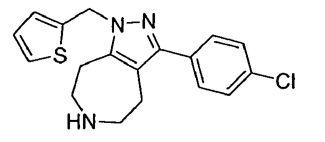

3-(4-Chloro-phenyl)-1-thiophen-2-ylmethyl-1,4,5,6,7,8-hexahydro-1,2,6-triaza-azulene.
Step A. 1-(4-Chlorophenyl)-2-diazo-ethanone. To a solution of diazomethane (33.2 mmmol) in Et₂O (70 mL) was added triethylamine (33. 2 mmol). The mixture was cooled to 0 °C, and 4-chlorobenzoyl chloride (30 mmol) in Et₂O (30 mL) was added slowly. The mixture was then warmed to RT and stirred for 1 h. After filtration of the mixture, the clear filtrate was concentrated to provide the crude desired compound (5.4 g).
Step B. 3-(4-Chloro-phenyl)-4,5.7,8-tetrahydro-1H-1,2,6-triaza-azulene-6-carboxylic acid tert-butyl ester. To a 0 °C mixture of 4-oxo-piperidine 1-carboxylic acid *tert*-butyl ester (20 mmol) in Et₂O (150 mL) was added a solution of BF₃•Et₂O (30 mmol) in Et₂O (150 mL) followed by a solution of the product from Step A (21 mmol) in Et₂O (150 mL). After the addition was complete, the mixture was warmed to 25 °C and stirred for 1 h. Satd. aq. NaHCO₃ (200 mL) was added, and the layers were separated. The organic layer was concentrated, and the resulting residue was diluted with MeOH (100 mL). Hydrazine (3 mL) was added and the mixture was stirred at 25 °C for 16 h. Purification by flash chromatography (EtOAc/CH₂Cl₂) provided the desired compound (1.8 g).
Step C. The product from Step B (0.2 mmol) was mixed with 2-chloromethyl-thiophene (0.3 mmol) in DMF (2 mL), and Cs₂CO₃ (0.3 mmol) was then added. The mixture was stirred at 25 °C for 16 h. After concentration and purification by SiO₂ chromatography (EtOAc/hexanes), 3-(4-chloro-phenyl)-1-thiophen-2-ylmethyl-1,4,5,6,7,8-hexahydro-1,2,6-triaza-azulene was obtained. The intermediate was treated with TFA (1 mL) in CH₂Cl₂ (10 mL) for 4 h. After concentration of the reaction mixture, the title compound was obtained (0.029 g). The reaction sequence also provided 3-(4-chloro-phenyl)-2-thiophen-2-ylmethyl-4,5,7,8-tetrahydro-2*H*-1,2,6-triaza-azulene-6-carboxylic acid *tert*-butyl ester in the alkylation step. MS (ESI): exact mass calculated for C₁₈H₁₈ClN₃O, 343.09; found, *m*/*z* 344.1 [M+H]⁺. ¹H NMR (500 MHz, CD₃OD): 7.46-7.44 (m, 2H), 7.41-7.39 (m, 2H), 7.29 (dd, *J* = 5.1, 1.1 Hz, 1 H), 7.00 (dd, *J* = 3.5. 1.1 Hz, 1 H), 6.91 (dd, J = 5.1, 3.5 Hz, 1 H), 5.52 (s, 2H), 3.36-3.34 (m, 2H), 3.30-3.28 (m, 2H), 3.24-3.18 (m, 2H), 2.99-2.97 (m, 2H).

Example 104 through 155 were prepared using the procedure described in Example 103 unless otherwise noted.

### Example 104

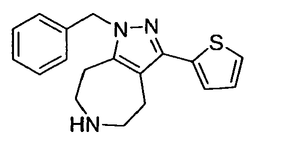

1-Benzyl-3-thiophen-2-yl-1,4,5,6,7,8-hexahydro-1,2,6-triaza-azulene.
The title compound (28 mg) was prepared from 3-thiophen-2-yl-4,5,7,8-tetrahydro-1H-1,2,6-triaza-azulene-6-carboxylic acid tert-butyl ester as described in Example 103, using thiophene-2-carbonyl chloride (5 mmol) in place of 4-chlorobenzoyl chloride, and benzyl chloride (0.3 mmol) in place of 2-chloromethyl-thiophene. MS (ESI): exact mass calculated for C₁₈H₁₉N₃S, 309.13; found, *m*/*z* 310.1 [M+H]⁺. ¹H NMR (500 MHz, CD₃OD): 7.27-7.01 (m, 8H), 5.28 (s, 2H), 3.26-3.24 (br m, 2H), 3.18-3.16 (br m, 2H), 3.11-3.09 (br m, 2H), 2.96-2.94 (br m, 2H).

### Example 105

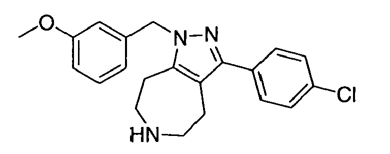

3-(4-Chloro-phenyl)-1-(3-methoxy-benzyl)-1,4,5,6,7,8-hexahydro-1,2,6-triaza-azulene.
The title compound (0.095 g) was prepared from 3-(4-chloro-phenyl)-4,5,7,8-tetrahydro-1*H*-1,2,6-triaza-azulene-6-carboxylic acid *tert*-butyl ester (Example 103, Step B; 1 mmol) using 3-methoxy-benzyl chloride (1.5 mmol) in place of 2-chloromethyl-thiophene. MS (ESI): exact mass calculated for C₂₁H₂₂ClN₃O, 367.15; found, *m*/*z* 368.1 [M+H]⁺. ¹H NMR (500 MHz, CD₃OD): 7.60 (d, J= 8.5 Hz, 2H), 7.56 (d, J = 8.5 Hz, 2H), 7.32 (d, J = 7.9 Hz, 1 H), 6.92 (dd, J = 8.2, 2.1 Hz, 1H),6.84(s, 1 H), 6.80 (d, J = 8.2 Hz, 1 H), 5.57 (s, 2H), 3.79 (s, 3H), 3.48-3.46 (br m, 2H), 3.44-3.42 (br m, 2H), 3.33-3.31 (br m, 2H), 3.16-3.14 (br m,2H).

### Example 106

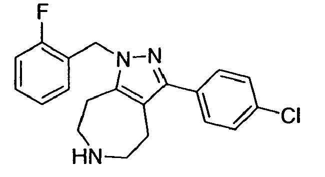

3-(4-Chloro-phenyl)-1-(2-fluoro-benzyl)-1,4,5,6,7,8-hexahydro-1,2,6-triaza-azulene.
The title compound (0.042 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2-fluorobenzyl chloride (0.3 mmol) in place of 2-chloromethyl-thiophene. MS (ESI): exact mass calculated for C₂₀H₁₉ClFN₃, 355.13; found, *m*/*z*356.2 [M+H]⁺. ¹H NMR (500 MHz, CD₃OD): 7.55-7.48 (br m, 4H), 7.39-3.37 (br m, 1 H), 7.20-7.14 (m, 3H), 5.54 (s, 2H), 3.48-3.46 (br m, 2H), 3.40-3.38 (br m, 2H), 3.31-3.29 (br m, 2H), 3.13-3.11 (br m, 2H).

### Example 107

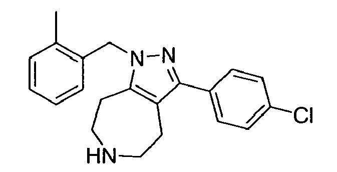

3-(4-Chloro-phenyl)-1-(2-methyl-benzyl)-1,4,5,6,7,8-hexahydro-1,2,6-triaza-azulene.
The title compound (0.03 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2-methylbenzyl chloride (0.3 mmol) in place of 2-chloromethyl-thiophene. The reaction sequence also yielded 3-(4-chlorophenyl)-2-(2-methyl-benzyl)-4,5,7,8-tetrahydro-2*H*-1,2,6-triaza-azulene-6-carboxylic acid *tert*-butyl ester in the alkylation step. MS (ESI): exact mass calculated for C₂₁H₂₂ClN₃, 351.15; found, *m*/*z* 352.2 [M+H]⁺. ¹H NMR (400 MHz, CD₃OD): 7.56 (d, *J =* 8.5 Hz, 2H), 7.49 (d, *J =* 8.5 Hz, 2H), 7.23-7.15 (m, 3H), 6.58 (d, J = 7.5, 1 H), 5.50 (s, 2H), 3.42-3.39 (br m, 4H), 3.15-3.12 (br m, 4H), 2.40 (s, 3H).

### Example 108

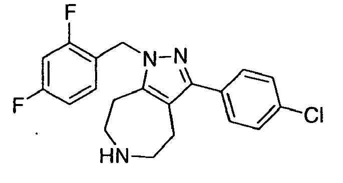

3-(4-Chloro-phenyl)-1-(2,4-difluoro-benzyl)-1,4,5,6,7,8-hexahydro-1,2,6-triaza-azulene.
The title compound (0.030g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2,4-difluorobenzyl bromide (0.3 mmol) in place of 2-chloromethyl-thiophene. The reaction sequence also yielded 3-(4-chlorophenyl)-2-(2,4-difluoro-benzyl)-4,5,7,8-tetrahydro-2*H*-1,2,6-triaza-azulene-6-carboxylic acid *tert*-butyl ester in the alkylation step. MS (ESI): exact mass calculated for C₂₀H₁₈CIF₂N₃, 373.12; found, *m*/*z*374.1 [M+H]⁺. ¹H NMR (400 MHz, CD₃OD): 7.52-7.49 (br m, 4H), 7.27-7.24 (br m, 1 H), 7.01-6.99 (br m, 2H), 5.52 (s, 2H), 3.51-3.49 (br m, 2H), 3.43-3.40 (br m, 2H), 3.34-3.31 (br m, 2H), 3.11-3.09 (br m, 2H).

### Example 109

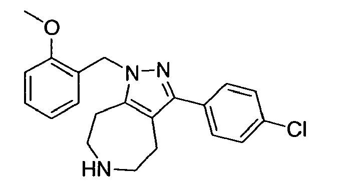

3-(4-Chloro-phenyl)-1-(2-methoxy-benzyl)-1,4,5,6,7,8-hexahydro-1,2,6-triaza-azulene.
The title compound (0.06 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.3 mmol) using 2-methoxybenzyl chloride (0.3 mmol) in place of 2-chloromethyl-thiophene. The reaction sequence also yielded 3-(4-chlorophenyl)-2-(2-methoxy-benzyl)-4,5,7,8-tetrahydro-2*H*-1,2,6-triaza-azulene-6-carboxylic acid *tert*-butyl ester in the alkylation step. MS (ESI): exact mass calculated for C₂₁H₂₂ClN₃O, 367.15; found, *m*/*z* 368.1 [M+H]⁺. ¹H NMR (500 MHz, CDCl₃): 7.45-7.43 (m, 2H), 7.30-7.27 (m, 2H), 7.18-7.17 (m, 1 H), 6.80-6.77 (m, 2H), 6.61-6.59 (m, 1 H), 5.26 (s, 2H), 3.80 (s, 3H), 2.92-2.86 (m, 4H), 2.74-2.69 (m, 4H).

### Example 110

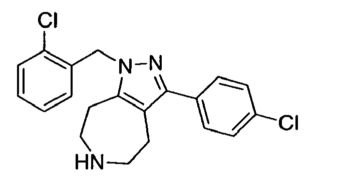

1-(2-Chloro-benzyl)-3-(4-chloro-phenyl)-1,4,5,6,7,8-hexahydro-1,2,6-triaza-azulene.
The title compound (0.01 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2-chlorobenzyl chloride (0.3 mmol) in place of 2-chloromethyl-thiophene. MS (ESI): exact mass calculated for C₁₇H₂₂ClN₃O, 371.10; found, *m*/*z* 372.1 [M+H]⁺. ¹H NMR (500 MHz, CD₃OD): 7.43-7.41 (m, 2H), 7.32-7.30 (m, 2H), 7.32 (d, J = 8.6 Hz, 2H), 6.76 (d, J = 8.6 Hz, 2H), 5.23 (s, 2H), 2.94-2.91 (br m, 4H), 2.79-7.77 (br m, 2H), 2.73-7.71 (br m, 2H).

### Example 111

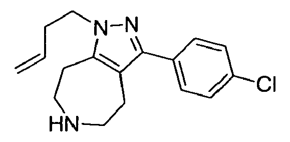

1-But-3-enyl-3-(4-chloro-phenyl)-1,4,5,6,7,8-hexahydro-1,2,6-triaza-azulene.
The title compound (0.028 g) was prepared from 3-(4-chloro-phenyl)-4,5,7,8-tetrahydro-1 *H*-1,2,6-triaza-azulene-6-carboxylic acid *tert*-butyl ester (Example 103, Step B; 0.2 mmol) using 1-but-3-enyl chloride (0.3 mmol) in place of 2-chloromethyl-thiophene. MS (ESI): exact mass calculated for C₁₇H₂₂ClN₃O, 301.13; found, m/z 302.1 [M+H]⁺. ¹H NMR (500 MHz, CDCl₃): 7.40-7.37 (m, 2H), 7.31-7.28 (m, 2H), 6.75-6.67 (m, 1 H), 5.02-5.00 (br m, 2H), 4.07 (t, J = 7.3 Hz, 2H), 2.99-2.97 (br m, 2H), 2.91-2.89 (br m, 2H), 2.80-2.78 (br m, 2H), 2.71-2.69 (br m, 2H), 2.48 (q, J = 7.3 Hz, 2H).

### Example 112

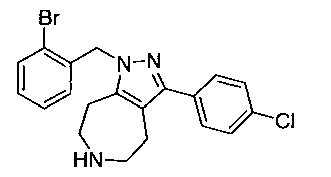

1-(2-Bromo-benzyl)-3-(4-chloro-phenyl)-1,4,5,6,7,8-hexahydro-1,2,6-triaza-azulene.
The title compound (0.035 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2-bromobenzyl bromide (0.3 mmol) in place of 2-chloromethyl-thiophene. MS (ESI): exact mass calculated for C₂₀H₁₉BrClN₃, 415.05; found, *m*/*z* 418.0 [M+H]⁺. ¹H NMR (500 MHz, CD₃OD): 7.69 (d, *J* = 7.8 Hz, 1 H), 7.53-7.27 (m, 6H), 6.78 (d, *J* = 7.8 Hz, 1 H), 5.58 (s, 2H), 3.50-3.48 (br m, 4H), 3.19-3.17 (br m, 4H).

### Example 113

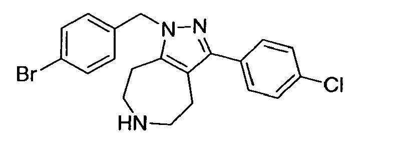

1-(4-Bromo-benzyl)-3-(4-chloro-phenyl)-1,4,5,6,7,8-hexahydro-1,2,6-triaza-azulene.
The title compound (0.032 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.3 mmol) using 4-bromobenzyl bromide (0.3 mmol) in place of 2-chloromethyl-thiophene. MS (ESI): exact mass calculated for C₂₀H₁₉BrClN₃, 415.05; found, *m*/*z* 418.0 [M+H]⁺. ¹H NMR (500 MHz, CD₃OD): 7.48-7.40 (m, 6H), 6.92 (d, J = 8.4 Hz, 2H), 5.28 (s, 2H), 3.32-3.30 (br m, 4H), 3.03-3.01 (br m, 4H).

### Example 114

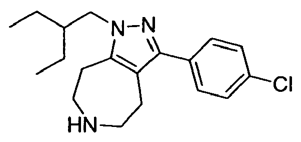

3-(4-Chloro-phenyl)-1-(2-ethyl-butyl)-1,4,5,6,7,8-hexahydro-1,2,6-triaza-azulene.
The title compound (0.010 g) was prepared from 3-(4-chloro-phenyl)-4,5,7,8-tetrahydro-1 *H*-1,2,6-triaza-azulene-6-carboxylic acid *tert*-butyl ester (Example 103, Step B; 0.2 mmol) using 1-bromo-2-ethyl-butane (0.3 mmol) in place of 2-chloromethyl-thiophene. The reaction sequence also yielded 3-(4-chlorophenyl)-2-(2-ethyl-butyl)-4,5,7,8-tetrahydro-2*H*-1,2,6-triaza-azulene-6-carboxylic acid *tert*-butyl ester in the alkylation step. MS (ESI): exact mass calculated for C₁₉H₂₆CIN₃, 331.18; found, *m*/*z* 332.3 [M+H]⁺. ¹H NMR (400 MHz, CD₃OD): 7.50-7.48 (br m, 4H), 4.11-4.09 (br m, 2H), 3.71-3.69 (br m, 2H), 3.33-3.31 (br m, 2H), 3.26-3.24 (br m, 2H), 3.06-3.04 (br m, 2H), 1.91-1.89 (m, 1 H), 1.36-1.34 (m, 4H), 0.93 (t, J = 7.3 Hz, 6H).

### Example 115

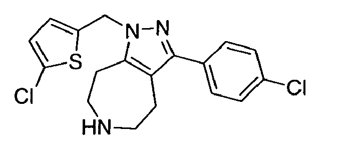

3-(4-Chloro-phenyl)-1-(5-chloro-thiophen-2-ylmethyl)-1,4,5,6,7,8-hexahydro-1,2,6-triaza-azulene.
The title compound (0.029 g) was prepared from 3-(4-chloro-phenyl)-4,5,7,8-tetrahydro-1 *H*-1,2,6-triaza-azulene-6-carboxylic acid *tert*-butyl ester (Example 103, Step B; 0.2 mmol) using 5-chloro-thiophen-2-ylmethyl chloride (0.3 mmol) in place of 2-chloromethyl-thiophene. The reaction sequence also yielded 3-(4-chloro-phenyl)-2-(5-chloro-thiophen-2-ylmethyl)-4,5,7,8-tetrahydro-2*H*-1,2,6-triaza-azulene-6-carboxylic acid *tert*-butyl ester in the alkylation step. MS (ESI): exact mass calculated for C₁₈H₁₇CI₂N₃S, 377.05; found, *m*/*z* 378.0 [M+H] ⁺. ¹H NMR (500 MHz, CD₃OD): 7.54-7.51 (m, 2H), 7.49-7.46 (m, 2H), 3.91 (d, *J* = 3.8 Hz, 1 H), 6.86 (d, *J* = 3.8 Hz, 1 H), 5.51 (s, 2H), 3.45-3.44 (m, 2H), 3.38-3.61 (m, 2H), 3.27-3.25 (m, 2H), 3.07-3.05 (m, 2H).

### Example 116

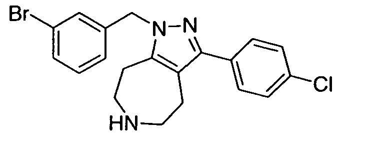

1-(3-Bromo-benzyl)-3-(4-chloro-phenyl)-1,4,5,6,7,8-hexahydro-1,2,6-triaza-azulene.
The title compound (0.04 g) was prepared from 3-(4-chloro-phenyl)-4,5,7,8-tetrahydro-1 *H*-1,2,6-triaza-azulene-6-carboxylic acid *tert-*butyl ester (Example 103, Step B; 0.2 mmol) using 3-bromobenzyl chloride (0.3 mmol) in place of 2-chloromethyl-thiophene. MS (ESI): exact mass calculated for C₂₀H₁₉BrClN₃, 415.05; found, *m*/*z* 416.0 [M+H]⁺. ¹H NMR (500 MHz, CD₃OD): 7.50-6.86 (m, 8H), 5.36 (s, 2H), 3.30-3.27 (br m, 4H), 3.06-3.04 (m, 4H).

### Example 117

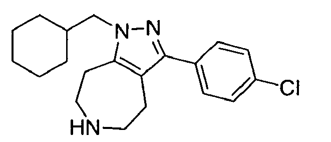

3-(4-Chloro-phenyl)-1-cyclohexylmethyl-1,4,5,6,7,8-hexahydro-1,2,6-triaza-azulene.
The title compound (0.09 g) was prepared from 3-(4-chloro-phenyl)-4,5,7,8-tetrahydro-1*H*-1,2,6-triaza-azulene-6-carboxylic acid *tert*-butyl ester (Example 103, Step B; 170 mg) using cyclohexylmethyl bromide (2 mmol) in place of 2-chloromethyl-thiophene. The reaction sequence also yielded 3-(4-chlorophenyl)-2-cyclohexylmethyl-4,5,7,8-tetrahydro-2*H*-1,2,6-triaza-azulene-6-carboxylic acid *tert*-butyl ester in the alkylation step. MS (ESI): exact mass calculated for C₂₀H₂₆ClN₃, 343.18; found, *m*/*z* 344.3 [M+H]⁺. ¹H NMR (500 MHz, CD₃OD): 7.37 (d, *J* = 6.6 Hz, 2H), 7.32 (d, *J* = 6.6 Hz, 2H), 3.94 (d, *J* = 7.3 Hz, 2H), 3.44-3.40 (br m, 2H), 3.35-3.32 (br m, 2H), 3.17-3.14 (br m, 2H), 3.01-2.99 (br m, 2H), 1.74-1.53 (m, 4 H), 1.52 (d, *J* =11.2 Hz, 2H), 1.17-1.10 (m, 3H), 0.94-0.90 (m, 2H).

### Example 118

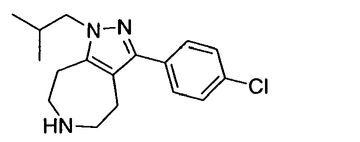

3-(4-Chloro-phenyl)-1-isobutyl-1,4,5,6,7,8-h exahydro-1,2,6-triaza-azulene.
The title compound (0.031 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using isobutyl bromide (0.3 mmol) in place of 2-chloromethyl-thiophene. The reaction sequence also yielded 3-(4-chlorophenyl)-2-isobutyl-4,5,7,8-tetrahydro-2*H*-1,2,6-triaza-azulene-6-carboxylic acid *tert*-butyl ester from the alkylation step. MS (ESI): exact mass calculated for C₁₇H₂₂ClN₃, 303.15; found, *m*/*z* 304.1 [M+H]⁺. ¹H NMR (500 MHz, CD₃OD): 7.64 (d, *J* = 6.6 Hz, 2H), 7.56 (d, *J* = 6.6 Hz, 2H), 4.20 (d, *J* = 7.4 Hz, 2H), 3.72-3.69 (br m, 2H), 3.62-3.60 (br m, 2H), 3.44-3.42 (br m, 2H), 3.29-3.27 (br m, 2H), 2.35 (m, 1 H), 1.14 (d, *J* = 6.7 Hz, 6H).

### Example 119

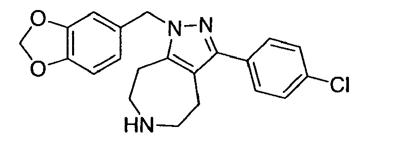

1-Benzo[1,3]dioxol-5-ylmethyl-3-(4-chloro-phenyl)-1,4,5,6,7,8-hexahydro-1,2,6-triaza-azulene.
The title compound (0.035 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using benzo[1,3]dioxol-5-ylmethyl chloride (0.3 mmol) in place of 2-chloromethyl-thiophene. The reaction sequence also yielded 2-benzo[1,3]dioxol-5-ylmethyl-3-(4-chloro-phenyl)-4,5,7,8-tetrahydro-2*H*-1,2,6-triaza-azulene-6-carboxylic acid *tert*-butyl ester in the alkylation step. MS (ESI): exact mass calculated for C₂₁H₂₀ClN₃O₂, 381.12; found, *m*/*z* 382.1 [M+H]⁺. ¹H NMR (500 MHz, CD₃OD): 7.39-7.32 (m, 4H), 6.67-6.65 (m, 1 H), 6.54-6.61 (m, 2H), 5.81 (s, 2H), 5.16 (s, 2H), 2.81-2.79 (m, 4H), 2.76-2.74 (m, 2H), 2.68-2.66 (m, 2H).

### Example 120

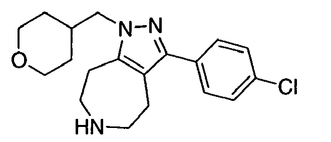

3-(4-Chloro-phenyl)-1-(tetrahydro-pyran-4-ylmethyl)-1,4,5,6,7,8-hexahydro-1,2,6-triaza-azulene.
The title compound was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using toluene-4-sulfonic acid tetrahydro-pyran-4-ylmethyl ester (0.3 mmol) in place of 2-chloromethyl-thiophene. The title compound was obtained as a 2:1 mixture (25 mg) with 3-(4-chloro-phenyl)-2-(tetrahydro-pyran-4-ylmethyl)-2,4,5,6,7,8-hexahydro-1,2,6-triaza-azulene. Data for the mixture: MS (ESI): exact mass calculated for C₁₉H₂₄ClN₃O, 345.16; found, *m*/*z* 346.1 [M+H]⁺. ¹H NMR (500 MHz, CD₃OD): 7.47-7.23 (m, 4H), 4.10-3.78 (m, 4H), 3.37-3.14 (m, 8H), 3.06-2.67 (m, 2H), 2.02-1.93 (m, 1 H), 1.42-0.97 (m, 4H)..

### Example 121

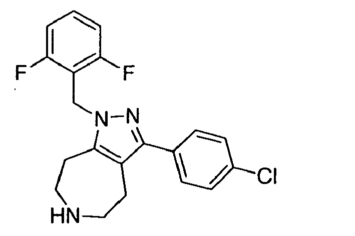

3-(4-Chloro-phenyl)-1-(2,6-difluoro-benzyl)-1,4,5,6,7,8-hexahydro-1,2,6-triaza-azulene.
The title compound (0.07 g) was prepared from 3-(4-chloro-phenyl)-4,5,7,8-tetrahydro-1*H*-1,2,6-triaza-azulene-6-carboxylic acid *tert*-butyl ester (Example 103, Step B; 1 mmol) using 2,6-difluorobenzyl chloride (1.5 mmol) in place of 2-chloromethyl-thiophene. The reaction sequence also yielded 3-(4-chlorophenyl)-2-(2,6-difluoro-benzyl)-4,5,7,8-tetrahydro-2*H*-1,2,6-triaza-azulene-6-carboxylic acid *tert*-butyl ester in the alkylation step. MS (ESI): exact mass calculated for C₂₀H₁₈ClF₂N₃, 373.12; found, *m*/*z*374.1 [M+H]⁺. ¹H NMR (500 MHz, CD₃OD): 7.34-7.30 (m, 5H), 6.93-6.90 (m, 2H), 5.34 (s, 2H), 3.59-3.57 (m, 2H), 3.39-3.37 (m, 4H), 2.94-2.92 (m, 2H).

### Example 122

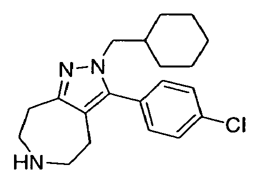

3-(4-Chloro-phenyl)-2-cyclohexylmethyl-2,4,5,6,7,8-hexahydro-1,2,6-triaza-azulene.
The title compound (0.06 g) was prepared from 3-(4-chloro-phenyl)-2-cyclohexylmethyl-4,5,7,8-tetrahydro-2*H*-1,2,6-triaza-azulene-6-carboxylic acid *tert*-butyl ester (Example 117) according to the deprotection method in Example 103, Step C. MS (ESI): exact mass calculated for C₂₀H₂₆ClN₃, 343.18; found, *m*/*z* 344.2 [M+H]⁺. ¹H NMR (500 MHz, CD₃OD): 7.39 (d, J= 8.5 Hz, 2H), 7.31 (d, *J* = 8.5 Hz, 2H), 4.10 (d, J = 7.3 Hz, 2H), 3.49-3.47 (br m, 2H), 3.37-3.35 (br m, 2H), 3.21-3.19 (br m, 2H), 3.03-3.01 (br m, 2H), 1.88-1.61 (m, 4 H), 1.52-1.49 (m, 2H), 1.17-1.10 (m, 3H), 0.94-0.90 (m, 2H).

### Example 123

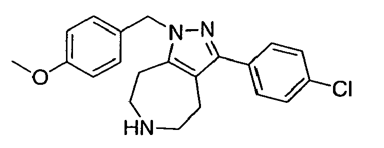

3-(4-Chloro-phenyl)-1-(4-methoxy-benzyl)-1,4,5,6,7,8-hexahydro-1,2,6-triaza-azulene.
The title compound (0.1 g) was prepared from 3-(4-chloro-phenyl)-4,5,7,8-tetrahydro-1*H*-1,2,6-triaza-azulene-6-carboxylic acid *tert*-butyl ester (Example 103, Step B; 1 mmol) using 4-methoxybenzyl chloride (1.5 mmol) in place of 2-chloromethyl-thiophene. MS (ESI): exact mass calculated for C₂₁ H₂₂ClN₃O, 367.15; found, *m*/*z* 368.1 [M+H]⁺. ¹H NMR (400 MHz, CD₃OD): 7.41-7.39 (m, 2H), 7.31 (d, *J* = 7.7 Hz, 2H), 6.70 (d, *J* = 7.7 Hz, 2H), 5.36 (s, 2H), 3.60 (s, 3H), 3.33-3.31 (br m, 2H), 3.21-3.19 (br m, 2H), 3.18-3.16 (br m, 2H), 2.96-2.94 (br m, 2H).

### Example 124

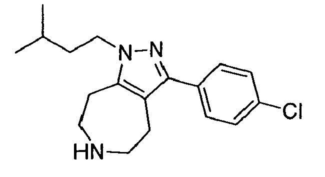

3-(4-Chloro-phenyl)-1-(3-methyl-butyl)-1,4,5,6,7,8-hexahydro-1,2,6-triaza-azulene.

The title compound (0.030 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 1-bromo-3-methyl-butane (0.3 mmol) in place of 2-chloromethyl-thiophene. MS (ESI): exact mass calculated for C₁₈H₂₄ClN₃, 317.17; found, *m*/*z* 318.3 [M+H]⁺. ¹H NMR (400 MHz, CD₃OD): 7.56-7.54 (m, 4H), 4.34 (s, 2H), 3.57-3.55 (br m, 2H), 3.44-3.42 (br m, 2H), 3.40-3.38 (br m, 2H), 3.29-3.27 (br m, 2H), 1.79-1.77 (br m, 1 H), 1.02 (d, J = 4.5 Hz, 6H).

### Example 125

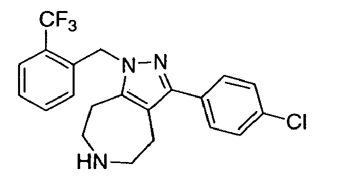

3-(4-Chloro-phenyl)-1-(2-trifluoromethyl-benzyl)-1,4,5,6,7,8-hexahydro-1,2,6-triaza-azulene.
The title compound (0.04 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2-trifluoromethylbenzyl bromide (0.3 mmol) in place of 2-chloromethyl-thiophene. The reaction sequence also yielded 3-(4-chloro-phenyl)-2-(2-trifluoromethyl-benzyl)-4,5,7,8-tetrahydro-2*H*-1,2,6-triaza-azulene-6-carboxylic acid *tert*-butyl ester in the alkylation step. MS (ESI): exact mass calculated for C₂₁H₁₉CIF₃N₃, 405.12; found, *m*/*z* 406.1 [M+H]⁺. ¹H NMR (400 MHz, CD₃OD): 7.45 (d, *J =* 7.7 Hz, 2H), 7.25-7.24 (br m, 3H), 7.18-7.16 (br m, 3H), 6.46-6.44 (br m, 1 H), 5.43-5.41 (s, 2H), 3.14-3.11 (br m, 4H), 2.89-2.87 (br m, 4H).

### Example 126

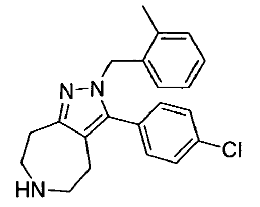

3-(4-Chloro-phenyl)-2-(2-methyl-benzyl)-2,4,5,6,7,8-hexahydro-1,2,6-triaza-azulene.
The title compound (0.020 g) was prepared from 3-(4-chloro-phenyl)-2-(2-methyl-benzyl)-4,5,7,8-tetrahydro-2*H*-1,2,6-triaza-azulene-6-carboxylic acid *tert-*butyl ester (Example 107) according to the deprotection method in Example 103, Step C. MS (ESI): exact mass calculated for C₂₁H₂₂ClN₃, 351.15; found, *m*/*z* 352.2 [M+H]⁺. ¹H NMR (400 MHz, CD₃OD): 7.47 (d, J= 8.4 Hz, 2H), 7.24 (d, J = 8.4 Hz, 2H), 7.15 (m, 3H), 6.60 (d, J = 7.6 Hz, 1 H), 5.23 (s, 2H), 3.46-3.44 (m, 2H), 3.36-3.34 (m, 2H), 3.21-3.19 (m , 2H), 2.89-2.87 (m, 2H), 2.13 (s, 3H).

### Example 127

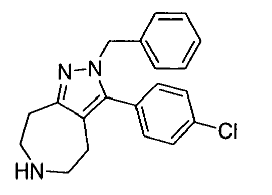

2-Benzyl-3-(4-chloro-phenyl)-2,4,5,6,7,8-hexahydro-1,2,6-triaza-azulene.
The title compound (0.018 g) was prepared from 2-benzyl-3-(4-chloro-phenyl)-4,5,7,8-tetrahydro-2*H*-1,2,6-triaza-azulene-6-carboxylic acid *tert*-butyl ester (Example 59, Step D) according to the deprotection method in Example 103, Step C. MS (ESI): exact mass calculated for C₂₀H₂₀ClN₃, 337.13; found, m/z 338.1 [M+H]⁺. ¹H NMR (500 MHz, CD₃OD): 7.30-7.28 (m, 2H), 7.20-7.15 (m, 3H), 7.03-7.01 (m, 2H), 6.91-6.89 (m, 2H), 5.06 (s, 2H), 3.03-3.01 (m, 2H), 2.94-2.90 (m, 4H), 2.51-2.49 (m, 2H).

### Example 128

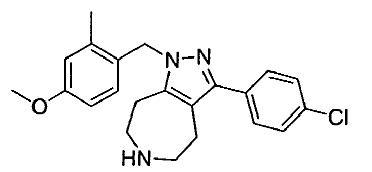

3-(4-Chloro-phenyl)-1-(4-methoxy-2-methyl-benzyl)-1,4,5,6,7,8-hexahydro-1,2,6-triaza-azulene.
To a solution of 3-(4-chloro-phenyl)-4,5,7,8-tetrahydro-1*H*-1,2,6-triaza-azulene-6-carboxylic acid *tert*-butyl ester (Example 103, Step B; 0.4 mmol) in toluene (3 mL) was added 4-methoxy-2-methyl-benzyl chloride (0.9 mmol) and cyanomethylene-tri-*n*-butylphosphorane (1 mmol). The mixture was heated at 110 °C for 16 h. After concentration and purification (SiO₂, EtOAc/hexanes), 3-(4-chloro-phenyl)-1-(4-methoxy-2-methyl-benzyl)-1,4,5,6,7,8-hexahydro-1,2,6-triaza-azulene-6-carboxylic acid *tert*-butyl ester was obtained (54 mg). The other regioisomer, 3-(4-chloro-phenyl)-2-(4-methoxy-2-methyl-benzyl)-1,4,5,6,7,8-hexahydro-1,2,6-triaza-azulene-6-carboxylic acid *tert*-butyl ester, was also obtained (86 mg). 3-(4-Chloro-phenyl)-1-(4-methoxy-2-methylbenzyl)-1,4,5,6,7,8-hexahydro-1,2,6-triaza-azulene-6-carboxylic acid *tert*-butyl ester (20 mg) was treated with TFA (1 mL) in CH₂Cl₂ (10 mL) for 4 h. After concentration of the reaction mixture, the title compound was obtained (0.02 g). MS (ESI): exact mass calculated for C₂₂H₂₄ClN₃O, 381.16; found, *m*/*z* 382.1 [M+H]⁺. ¹H NMR (500 MHz, CD₃OD): 7.39-7.37 (m, 2H), 7.34-7.32 (m, 2H), 6.68-6.66 (br m, 1 H), 6.54-6.52 (br m, 1 H), 6.37 (d, J = 8.3 Hz, 1 H), 5.21 (s, 2H), 2.81-2.79 (m, 4H), 2.71-2.69 (m, 4H).

### Example 129

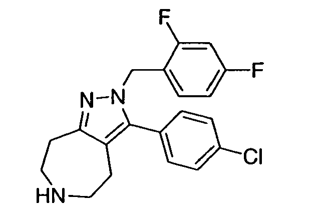

3-(4-Chloro-phenyl)-2-(2,4-difluoro-benzyl)-2,4,5,6,7,8-hexahydro-1,2,6-triaza-azulene.
The title compound (0.016 g) was prepared from 3-(4-chloro-phenyl)-2-(2,4-difluoro-benzyl)-4,5,7,8-tetrahydro-2*H*-1,2,6-triaza-azulene-6-carboxylic acid *tert*-butyl ester (Example 108; 0.2 mmol) according to the deprotection method in Example 103, Step C. MS (ESI): exact mass calculated for C₂₀H₁₈ClF₂N₃, 373.12; found, *m*/*z*374.1 [M+H]⁺. ¹H NMR (400 MHz, CD₃OD): 7.54 (d, J= 8.0 Hz, 2H), 7.49 (d, J = 8.0 Hz, 2H), 6.96-6.88 (m, 3H), 5.27 (s, 2H), 3.46-3.44 (br m, 2H), 3.34-3.32 (br m, 2H), 3.23-3.21 (br m, 2H), 2.86-2.84 (br m, 2H).

### Example 130

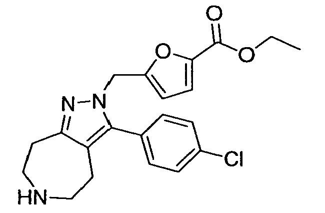

5-[3-(4-Chloro-phenyl)-5,6,7,8-tetrahydro-4*H*-1,2,6-triaza-azulen-2-ylmethyl]-furan-2-carboxylic acid ethyl ester.
The title compound (0.008 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 5-chloro-furan-2-carboxylic acid ethyl ester (0.3 mmol) in place of 2-chloromethyl-thiophene. The reaction sequence also provided 3-(4-chloro-phenyl)-1-(5-ethoxycarbonyl-furan-2-ylmethyl)-4,5,7,8-tetrahydro-1*H*-1,2,6-triaza-azulene-6-carboxylic acid *tert*-butyl ester in the alkylation step. MS (ESI): exact mass calculated for C₂₁H₂₂ClN₃O₃, 399.13; found, *m*/*z* 400.2 [M+H]⁺. ¹H NMR (500 MHz, CD₃OD): 7.44 (d, *J* = 8.4 Hz, 2H), 7.30 (d, *J* = 8.4 Hz, 2H), 7.00 (d, *J* = 3.5 Hz, 1 H), 6.21 (d, *J* = 3.5 Hz, 1 H), 5.09 (s, 2H), 4.21 (q, J = 7.1 Hz, 2H), 3.21-3.19 (m, 2H), 3.11-3.09 (m, 2H), 2.96-2.94 (m, 2H), 2.61-2.59 (m, 2H), 1.24 (t, J = 7.1 Hz, 2H).

### Example 131

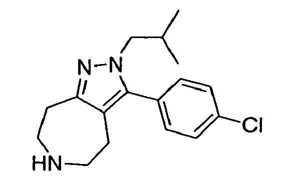

3-(4-Chloro-phenyl)-2-isobutyl-2,4,5,6,7,8-hexahydro-1,2,6-triaza-azulene.
The title compound (0.010 g) was prepared from 3-(4-chloro-phenyl)-2-isobutyl-4,5,7,8-tetrahydro-2*H*-1,2,6-triaza-azulene-6-carboxylic acid *tert*-butyl ester (Example 118, Step C) according to the deprotection method from Example 103, Step C. MS (ESI): exact mass calculated for C₁₇H₂₂ClN₃, 303.15; found, *m*/*z* 304.1 [M+H]⁺. ¹H NMR (400 MHz, CD₃OD): 7.58-7.56 (m, 2H), 7.37-7.34 (m, 2H), 3.81 (d, *J* = 7.5 Hz, 2H), 3.42-3.40 (m, 2H), 3.34-3.30 (m, 2H), 3.18-3.15 (m, 2H), 2.81-2.78 (m, 2H), 2.02-2.00 (m, 1 H), 0.74 (d, J= 6.7 Hz, 6H).

### Example 132

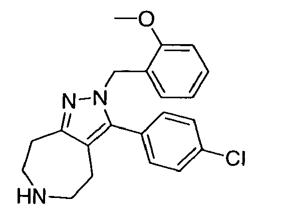

3-(4-Chloro-phenyl)-2-(2-methoxy-benzyl)-2,4,5,6,7,8-hexahydro-1,2,6-triaza-azulene.
The title compound (0.040 g) was prepared from 3-(4-chloro-phenyl)-2-(2-methoxy-benzyl)-4,5,7,8-tetrahydro-2*H*-1,2,6-triaza-azulene-6-carboxylic acid *tert*-butyl ester (Example 109) according to the deprotection method in Example 103, Step C. MS (ESI): exact mass calculated for C₂₁H₂₂CIN₃O₃, 399.13; found, *m*/*z* 368.2 [M+H]⁺. ¹H NMR (500 MHz, CDCl₃): 7.26 (d, *J* = 6.5 Hz, 2H), 7.12 (t, J = 7.6 Hz, 1 H), 7.03 (d, J = 6.5 Hz, 2H), 6.80 (t, J = 7.6 Hz, 1 H), 6.71 (d, J = 7.6 Hz, 1 H), 6.62 (d, J = 7.6 Hz, 1 H), 5.08 (s, 2H), 2.99-2.97 (m, 2H), 2.89-2.87 (m, 4H), 2.49-2.47 (m, 2H).

### Example 133

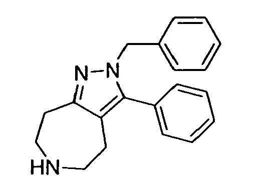

2-Benzyl-3-phenyl-2,4,5,6,7,8-hexahydro-1,2,6-triaza-azulene.
To a solution of 2-benzyl-3-(4-chloro-phenyl)-4,5,7,8-tetrahydro-2*H*-1,2,6-triaza-azulene-6-carboxylic acid *tert*-butyl ester (0.1 mmol) (Example 59, Step D) in THF (25 mL) was added lithium aluminum hydride (100 mg). The mixture was heated at reflux for 4 h. Water (1 mL) was added, the mixture was filtered, and the filtrate was concentrated. After purification (SiO₂, EtOAc/hexanes), 2-benzyl-3-phenyl-2,4,5,6,7,8-hexahydro-1,2,6-triaza-azulene-6-carboxylic acid *tert*-butyl ester was obtained. The intermediate was diluted with CH₂Cl₂ (5 mL) and TFA (1 mL) was added. The reaction mixture was stirred at RT for 4 h. The reaction mixture was concentrated to obtain the title compound (0.018 g). MS (ESI): exact mass calculated for C₂₀H₂₁N₃, 303.17; found, *m*/*z* 304.3 [M+H]⁺. ¹H NMR (400 MHz, CD₃OD): 7.38-7.35 (m, 3H), 7.19-7.18 (m, 3H), 7.12-7.10 (m, 2H), 5.13 (s, 2H), 3.35-3.21 (br m, 6H), 3.34-3.30 (br m, 2H), 2.81-2.79 (br m, 2H).

### Example 134

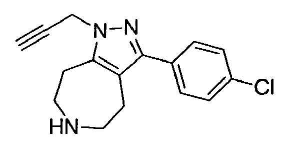

3-(4-Chloro-phenyl)-1-prop-2-ynyl-1,4,5,6,7,8-hexahydro-1,2,6-triaza-azulene. The title compound (0.014 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2-propynyl chloride (0.3 mmol) in place of 2-chloromethyl-thiophene. MS (ESI): exact mass calculated for C₁₆H₁₆CIN₃, 285.10; found, *m*/*z* 286.2 [M+H]⁺. ¹H NMR (500 MHz, CD₃OD): 7.38-7.32 (m, 4H), 7.13 (t, *J* = 6.4 Hz, 1 H), 5.48 (d, *J* = 6.4 Hz, 2H), 2.93-2.91 (m, 4H), 2.84-2.81 (m, 2H), 2.68-2.65 (m, 2H).

### Example 135

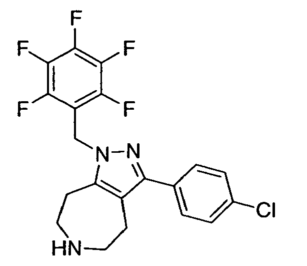

3-(4-Chloro-phenyl)-1-pentafluorophenylmethyl-1,4,5,6,7,8-hexahydro-1,2,6-triaza-azulene.
The title compound (0.02 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using pentafluorophenylmethyl chloride (0.3 mmol) in place of 2-chloromethyl-thiophene. The reaction sequence also yielded 3-(4-chloro-phenyl)-2-pentafluorophenylmethyl-4,5,7,8-tetrahydro-2*H*-1,2,6-triaza-azulene-6-carboxylic acid *tert*-butyl ester in the alkylation step. MS (ESI): exact mass calculated for C₂₀H₁₅ClF₅N₃, 427.09; found, *m*/*z* 428.1 [M+H]⁺. ¹H NMR (500 MHz, CD₃OD): 7.30 (br s, 4H), 5.34 (s, 2H), 3.01 (br s, 4H), 2.90-2.88 (m, 2H), 2.71-2.69 (m, 2H).

### Example 136

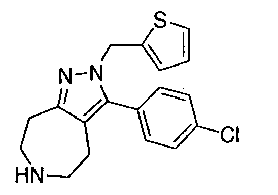

3-(4-Chloro-phenyl)-2-thiophen-2-ylmethyl-2,4,5,6,7,8-hexahydro-1,2,6-triaza-azulene.
The title compound (0.010 g) was prepared from 3-(4-chloro-phenyl)-2-thiophen-2-ylmethyl-4,5,7,8-tetrahydro-2*H*-1,2,6-triaza-azulene-6-carboxylic acid *tert*-butyl ester according to the method in Example 103. MS (ESI): exact mass calculated for C₁₈H₁₈ClN₃S, 343.09; found, *m*/*z* 344.1 [M+H]⁺. ¹H NMR (500 MHz, CD₃OD): 7.60-7.58 (m, 2H), 7.38-7.35 (m, 2H), 7.32 (dd, *J* = 5.1, 1.1 Hz, 1H), 6.92 (dd, *J* = 5.1, 3.5 Hz, 1H), 6.78 (dd, *J* = 3.5, 1.1 Hz, 1H), 5.41 (s, 2H), 3.47-3.45 (m, 2H), 3.37-3.35 (m, 2H), 3.23-3.21 (m, 2H), 2.85-2.83 (m, 2H).

### Example 137

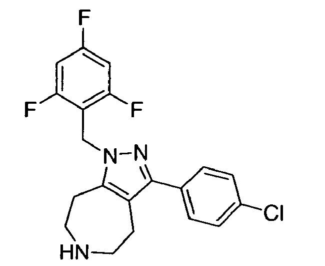

3-(4-Chloro-phenyl)-1-(2,4,6-trifluoro-benzyl)-1,4,5,6,7,8-hexahydro-1,2,6-triaza-azulene.

The title compound (0.027 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2,4,6-trifluorobenzyl chloride (0.3 mmol) in place of 2-chloromethyl-thiophene. MS (ESI): exact mass calculated for C₂₀H₁₇ClF₃N₃, 391.11; found, *m*/*z* 392.1 [M+H]⁺. ¹H NMR (500 MHz, CD₃OD): 7.30-7.26 (m, 4H), 6.80-6.78 (br m, 2H), 5.25 (s, 2H), 2.94-2.92 (m, 4H), 2.82-2.80 (m, 2H), 2.66-2.62 (m, 2H).

### Example 138

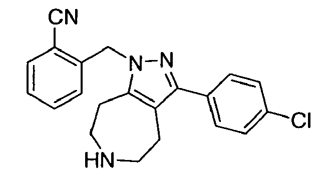

2-[3-(4-Chloro-phenyl)-5,6,7,8-tetrahydro-4*H*-1,2,6-triaza-azulen-1-ylmethyl]-benzonitrile.
The title compound (0.032 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 2-chloro-benzonitrile (0.3 mmol) in place of 2-chloromethyl-thiophene. MS (ESI): exact mass calculated for C₂₁H₁₉ClN₄, 362.13; found, *m*/*z* 363.2 [M+H]⁺. ¹H NMR (400 MHz, CD₃OD): 7.81 (d, J= 7.6 Hz, 1 H), 7.68-7.66 (br m, 1 H), 7.54-7.51 (m, 3H), 7.46 (d, J = 8.4 Hz, 2H), 7.23 (d, J = 7.6 Hz, 1 H), 5.64 (s, 2H), 3.51-3.49 (br m, 2H), 3.43-3.41 (br m, 2H), 3.31-3.29 (br m, 2H), 3.13-3.11 (br m, 2H).

### Example 139

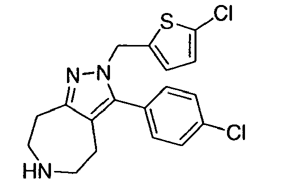

3-(4-Chloro-phenyl)-2-(5-chloro-thiophen-2-ylmethyl)-2,4,5,6,7,8-hexahydro-1,2,6-triaza-azulene.
The title compound (0.009 g) was prepared from 3-(4-chloro-phenyl)-2-(5-chloro-thiophen-2-ylmethyl)-4,5,7,8-tetrahydro-2*H*-1,2,6-triaza-azulene-6-carboxylic acid *tert*-butyl ester (Example 115) according to the deprotection method in Example 103, Step C. MS (ESI): exact mass calculated for C₁₈H₁₇Cl₂N₃S, 377.05; found, *m*/*z* 378.0 [M+H]⁺. ¹H NMR (500 MHz, CD₃OD): 7.47-7.44 (m, 2H), 7.22-7.20 (m, 2H), 6.65 (d, *J* = 3.8 Hz, 1 H), 6.44 (d, *J* = 3.8 Hz, 1 H), 5.17 (s, 2H), 3.32-3.30 (m, 2H), 3.21-3.19 (m, 2H), 3.07-3.05 (m, 2H), 2.70-2.68 (m, 2H).

### Example 140

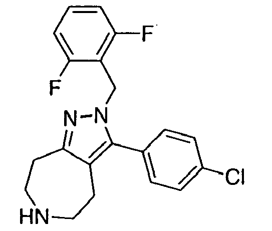

3-(4-Chloro-phenyl)-2-(2,6-difluoro-benzyl)-2,4,5,6,7,8-hexahydro-1,2,6-triaza-azulene.
The title compound (0.036 g) was prepared from 3-(4-chloro-phenyl)-2-(2,6-difluoro-benzyl)-4,5,7,8-tetrahydro-2*H*-1,2,6-triaza-azulene-6-carboxylic acid *tert*-butyl ester (Example 121, Step C) according to the deprotection method in Example 103, Step C. MS (ESI): exact mass calculated for C₂₀H₁₈ClF₂N₃, 373.12; found, *m*/*z* 374.2 [M+H]⁺. ¹H NMR (500 MHz, CD₃OD): 7.44-7.42 (m, 2H), 7.27-7.23 (m, 3H), 6.79 (m, 2H), 5.14 (s, 2H), 3.27-3.25 (m, 2H), 3.21-3.18 (m, 2H), 3.02-3.00 (m, 2H), 2.69-2.67 (m, 2H).

### Example 141

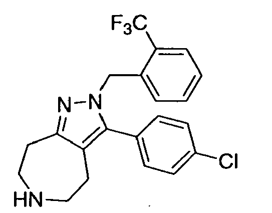

3-(4-Chloro-phenyl)-2-(2-trifluoromethyl-benzyl)-2,4,5,6,7,8-hexahydro-1,2,6-triaza-azulene.
The title compound (0.021 g) was prepared from 3-(4-chloro-phenyl)-2-(2-trifluoromethyl-benzyl)-4,5,7,8-tetrahydro-2*H*-1,2,6-triaza-azulene-6-carboxylic acid *tert*-butyl ester (Example 125) according to the deprotection method in Example 103, Step C. MS (ESI): exact mass calculated for C₂₁H₁₉ClF₃N₃, 405.12; found, *m*/*z* 406.1 [M+H]⁺. ¹H NMR (400 MHz, CD₃OD): 7.69 (d, J= 7.8 Hz, 1 H), 7.59 (t, J = 7.2 Hz, 1 H), 7.53-7.46 (m, 3H), 7.27 (d, J = 8.1, 2H), 6.83 (d, J = 7.2 Hz, 1 H), 5.47 (s, 2H), 3.51-3.49 (br m, 2H), 3.42-3.40 (br m, 2H), 3.31-3.29 (br m, 2H), 2.94-2.92 (br m, 2H).

### Example 142

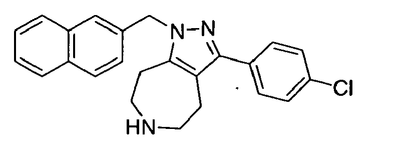

3-(4-Chloro-phenyl)-1-naphthalen-2-ylmethyl-1,4,5,6,7,8-hexahydro-1,2,6-triaza-azulene.
The title compound (0.043 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using naphthalen-2-ylmethyl chloride (0.3 mmol) in place of 2-chloromethyl-thiophene. MS (ESI): exact mass calculated for C₂₄H₂₂CIN₃, 387.15; found, *m*/*z* 388.1 [M+H]⁺. ¹H NMR (500 MHz, CDCl₃): 7.74-7.69 (m, 3H), 7.45-7.38 (m, 5H), 7.34-7.32 (m, 1 H), 7.18-7.16 (m, 2H), 5.43 (s, 2H), 3.04 (m, 2H), 2.99-2.97 (m, 2H), 2.87-2.85 (m, 4H).

### Example 143

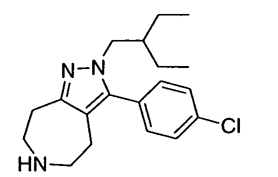

3-(4-Chloro-phenyl)-2-(2-ethyl-butyl)-2,4,5,6,7,8-hexahydro-1,2,6-triaza-azulene.
The title compound (0.012 g) was prepared from 3-(4-chloro-phenyl)-2-(2-ethylbutyl)-4,5,7,8-tetrahydro-2*H*-1,2,6-triaza-azulene-6-carboxylic acid *tert*-butyl ester (Example 114) according to the deprotection method in Example 103, Step C. MS (ESI): exact mass calculated for C₁₉H₂₆ClN₃, 331.18; found, *m*/*z* 332.2 [M+H]⁺. ¹H NMR (400 MHz, CD₃OD): 7.50-7.25 (br m, 4H), 3.76-3.74 (m, 2H), 3.33-3.31 (br m, 2H), 3.22-3.20 (br m, 2H), 3.09-3.07 (br m, 2H), 2.73-2.71 (br m, 2H), 1.56-1.54 (m, 1 H), 1.16-1.14 (m, 4H), 0.82-0.55 (m, 6H).

### Example 144

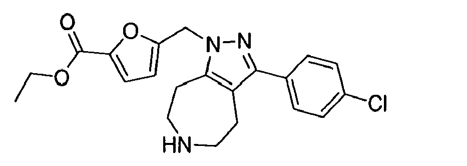

5-[3-(4-Chloro-phenyl)-5,6,7,8-tetrahydro-4*H*-1,2,6-triaza-azulen-1-ylmethyl]-furan-2-carboxylic acid ethyl ester.
The title compound (0.017 g) was prepared from 3-(4-chloro-phenyl)-1-(5-ethoxycarbonyl-furan-2-ylmethyl)-4,5,7,8-tetrahydro-1 1*H*-1,2,6-triaza-azulene-6-carboxylic acid *tert*-butyl ester (Example 130) according to the deprotection method in Example 103, Step C. MS (ESI): exact mass calculated for C₂₁H₂₂ClN₃O₃, 399.13; found, *m*/*z* 400.1 [M+H]⁺. ¹H NMR (500 MHz, CD₃OD): 7.37-7.32 (m, 4H), 7.06 (d, *J* = 3.5 Hz, 1 H), 6.41 (d, *J* = 3.5 Hz, 1 H), 5.34 (s, 2H), 4.21 (q, *J* = 7.1 Hz, 2H), 3.26-3.24 (m, 2H), 3.19-3.17 (m, 2H), 3.13-3.11 (m, 2H), 2.86-2.84 (m, 2H), 1.24 (t, *J* = 7.1 Hz, 2H).

### Example 145

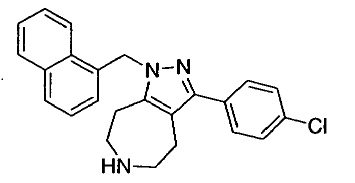

3-(4-Chloro-phenyl)-1-naphthalen-1-ylmethyl-1,4,5,6,7,8-hexahydro-1,2,6-triaza-azulene.
The title compound (0.015 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 1-naphthalen-methyl chloride (0.3 mmol) in place of 2-chloromethyl-thiophene. The reaction sequence also provided 3-(4-chlorophenyl)-2-naphthalen-1-ylmethyl-4,5,7,8-tetrahydro-2*H*-1,2,6-triaza-azulene-6-carboxylic acid *tert*-butyl ester in the alkylation step. MS (ESI): exact mass calculated for C₂₄H₂₂CIN₃, 387.15; found, *m*/*z* 388.1 [M+H]⁺. ¹H NMR (500 MHz, CD₃OD): 7.79-7.18 (m, 11 H), 5.74 (d, J = 7.3 Hz, 2H), 3.42 (s, 2H), 3.21-3.19 (br m, 2H), 3.10-3.08 (br m, 2H), 2.92-2.90 (br m, 2H), 2.84-2.82 (br m, 2H).

### Example 146

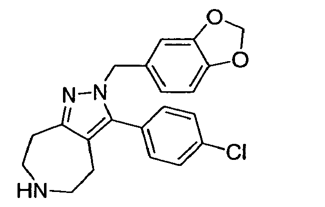

2-Benzo[1,3]dioxol-5-ylmethyl-3-(4-chloro-phenyl)-2,4,5,6,7,8-hexahydro-1,2,6-triaza-azulene.
The title compound (0.021 g) was prepared from 2-benzo[1,3]dioxol-5-ylmethyl-3-(4-chloro-phenyl)-4,5,7,8-tetrahydro-2*H*-1,2,6-triaza-azulene-6-carboxylic acid *tert*-butyl ester (Example 119) according to the deprotection method in Example 103, Step C. MS (ESI): exact mass calculated for C₂₁H₂₀ClN₃O₂, 381.12; found, *m*/*z* 382.0 [M+H]⁺. ¹H NMR (500 MHz, CD₃OD): 7.37-7.34 (m, 2H), 7.11-7.10 (m, 2H), 6.57 (d, J= 7.9 Hz, 1 H), 6.31-6.29 (m, 2H), 5.79 (s, 2H), 4.95 (s, 2H), 3.55-3.40 (m, 1 H), 2.82-2.80 (m, 5H), 2.42-2.41 (m, 2H).

### Example 147

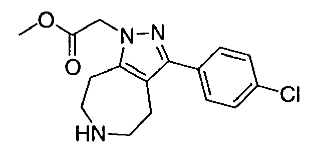

[3-(4-Chloro-phenyl)-5,6,7,8-tetrahydro-4*H*-1,2,6-triaza-azulen-1-yl]-acetic acid methyl ester.
The title compound (0.09 g) was prepared from 3-(4-chloro-phenyl)-4,5,7,8-tetrahydro-1 *H*-1,2,6-triaza-azulene-6-carboxylic acid *tert*-butyl ester (Example 103, Step B; 1 mmol) using 2-bromoacetic acid methyl ester (1.5 mmol) in place of 2-chloromethyl-thiophene. MS (ESI): exact mass calculated for C₁₆H₁₈ClN₃O₂, 319.11; found, *m*/*z* 320.2 [M+H]⁺. ¹H NMR (500 MHz, CD₃OD): 7.31 (d, J = 8.1 Hz, 2H), 7.26 (d, J = 8.1 Hz, 2H), 4.93 (s, 2H), 3.56 (s, 3H), 3.27-3.25 (br m, 2H), 3.19-3.17 (br m, 2H), 3.04-3.03 (br m, 2H), 2.90-2.88 (br m, 2H).

### Example 148

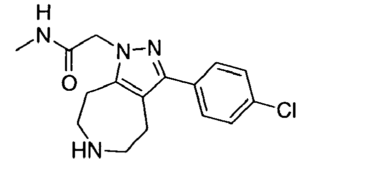

2-[3-(4-Chloro-phenyl)-5,6,7,8-tetrahydro-4*H*-1,2,6-triaza-azulen-1-yl]-*N*-methylacetamide.
To a solution of 3-(4-chloro-phenyl)-1-methylcarbamoylmethyl-4,5,7,8-tetrahydro-1 *H*-1,2,6-triaza-azulene-6-carboxylic acid *tert*-butyl ester (44 mg) (Example 147) in THF (0.5 mL) was added 8% aq. NaOH (0.3 mL). The mixture was stirred at RT for 16 h, and then was acidified with 1 N HCl (0.5 mL). The mixture was extracted with CH₂Cl₂ (2x2 mL). The combined organic layers were washed with brine, dried over Na₂SO₄, and concentrated. The residue was diluted with CH₃CN (0.5 mL) and treated with DCC (26 mg) and HOBt (18 mg). After 2 h at RT, a solution of methylamine hydrochloride (70 mg) in H₂O (0.3 mL) was added. The mixture was stirred at RT for 16 h. Concentration of the reaction mixture and purification of the residue by SiO₂ chromatography (EtOAc/hexanes) gave 3-(4-chloro-phenyl)-1-methylcarbamoylmethyl-4,5,7,8-tetrahydro-1 *H*-1,2,6-triaza-azulene-6-carboxylic acid *tert*-butyl ester. The intermediate was treated with TFA (1 mL) in CH₂Cl₂ (10 mL) for 4 h, and the solution was concentrated to obtain the title compound (0.015 g). MS (ESI): exact mass calculated for C₁₆H₁₉ClN₄O, 318.12; found, *m*/*z*319.1 [M+H]⁺. ¹H NMR (500 MHz, CD₃OD): 7.41-7.32 (m, 4H), 5.94 (br s, 1 H), 4.70 (s, 2H), 2.98-2.90 (m, 4H), 2.77-2.71 (m, 7H).

### Example 149

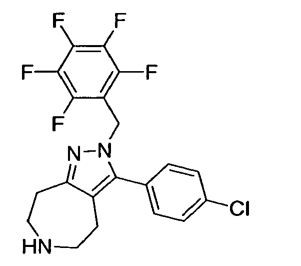

3-(4-Chloro-phenyl)-2-pentafluorophenylmethyl-2,4,5,6,7,8-hexahydro-1,2,6-triaza-azulene.
The title compound (0.016 g) was prepared from 3-(4-chloro-phenyl)-2-pentafluorophenylmethyl-4,5,7,8-tetrahydro-2H- 1,2,6-triaza-azulene-6-carboxylic acid *tert*-butyl ester (Example 135) according to the deprotection method in Example 103, Step C. MS (ESI): exact mass calculated for C₂₀H₁₅ClF₅N₃, 427.09; found, *m*/z 428.1 [M+H]⁺.¹H NMR (500 MHz, CD₃OD): 7.45-7.43 (m, 2H), 7.26-7.23 (m, 2H), 5.15 (s, 2H), 2.91-2.89 (m, 2H), 2.83-2.81 (m, 2H), 2.79-2.77 (m, 2H), 2.46-2.44(m, 2H).

### Example 150

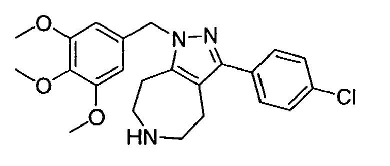

3-(4-Chloro-phenyl)-1-(3,4,5-trimethoxy-benzyl)-1,4,5,6,7,8-hexahydro-1,2,6-triaza-azulene.
The title compound (0.006 g) was prepared from 3-(4-chloro-phenyl)-4,5,7,8-tetrahydro-1 *H*-1,2,6-triaza-azulene-6-carboxylic acid *tert*-butyl ester (Example 103, Step B; 0.2 mmol) using 3,4,5-trimethoxybenzyl chloride (0.3 mmol) in place of 2-chloromethyl-thiophene. The reaction sequence also yielded 3-(4-chloro-phenyl)-2-(3,4,5-trimethoxy-benzyl)-4,5,7,8-tetrahydro-2*H*-1,2,6-triaza-azulene-6-carboxylic acid *tert*-butyl ester in the alkylation step. MS (ESI): exact mass calculated for C₂₃H₂₆ClN₃O₃, 427.17; found, *m*/*z* 428.1 [M+H]⁺. ¹H NMR (500 MHz, CD₃OD): 7.51-7.49 (m, 2H), 7.46-7.44 (m, 2H), 6.44 (s, 2H), 5.32 (s, 2H), 3.78 (s, 6H), 3.74 (s, 3H), 2.95-2.89 (m, 6H), 2.81-2.79 (m, 2H).

### Example 151

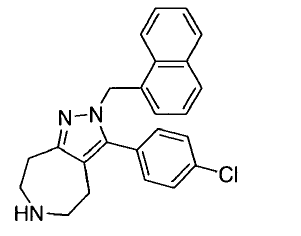

3-(4-Chloro-phenyl)-2-naphthalen-1-ylmethyl-2,4,5,6,7,8-hexahydro-1,2,6-triaza-azulene.
The title compound (0.015 g) was prepared from 3-(4-chloro-phenyl)-2-naphthalen-1-ylmethyl-4,5,7,8-tetrahydro-2*H*-1,2,6-triaza-azulene-6-carboxylic acid *tert*-butyl ester (Example 145) according to the deprotection method in Example 103, Step C. MS (ESI): exact mass calculated for C₂₄H₂₂ClN₃, 387.15; found, *m*/*z* 388.2 [M+H]⁺. ¹H NMR (500 MHz, CDCl₃): 7.78-7.75 (m, 2H), 7.67 (d, J = 8.3 Hz, 1 H), 7.41-7.39 (m, 2H), 7.00 (td, J = 8.0, 1.0 Hz, 1 H), 7.21-7.19 (m, 2H), 7.02-7.01 (m, 2H), 6.69-6.67 (dd, J = 7.1, 1.0 Hz, 1 H), 5.56 (s, 2H), 3.31-3.29 (m, 2H), 2.90-2.88 (m, 4H), 2.49-2.47 (m, 2H).

### Example 152

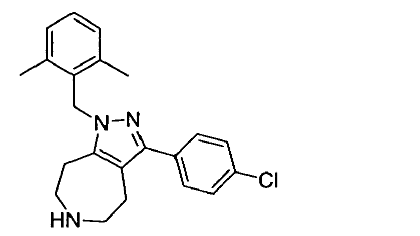

3-(4-Chloro-phenyl)-1-(2,6-dimethyl-benzyl)-1,4,5,6,7,8-hexahydro-1,2,6-triaza-azulene.
The title compound (0.018 g) was prepared from 3-(4-chloro-phenyl)-4,5,7,8-tetrahydro-1*H*-1,2,6-triaza-azulene-6-carboxylic acid *tert*-butyl ester (Example 103, step B; 0.2 mmol) using 2,6-dimethylbenzyl chloride (0.3 mmol) in place of 2-chloromethyl-thiophene. MS (ESI): exact mass calculated for C₂₂H₂₄CIN₃, 365.17; found, *m*/*z* 366.2 [M+H]⁺. ¹H NMR (500 MHz, CD₃OD): 7.48-7.45 (m, 4H), 7.17-7.15 (br m, 1 H), 7.11-7.09 (m, 2H), 5.51 (s, 2H), 3.43-3.41 (br m, 2H), 3.39-3.37 (br m, 2H), 3.31-3.29 (br m, 2H), 3.10-3.08 (br m, 2H), 2.31 (s, 3H).

### Example 153

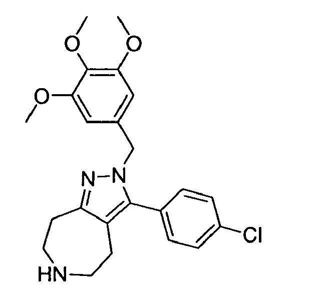

3-(4-Chloro-phenyl)-2-(3,4,5-trimethoxy-benzyl)-2,4,5,6,7,8-hexahydro-1,2,6-triaza-azulene.
The title compound (25 mg) was prepared from 3-(4-chloro-phenyl)-2-(3,4,5-trimethoxy-benzyl)-4,5,7,8-tetrahydro-2*H*-1,2,6-triaza-azulene-6-carboxylic acid *tert*-butyl ester (Example 150) according to the deprotection method in Example 103, Step C. MS (ESI): exact mass calculated for C₂₃H₂₆CIN₃O₃, 427.17; found, *m*/*z* 428.1 [M+H]⁺. ¹H NMR (500 MHz, CD₃OD): 7.39-7.36 (m, 2H), 7.13-7.11 (m, 2H), 6.07 (s, 2H), 5.00 (s, 2H), 3.59 (s, 9H), 2.90-2.70 (m, 6H), 2.46-2.44 (m, 2H).

### Example 154

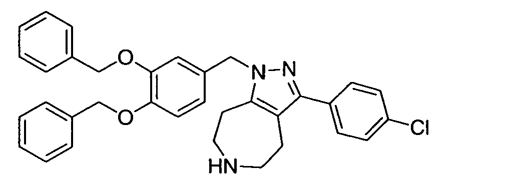

1-(3,4-Bis-benzyloxy-benzyl)-3-(4-chloro-phenyl)-1,4,5,6,7,8-hexahydro-1,2,6-triaza-azulene.
The title compound (22 mg) was prepared from 3-(4-chloro-phenyl)-4,5,7,8-tetrahydro-1*H*-1,2,6-triaza-azulene-6-carboxylic acid *tert*-butyl ester (Example 103, Step B; 0.2 mmol) using 3,4-bis(benzyloxy)benzyl chloride (0.3 mmol) in place of 2-chloromethyl-thiophene. The reaction sequence also provided 2-(3,4-bis-benzyloxy-benzyl)-3-(4-chloro-phenyl)-4,5,7,8-tetrahydro-2*H*-1,2,6-triaza-azulene-6-carboxylic acid *tert*-butyl ester in the alkylation step. MS (ESI): exact mass calculated for C₃₄H₃₂ClN₃O₂, 549.22; found, *m*/*z* 550.1 [M+H]⁺. ¹H NMR (500 MHz, CD₃OD): 7.36-7.16 (m, 14H), 6.86 (d, J= 8.3 Hz, 1 H), 6.65 (d, J = 1.9 Hz, 1 H), 6.56 (dd, J = 8.3, 1.9 Hz, 1 H), 5.13 (s, 2H), 4.99 (s, 2H), 4.97 (s, 2H), 2.78-2.76 (m, 2H), 2.73-2.71 (m, 2H), 2.65 (m, 4H).

### Example 155

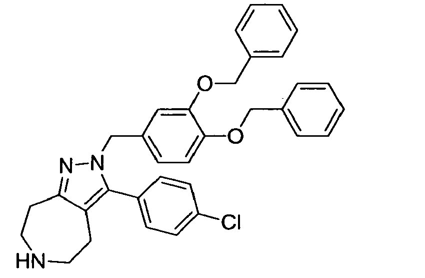

2-(3,4-Bis-benzyloxy-benzyl)-3-(4-chloro-phenyl)-2,4,5,6,7,8-hexahydro-1,2,6-triaza-azulene.
The title compound (20 mg) was prepared from 2-(3,4-bis-benzyloxy-benzyl)-3-(4-chloro-phenyl)-4,5,7,8-tetrahydro-2*H*-1,2,6-triaza-azulene-6-carboxylic acid *tert*-butyl ester (Example 154) according to the deprotection method in Example 103, Step C. MS (ESI): exact mass calculated for C₃₄H₃₂CIN₃O₂, 549.22; found, *m*/*z* 550.1 [M+H]⁺. ¹H NMR (500 MHz, CD₃OD): 7.43-7.30 (m, 12H), 7.07-7.05 (m, 2H), 6.89-6.87 (m, 1 H), 6.47-6.46 (m, 2H), 5.09 (s, 2H), 5.04 (s, 2H), 5.01 (s, 2H), 2.99-2.97 (m, 2H), 2.93-2.91 (m, 2H), 2.88-2.86 (m, 2H), 2.52-2.50 (m, 2H).

### Example 156

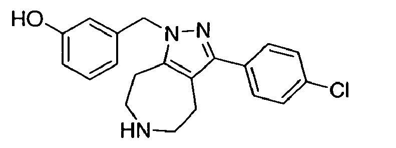

3-[3-(4-Chloro-phenyl)-5,6,7,8-tetrahydro-4*H*-1,2,6-triaza-azulen-1-ylmethyl]-phenol.
A solution of 3-(4-chloro-phenyl)-1-(3-methoxy-benzyl)-1,4,5,6,7,8-hexahydro-1,2,6-triaza-azulene (Example 105, 0.1 mmol) in CH₂Cl₂ (5 mL) was cooled to 0 °C, and 1 M BBr₃ in CH₂Cl₂ (0.5 mL) was added. The mixture was allowed to warm to 25 °C. After 2 h, satd. aq. NaHCO₃ (5 mL) was added. The layers were separated, and the aqueous layer was extracted with EtOAc (2x5 mL). The combined organic layers were dried over Na₂SO₄ and concentrated. Purification by flash chromatography (2 M NH₃ in MeOH/CH₂Cl₂) provided the title compound (10 mg). MS (ESI): exact mass calculated for C₂₀H₂₀ClN₃O, 353.13; found, *m*/*z* 354.1 [M+H]⁺. ¹H NMR (500 MHz, CD₃OD): 7.40-7.38 (m, 2H), 7.35-7.32 (m, 2H), 7.03 (t, J = 7.9 Hz, 1 H), 6.57 (dd, J = 8.1, 2.0 Hz, 1 H), 6.49 (d, J = 8.1 Hz, 1 H), 6.39-6.37 (br m, 1 H), 5.20 (s, 2H), 2.81-2.78 (br m, 4H), 2.74-2.72 (br m, 2H), 2.70-2.68 (br m, 2H).

### Example 157

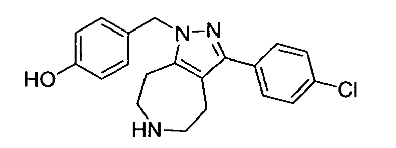

4-[3-(4-Chloro-phenyl)-5,6,7,8-tetrahydro-4H-1,2,6-triaza-azulen-1 -ylmethyl]-phenol.
The title compound (10 mg) was prepared from (4-chloro-phenyl)-1-(4-methoxy-benzyl)-1,4,5,6,7,8-hexahydro-1,2,6-triaza-azulene-6-carboxylic acid *tert*-butyl ester (Example 123, 0.1 mmol) as in Example 156. MS (ESI): exact mass calculated for C₂₀H₂₀ClN₃O, 353.13; found, *m*/*z* 354.1 [M+H]⁺. ¹H NMR (500 MHz, CD₃OD): 7.39-7.37 (m, 2H), 7.34-7.31 (m, 2H), 6.89-6.86 (m, 2H), 6.64-6.61 (m, 2H), 5.16 (s, 2H), 2.77-2.75 (br m, 6H), 2.67-2.65 (br m, 2H).

### Example 158

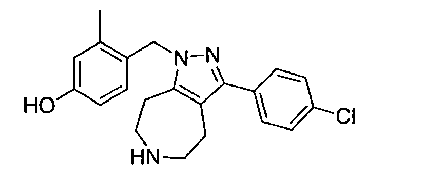

4-[3-(4-Chloro-phenyl)-5,6,7,8-tetrahydro-4*H*-1,2,6-triaza-azulen-1-ylmethyl]-3-methyl-phenol.
The title compound (8 mg) was prepared from (4-chloro-phenyl)-1-(4-methoxy-2-methyl-benzyl)-1,4,5,6,7,8-hexahydro-1,2,6-triaza-azutene-6-carboxylic acid *tert-*butyl ester (Example 128, 34 mg) as in Example 156. MS (ESI): exact mass calculated for C₂₁H₂₂ClN₃O, 367.15; found, *m*/*z* 368.0 [M+H]⁺. ¹H NMR (500 MHz, CD₃OD): 7.39-7.37 (m, 2H), 7.33-7.32 (m, 2H), 6.54-6.52 (br m, 1 H), 6.41-6.39 (br m, 1 H), 6.28 (d, J = 8.3 Hz, 1 H), 5.17 (s, 2H), 2.83-2.78 (m, 4H), 2.71-2.67 (m, 2H).

### Example 159

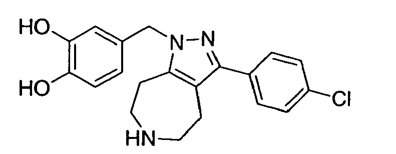

4-[3-(4-Chloro-phenyl)-5,6,7,8-tetrahydro-4*H*-1,2,6-triaza-azulen-1-ylmethyl]-benzene-1,2-diol.
A solution of 1-(3,4-bis-benzyloxy-benzyl)-3-(4-chloro-phenyl)-1,4,5,6,7,8-hexahydro-1,2,6-triaza-azulene-carboxylic acid *tert*-butyl ester (Example 154, 0.1 mmol) in CH₂Cl₂ (5 mL) was cooled to 0 °C, and 1 M BBr₃ in CH₂Cl₂ (0.5 mL) was added. The mixture was allowed to warm to RT and was stirred at RT for 1 h. The precipitate that had formed was collected by filtration, washed with water, and dried under vacuum to provide the title compound (25 mg). MS (ESI): exact mass calculated for C₂₀H₂₀CIN₃O₂, 369.12; found, *m*/*z* 370.0 [M+H]⁺. ¹H NMR (500 MHz, CD₃OD): 7.44-7.42 (m, 4H), 7.39-7.37 (m, 2H), 6.63 (d, J = 8.1 Hz, 1 H), 6.50 (d, J = 2.1 Hz, 1 H), 6.45 (dd, J = 8.1, 2.1 Hz, 1 H), 5.18 (s, 2H), 3.29-3.25 (m, 4H), 3.06-3.04 (m, 2H), 2.97-2.95 (m, 2H).

### Example 160

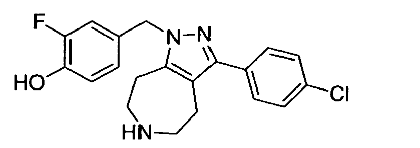

4-[3-(4-Chloro-phenyl)-5,6,7,8-tetrahydro-4*H*-1,2,6-triaza-azulen-1-ylmethyl]-2-fluoro-phenol.
BBr₃ (0.13 mL) was added slowly to a 0 °C solution of 0.022 g of 3-(4-chlorophenyl)-1-(3-fluoro-4-methoxy-benzyl)-1,4,5,6,7,8-hexahydro-1,2,6-triaza-azulene (Example 96) in CH₂Cl₂ (20 mL). After 1 h, the mixture was warmed to RT and stirred for 18 h. The reaction was then cooled back to 0 °C and quenched by the addition of 5 mL of satd. aq. NaHCO₃. The aqueous layer was extracted with methanolic CH₂Cl₂ (2x). The combined organic layers were dried over Na₂SO₄ and concentrated. The crude oil was purified by preparative TLC (9:1 CH₂Cl₂/2 M NH₃ in MeOH) to afford the title compound (0.016 g) as a tan solid. MS (ESI): exact mass calculated for C₂₀H₁₉CIFN₃O, 371.12; found, *m*/*z* 372.1 [M+H]⁺. ¹H NMR (400 MHz, CD₃OD): 7.50-7.42 (m, 4H), 6.86-6.79 (m, 3H), 5.26 (s, 2H), 3.31-3.26 (m, 2H), 2.96-2.95 (m, 4H), 2.90-2.87 (m, 2H), 2.81-2.79 (m, 2H). ¹³C NMR (100 MHz, CD₃OD): 154.2, 151.8, 149.6, 146.1, 146.0, 143.8, 134.8, 133.4, 131.1, 130.3, 130.2, 129.7, 124.0, 119.1, 115.6, 115.4, 53.1, 50.4, 29.0, 27.5.

### Example 161

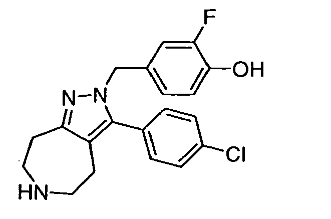

4-[3-(4-Chloro-phenyl)-5,6,7,8-tetrahydro-4*H*-1,2,6-triaza-azulen-2-ylmethyl]-2-fluoro-phenol.
3-(4-Chloro-phenyl)-2-(3-fluoro-4-methoxy-benzyl)-2,4,5,6,7,8-hexahydro-1,2,6-triaza-azulene (Example 97, 0.12 g) was de-methylated as in Example 160 to afford the title compound (0.027 g) as an off-white solid. MS (ESI): exact mass calculated for C₂₀H₁₉ClFN₃O, 371.12; found, *m*/*z* 372.0 [M+H]⁺. ¹H NMR (500 MHz, CD₃OD): 7.48-7.44 (m, 2H), 7.21-7.18 (m, 2H), 6.75 (t, J= 8.6 Hz, 1 H), 6.61-6.58 (m, 1 H), 6.53-6.50 (m 1 H), 5.04 (s, 2H), 3.31-3.30 (m, 2H), 3.01-2.99 (m, 2H), 2.94-2.88 (m, 4H), 2.55-2.52 (m, 2H). ¹³C NMR (125 MHz, CD₃OD): 154.2, 153.7, 152.3, 146.5, 146.4, 142.4, 136.6, 133.1, 130.6, 130.5, 130.1, 124.5, 120.7, 119.2, 116.0, 115.9, 53.4, 51.2, 32.6, 28.0.

### Example 162

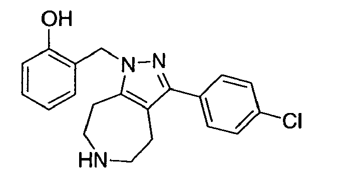

2-[3-(4-Chloro-phenyl)-5,6,7,8-tetrahydro-4*H*-1,2,6-triaza-azulen-1-ylmethyl]-phenol.
The title compound (13 mg) was prepared from 3-(4-chloro-phenyl)-1-(2-methoxy-benzyl)-1,4,5,6,7,8-hexahydro-1,2,6-triaza-azulene-6-carboxylic acid *tert*-butyl ester (Example 109, 0.1 mmol) as in Example 156. MS (ESI): exact mass calculated for C₂₀H₂₀ClN₃O, 353.13; found, *m*/*z* 354.2 [M+H]⁺. ¹H NMR (500 MHz, CDCl₃): 7.37 (d, J= 6.5 Hz, 2H), 7.33 (d, J= 6.5 Hz, 2H), 7.19 (t, J = 7.6 Hz, 1 H), 7.10 (d, J = 7.6 Hz, 1 H), 6.91 (d, J = 7.6 Hz, 1 H), 6.62 (t, J = 7.6 Hz, 1 H), 5.12 (s, 2H), 2.91-2.80 (br m, 6H), 2.68-2.66 (m, 2H).

### Example 163

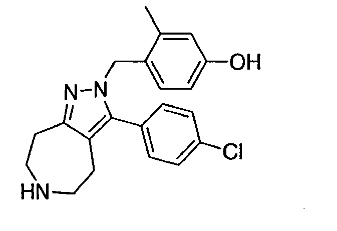

4-[3-(4-Chloro-phenyl)-5,6,7,8-tetrahydro-4*H*-1,2,6-triaza-azulen-2-ylmethyl]-3-methyl-phenol.
The title compound (14 mg) was prepared from (4-chloro-phenyl)-2-(4-methoxy-2-methyl-benzyl)-1,4,5,6,7,8-hexahydro-1,2,6-triaza-azulene-6-carboxylic acid *tert*-butyl ester (Example 128, 42 mg) as in Example 156. MS (ESI): exact mass calculated for C₂₁H₂₂CIN₃O, 367.15; found, *m*/*z* 368.1 [M+H]⁺. ¹H NMR (500 MHz, CD₃OD): 7.33-7.30 (m, 2H), 7.07-7.06 (m, 2H), 6.43 (d, J = 2.3 Hz, 1 H), 6.36 (dd, J = 8.4, 2.3 Hz, 1 H), 6.30 (d, J = 8.4 Hz, 1 H), 4.96 (s, 2H), 2.89-2.86 (m, 2H), 2.82-2.77 (m, 4H), 2.44 (m, 2H), 1.89 (s, 3H).

### Example 164

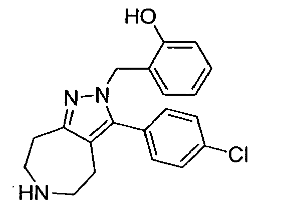

2-[3-(4-Chloro-phenyl)-5,6,7,8-tetrahydro-4*H*-1,2,6-triaza-azulen-2-ylmethyl]-phenol.
The title compound (8 mg) was prepared from 3-(4-chloro-phenyl)-2-(2-methoxy-benzyl)-1,4,5,6,7,8-hexahydro-1,2,6-triaza-azulene-6-carboxylic acid *tert*-butyl ester (Example 132, 30 mg) as in Example 156. MS (ESI): exact mass calculated for C₂₀H₂₀ClN₃O, 353.13; found, *m*/*z* 354.1 [M+H]⁺. ¹H NMR (500 MHz, CDCl₃): 7.62 (d, J = 6.5 Hz, 2H), 7.36-7.34 (m, 3H), 7.13 (d, J = 8.0 Hz, 1 H), 7.00 (d, J = 8.0 Hz, 1 H), 6.82 (t, J = 8.0 Hz, 1 H), 5.08 (s, 2H), 3.11-3.00 (br m, 6H), 2.60-2.58 (m, 2H).

### Example 165

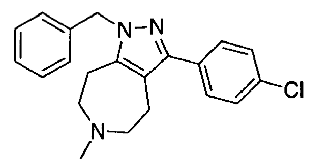

1-Benzyl-3-(4-chloro-phenyl)-6-methyl-1,4,5,6,7,8-hexahydro-1,2,6-triaza-azulene.
To a solution of 1-benzyl-3-(4-chloro-phenyl)-1,4,5,6,7,8-hexahydro-1,2,6-triaza-azulene (Example 59, Step E; 0.1 mmol) in 1,2-dichloroethane (5 mL) was added acetic acid (0.2 mmol), formaldehyde (37% water solution, 0.037 mL), and NaBH(OAc)₃ (0.2 mmol). The mixture was stirred at RT for 15 h. The mixture was diluted with CH₂Cl₂ and washed with satd. aq. NaHCO₃ (2x).
The combined organic layers were dried over Na₂SO₄, filtered, and concentrated *in vacuo.* Chromatography on SiO₂ (2 M NH₃ in MeOH/CH₂Cl₂) afforded 0.015 g of the title compound. MS (ESI): exact mass calculated for C₂₁H₂₂ClN₃, 351.15; found, *m*/*z* 352.2 [M+H]⁺. ¹H NMR (400 MHz, CDCl₃): 7.45-7.42 (m, 2H), 7.32-7.29 (m, 2H), 7.26-7.19 (m, 3H), 7.03-7.01 (br m, 2H), 5.27 (s, 2H), 2.75-2.68 (m, 4H), 2.64-2.58 (m, 4H), 2.36 (s, 3H).

Examples 166 through 169 were synthesized using the procedure described in Example 165 unless otherwise noted.

### Example 166

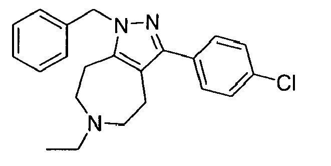

1-Benzyl-3-(4-chloro-phenyl)-6-ethyl-1,4,5,6,7,8-hexahydro-1,2,6-triaza-azulene.
The title compound (18 mg) was prepared using acetaldehyde (0.2 mmol) in place of formaldehyde. MS (ESI): exact mass calculated for C₂₂H₂₄ClN₃, 365.17; found, *m*/*z* 366.2 [M+H]⁺. ¹H NMR (400 MHz, CDCl₃): 7.45-7.43 (m, 2H), 7.31-7.29 (m, 2H), 7.26-7.19 (m, 3H), 7.03-7.01 (br m, 2H), 5.26 (s, 2H), 2.74-2.71 (m, 10H), 1.01 (t, *J* = 7.1 Hz, 3H).

### Example 167

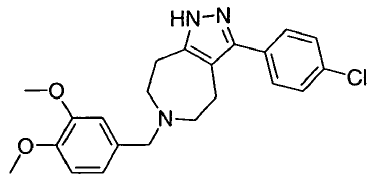

3-(4-Chloro-phenyl)-6-(3,4-dimethoxy-benzyl)-1,4,5,6,7,8-hexahydro-1,2,6-triaza-azulene.
To a solution of 3-(4-chloro-phenyl)-4,5,7,8-tetrahydro-1 *H*-1,2,6-triaza-azulene-6-carboxylic acid *tert*-butyl ester (Example 59, Step C; 0.1 mmol) in CH₂Cl₂ (5 mL) was added TFA (1 mL). The mixture was stirred at RT for 16 h. After concentration, the intermediate 3-(4-chloro-phenyl)-1,4,5,6,7,8-hexahydro-1,2,6-triaza-azulene was obtained. The intermediate (0.1 mmol) was converted to the title compound (16 mg) according to the procedure described in Example 165 using 3,4-dimethoxy-benzaldehyde (0.2 mmol) in place of formaldehyde. MS (ESI): exact mass calculated for C₂₂H₂₄ClN₃O₂, 397.16; found, *m*/*z* 398.2 [M+H]⁺. ¹H NMR (500 MHz, CD₃OD): 7.38-7.35 (br m, 4H), 7.91 (d, J = 1.8 Hz, 1 H), 6.82-6.80 (dd, J = 8.1, 1.8 Hz, 1 H), 6.76-6.74 (d, J = 8.1 Hz, 1 H), 3.83-3.80 (s, 6H), 2.84-2.82 (m, 4H), 2.71-2.69 (m, 4H).

### Example 168

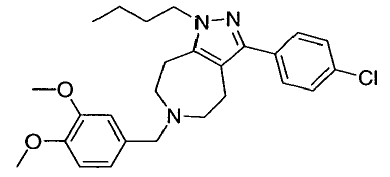

1-Butyl-3-(4-chloro-phenyl)-6-(3,4-dimethoxy-benzyl)-1,4,5,6,7,8-hexahydro-1,2,6-triaza-azulene.
The title compound (8 mg) was prepared from 1-butyl-3-(4-chloro-phenyl)-1,4,5,6,7,8-hexahydro-1,2,6-triaza-azulene (Example 67, 14 mg) using 3,4-dimethoxy-benzaldehyde (0.2 mmol) in place of formaldehyde. MS (ESI): exact mass calculated for C₂₆H₃₂ClN₃O₂, 453.22; found, *m*/*z* 454.2 [M+H]⁺. ¹H NMR (400 MHz, CDCl₃): 7.38 (d, *J* = 8.4 Hz, 2H), 7.28 (d, *J*= 8.4 Hz, 2H), 7.20 (s, 1H), 6.91-6.90 (br m, 1H), 6.81 (d, *J* = 8.2 Hz, 1H), 6.75 (d, *J* = 8.2 Hz, 1H), 3.98 (t, J = 7.3 Hz, 2H), 3.82 (d, J = 7.0 Hz, 6H), 3.66 (s, 2H), 2.78-2.72 (br m, 8H), 1.67 (m, 2H), 1.27 (m, 2H), 0.86 (t, J = 7.3 Hz, 6H).

### Example 169

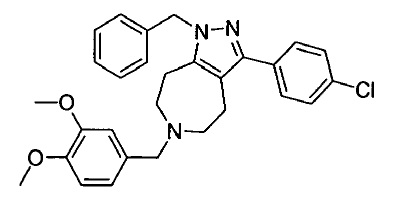

1-Benzyl-3-(4-chloro-phenyl)-6-(3,4-dimethoxy-benzyl)-1,4,5,6,7,8-hexahydro-1,2,6-triaza-azulene.
The title compound was prepared (12 mg) from 1-benzyl-3-(4-chloro-phenyl)-1,4,5,6,7,8-hexahydro-1,2,6-triaza-azulene (Example 59, Step E; 0.1 mmol) using 3,4-dimethoxy-benzaldehyde (0.2 mmol) in place of formaldehyde. MS (ESI): exact mass calculated for C₂₉H₃₀CIN₃O₂, 487.20; found, *m*/*z* 488.2 [M+H]⁺. ¹H NMR (500 MHz, CDCl₃): 7.44-7.43 (m, 2H), 7.30-7.29 (m, 2H), 7.25-7.22 (m, 2H), 7.20-7.18 (m, 1 H), 7.03-7.02 (m, 2H), 6.86 (d, J = 1.7 Hz, 1 H), 6.76-6.71 (m, 2H), 5.25 (s, 2H), 3.79 (s, 6H), 3.63 (s, 2H), 2.72 (s, 4H), 2.68-2.66 (m, 4H).

### Example 170

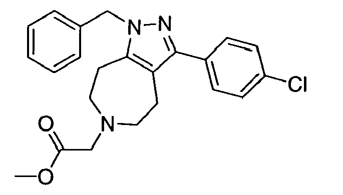

[1-Benzyl-3-(4-chloro-phenyl)-4,5,7,8-tetrahydro-1*H*-1,2,6-triaza-azulen-6-yl]-acetic acid methyl ester.
To a solution of 1-benzyl-3-(4-chloro-phenyl)-1,4,5,6,7,8-hexahydro-1,2,6-triaza-azulene (Example 59, Step E; 1 mmol) in acetone (3 mL) was added Na₂CO₃ (2 mmol) and bromoacetic methyl ester (2 mmol). The mixture was stirred at RT for 1 h. After concentration and purification (SiO₂, 2 M NH₃ in MeOH/CH₂Cl₂), the title compound was obtained (60 mg). MS (ESI): exact mass calculated for C₂₃H₂₄ClN₃O₂, 409.16; found, *m*/*z* 410.1 [M+H]⁺. ¹H NMR (500 MHz, CDCl₃): 7.44-7.42 (m, 2H), 7.31-7.29 (m, 2H), 7.25-7.23 (br m, 2H), 7.20-7.18 (br m, 1 H), 7.02-6.99 (br m, 2H), 5.26 (s, 2H), 3.64 (s, 2H), 3.41 (s, 2H), 2.81-2.79 (br m, 4H), 2.75-2.73 (br m, 2H), 2.70-2.68 (br m, 2H).

### Example 171

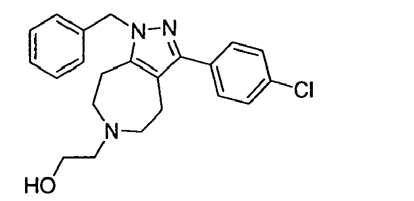

2-[1-Benzyl-3-(4-chloro-phenyl)-4,5,7,8-tetrahydro-1*H*-1,2,6-triaza-azuien-6-yl]-ethanol.
To a solution of [1-benzyl-3-(4-chloro-phenyl)-4,5,7,8-tetrahydro-1 *H*-1,2,6-triaza-azulen-6-yl]-acetic acid methyl ester (Example 170, 16 mg) in THF (1 mL) was added lithium aluminum hydride (100 mg). The mixture was stirred at RT for 16 h. The reaction was quenched by the addition of H₂O (0.1 mL). Concentration and purification (SiO₂, 2 M NH₃ in MeOH/CH₂Cl₂) provided the title compound (5 mg). MS (ESI): exact mass calculated for C₂₂H₂₄ClN₃O, 381.16; found, *m*/*z* 382.1 [M+H]⁺. ¹H NMR (500 MHz, CDCl₃): 7.50 -7.20 (m, 7H), 7.04 (d, J = 7.2 Hz, 1 H), 5.29 (s, 2H), 3.07-3.04 (m, 2H), 2.89-2.77 (m, 10H).

### Example 172

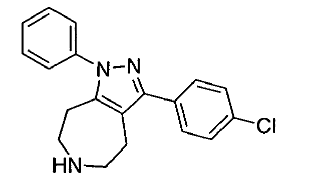

3-(4-Chloro-phenyl)-1-phenyl-1,4,5,6,7,8-hexahydro-1,2,6-triaza-azulene.
A solution of 3-(4-chloro-phenyl)-4,5,7,8-tetrahydro-1*H*-1,2,6-triaza-azulene-6-carboxylic acid *tert*-butyl ester (Example 59, Step C; 0.3 mmol) in CH₂Cl₂ (5 mL) was treated with phenylboronic acid (0.6 mmol), pyridine (0.6 mmol), and copper(II) acetate (4.5 mmol). The mixture was stirred at RT for 16 h. After concentration and purification (SiO₂, EtOAc/hexanes), 3-(4-chloro-phenyl)-1-phenyl-1,4,5,6,7,8-hexahydro-1,2,6-triaza-azulene-6-carboxylic acid *tert*-butyl ester was obtained. This intermediate was then diluted with CH₂Cl₂ (10 mL), and TFA (1 mL) was added. The mixture was stirred at RT for 4 h. The mixture was concentrated and the residue was purified (SiO₂, 2 M NH₃ in MeOH/CH₂Cl₂) to provide the title compound (40 mg). MS (ESI): exact mass calculated for C₁₉H₁₈ClN₃, 323.12; found, *m*/*z* 324.1 [M+H]⁺. ¹H NMR (500 MHz, CDCl₃): 7.46-7.40 (m, 4H), 7.36-7.32 (m, 5H), 3.09-3.07 (br m, 4H), 3.00-2.98 (br m, 2H), 3.92-2.90 (br m, 2H).

### Example 173

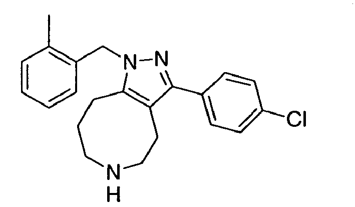

3-(4-Chloro-phenyl)-1-(2-methyl-benzyl)-4,5,6,7,8,9-hexahydro-1*H*-1,2,6-triaza-cyclopentacyclooctene.
Step A. 3-(4-Chloro-phenyl)-1,4,5,7,8.9-hexahydro-1,2,6-triaza-cyclopentacyclooctene-6-carboxylic acid *tert*-butyl ester. To a 0 °C solution of 4-oxo-azepane-1-carboxylic acid *tert*-butyl ester (Example 59, Step B; 0.915 g) in Et₂O (30 mL) was added BF₃•Et₂O (0.733 mL) followed by a solution of 1-(4-chlorophenyl)-2-diazo-ethanone (Example 103, Step A; 4.5 mmol) in Et₂O (30 mL). The mixture was warmed to 25 °C and stirred for 1 h. Satd. aq. NaHCO₃ (40 mL) was added, and the organic layer was separated and concentrated. The resulting residue was diluted with MeOH (50 mL) and treated with hydrazine (1.5 mL). The reaction mixture was stirred at 25 °C for 16 h. Concentration and purification by flash chromatography (SiO₂, EtOAc/CH₂Cl₂) provided the desired ester.
Step B. 3-(4-Chloro-phenyl)-1-(2-methyl-benzyl)-1,4,5,7,8,9-hexahydro-1,2,6-triaza-cyclopentacyclooctene-6-carboxylic acid tert-butyl ester. A solution of the product from Step A (0.2 mmol) in DMF (2 mL) was treated with 2-methylbenzyl chloride (0.3 mmol) followed by Cs₂CO₃ (0.3 mmol). The mixture was stirred at 25 °C for 16 h. Concentration and purification by chromatography (SiO₂, EtOAc/hexanes) provided the target intermediate.
Step C. A solution of the product from Step B in MeOH (20 mL) was treated with HCl (2 M in Et₂O, 1 mL) for 16 h. After concentration and purification by chromatography (SiO₂, 2 M NH₃ in MeOH/CH₂Cl₂), the title compound was obtained (24 mg). The reaction sequence also yielded 3-(4-chloro-phenyl)-1-(2-methyl-benzyl)-4,5,6,7,8,9-hexahydro-1*H*-1,2,7-triaza-cyclopentacyclooctene (20 mg). MS (ESI): exact mass calculated for C₂₂H₂₄CIN₃, 365.17; found, *m*/*z* 366.2 [M+H]⁺. ¹H NMR (500 MHz, CD₃OD): 7.51-7.50 (m, 2H), 7.42-7.40 (m, 2H), 7.14-7.05 (m, 3H), 6.58 (d, J = 7.6 Hz, 1 H), 5.42 (s, 2H), 3.25 (t, J = 5.6 Hz, 2H), 3.13 (t, J = 5.6 Hz, 2H), 3.01 (t, J = 5.6 Hz, 2H), 2.89 (t, J = 5.6 Hz, 2H), 2.30 (s, 3H), 1.78-1.76 (m, 2H).

### Example 174

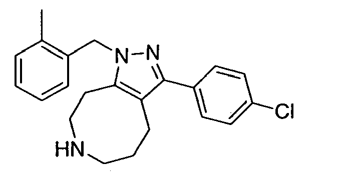

3-(4-Chloro-phenyl)-1-(2-methyl-benzyl)-4,5,6,7,8,9-hexahydro-1*H*-1,2,7-triaza-cyclopentacyclooctene.
The title compound (20 mg) was obtained as in Example 173. MS (ESI): exact mass calculated for C₂₂H₂₄CIN₃, 365.17; found, *m*/*z* 366.2 [M+H]⁺. ¹H NMR (500 MHz, CD₃OD): 7.48-7.46 (m, 2H), 7.39-7.36 (m, 2H), 7.14-7.05 (m, 3H), 6.55-6.54 (m, 1 H), 5.39 (s, 2H), 3.02-3.00 (m, 2H), 2.98-2.96 (m, 4H), 2.80-2.78 (m, 2H), 2.30-2.28 (s, 3H), 1.99-1.97 (m, 2H).

### Example 175

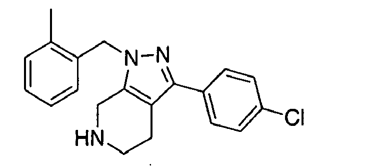

3-(4-Chloro-phenyl)-1-(2-methyl-benzyl)-4,5,6,7-tetrahydro-1*H*-pyrazolo[3,4-c]pyridine.
The title compound (22 mg) was prepared from 3-oxo-pyrrolidine-1-carboxylic acid *tert*-butyl ester (0.858 g) and 1-(4-chlorophenyl)-2-diazo-ethanone (Example 103, Step A; 5.79 mmol) as in Example 173. MS (ESI): exact mass calculated for C₁₇H₂₂ClN₃O, 337.13; found, *m*/*z* 338.2 [M+H]⁺. ¹H NMR (500 MHz, CD₃OD): 7.62-7.60 (m, 2H), 7.36-7.34 (m, 2H), 7.14-7.06 (m, 3H), 6.76 (d, *J* = 7.5 Hz, 1 H), 5.33 (s, 2H), 4.09 (s, 2H), 3.38 (t, *J* = 6.1 Hz, 2H), 3.10 (t, *J* = 6.1 Hz, 2H), 2.25 (s, 3H).

### Example 176

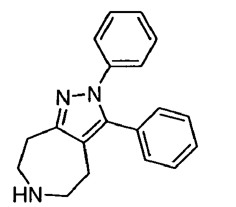

2,3-Diphenyl-2,4,5,6,7,8-hexahydro-1,2,6-triaza-azulene.
Step A. 3-Oxo-2-phenyl-2,3,4,5,7,8-hexahydro-1*H*-1,2,6-triaza-azulene-6-carboxylic acid *tert*-butvi ester. To a solution of the compound (3.13 g) from Example 59, Step A in 80 mL of EtOH was added 1.2 mL of phenylhydrazine. The resulting solution was heated at reflux for 3 days and then was cooled to RT and the solvent was removed *in vacuo.* The residue was chromatographed on SiO₂ (0 to 80% EtOAc/hexanes) to afford 3.13 g of the desired compound. MS (ESI): exact mass calculated for C₁₈H₂₃N₃O₃, 329.17; found, *m*/*z* 330.2 [M+H]⁺.
Step B. 2-Phenyl-3-trifluoromethanesulfonyloxy-4,5,7,8-tetrahydro-2H-1,2,6-triaza-azulene-6-carboxylic acid *tert*-butyl ester. To a stirred solution of the above compound (1.79 g) in 35 mL of CH₂Cl₂ was added 3.0 mL of *i*-Pr₂NEt and 3.05 g of *N*-phenyltrifluoromethanesulfonimide. The mixture was heated at reflux for 24 h and then was concentrated *in vacuo.* Chromatography on SiO₂ (0 to 75% EtOAc/hexanes) afforded 1.88 g of the desired compound. MS (ESI): exact mass calculated for C₁₉H₂₂F₃N₃O₅S, 461.12; found, *m*/*z* 407.1 [M+H]⁺.
Step C. 2,3-Diphenyl-4,5,7,8-tetrahydro-2*H*-1,2,6-triaza-azulene-6-carboxylic acid *tert*-butyl ester. To a solution of the above compound (0.28 g) in 5 mL of 1,4-dioxane was added 0.29 g of K₃PO₄, 104.3 mg of phenylboronic acid and 43.0 mg of PdCl₂dppf. The mixture was heated at 80 °C for 3 h. More phenylboronic acid (0.10 g) and PdCl₂dppf (26 mg) were added and the temperature was increased to 100 °C. After an additional 12 h, the mixture was poured into water (100 mL) and extracted with CH₂Cl₂ (3 x 20 mL). The combined organic layers were filtered through diatomaceous earth and the filtrate was concentrated *in vacuo.* Chromatography on SiO₂ (0 to 20% EtOAc/hexanes) afforded 158.8 mg of the desired compound. MS (ESI): exact mass calculated for C₂₄H₂₇N₃O₂, 389.21; found, *m*/*z* 390.2 [M+H]⁺.
Step D. To a stirred solution of the above compound (158.8 mg) in 5 mL of EtOH was added 2 mL of 1.0 M HCl in Et₂O. The mixture was stirred at RT for 12 h and concentrated *in vacuo* to give 75.6 mg of the title compound. MS (ESI): exact mass calculated for C₁₉H₁₉N₃, 289.16; found, *m*/*z* 290.2 [M+H]⁺. ¹H NMR (500 MHz, CD₃OD): 7.41-7.38 (m, 3H), 7.36-7.32 (m, 3H), 7.23-7.18 (m, 4H), 3.49-3.45 (m, 2H), 3.38-3.34 (m, 2H), 3.26-3.23 (m, 2H), 2.96-2.93 (m, 2H).

### Example 177

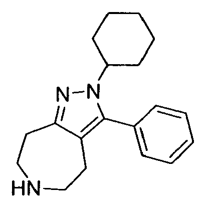

2-Cyclohexyl-3-phenyl-2,4,5,6,7,8-hexahydro-1,2,6-triaza-azulene.
Step A. Cyclohexyl-hydrazine hydrochloride. To a solution of cyclohexanone (1.25 mL) in hexanes (8 mL) was added 1.59 g of *tert*-butyl carbazate. The mixture was heated at reflux for 10 min and then allowed to cool to RT. The white precipitate that had formed was removed by filtration and washed with cold hexanes. The white solid was then treated with BH₃ (1.0 M in THF, 12 mL). After stirring at RT for 20 min, the mixture was treated with 16 mL of 6 N HCl. The mixture was heated at 110°C for 20 min and then was concentrated *in vacuo.* The residue was treated with 30 mL of THF. The title compound (1.82 g), a white solid, was collected from this mixture by filtration. MS (ESI): exact mass calculated for C₆H₁₄N₂, 114.12; found, *m*/*z* 115.1 [M+H]⁺. ¹H NMR (500 MHz, CDCl₃): 3.05-2.99 (m, 1 H), 2.11-2.09 (m, 2H), 1.88-1.86 (m, 2H), 1.72-1.69 (m, 1 H), 1.37-1.19 (m, 5H).
Step B. 2-Cyclohexyl-3-trifluoromethanesulfonyloxy-4,5,7,8-tetrahydro-2*H-*1,2,6-triaza-azulene-6-carboxylic acid *tert*-butyl ester. The desired compound was made as in Steps A and B of Example 176, with cyclohexylhydrazine hydrochloride from Step A in place of phenylhydrazine. The hydrazine salt was neutralized with Dowex^{®} 550 resin prior to use.
Step C. 2-Cyclohexyl-3-phenyl-4,5,7,8-tetrahydro-2*H*-1,2,6-triaza-azulene-6-carboxylic acid *tert*-butyl ester. To a solution of 126 mg of the compound from Step A in 3 mL of 1,4-dioxane were added 229 mg of K₃PO₄, 131 mg of phenylboronic acid, and 7.5 mg of dppf. PdCl₂dppf (22 mg) was then added and the mixture was heated at reflux overnight. The mixture was concentrated *in vacuo* and the residue was dissolved in toluene. The solution was filtered through diatomaceous earth and the filtrate was concentrated to afford 202 mg of an oil. Chromatography on SiO₂ (5 to 25% EtOAc/hexanes) provided 98.7 mg of the desired compound. MS (ESI): exact mass calculated for C₂₄H₃₃N₃O₂, 395.26; found, m/z 396.2 [M+H]⁺.
Step D. The above compound (98.7 mg) was converted to the title compound (71.0 mg) as in Example 43, Step E, and the crude product was chromatographed on SiO₂ (2 to 8% 2 M NH₃ in MeOH/EtOAc). MS (ESI): exact mass calculated for C₁₉H₂₅N₃, 295.20; found, *m*/*z* 296.2 [M+H]⁺. ¹H NMR (500 MHz, CDCl₃): 7.59-7.49 (m, 3H), 7.35-7.29 (m, 2H), 3.97-3.88 (m, 1 H), 3.44-3.38 (m, 2H), 3.34-3.27 (m, 2H), 3.20-3.14 (m, 2H), 2.81-2.73 (m, 2H), 1.99-1.76 (m, 6H), 1.65 (br s, 1 H), 1.28-1.17 (m, 3H).

### Example 178

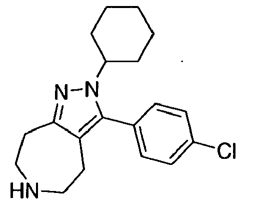

3-(4-Chloro-phenyl)-2-cyclohexyl-2,4,5,6,7,8-hexahydro-1,2,6-triaza-azulene.
The title compound (48 mg) was prepared as in Example 177, Steps C and D, using 129 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 173 mg of 4-chlorophenylboronic acid. MS (ESI): exact mass calculated for Cₗ₉H₂₄ClN₃, 329.17; found, *m*/*z* 330.1 [M+H]⁺. ¹H NMR (500 MHz, CDCI₃): 7.60-7.53 (m, 2H), 7.36-7.27 (m, 2H), 3.94-3.83 (m, 1 H), 3.43-3.36 (m, 2H), 3.34-3.26 (m, 2H), 3.2-3.12 (m, 2H), 2.80-2.72 (m, 2H), 1.98-1.76 (m, 6H), 1.67 (br s, 1 H), 1.32-1.17 (m, 3H).

### Example 179

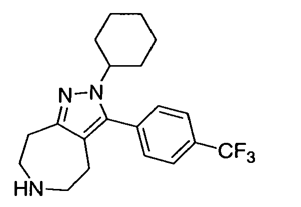

2-Cyclohexyl-3-(4-trifluoromethyl-phenyl)-2,4,5,6,7,8-hexahydro-1,2,6-triaza-azulene.
The title compound (68 mg) was prepared as in Example 177, Steps C and D, using 130 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 132 mg of 4-trifluoromethylphenylboronic acid. MS (ESI): exact mass calculated for C₂₀H₂₄F₃N₃, 363.19; found, *m*/*z* 364.2 [M+H]⁺. ¹H NMR (500 MHz, CDCl₃): 7.90-7.84 (m, 2H), 7.57-7.50 (m, 2H), 4.64 (br s, 2H), 3.94-3.85 (m, 1 H), 3.33-3.05 (m, 4H), 2.93-2.72 (m, 2H), 2.00-1.76 (m, 6H), 1.67 (br s, 1 H), 1.38-1.17 (m, 3H).

### Example 180

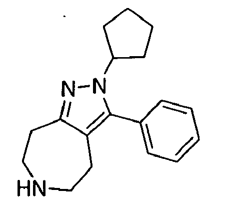

2-Cyclopentyl-3-phenyl-2,4,5,6,7,8-hexahydro-1,2,6-triaza-azulene.
Step A. 2-Cyclopentyl-3-trifluoromethanesulfonyloxy-4,5,7,8-tetrahydro-2*H-*1,2,6-triaza-azulene-6-carboxylic acid tert-butyl ester. The desired triflate was prepared as in Steps A and B of Example 176, using cyclopentylhydrazine hydrochloride (made according to the procedure of Example 177, Step A using cyclopentanone in place of cyclohexanone) in place of phenylhydrazine, *t-*butanol in place of EtOH, with the addition of 3 equiv. of triethylamine.
Step B. The title compound (52 mg) was prepared from the product of Step A (101 mg) according to the procedure of Example 177, Steps C and D, using 109 mg of phenylboronic acid. MS (ESI): exact mass calculated for C₁₈H₂₃N₃, 281.19; found, *m*/*z* 282.1 [M+H]⁺. ¹H NMR (500 MHz, CDCl₃): 7.58-7.48 (m, 3H), 7.36-7.30 (m, 2H), 4.50 (m, 1 H), 3.44-3.38 (m, 2H), 3.34-3.27 (m, 2H), 3.22-3.16 (m, 2H), 2.81-2.75 (m, 2H), 2.06-1.84 (m, 6H), 1.65-1.54 (m, 2H).

### Example 181

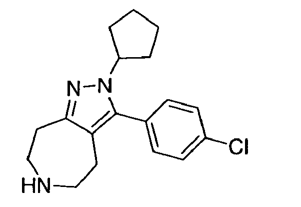

3-(4-Chloro-phenyl)-2-cyclopentyl-2,4,5,6,7,8-hexahydro-1,2,6-triaza-azulene.
The title compound (74 mg) was prepared as in Example 177, Steps C and D, using 215 mg of 2-cyclopentyl-3-trifluoromethanesulfonyloxy-4,5,7,8-tetrahydro-2*H*-1,2,6-triaza-azulene-6-carboxylic acid *tert*-butyl ester (Example 180, Step A) and 296 mg of 4-chlorophenylboronic acid. MS (ESI): exact mass calculated for C₁₈H₂₂ClN₃, 315.15; found, *m*/*z* 316.1 [M+H]⁺. ¹H NMR (500 MHz, CDCl₃): 7.59-7.53 (m, 2H), 7.36-7.30 (m, 2H), 4.48 (m, 1 H), 3.44-3.37 (m, 2H), 3.34-3.27 (m, 2H), 3.22-3.15 (m, 2H), 2.81-2.74 (m, 2H), 2.06-1.84 (m, 6H), 1.65-155 (m, 2H).

### Example 182

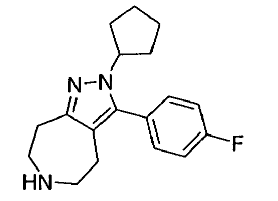

2-Cyclopentyl-3-(4-fluoro-phenyl)-2,4,5,6,7,8-hexahydro-1,2,6-triaza-azulene.
The title compound (113 mg) was prepared as in Example 177, Steps C and D, using 200 mg of 2-cyclopentyl-3-trifluoromethanesulfonyloxy-4,5,7,8-tetrahydro-2*H*-1,2,6-triaza-azulene-6-carboxylic acid *tert*-butyl ester (Example 180, Step A) and 185 mg of 4-fluorophenylboronic acid. MS (ESI): exact mass calculated for C₁₈H₂₂FN₃, 299.18; found, *m*/*z* 300.5 [M+H]⁺. ¹H NMR (500 MHz, CDCl₃): 7.45-7.39 (m, 2H), 7.35-7.29 (m, 2H), 4.53 (m, 1 H), 3.48-3.42 (m, 2H), 3.36-3.28 (m, 2H), 3.28-3.23 (m, 2H), 2.84-2.78 (m, 2H), 2.08-1.85 (m, 6H), 1.67-1.56 (m, 2H).

### Example 183

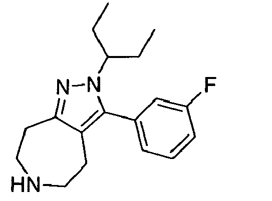

2-(1-Ethyl-propyl)-3-(3-fluoro-phenyl)-2,4,5,6,7,8-hexahydro-1,2,6-triaza-azulene.
Step A. 2-(1-Ethyl-propyl-3-trifluoromethanesulfonyoxy-4,5,7,8-tetrahydro-2*H-*1,2,6-triaza-azulene-6-carboxylic acid tert-butyl ester. The desired triflate was prepared as in Steps A and B of Example 176, using (1-ethyl-propyl)-hydrazine hydrochloride (made from 3-pentanone as described in Example 177, Step A) in place of phenylhydrazine. The hydrazine was neutralized with NaH in DMF prior to use.
Step B. The title compound (82 mg) was prepared as in Example 177, Steps C and D, using 150 mg of the triflate from Step A and 138 mg of 3-fluorophenylboronic acid. MS (ESI): exact mass calculated for C₁₈H₂₄FN₃, 301.20; found, *m*/*z* 302.4 [M+H]⁺. ¹H NMR (500 MHz, CDCl₃): 7.61-7.55 (m, 1 H), 7.31-7.25 (m, 1 H), 7.16-7.12 (m, 1 H), 7.10-7.05 (m, 1 H), 3.85-3.77 (m, 1 H), 3.45-3.40 (m, 2H), 3.35-3.29 (m, 2H), 3.23-3.18 (m, 2H), 2.82-2.76 (m, 2H), 1.97-1.80 (m, 2H), 1.79-1.70 (m, 2H), 0.71 (t, J= 7.4 Hz, 3H).

### Example 184

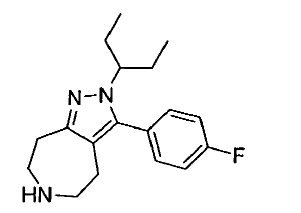

2-(1-Ethyl-propyl)-3-(4-fluoro-phenyl)-2,4,5,6,7,8-hexahydro-1,2,6-triaza-azulene.
The title compound (93 mg) was prepared as in Example 177, Steps C and D, using 150 mg of 2-(1-ethyl-propyl)-3-trifluoromethanesulfonyloxy-4,5,7,8-tetrahydro-2*H*-1,2,6-triaza-azulene-6-carboxylic acid *tert*-butyl ester (Example 183, Step A) and 138 mg of 3-fluorophenylboronic acid. MS (ESI): exact mass calculated for C₁₈H₂₄FN₃, 301.20; found, *m*/*z* 302.5 [M+H]⁺. ¹H NMR (500 MHz, CDCl₃): 7.44-7.30 (m, 4H), 3.92-3.85 (m, 1 H), 3.51-3.43 (m, 2H), 3.38-3.33 (m, 2H), 3.30-3.24 (m, 2H), 2.86-2.78 (m, 2H), 1.98-1.85 (m, 2H), 1.84-1.73 (m, 2H), 0.73 (t, *J* = 7.4 Hz, 3H).

### Example 185

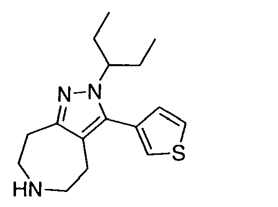

2-(1-Ethyl-propyl)-3-thiophen-3-yl-2,4,5,6,7,8-hexahydro-1,2,6-triaza-azulene.
The title compound (95 mg) was prepared as in Example 177, Steps C and D, using 150 mg of 2-(1-ethyl-propyl)-3-trifluoromethanesulfonyloxy-4,5,7,8-tetrahydro-2*H*-1,2,6-triaza-azulene-6-carboxylic acid *tert*-butyl ester (Example 183, Step A) and 126 mg of 3-thiopheneboronic acid. MS (ESI): exact mass calculated for C₁₆H₂₃N₃S, 289.16; found, *m*/*z* 290.4 [M+H]⁺. ¹H NMR (500 MHz, CDCl₃): 7.68-7.64 (m, 1 H), 7.52-7.48 (m, 1 H), 7.12-7.07 (m, 1 H), 3.93-3.86 (m, 1H), 3.44-3.39 (m, 2H), 3.34-3.28 (m, 2H), 3.22-3.17 (m, 2H), 2.85-2.79 (m, 2H), 1.95-1.84 (m, 2H), 1.79-1.69 (m, 2H), 0.71 (t, J = 7.4 Hz, 3H).

### Example 186

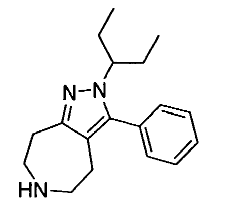

2-(1-Ethyl-propyl)-3-phenyl-2,4,5,6,7,8-hexahydro-1,2,6-triaza-azulene.
The title compound (40 mg) was prepared as in Example 177, Steps C and D, using 150 mg of 2-(1-ethyl-propyl)-3-trifluoromethanesulfonyloxy-4,5,7,8-tetrahydro-2*H*-1,2,6-triaza-azulene-6-carboxylic acid *tert*-butyl ester (Example 183, Step A) and 120 mg of phenylboronic acid. MS (ESI): exact mass calculated for C₁₈H₂₅N₃, 283.20; found, *m*/*z* 284.4 [M+H]⁺. ¹H NMR (500 MHz, CDCl₃): 7.48-7.38 (m, 3H), 7.24-7.19 (m, 2H), 3.77-3.70 (m, 1 H), 3.36-3.31 (m, 2H), 3.24-3.19 (m, 2H), 3.14-3.09 (m, 2H), 2.71-2.65 (m, 2H), 1.85-1.75 (m, 2H), 1.68-1.58 (m, 2H), 0.61 (t, J= 7.4 Hz, 3H).

### Example 187

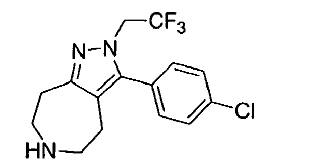

3-(4-Chloro-phenyl)-2-(2,2,2-trifluoro-ethyl)-2,4,5,6,7,8-hexahydro-1,2,6-triaza-azulene.
Step A. 2-(2,2,2-Trifluoro-ethyl)-3-trifluoromethanesulfonyloxy-4,5,7,8-tetrahydro-2*H*-1,2,6-triaza-azulene-6-carboxylic acid *tert*-butyl ester. The desired triflate was prepared as in Steps A and B of Example 176, using 2,2,2-trifluoroethylhydrazine in place of phenylhydrazine.
Step B. The title compound (40 mg) was prepared as in Example 177, Steps C and D, using 304 mg of the triflate from Step A and 407 mg of 4-chlorophenylboronic acid. MS (ESI): exact mass calculated for C₁₅H₁₅ClF₃N₃, 329.09; found, *m*/*z* 330.0 [M+H]⁺. ¹H NMR (500 MHz, CDCl₃): 7.62-7.52 (m, 2H), 7.40-7.29 (m, 2H), 4.70 (q, J = 8.6 Hz, 2H), 3.44-3.37 (m, 2H), 3.36-3.25 (m, 2H), 3.22-3.13 (m, 2H), 2.83-2.73 (m, 2H).

### Example 188

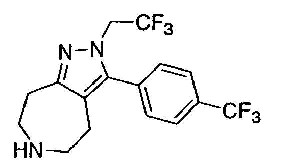

2-(2,2,2-Trifluoro-ethyl)-3-(4-trifluoromethyl-phenyl)-2,4,5,6,7,8-hexahydro-1,2,6-triaza-azulene.
The title compound (128 mg) was prepared as in Example 177, Steps C and D, using 288 mg of 2-(2,2,2-trifluoro-ethyl)-3-trifluoromethanesulfonyloxy-4,5,7,8-tetrahydro-2*H*-1,2,6-triaza-azulene-6-carboxylic acid *tert*-butyl ester (Example 187, Step A) and 468 mg of 4-trifluoromethylphenylboronic acid. MS (ESI): exact mass calculated for C₁₆H₁₅F₆N₃, 363.12; found, *m*/*z* 364.0 [M+H]⁺. ¹H NMR (500 MHz, CDCl₃): 7.93-7.83 (m, 2H), 7.62-7.54 (m, 2H), 4.75 (q, J= 8.6 Hz, 2H), 3.47-3.39 (m, 2H), 3.38-3.27 (m, 2H), 3.24-3.15 (m, 2H), 2.87-2.76 (m, 2H).

### Example 189

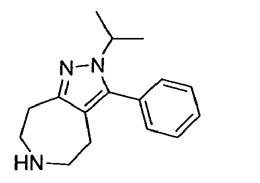

2-lsopropyl-3-phenyl-2,4,5,6,7,8-hexahydro-1,2,6-triaza-azulene.
Step A. 2-lsopropyl-3-trifluoromethanesulfonyloxy-4,5,7,8-tetrahydro-2*H*-1,2,6-triaza-azulene-6-carboxylic acid *tert*-butyl ester. The desired triflate was prepared as in Steps A and B of Example 176, using isopropylhydrazine hydrochloride in place of phenylhydrazine, *t*-butanol in place of EtOH, with the addition of 3 equiv. of triethylamine.
Step B. The title compound (93 mg) was prepared as in Example 177, Steps C and D, using 172 mg of the triflate from Step A and 147 mg of phenylboronic acid. MS (ESI): exact mass calculated for C₁₆H₂₁N₃, 255.17; found, *m*/*z* 256.5 [M+H]⁺. ¹H NMR (500 MHz, CDCl₃): 7.58-7.49 (m, 3H), 7.36-7.30 (m, 2H), 4.40 (m, 1H), 3.45-3.40 (m, 2H), 3.34-3.28 (m, 2H), 3.23-3.18 (m, 2H), 2.82-2.75 (m, 2H), 1.40 (d, *J* = 6.9 Hz, 6H).

### Example 190

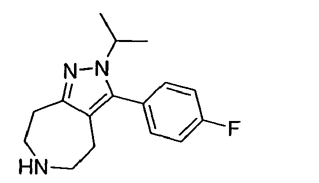

3-(4-Fluoro-phenyl)-2-isopropyl-2,4,5,6,7,8-hexahydro-1,2,6-triaza-azulene.
The title compound (92 mg) was prepared as in Example 177, Steps C and D, using 159 mg of 2-isopropyl-3-trifluoromethanesulfonyloxy-4,5,7,8-tetrahydro-2*H*-1,2,6-triaza-azulene-6-carboxylic acid *tert*-butyl ester (Example 189, Step A) and 156 mg of 4-fluorophenylboronic acid. MS (ESI): exact mass calculated for C₁₆H₂₀FN₃, 273.16; found, *m*/*z* 274.4 [M+H]⁺. ¹H NMR (500 MHz, CDCl₃): 7.42-7.35 (m, 2H), 7.33-7.27 (m, 2H), 4.37 (m, 1H), 3.46-3.39 (m, 2H), 3.34-3.28 (m, 2H), 3.23-3.18 (m, 2H), 2.81-2.74 (m, 2H), 1.41 (d, J= 6.9 Hz,6H).

### Example 191

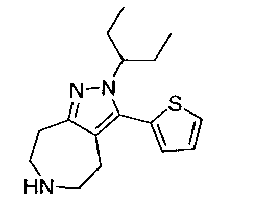

2-(1-Ethyl-propyl)-3-thiophen-2-yl-2,4,5,6,7,8-hexahydro-1,2,6-triaza-azulene.
The title compound (35 mg) was prepared as in Example 177, Steps C and D, using 148 mg of 2-(1-ethyl-propyl)-3-trifluoromethanesulfonyloxy-4,5,7,8-tetrahydro-2*H*-1,2,6-triaza-azulene-6-carboxylic acid *tert*-butyl ester (Example 183, Step A) and 122 mg of 2-thiopheneboronic acid. MS (ESI): exact mass calculated for C₁₆H₂₃N₃S, 289.16; found, *m*/*z* 290.5 [M+H]⁺. ¹H NMR (500 MHz, CDCl₃): 7.72-7.67 (m, 1 H), 7.25-7.21 (m, 1 H), 7.13-7.09 (m, 1 H), 4.01-3.94 (m, 1 H), 3.43-3.38 (m, 2H), 3.34-3.28 (m, 2H), 3.20-3.14 (m, 2H), 2.86-2.80 (m, 2H), 1.95-1.85 (m, 2H), 1.79-1.69 (m, 2H), 0.71 (t, J = 7.4 Hz, 3H).

### Example 192

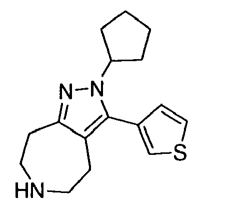

2-Cyclopentyl-3-thiophen-3-yl-2,4,5,6,7,8-hexahydro-1,2,6-triaza-azulene.
The title compound (114 mg) was prepared as in Example 177, Steps C and D, using 200 mg of 2-cyclopentyl-3-trifluoromethanesulfonyloxy-4,5,7,8-tetrahydro-2*H*-1,2,6-triaza-azulene-6-carboxylic acid *tert*-butyl ester (Example 180, Step A) and 169 mg of 3-thiopheneboronic acid. MS (ESI): exact mass calculated for C₁₆H₂₁N₃S, 287.15; found, *m*/*z* 288.4 [M+H]⁺. ¹H NMR (500 MHz, CDCl₃): 7.68-7.63 (m, 1 H), 7.56-7.51 (m, 1 H), 7.17-7.12 (m, 1 H), 4.58 (m, 1 H), 3.43-3.37 (m, 2H), 3.34-3.28 (m, 2H), 3.19-3.14 (m, 2H), 2.86-2.80 (m, 2H), 2.04-1.85 (m, 6H), 1.67-1.57 (m, 2H).

### Example 193

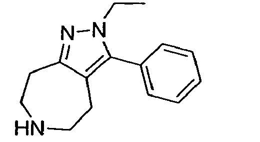

2-Ethyl-3-phenyl-2,4,5,6,7,8-hexahydro-1,2,6-triaza-azulene.
Step A. 2-Ethyl-3-trifluoromethanesulfonyloxy-4,5,7,8-tetrahydro-2H-1,2,6-triaza-azulene-6-carboxylic acid *tert*-butyl ester. The desired triflate was prepared as in Steps A and B of Example 176, using ethylhydrazine oxalate in place of phenylhydrazine, *t*-butanol in place of EtOH, with the addition of 3 equiv. of triethylamine.
Step B. The title compound (106 mg) was prepared as in Example 177, Steps C and D, using 198 mg of the triflate from Step A and 122 mg of phenylboronic acid. MS (ESI): exact mass calculated for C₁₅H₁₉N₃, 241.16; found, *m*/*z* 242.4 [M+H]⁺. ¹H NMR (500 MHz, CDCl₃): 7.61-7.54 (m, 3H), 7.43-7.39 (m, 2H), 4.11 (q, J = 7.1 Hz, 2H), 3.49-3.44 (m, 2H), 3.37-3.32 (m, 2H), 3.28-3.22 (m, 2H), 2.89-2.82 (m, 2H), 1.33 (t, J = 7.1 Hz, 3H).

### Example 194

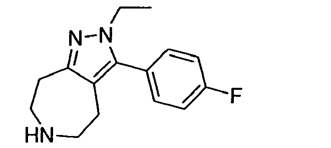

2-Ethyl-3-(4-fluoro-phenyl)-2,4,5,6,7,8-hexahydro-1,2,6-triaza-azulene.
The title compound (114 mg) was prepared as in Example 177, Steps C and D, using 208 mg of 2-ethyl-3-trifluoromethanesulfonyloxy-4,5,7,8-tetrahydro-2*H-*1,2,6-triaza-azulene-6-carboxylic acid *tert*-butyl ester (Example 193, Step A) and 211 mg of 4-fluorophenylboronic acid. MS (ESI): exact mass calculated for C₁₅H₁₈FN₃, 259.15; found, *m*/*z* 260.4 [M+H]⁺. ¹H NMR (500 MHz, CDCl₃): 7.41-7.35 (m, 2H), 7.32-7.26 (m, 2H), 3.99 (q, J = 7.1 Hz, 2H), 3.43-3.38 (m, 2H), 3.33-3.28 (m, 2H), 3.18-3.12 (m, 2H), 2.80-2.75 (m, 2H), 1.27 (t, J = 7.1 Hz, 3H).

### Example 195

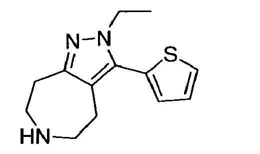

2-Ethyl-3-thiophen-2-yl-2,4,5,6,7,8-hexahydro-1,2,6-triaza-azulene.
The title compound (101 mg) was prepared as in Example 177, Steps C and D, using 148 mg of 2-ethyl-3-trifluoromethanesulfonyloxy-4,5,7,8-tetrahydro-2*H-*1,2,6-triaza-azulene-6-carboxylic acid *tert*-butyl ester (Example 193, Step A) and 306 mg of 2-thiopheneboronic acid. MS (ESI): exact mass calculated for C₁₃H₁₇N₃S, 247.11; found, *m*/*z* 248.4 [M+H]⁺. ¹H NMR (500 MHz, CDCl₃): 7.62-7.57 (m, 1 H), 7.16-7.11 (m, 1 H), 7.10-7.05 (m, 1 H), 3.98 (q, J = 7.1 Hz, 2H), 3.33-3.27 (m, 2H), 3.24-3.18 (m, 2H), 3.07-3.01 (m, 2H), 2.80-2.73 (m, 2H), 1.22 (t, J = 7.1 Hz, 3H).

### Example 196

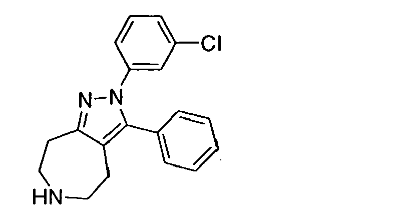

2-(3-Chloro-phenyl)-3-phenyl-2,4,5,6,7,8-hexahydro-1,2,6-triaza-azulene.
Step A. 2-(3-Chloro-phenyl)-3-phenyl-4,5,7,8-tetrahydro-2H-1,2,6-triaza-azulene-6-carboxylic acid *tert*-butyl ester. The desired compound (53.9 mg) was prepared from 142.7 mg of 2-(3-chloro-phenyl)-3-trifluoromethanesulfonyloxy-4,5,7,8-tetrahydro-2*H*-1,2,6-triaza-azulene-6-carboxylic acid *tert*-butyl ester (made as in Example 176, Steps A and B) replacing phenylhydrazine with (3-chloro-phenyl)-hydrazine, as described in Example 43, Step D, using 102.1 mg of phenylboronic acid. MS (ESI): exact mass calculated for C₂₄H₂₆ClN₃O₂, 423.17; found, *m*/*z* 424.1 [M+H]⁺.
Step B. The above compound (53.9 mg) was converted to the title compound (37.6 mg) as in Example 26, Step B. MS (ESI): exact mass calculated for C₁₉H₁₈ClN₃, 323.12; found, *m*/*z* 324.1 [M+H]⁺. ¹H NMR (500 MHz, CDCl₃): 7.38-7.32 (m, 4H), 7.16-7.09 (m, 4H), 6.96-6.93 (m, 1 H), 3.09-3.05 (m, 2H), 3.02-2.98 (m, 2H), 2.97-2.94 (m, 2H), 2.65-2.62 (m, 2H), 2.07 (br s, 1H).

### Example 197

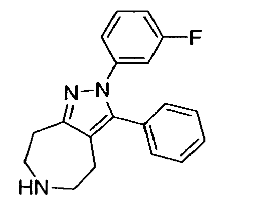

2-(3-Fluoro-phenyl)-3-phenyl-2,4,5,6,7,8-hexahydro-1,2,6-triaza-azulene.
The title compound (37.6 mg) was prepared from 339.2 mg of 2-(3-fluorophenyl)-3-trifluoromethanesulfonyloxy-4,5,7,8-tetrahydro-2*H*-1,2,6-triaza-azulene-6-carboxylic acid *tert*-butyl ester (made as in Example 176, Steps A and B from (3-fluoro-phenyl)-hydrazine) as described in Example 196, using 1,4-dioxane as the solvent. MS (ESI): exact mass calculated for C₁₉H₁₈FN₃, 307.15; found, *m*/*z* 308.1 [M+H]⁺. ¹H NMR (400 MHz, CDCl₃): 7.39-7.35 (m, 3H), 7.20-7.14 (m, 3H), 7.00-6.96 (m, 1 H), 6.94-6.86 (m, 2H), 3.13-3.09 (m, 2H), 3.06-3.02 (m, 2H), 3.01-2.96 (m, 2H), 2.69-2.66 (m, 2H).

### Example 198

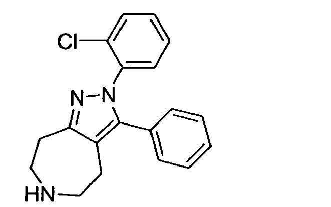

2-(2-Chloro-phenyl)-3-phenyl-2,4,5,6,7,8-hexahydro-1,2,6-triaza-azulene.
The title compound (17.2 mg) was prepared from 199.8 mg of 2-(2-chlorophenyl)-3-trifluoromethanesulfonyloxy-4,5,7,8-tetrahydro-2*H*-1,2,6-triaza-azulene-6-carboxylic acid *tert*-butyl ester (made from (2-chloro-phenyl)-hydrazine as in Example 176, Steps A and B), as described in Example 196 using 1,4-dioxane as the solvent. MS (ESI): exact mass calculated for C₁₉H₁₈ClN₃, 323.12; found, *m*/*z* 324.1 [M+H]⁺. ¹H NMR (500 MHz, CDCl₃): 7.37-7.34 (m, 2H), 7.29-7.24 (m, 5H), 7.14-7.10 (m, 2H), 3.12-3.09 (m, 2H), 3.04-2.99 (m, 4H), 2.74-2.71 (m, 2H), 2.13 (br s, 1H).

### Example 199

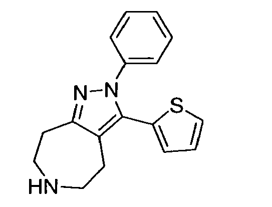

2-Phenyl-3-thiophen-2-yl-2,4,5,6,7,8-hexahydro-1,2,6-triaza-azulene.
Step A. 2-Phenyl-3-thiophen-2-yl-4,5,7,8-tetrahydro-2H-1,2,6-triaza-azulene-6-carboxylic acid tert-butyl ester. To a solution of 199.8 mg of 2-phenyl-3-trifluoromethanesulfonyloxy-4,5,7,8-tetrahydro-2*H*-1,2,6-triaza-azulene-6-carboxylic acid *tert*-butyl ester (Example 176, Step B) in 3.5 mL of DMF were added 0.6 mL of 2 M aq. Na₂CO₃ and 75.6 mg of thiophene-2-boronic acid. PdCl₂dppf (20.2 mg) was added and the mixture was heated at 80 °C for 16 h.
   The mixture was poured into water (50 mL) and extracted with CH₂Cl₂ (3 x 15 mL) and the combined organic layers were concentrated *in vacuo.* Chromatography on SiO₂ (0 to 50% EtOAc/hexanes) afforded 58.9 mg of the desired compound as a white solid. MS (ESI): exact mass calculated for C₂₂H₂₅N₃O₂S, 395.17; found, *m*/*z* 396.1 [M+H]⁺.
Step B. The above compound (58.9 mg) was converted to the title compound (28.1 mg) as in Example 26, Step B. MS (ESI): exact mass calculated for C₁₇H₁₇N₃S, 295.11; found, *m*/*z* 296.1 [M+H]⁺. ¹H NMR (500 MHz, CDCl₃): 7.36 (dd, J = 5.2, 1.3 Hz, 1 H), 7.32-7.23 (m, 5H), 7.01 (dd, J = 5.2, 3.3 Hz, 1 H), 6.85 (dd, J = 3.3, 1.3 Hz, 1 H), 3.11-3.08 (m, 2H), 3.03-2.99 (m, 4H), 2.76-2.73 (m, 2H), 2.12 (br s, 1 H).

### Example 200

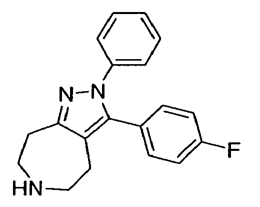

3-(4-Fluoro-phenyl)-2-phenyl-2,4,5,6,7,8-hexahydro-1,2,6-triaza-azulene.
The title compound (70.0 mg) was prepared from 207.0 mg of 2-phenyl-3-trifluoromethanesulfonyloxy-4,5,7,8-tetrahydro-2*H*-1,2,6-triaza-azulene-6-carboxylic acid *tert*-butyl ester (Example 176, Step B) and 98.5 mg of 4-fluorophenylboronic acid as in Example 199. MS (ESI): exact mass calculated for C₁₉H₁₈FN₃, 307.15; found, *m*/*z* 308.2 [M+H]⁺. ¹H NMR (500 MHz, CDCl₃): 7.28-7.24 (m, 2H), 7.22-7.16 (m, 3H), 7.13-7.10 (m, 2H), 7.05-7.01 (m, 2H), 3.12-3.08 (m, 2H), 3.05-3.02 (m, 2H), 3.01-2.98 (m, 2H), 2.68-2.64 (m, 2H).

### Example 201

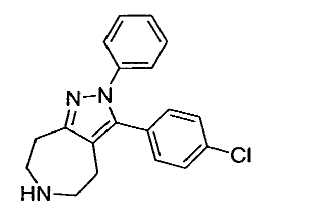

3-(4-Chloro-phenyl)-2-phenyl-2,4,5,6,7,8-hexahydro-1,2,6-triaza-azulene.
The title compound (9.3 mg) was prepared from 164.0 mg of 2-phenyl-3-trifluoromethanesulfonyloxy-4,5,7,8-tetrahydro-2*H*-1,2,6-triaza-azulene-6-carboxylic acid *tert*-butyl ester (Example 176, Step B) and 63.8 mg of 4-chlorophenylboronic acid as in Example 196. MS (ESI): exact mass calculated for C₁₉H₁₈ClN₃, 323.12; found, *m*/*z* 324.1 [M+H]⁺. ¹H NMR (500 MHz, CDCl₃): 7.33-7.25 (m, 4H), 7.23-7.16 (m, 3H), 7.09-7.06 (m, 2H), 3.10-3.07 (m, 2H), 3.03-3.00 (m, 2H), 2.99-2.96 (m, 2H), 2.66-2.63 (m, 2H).

### Example 202

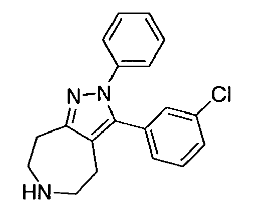

3-(3-Chloro-phenyl)-2-phenyl-2,4,5,6,7,8-hexahydro-1,2,6-triaza-azulene.
The title compound (37.5 mg) was prepared from 192.3 mg of 2-phenyl-3-trifluoromethanesulfonyloxy-4,5,7,8-tetrahydro-2*H*-1,2,6-triaza-azulene-6-carboxylic acid *tert*-butyl ester (Example 176, Step B) and 84.7 mg of 3-chlorophenylboronic acid as in Example 199. MS (ESI): exact mass calculated for C₁₉H₁₈CIN₃, 323.12; found, *m*/*z* 324.1 [M+H]⁺. ¹H NMR (500 MHz, CDCl₃): 7.32-7.16 (m, 8H), 7.01-6.98 (m, 1 H), 3.12-3.08 (m, 2H), 3.05-3.02 (m, 2H), 3.01-2.98 (m, 2H), 2.69-2.66 (m, 2H).

### Example 203

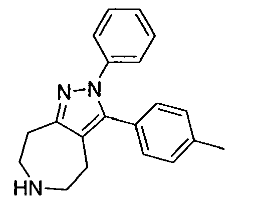

2-Phenyl-3-p-tolyl-2,4,5,6,7,8-hexahydro-1,2,6-triaza-azulene.
The title compound (17.5 mg) was prepared from 188.9 mg of 2-phenyl-3-trifluoromethanesulfonyloxy-4,5,7,8-tetrahydro-2*H*-1,2,6-triaza-azulene-6-carboxylic acid *tert*-butyl ester (Example 176, Step B) and 93.3 mg of *p-*tolylboronic acid as in Example 199, using DME as the solvent. MS (ESI): exact mass calculated for C₂₀H₂₁N₃, 303.17; found, *m*/*z* 304.2 [M+H]⁺. ¹H NMR (500 MHz, CDCl₃): 7.26-7.23 (m, 2H), 7.21-7.16 (m, 3H), 7.15-7.12 (m, 2H), 7.04-7.01 (m, 2H), 3.11-3.07 (m, 2H), 3.04-3.00 (m, 2H), 2.98-2.96 (m, 2H), 2.68-2.65 (m, 2H), 2.35 (s, 3H).

### Example 204

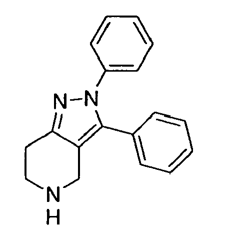

2,3-Diphenyl-4,5,6,7-tetrahydro-2H pyrazolo[4,3-c]pyridine.
Step A. 2,3-Diphenyl-2,4,6,7-tetrahydro-pyrazolof4,3-clpyridine-5-carboxylic acid tert-butyl ester. To a solution of 156.6 mg of 2-phenyl-3-trifluoromethanesulfonyloxy-2,4,6,7-tetrahydro-pyrazolo[4,3-c]pyridine-5-carboxylic acid *tert*-butyl ester (made from 4-oxo-piperidine-1,3-dicarboxylic acid 1-*tert*-butyl ester 3-methyl ester as in Example 176, Steps A and B) in THF/H₂O (10:1, 4 mL) were added 148.4 mg of K₂CO₃ and 56.2 mg of phenylboronic acid. PdCl₂dppf (23.4 mg) was added and the mixture was heated at reflux for 16 h. The mixture was concentrated *in vacuo.* The residue was chromatographed on SiO₂ (0 to 75% EtOAc/hexanes) to afford 45.6 mg of the desired ester as an off-white solid. MS (ESI): exact mass calculated for C₂₃H₂₅N₃O₂, 375.19; found, *m*/*z* 376.2 [M+H]⁺.
Step B. The above compound (45.6 mg) was converted to the title compound (24.5 mg) as in Example 26, Step B. MS (ESI): exact mass calculated for C₁₈H₁₇N₃, 275.14; found, *m*/*z* 276.2 [M+H]⁺. ¹H NMR (500 MHz, CDCl₃): 7.34-7.22 (m, 8H), 7.16-7.12 (m, 2H), 3.96 (s, 2H), 3.23 (t, J = 6.0 Hz, 2H), 2.88 (t, J = 6.0 Hz, 2H).

### Example 205

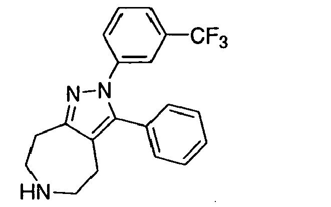

3-Phenyl-2-(3-trifluoromethyl-phenyl)-2,4,5,6,7,8-hexahydro-1,2,6-triaza-azulene.
Step A. 3-Phenyl-2-(3-trifluoromethyl-phenyl)-4,5,7,8-tetrahydro-2*H*-1,2,6-triaza-azulene-6-carboxylic acid *tert*-butyl ester. The desired compound (172.0 mg) was prepared from 279.1 of mg of 3-trifluoromethanesulfonyloxy-2-(3-trifluoromethyl-phenyl)-4,5,7,8-tetrahydro-2*H*-1,2,6-triaza-azulene-6-carboxylic acid *tert*-butyl ester (made from (3-trifluoromethyl-phenyl)-hydrazine as in Example 176, Steps A and B) and 0.21 g of phenylboronic acid, as described in Example 177, Step C. MS (ESI): exact mass calculated for C₂₅H₂₆F₃N₃O₂, 457.20; found, *m*/*z* 458.1 [M+H]⁺.
Step B. The above compound (172.0 mg) was converted to the title compound (106.4 mg) as in Example 26, Step B. MS (ESI): exact mass calculated for C₂₀H₁₈F₃N₃, 357.15; found, *m*/*z* 358.1 [M+H]⁺. ¹H NMR (500 MHz, CDCl₃): 7.53 (s, 1 H), 7.44-7.41 (m, 1 H), 7.39-7.29 (m, 5H), 7.17-7.13 (m, 2H), 3.12-3.09 (m, 2H), 3.06-3.02 (m, 2H), 3.00-2.97 (m, 2H), 2.69-2.66 (m, 2H).

### Example 206

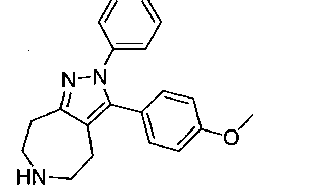

3-(4-Methoxy-phenyl)-2-phenyl-2,4,5,6,7,8-hexahydro-1,2,6-triaza-azulene.
The title compound (43.6 mg) was prepared from 198.3 mg of 2-phenyl-3-trifluoromethanesulfonyloxy-4,5,7,8-tetrahydro-2*H*-1,2,6-triaza-azulene-6-carboxylic acid *tert*-butyl ester (Example 176, Step B) and 94.7 mg of 4-methoxyphenylboronic acid as described in Example 199. MS (ESI): exact mass calculated for C₂₀H₂₁N₃O, 319.17; found, *m*/*z* 320.2 [M+H]⁺. ¹H NMR (500 MHz, CDCl₃): 7.28-7.24 (m, 2H), 7.21-7.17 (m, 3H), 7.07 (d, J = 8.8 Hz, 2H), 6.87 (d, J = 8.8 Hz, 2H), 3.81 (s, 3H), 3.11-3.08 (m, 2H), 3.04-3.01 (m, 2H), 3.00-2.97 (m, 2H), 2.68-2.65 (m, 2H).

### Example 207

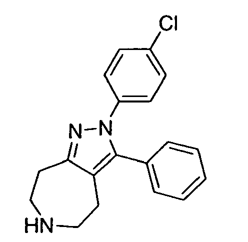

2-(4-Chloro-phenyl)-3-phenyl-2,4,5,6,7,8-hexahydro-1,2,6-triaza-azulene.
The title compound (50.4 mg) was prepared from 201.1 mg of 2-(4-chlorophenyl)-3-trifluoromethanesulfonyloxy-4,5,7,8-tetrahydro-2*H*-1,2,6-triaza-azulene-6-carboxylic acid *tert*-butyl ester (made from (4-chloro-phenyl)-hydrazine as in Example 176, Steps A and B), and 65.1 mg of phenylboronic acid as described in Example 204. MS (ESI): exact mass calculated for C₁₉H₁₈ClN₃, 323.12; found, *m*/*z* 324.1 [M+H]⁺. ¹H NMR (500 MHz, CDCl₃): 7.39-7.34 (m, 3H), 7.22-7.19 (m, 2H), 7.15-7.11 (m, 4H), 3.11-3.07 (m, 2H), 3.04-3.00 (m, 2H), 2.99-2.96 (m, 2H), 2.67-2.64 (m, 2H).

### Example 208

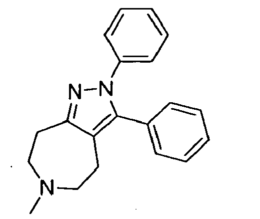

6-Methyl-2,3-diphenyl-2,4,5,6,7,8-hexahydro-1,2,6-triaza-azulene.
To a solution of 33.5 mg of 2,3-diphenyl-2,4,5,6,7,8-hexahydro-1,2,6-triaza-azulene (Example 176, Step D) in 5 mL of CH₂Cl₂ were added 0.15 g of paraformaldehyde and 0.15 g of NaBH(OAc)₃. The mixture was stirred at RT for 12 h and was diluted with 20 mL of 1 M NaOH. After stirring for 3 h, the mixture was extracted with CH₂CI₂ (2x10 mL) and the combined organic layers were concentrated. Chromatography on SiO₂ (0 to 5% 2 M NH₃ in MeOH/CH₂CI₂) gave 22.3 mg of the title compound as a white solid. MS (ESI): exact mass calculated for C₂₀H₂₁N₃, 303.17; found, *m*/*z* 304.2 [M+H]⁺. ¹H NMR (500 MHz, CDCl₃): 7.35-7.30 (m, 3H), 7.27-7.21 (m, 2H), 7.20-7.16 (m, 3H), 7.15-7.12 (m, 2H), 3.06-3.02 (m, 2H), 2.83-2.79 (m, 2H), 2.72-2.67 (m, 4H), 2.50 (s, 3H).

### Example 209

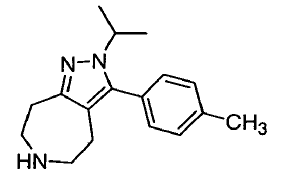

2-lsopropyl-3-p-tolyl-2,4,5,6,7,8-hexahydro-1,2,6-triaza-azulene.
The title compound (129 mg) was prepared as in Example 177, Steps C and D, using 204 mg of 2-isopropyl-3-trifluoromethanesulfonyloxy-4,5,7,8-tetrahydro-2*H*-1,2,6-triaza-azulene-6-carboxylic acid *tert*-butyl ester (Example 189, Step A) and 194 mg of 4-methylphenylboronic acid. MS (ESI): exact mass calculated for C₁₇H₂₃N₃, 269.19; found, *m*/*z* 270.5 [M+H]⁺. ¹H NMR (500 MHz, CD₃OD): 7.28 (d, *J =* 7.7 Hz, 2H), 7.12 (d, *J =* 7.7 Hz, 2H), 4.32 (m, 1 H), 3.34-3.33 (m, 2H), 3.12-3.10 (m, 2H), 2.70-2.68 (m, 2H), 2.33 (s, 3H), 1.30 (d, *J* = 6.6 Hz, 6H).

### Example 210

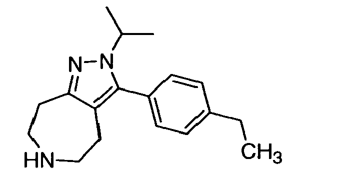

3-(4-Ethyl-phenyl)-2-isopropyl-2,4,5,6,7,8-hexahydro-1,2,6-triaza-azulene.
The title compound (134 mg) was prepared as in Example 177, Steps C and D, using 202 mg of 2-isopropyl-3-trifluoromethanesulfonyloxy-4,5,7,8-tetrahydro-2*H*-1,2,6-triaza-azulene-6-carboxylic acid *tert*-butyl ester (Example 189, Step A) and 212 mg of 4-ethylphenylboronic acid. MS (ESI): exact mass calculated for C₁₈H₂₅N₃, 283.20; found, *m*/*z* 284.5 [M+H]⁺. ¹H NMR (500 MHz, CD₃OD): 7.44 (d, *J* = 7.7 Hz, 2H), 7.31 (d, *J* = 7.7 Hz, 2H), 4.52 (m, 1 H), 3.50-3.48 (m, 2H), 3.36-3.34 (m, 2H), 2.85-2.83 (m, 2H), 2.75 (q, J = 7.7 Hz, 2H), 1.47 (d, J = 6.6 Hz, 6H), 1.29 (t, J = 7.7 Hz, 3H).

### Example 211

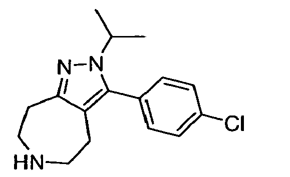

3-(4-Chloro-phenyl)-2-isopropyl-2,4,5,6,7,8-hexahydro-1,2,6-triaza-azulene.
The title compound (82 mg) was prepared as in Example 177, Steps C and D, using 205 mg of 2-isopropyl-3-trifluoromethanesulfonyloxy-4,5,7,8-tetrahydro-2H 1,2,6-triaza-azulene-6-carboxylic acid *tert*-butyl ester (Example 189, Step A) and 332 mg of 2-(4-chloro-phenyl)-benzo[1,3,2]dioxaborole. MS (ESI): exact mass calculated for C₁₆H₂₀ClN₃, 289.13; found, *m*/*z* 290.4 [M+H]⁺, 292.4 [M+H]⁺. ¹H NMR (500 MHz, CD₃OD): 7.57 (d, *J*= 8.5 Hz, 2H), 7.33 (d, *J*= 8.5 Hz, 2H), 4.36 (m, 1 H), 3.44-3.40 (m, 2H), 3.20-3.18 (m, 2H), 2.81-2.76 (m, 2H), 1.40 (d, J = 6.6 Hz, 6H).

### Example 212

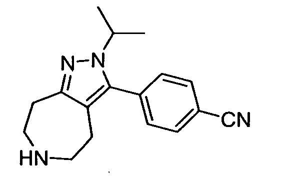

4-(2-Isopropyl-2,4,5,6,7,8-hexahydro-1,2,6-triaza-azulen-3-yl)-benzonitrile.
The title compound (95 mg) was prepared as in Example 177, Steps C and D, using 205 mg of 2-isopropyl-3-trifluoromethanesulfonyloxy-4,5,7,8-tetrahydro-2*H*-1,2,6-triaza-azulene-6-carboxylic acid *tert*-butyl ester (Example 189, Step A) and 211 mg of 4-cyanophenylboronic acid. MS (ESI): exact mass calculated for C₁₇H₂₀N₄, 280.17; found, *m*/*z* 281.4 [M+H]⁺. ¹H NMR (500 MHz, CD₃OD): 7.57 (d, J = 8.5 Hz, 2H), 7.33 (d, J = 8.5 Hz, 2H), 4.36 (m, 1H), 3.42-3.40 (m, 2H), 3.19-3.17 (m, 2H), 2.79-2.77 (m, 2H), 1.40 (d, *J* = 6.6 Hz, 6H).

### Example 213

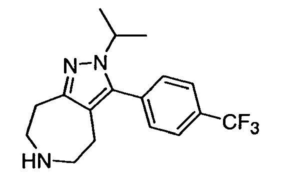

2-Isopropyl-3-(4-trifluoromethyl-phenyl)-2,4,5,6,7,8-hexahydro-1,2,6-triaza-azulene.
The title compound (103 mg) was prepared as in Example 177, Steps C and D, using 199 mg of 2-isopropyl-3-trifluoromethanesulfonyloxy-4,5,7,8-tetrahydro-2*H*-1,2,6-triaza-azulene-6-carboxylic acid *tert*-butyl ester (Example 189, Step A) and 265 mg of 4-trifluoromethylphenylboronic acid. MS (ESI): exact mass calculated for C₁₇H₂₀F₃N₃, 323.16; found, *m*/*z* 324.4 [M+H]⁺. ¹H NMR (500 MHz, CD₃OD): 7.86 (d, J= 8.0 Hz, 2H), 7.55 (d, J = 8.0 Hz, 2H), 4.34 (m, 1 H), 3.43-3.40 (m, 2H), 3.20-3.18 (m, 2H), 2.80-2.78 (m, 2H), 1.40 (d, J = 6.6 Hz, 6H).

### Example 214

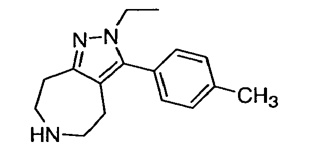

2-Ethyl-3-p-tolyl-2,4,5,6,7,8-hexahydro-1,2,6-triaza-azulene.
The title compound (136 mg) was prepared as in Example 177, Steps C and D, using 201 mg of 2-ethyl-3-trifluoromethanesulfonyloxy-4,5,7,8-tetrahydro-2H 1,2,6-triaza-azulene-6-carboxylic acid tert-butyl ester (Example 193, Step A) and 198 mg of 4-methylphenylboronic acid. MS (ESI): exact mass calculated for C₁₆H₂₁N₃, 255.17; found, *m*/*z* 256.5 [M+H]⁺. ¹H NMR (500 MHz, CD₃OD): 7.38 (d, J = 8.0 Hz, 2H), 7.25 (d, J = 8.0 Hz, 2H), 4.67 (br s, 1 H), 4.05 (q, J = 7.1 Hz, 2H), 3.92-3.41 (m, 2H), 3.28-3.18 (m, 3H), 2.89-2.80 (m, 2H), 2.43 (s, 3H), 1.29 (t, J = 7.1 Hz, 3H).

### Example 215

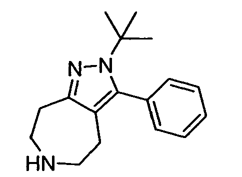

2-tert-Butyl-3-phenyl-2,4,5,6,7,8-hexahydro-1,2,6-triaza-azulene.
Step A. 2-(*tert*-Butyl)-3-trifluoromethanesulfonyloxy-4,5,7,8-tetrahydro-2*H-*1,2,6-triaza-azulene-6-carboxylic acid tert-butyl ester. The desired triflate was prepared as in Steps A and B of Example 176, using *tert*-butyl hydrazine hydrochloride in place of phenylhydrazine, *t*-butanol in place of EtOH, with the addition of 3 equiv. of triethylamine.
Step B. The title compound (53 mg) was prepared as in Example 177, Steps C and D, using 200 mg of the triflate from Step A and 166 mg of phenylboronic acid. MS (ESI): exact mass calculated for C₁₇H₂₃N₃, 269.19; found, *m*/*z* 270.5 [M+H]⁺, 214.4 [M-*^{t}*Bu]⁺. ¹H NMR (500 MHz, CD₃OD): 7.49-7.47 (m, 3H), 7.32-7.30 (m, 2H), 3.41-3.39 (m, 2H), 3.25-3.23 (m, 2H), 3.18-3.15 (m, 2H), 2.52-2.520 (m, 2H), 1.41 (s, 9H).

### Example 216

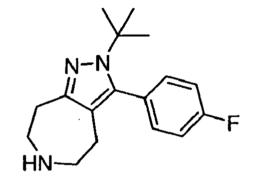

2-tert-Butyl-3-(4-fluoro-phenyl)-2,4,5,6,7,8-hexahydro-1,2,6-triaza-azulene.
The title compound (88 mg) was prepared as in Example 177, Steps C and D, using 204 mg of 2-(tert-butyl)-3-trifluoromethanesulfonyloxy-4,5,7,8-tetrahydro-2*H*-1,2,6-triaza-azulene-6-carboxylic acid tert-butyl ester (Example 215 Step A) and 194 mg of 4-fluorophenylboronic acid. MS (ESI): exact mass calculated for C₁₇H₂₂FN₃, 287.18; found, *m*/*z* 288.4 [M+H]⁺, 232.4 [M-*^{t}*Bu]⁺. ¹H NMR (500 MHz, CD₃OD): 7.37-7.33 (m, 2H), 7.26-7.22 (m, 2H), 3.41-3.38 (m, 2H), 3.26-3.24 (m, 2H), 3.18-3.15 (m, 2H), 2.53-2.51 (m, 2H), 1.42 (s, 9H).

### Example 217

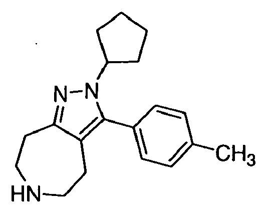

2-Cyclopentyl-3-p-tolyl-2,4,5,6,7,8-hexahydro-1,2,6-triaza-azulene.
The title compound (70.4 mg) was prepared as in Example 177, Steps C and D, using 204.3 mg of 2-cyclopentyl-3-trifluoromethanesulfonyloxy-4,5,7,8-tetrahydro-2*H*-1,2,6-triaza-azulene-6-carboxylic acid *tert*-butyl ester (Example 180, Step A) and 204.1 mg of 4-methylphenylboronic acid. MS (ESI): exact mass calculated for C₁₉H₂₅N₃, 295.42; found, *m*/*z* 296.5 [M+H]⁺. ¹H NMR (500 MHz, CD₃OD): 7.37 (d, J = 7.9 Hz, 2H), 7.21 (d, J = 7.9 Hz, 2H), 4.50 (m, 1 H), 3.43-3.40 (m, 2H), 3.32-3.28 (m, 2H), 3.20-3.17 (m, 2H), 2.80-2.77 (m, 2H), 2.43 (s, 3H), 2.04-1.86 (m, 6H), 1.64-1.55 (m, 2H).

### Example 218

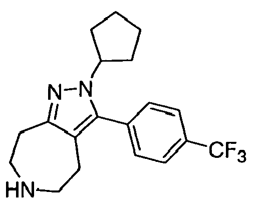

2-Cyclopentyl-3-(4-trifluoromethyl-phenyl)-2,4,5,6,7,8-hexahydro-1,2,6-triaza-azulene.
The title compound (45.2 mg) was prepared as in Example 177, Steps C and D, using 269.2 mg of 2-cyclopentyl-3-trifluoromethanesulfonyloxy-4,5,7,8-tetrahydro-2*H*-1,2,6-triaza-azulene-6-carboxylic acid *tert*-butyl ester (Example 180, Step A) and 359.2 mg of 4-trifluoromethylphenylboronic acid. MS (ESI): exact mass calculated for C₁₉H₂₂F₃N₃, 349.49; found, *m*/*z* 350.3 [M+H]⁺. ¹H NMR (500 MHz, CD₃OD): 7.87 (d, *J =* 7.9 Hz, 2H), 7.55 (d, *J =* 7.9 Hz, 2H), 4.48 (m, 1 H), 3.43-3.40 (m, 2H), 3.21-3.17 (m, 2H), 2.81-2.77 (m, 2H), 2.07-1.86 (m, 6H), 1.66-1.57 (m, 2H).

### Example 219

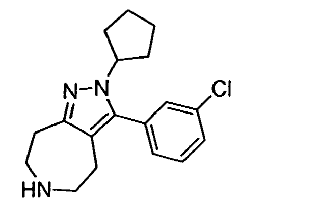

3-(3-Chloro-phenyl)-2-cyclopentyl-2,4,5,6,7,8-hexahydro-1,2,6-triaza-azulene.
The title compound (34.9 mg) was prepared as in Example 177, Steps C and D, using 204.4 mg of 2-cyclopentyl-3-trifluoromethanesulfonyloxy-4,5,7,8-tetrahydro-2*H*-1,2,6-triaza-azulene-6-carboxylic acid *tert*-butyl ester (Example 180, Step A) and 234.5 mg of 3-chlorophenylboronic acid. MS (ESI): exact mass calculated for C₁₈H₂₂ClN₃, 315.84; found, *m*/*z* 316.4 [M+H]⁺. ¹H NMR (500 MHz, CD₃OD): 7.57-7.52 (m, 2H), 7.37-7.35 (m, 1 H), 7.29-7.26 (m, 1 H), 4.46 (m, 1 H), 3.43-3.39 (m, 2H), 3.20-3.16 (m, 2H), 2.80-2.76 (m, 2H), 2.06-1.86 (m, 6H), 1.66-1.57 (m, 2H).

### Example 220

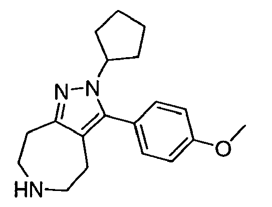

2-Cyclopentyl-3-(4-methoxy-phenyl)-2,4,5,6,7,8-hexahydro-1,2,6-triaza-azulene.
The title compound (34.9 mg) was prepared as in Example 177, Steps C and D, using 299.2 mg of 2-cyclopentyl-3-trifluoromethanesulfonyloxy-4,5,7,8-tetrahydro-2*H*-1,2,6-triaza-azulene-6-carboxylic acid *tert*-butyl ester (Example 180, Step A) and 329.2 mg of 4-methoxyphenylboronic acid. MS (ESI): exact mass calculated for C₁₉H₂₅N₃O, 311.42; found, *m*/*z* 312.3 [M+H]⁺. ¹H NMR (500 MHz, CD₃OD): 7.26-7.23 (m, 2H), 7.11-7.08 (m, 2H), 4.51 (m, 1 H), 3.87 (s, 3H), 3.43-3.40 (m, 2H), 3.20-3.16 (m, 2H), 2.80-2.76 (m, 2H), 2.02-1.86 (m, 6H), 1.64-1.55 (m, 2H).

### Example 221

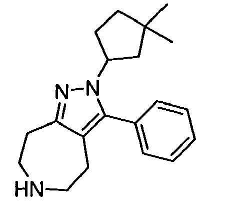

2-(3,3-Dimethyl-cyclopentyl)-3-phenyl-2,4,5,6,7,8-hexahydro-1,2,6-triaza-azulene.
Step A. 2-(3,3-Dimethyl-cyclopentyl)-3-trifluoromethanesulfonyloxy-4,5,7,8-tetrahydro-2*H*-1,2,6-triaza-azulene-6-carboxylic acid tert-butyl ester. The desired triflate was prepared as in Steps A and B of Example 176, using (3,3-dimethyl-cyclopentyl)-hydrazine hydrochloride in place of phenylhydrazine, *t-*butanol in place of EtOH, with the addition of 3 equiv. of triethylamine.
Step B. The title compound (92.8 mg) was prepared as in Example 177, Steps C and D, using 197.5 mg of the triflate from Step A and 150 mg of phenylboronic acid. MS (ESI): exact mass calculated for C₂₀H₂₇N₃, 309.45; found, *m*/*z* 310.5 [M+H]⁺. ¹H NMR (500 MHz, CD₃OD): 7.58-7.50 (m, 3H), 7.34-7.31 (m, 2H), 4.66-4.58 (m, 1 H), 3.44-3.40 (m, 2H), 3.32-3.28 (m, 2H), 3.21-3.17 (m, 2H), 2.81-2.77 (m, 2H), 2.21-2.03 (m, 2H), 2.01-1.95 (m, 1 H), 1.80-1.73 (m, 2H), 1.48-1.39 (m, 1 H), 1.16 (s, 3H), 0.92 (s, 3H).

### Example 222

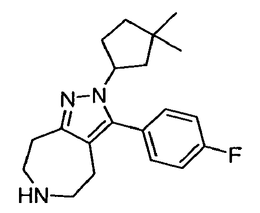

2-(3,3-Dimethyl-cyclopentyl)-3-(4-fluoro-phenyl)-2,4,5,6,7,8-hexahydro-1,2,6-triaza-azulene.
The title compound (52.6 mg) was prepared as in Example 177, Steps C and D, using 201.7 mg of 2-(3,3-dimethyl-cyclopentyl)-3-trifluoromethanesulfonyloxy-4,5,7,8-tetrahydro-2*H*-1,2,6-triaza-azulene-6-carboxylic acid tert-butyl ester (Example 221, Step A) and 180 mg of 4-fluorophenylboronic acid. MS (ESI): exact mass calculated for C₂₀H₂₆FN₃, 327.44; found, *m*/z 328.5 [M+H]⁺. ¹H NMR (500 MHz, CD₃OD): 7.39-7.34 (m, 2H), 7.33-7.28 (m, 2H), 4.62-4.53 (m, 1 H), 3.44-3.39 (m, 2H), 3.31-3.29 (m, 2H), 3.21-3.17 (m, 2H), 2.80-2.75 (m, 2H), 2.20-2.03 (m, 2H), 2.00-1.94 (m, 1 H), 1.80-1.73 (m, 2H), 1.49-1.41 (m, 1 H), 1.16 (s, 3H), 0.93 (s, 3H).

### Example 223

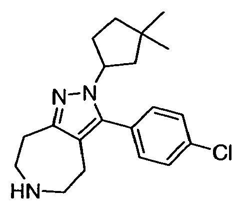

3-(4-Chloro-phenyl)-2-(3,3-dimethyl-cyclopentyl)-2,4,5,6,7,8-hexahydro-1,2,6-triaza-azulene.
The title compound (25.6 mg) was prepared as in Example 177, Steps C and D, using 203.3 mg of 2-(3,3-dimethyl-cyclopentyl)-3-trifluoromethanesulfonyloxy-4,5,7,8-tetrahydro-2H-1,2,6-triaza-azulene-6-carboxylic acid tert-butyl ester (Example 221 Step A) and 204.1 mg of 4-chlorophenylboronic acid. MS (ESI): exact mass calculated for C₂₀H₂₆ClN₃, 343.89; found, *m*/*z* 344.5 [M+H]⁺. ¹H NMR (500 MHz, CD₃OD): 7.57 (d, J= 8.5 Hz, 2H), 7.32 (d, J = 8.5 Hz, 2H), 4.62-4.54 (m, 1 H), 3.43-3.39 (m, 2H), 3.32-3.28 (m, 2H), 3.20-3.16 (m, 2H), 2.79-2.75 (m, 2H), 2.19-2.03 (m, 2H), 1.99-1.94 (m, 1 H), 1.80-1.73 (m, 2H), 1.49-1.40 (m, 1 H), 1.16 (s, 3H), 0.94 (s, 3H).

### Example 224

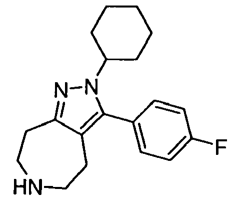

2-Cyclohexyl-3-(4-fluoro-phenyl)-2,4,5,6,7,8-hexahydro-1,2,6-triaza-azulene.
The title compound (17.2 mg) was prepared as in Example 177, Steps C and D, using 206.5 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 193.2 mg of 4-fluorophenylboronic acid. MS (ESI): exact mass calculated for C₁₉H₂₄FN₃, 313.41; found, *m*/*z* 314.5 [M+H]⁺. ¹H NMR (500 MHz, CD₃OD): 7.37-7.28 (m, 4H), 3.91-3.84 (m, 1H), 3.43-3.38 (m, 2H), 3.32-3.27 (m, 2H), 3.18-3.14 (m, 2H), 2.78-2.74 (m, 2H), 1.96-1.79 (m, 6H), 1.69-1.63 (m, 1 H), 1.30-1.19 (m, 3H).

### Example 225

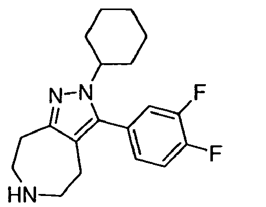

2-Cyclohexyl-3-(3,4-difluoro-phenyl)-2,4,5,6,7,8-hexahydro-1,2,6-triaza-azulene.
The title compound (42.7 mg) was prepared as in Example 177, Steps C and D, using 205.2 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 224.9 mg of 3,4-difluorophenylboronic acid. MS (ESI): exact mass calculated for C₁₉H₂₃F₂N₃, 331.40; found, *m*/*z* 332.5 [M+H]⁺. ¹H NMR (500 MHz, CD₃OD): 7.51-7.44 (m, 1 H), 7.34-7.28 (m, 1 H), 7.17-7.13 (m, 1 H), 3.91-3.84 (m, 1 H), 3.42-3.38 (m, 2H), 3.18-3.14 (m, 2H), 2.78-2.74 (m, 2H), 1.96-1.78 (m, 6H), 1.70-1.64 (m, 1 H), 1.32-1.19 (m, 3H).

### Example 226

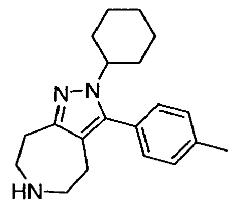

2-Cyclohexyl-3-p-tolyl-2,4,5,6,7,8-hexahydro-1,2,6-triaza-azulene.
The title compound (60.2 mg) was prepared as in Example 177, Steps C and D, using 203.8 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 181.6 mg of 4-methylphenylboronic acid. MS (ESI): exact mass calculated for C₂₀H₂₇N₃, 309.45; found, *m*/*z* 310.5 [M+H]⁺. ¹H NMR (500 MHz, CD₃OD): 7.37 (d, *J* = 7.7 Hz, 2H), 7.19 (d, *J* = 7.7 Hz, 2H), 3.97-3.89 (m, 1 H), 3.44-3.39 (m, 2H), 3.31-3.26 (m, 2H), 3.20-3.15 (m, 2H), 2.80-2.75 (m, 2H), 1.96-1.78 (m, 6H), 1.70-1.62 (m, 1 H), 1.28-1.18 (m, 3H).

### Example 227

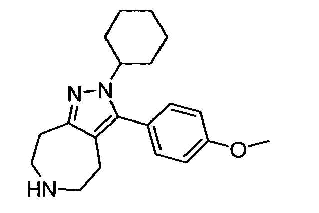

2-Cyclohexyl-3-(4-methoxy-phenyl)-2,4,5,6,7,8-hexahydro-1,2,6-triaza-azulene.
The title compound (96.8 mg) was prepared as in Example 177, Steps C and D, using 207 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 224.1 mg of 4-methoxyphenylboronic acid. MS (ESI): exact mass calculated for C₂₀H₂₇N₃O, 325.45; found, *m*/*z* 326.5 [M+H]⁺. ¹H NMR (500 MHz, CD₃OD): 7.25 (d, J= 8.7 Hz, 2H), 7.11 (d, J= 8.7 Hz, 2H), 4.00-3.92 (m, 1H), 3.87 (s, 3H), 3.45-3.40 (m, 2H), 3.22-3.17 (m, 2H), 2.81-2.75 (m, 2H), 1.96-1.65 (m, 7H), 1.29-1.19 (m, 3H).

### Example 228

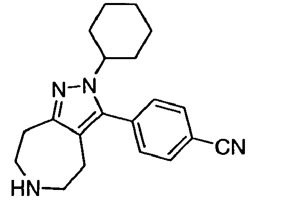

4-(2-Cyclohexyl-2,4,5,6,7,8-hexahydro-1,2,6-triaza-azulen-3-yl)-benzonitrile.
The title compound (135.4 mg) was prepared as in Example 177, Steps C and D, using 203.8 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 198 mg of 4-cyanophenylboronic acid. MS (ESI): exact mass calculated for C₂₀H₂₄N₄, 320.43; found, *m*/*z* 321.5 [M+H]⁺. ¹H NMR (500 MHz, CD₃OD): 7.93 (d, *J* = 8.5 Hz, 2H), 7.53 (d, *J =* 8.5 Hz, 2H), 3.92-3.84 (m, 1 H), 3.43-3.38 (m, 2H), 3.19-3.15 (m, 2H), 2.80-2.75 (m, 2H), 1.98-1.80 (m, 6H), 1.71-1.64 (m, 1 H), 1.32-1.20 (m, 3H).

### Example 229

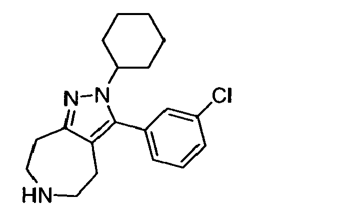

3-(3-Chloro-phenyl)-2-cyclohexyl-2,4,5,6,7,8-hexahydro-1,2,6-triaza-azulene.
The title compound (14.4 mg) was prepared as in Example 177, Steps C and D, using 199.3 mg of 2-cyclohexyl-3-trifluoromethanesulfonyloxy-4,5,7,8-tetrahydro-2*H*-1,2,6-triaza-azulene-6-carboxylic acid *tert*-butyl ester (Example 177, Step B) and 216.2 mg of 3-chlorophenylboronic acid. MS (ESI): exact mass calculated for C₁₉H₂₄ClN₃, 329.87; found, *m*/*z* 330.5 [M+H]⁺. ¹H NMR (500 MHz, CD₃OD): 7.57-7.54 (m, 2H), 7.35 (s, 1H), 7.28-7.25 (m, 1H), 3.91-3.84 (m, 1H), 3.43-3.38 (m, 2H), 3.19-3.14 (m, 2H), 2.79-2.74 (m, 2H), 1.97-1.80 (m, 6H), 1.71-1.64 (m, 1H), 1.28-1.19 (m, 3H).

### Example 230

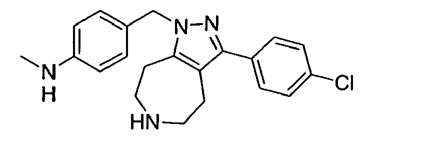

{4-[3-(4-Chloro-phenyl)-5,6,7,8-tetrahydro-4H-1,2,6-triaza-azulen-1-ylmethyl]-phenyl}-methyl-amine.
A mixture of 1-(4-bromo-benzyl)-3-(4-chloro-phenyl)-4,5,7,8-tetrahydro-1H-1,2,6-triaza-azulene-6-carboxylic acid *tert-*butyl ester (Example 113; 0.04 mmol), *tert*-butyl carbamate (0.05 mmol), sodium phenoxide trihydrate (0.05 mmol), tris(dibenzylideneacetone)dipalladium(0) (0.001 mmol), and tri-*tert-*butylphosphine (0.05 mmol) in anhydrous toluene (3 mL) was heated under N₂ at 100 °C for 6 h, 70 °C for 15 h and 100 °C for 2.5 h. After cooling to RT, the reaction mixture was purified directly by preparative TLC (2:1 hexanes/EtOAc) to yield 0.008 g of 1-(4-tert-butoxycarbonylamino-benzyl)-3-(4-chloro-phenyl)-4,5,7,8-tetrahydro-1H-1,2,6-triaza-azulene-6-carboxylic acid *tert*-butyl ester, which was then diluted with DMF (1 mL) and treated with NaH (60%, 1.5 equiv.). After 15 min, methyl iodide (1.5 equiv.) was added. After 1 h, the reaction was quenched with H₂O and the mixture was extracted with EtOAc (2x). The combined organic layers were dried over Na₂SO₄ and concentrated. The resulting semi-solid was then dissolved in CH₂Cl₂/MeOH (9:1, 1 mL) and treated with HCI (1 N in Et₂O, 4 mL). The mixture was stirred at RT for 3 h, then was concentrated. The resulting oil was purified by preparative TLC (10% 2 M NH₃ in MeOH/CH₂Cl₂) to yield 0.002 mg of the title compound as a white solid. MS (ESI): exact mass calculated for C₂₁H₂₃ClN₄, 366.16; found, *m*/*z* 367.1 [M+H]⁺. ¹H NMR (500 MHz, CD₃OD): 7.39-7.32 (m, 4H), 6.84 (d, J = 8.6 Hz, 1 H), 6.48-6.45 (m, 2H), 5.12 (s, 2H), 2.84-2.81 (m, 4H), 2.79-2.77 (m, 2H), 2.69-2.66 (m, 2H), 2.63 (s, 3H).

### Example 231

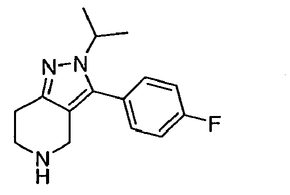

3-(4-Fluoro-phenyl)-2-isopropyl-4,5,6,7-tetrahydro-2H-pyrazolo[4,3-c]pyridine.
Step A. 2-lsopropyl-3-trifluoromethanesulfonyloxy-2,4,6,7-tetrahydropyrazolo[4,3-c]pyridine-5-carboxylic acid *tert-*butyl ester. The desired triflate was prepared according to Example 189, Step A, starting with 4-oxo-piperidine-1,3-dicarboxylic acid 1-*tert*-butyl ester 3-methyl ester.
Step B. The title compound (26 mg) was prepared as in Example 177, Steps C and D, using 221 mg of the triflate from Step A and 140 mg of 4-fluorophenylboronic acid. MS (ESI): exact mass calculated for C₁₅H₁₈FN₃, 259.32; found, *m*/*z* 260.4 [M+H]⁺. ¹H NMR (500 MHz, CD₃OD): 7.44-7.39 (m, 2H), 7.33-7.28 (m, 2H), 4.48 (m, 1 H), 4.15 (s, 2H), 3.58 (t, J = 6.3 Hz, 2H), 3.08 (t, J = 6.3 Hz, 2H), 1.42 (d, J = 6.6 Hz, 6H).

### Example 232

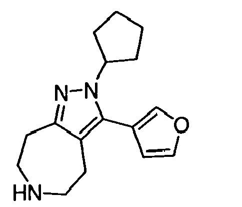

2-Cyclopentyl-3-furan-3-yl-2,4,5,6,7,8-hexahydro-1,2,6-triaza-azulene.
The title compound (101 mg) was prepared according to Example 180 using 202 mg of 2-cyclopentyl-3-trifluoromethanesulfonyloxy-4,5,7,8-tetrahydro-2H-1,2,6-triaza-azulene-6-carboxylic acid *tert*-butyl ester (Example 180, Step A) and 149 mg of 3-furanboronic acid. MS (ESI): exact mass calculated for C₁₆H₂₁N₃O, 271.17; found, *m*/*z* 272.5 [M+H]⁺. ¹H NMR (500 MHz, CD₃OD): 7.73-7.72 (m, 2H), 6.57-6.56 (m, 1 H), 4.64 (m, 1 H), 3.40-3.38 (m, 2H), 3.16-3.14 (m, 2H), 2.86-2.84 (m, 2H), 2.03-1.91 (m, 6H), 1.66-1.64 (m, 2H).

### Example 233

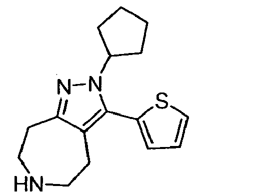

2-Cyclopentyl-3-thiophen-2-yl-2,4,5,6,7,8-hexahydro-1,2,6-triaza-azulene.
The title compound (83 mg) was prepared according to Example 180 using 200 mg of 2-cyclopentyl-3-trifluoromethanesulfonyloxy-4,5,7,8-tetrahydro-2H-1,2,6-triaza-azulene-6-carboxylic acid *tert*-butyl ester (Example 180, Step A) and 282 mg of 2-thiopheneboronic acid. MS (ESI): exact mass calculated for C₁₆H₂₁N₃S, 287.15; found, *m*/*z* 288.4 [M+H]⁺. ¹H NMR (500 MHz, CD₃OD): 7.70-7.68 (m, 1 H), 7.24-7.22 (m, 1 H), 7.15-7.14 (m, 1 H), 4.64 (m, 1 H), 3.41-3.39 (m, 2H), 3.16-3.15 (m, 2H), 2.85-2.83 (m, 2H), 2.01-1.88 (m, 6H), 1.64-1.60 (m, 2H).

### Example 234

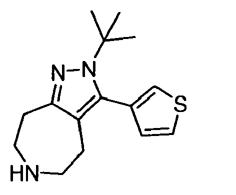

2-tert-Butyl-3-thiophen-3-yl-2,4,5,6,7,8-hexahydro-1,2,6-triaza-azulene.
The title compound (83 mg) was prepared according to Example 215 using 204 mg of 2-(*tert*-butyl)-3-trifluocomethanesulfonyloxy-4,5,7,8-tetrahydro-2H-1,2,6-triaza-azulene-6-carboxylic acid *tert*-butyl ester (Example 215, Step A) and 177 mg of 3-thiopheneboronic acid. MS (ESI): exact mass calculated for C₁₅H₂₁N₃S, 275.15; found, *m*/*z* 276.4 [M+H]⁺. ¹H NMR (500 MHz, CD₃OD): 7.59-7.57 (m, 1H), 7.43-7.42 (m, 1H), 7.08-7.06 (m, 1H), 3.38-3.36 (m, 2H), 3.25-3.23 (m, 2H), 3.13-3.11 (m, 2H), 2.56-2.54 (m, 2H), 1.43 (s, 9H).

### Example 235

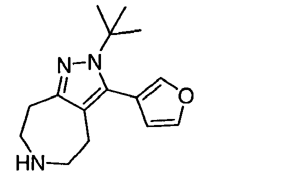

2-tert-Butyl-3-furan-3-yl-2,4,5,6,7,8-hexahydro-1,2,6-triaza-azulene.
The title compound (60 mg) was prepared according to Example 215 using 203 mg of 2-(*tert*-butyl)-3-trifluoromethanesulfonyloxy-4,5,7,8-tetrahydro-2H-1,2,6-triaza-azulene-6-carboxylic acid *tert*-butyl ester (Example 215, Step A) and 154 mg of 3-furanboronic acid. MS (ESI): exact mass calculated for C₁₅H₂₁N₃O, 259.17; found, *m*/*z* 260.5 [M+H]⁺. ¹H NMR (500 MHz, CD₃OD): 7.69-7.68 (m, 1H), 7.61 (br s, 1H), 6.50-6.49 (m, 1H), 3.38-3.36 (m, 2H), 3.27-3.25 (m, 2H), 3.13-3.11 (m, 2H), 2.63-2.61 (m, 2H), 1.50 (s, 9H).

### Example 236

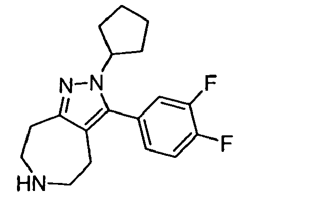

2-Cyclopentyl-3-(3,4-difluoro-phenyl)-2,4,5,6,7,8-hexahydro-1,2,6-triaza-azulene.
The title compound (70 mg) was prepared according to Example 180 using 209 mg of 2-cyclopentyl-3-trifluoromethanesulfonyloxy-4,5,7,8-tetrahydro-2H-1,2,6-triaza-azulene-6-carboxylic acid *tert*-butyl ester (Example 180, Step A) and 218 mg of 3,4-difluorophenylboronic acid. MS (ESI): exact mass calculated for C₁₈H₂₁F₂N₃, 317.17; found, *m*/*z* 318.4 [M+H]⁺. ¹H NMR (500 MHz, CD₃OD): 7.50-7.45 (m, 1H), 7.36-7.32 (m, 1H), 7.18-7.17 (m, 1H), 4.49 (m, 1H), 3.43-3.41 (m, 2H), 3.21-3.19 (m, 2H), 2.80-2.78 (m, 2H), 2.15-1.87 (m, 6H), 1.66-1.61 (m, 2H).

### Example 237

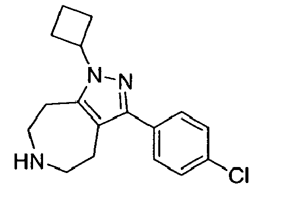

3-(4-CNoro-phenyl)-1-cyclobutyl-1,4,5,6,7,8-hexahydro-1,2,6-triaza-azulene.
The title compound (0.01 g) was prepared from 3-(4-chloro-phenyl)-1-cyclobutyl-1,4,5,6,7,8-hexahydro-1,2,6-triaza-azulene-6-carboxylic acid *tert-*butyl ester (Example 238) according to Example 103, Step C. (MS (ESI): exact mass calculated for C₁₇H₂₀ClN₃, 301.13; found, *m*/*z* 302.4 [M+H]⁺. ¹H NMR (500 MHz, CD₃OD): 7.54-7.48 (m, 4H), 5.15-5.05 (m, 1H), 3.47-3.46 (m, 2H), 3.35-3.30 (m, 4H), 3.22-3.21 (m, 2H), 2.70-2.60 (m, 2H), 2.50-2.40 (m, 2H), 1.90-1.80 (m, 2H).

### Example 238

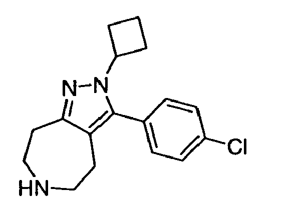

3-(4-Chloro-phenyl)-2-cyclobutyl-2,4,5,6,7,8-hexahydro-1,2,6-triaza-azulene.
To a solution of 3-(4-chloro-phenyl)-4,5,7,8-tetrahydro-1*H*-1,2,6-triaza-azulene-6-carboxylic acid *tert*-butyl ester (Example 103, Step B; 0.40 mmol) in DMF (2 mL) was added NaH (60% dispersion in oil, 60 mg) at 25 °C . After 10 min, the mixture was heated to 80 °C, and chloro-cyclobutane (1.5 mmol) was added. The mixture was heated at this temperature for 16 h. The mixture was concentrated and purified by chromatography (SiO₂ EtOAc/hexanes) to provide 3-(4-chloro-phenyl)-2-cyclobutyl-2,4,5,6,7,8-hexahydro-1,2,6-triaza-azulene-6-carboxylic acid *tert*-butyl ester. The title compound (0.030 mg) was obtained from this ester according to the deprotection method in Example 103, Step C. The reaction sequence also yielded 3-(4-chloro-phenyl)-1-cyclobutyl-2,4,5,6,7,8-hexahydro-1,2,6-triaza-azulene-6-carboxylic acid *tert*-butyl ester in the alkylation step. MS (ESI): exact mass calculated for C₁₇H₂₀ClN₃, 301.13; found, *m*/*z* 302.4 [M+H]⁺. ¹H NMR (500 MHz, CD₃OD): 7.52-7.51 (m, 2H), 7.30-7.29 (m, 2H), 4.70-4.60 (m, 1 H), 3.40-3.89 (m, 2H), 3.27-3.21 (m, 4H), 2.79-2.76 (m, 2H), 2.60-2.50 (m, 2H), 2.30-2.20 (m, 2H), 1.81-1.65 (m, 2H).

### Example 239

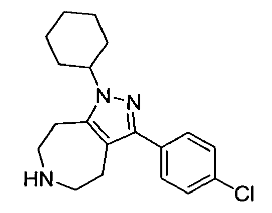

3-(4-Chloro-phenyl)-1-cyclohexyl-1,4,5,6,7,8-hexahydro-1,2,6-triaza-azulene.
The title compound (15 mg) was prepared from 3-(4-chloro-phenyl)-4,5,7,8-tetrahydro-1 *H*-1,2,6-triaza-azulene-6-carboxylic acid *tert*-butyl ester (Example 103, Step B; 0.40 mmol) using bromo-cyclohexane (1.5 mmol) in place of chloro-cyclobutane according to Example 238. MS (ESI): exact mass calculated for C₁₉H₂₄ClN₃, 329.17; found, *m*/*z* 330.4 [M+H]⁺. ¹H NMR (500 MHz, CD₃OD): 7.45-7.41 (m, 4H), 4.30-4.27 (m, 1 H), 3.44-3.42 (m, 2H), 3.31-3.26 (m, 4H), 2.98-2.96 (m, 2H), 1.93-1.84 (m, 5H), 1.70-1.65 (m, 1H), 1.46-1.40 (m, 2H), 1.24-1.89 (m, 2H).

### Example 240

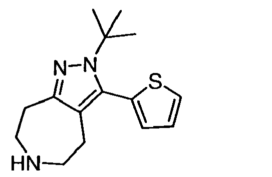

2-tert-Butyl-3-thiophen-2-yl-2,4,5,6,7,8-hexahydro-1,2,6-triaza-azulene.
The title compound (83 mg) was prepared according to Example 215 using 203 mg of 2-(tert-butyl)-3-trifluoromethanesulfonyloxy-4,5,7,8-tetrahydro-2H-1,2,6-triaza-azulene-6-carboxylic acid *tert*-butyl ester (Example 215, Step A) and 176 mg of 2-thiopheneboronic acid. MS (ESI): exact mass calculated for C₁₅H₂₁N₃S, 275.15; found, *m*/*z* 276.4 [M+H]⁺. ¹H NMR (500 MHz, CD₃OD): 7.57-7.56 (m, 1H), 7.08-7.06 (m, 1H), 7.01-7.00 (m, 1H), 3.29-3.27 (m, 2H), 3.17-3.14 (m, 2H), 2.51-2.48 (m, 2H), 1.37 (s, 9H).

### Example 241

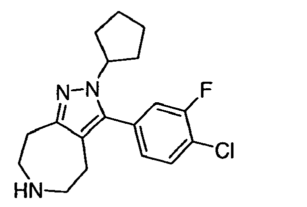

3-(4-Chloro-3-fluoro-phenyl)-2-cyclopentyl-2,4,5,6,7,8-hexahydro-1,2,6-triaza-azulene.
The title compound (31 mg) was prepared according to Example 180 using 146 mg of 2-cyclopentyl-3-trifluoromethanesulfonyloxy-4,5,7,8-tetrahydro-2H-1,2,6-triaza-azulene-6-carboxylic acid *tert*-butyl ester (Example 180, Step A) and 168 mg of 3-chloro-4-fluorophenylboronic acid. MS (ESI): exact mass calculated for C₁₈H₂₁ClFN₃, 333.14; found, *m*/*z* 334.4 [M+H]⁺, 336.4 [M+H]⁺. ¹H NMR (500 MHz, CD₃OD): 7.39-7.31 (m, 2H), 7.22-7.18 (m, 1 H), 4.37 (m; 1 H), 3.31-3.28 (m, 2H), 3.07-3.03 (m, 2H), 2.67-2.64 (m, 2H), 2.11-1.78 (m, 6H), 1.56-1.47 (m, 2H).

### Example 242

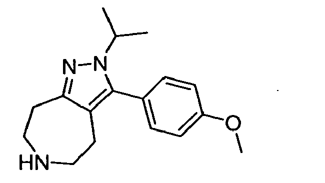

2-lsopropyl-3-(4-methoxy-phenyl)-2,4,5,6,7,8-hexahydro-1,2,6-triaza-azulene.
The title compound (148 mg) was prepared according to Example 189 using 206 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 219 mg of 4-methoxyphenylboronic acid. MS (ESI): exact mass calculated for C₁₇H₂₃N₃O, 285.18; found, *m*/*z* 286.5 [M+H]⁺. ¹H NMR (500 MHz, CD₃OD): 7.30-7.28 (m, 2H), 7.13-7.11 (m, 2H), 4.68 (m, 1 H), 4.47 (m, 1 H), 3.87 (s, 3H), 3.47-3.44 (m, 1 H), 3.25-3.23 (m, 1 H), 2.89-2.81 (m, 2H), 1.43 (d, J = 6.6 Hz, 6H).

### Example 243

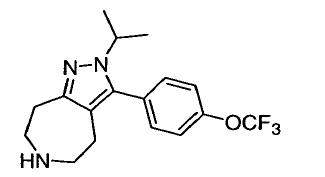

2-Isopropyl-3-(4-trifluoromethoxy-phenyl)-2,4,5,6,7,8-hexahydro-1,2,6-triaza-azulene.
The title compound (196 mg) was prepared according to Example 189 using 278 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 402 mg of 4-trifluoromethoxyphenylboronic acid. MS (ESI): exact mass calculated for C₁₇H₂₀F₃N₃O, 339.36; found, *m*/*z* 340.5 [M+H]⁺. ¹H NMR (500 MHz, CD₃OD): 7.53-7.45 (m, 4H), 4.66 (br s, 1 H), 4.40 (J = 6.68 Hz, 1 H), 3.45-3.43 (m, 1 H), 3.23-3.21 (m, 1 H), 2.88-2.79 (m, 2H), 1.42 (d, J = 6.7 Hz, 6H).

### Example 244

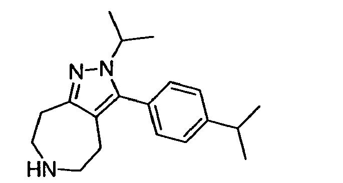

2-Isopropyl-3-(4-isopropyl-phenyl)-2,4,5,6,7,8-hexahydro-1,2,6-triaza-azulene.
The title compound (177 mg) was prepared according to Example 189 using 270 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 311 mg of 4-isopropylphenylboronic acid. MS (ESI): exact mass calculated for C₁₉H₂₇N₃, 297.44; found, *m*/*z* 298.5 [M+H]⁺. ¹H NMR (500 MHz, CD₃OD): 7.35-7.34 (m, 2H), 7.19-7.17 (m, 2H), 4.57 (br s, 1 H), 4.38-4.32 (m, 1H), 3.36-3.34 (m, 1H), 3.15-3.13 (m, 1H), 2.90 (m, 1H), 2.79-2.70 (m, 2H), 1.31 (d, J = 13.3 Hz, 6H), 1.20 (d, J = 6.9 Hz, 6H).

### Example 245

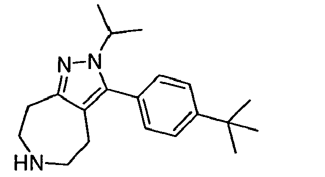

3-(4-*tert*-Butyl-phenyl)-2-isopropyl-2,4,5,6,7,8-hexahydro-1,2,6-triaza-azulene.
The title compound (28 mg) was prepared according to Example 189 using 215 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 268 mg of 4-tert-butylphenylboronic acid. MS (ESI): exact mass calculated for C₂₀H₂₉N₃, 311.24; found, *m*/*z* 312.5 [M+H]⁺. ¹H NMR (500 MHz, CD₃OD): 7.61-7.59 (m, 2H), 7.27-7.25 (m, 2H), 4.40 (m, 1 H), 3.43-3.40 (m, 2H), 3.19-3.17 (m, 2H), 2.79-2.77 (m, 2H), 1.50-1.25 (m, 15H).

### Example 246

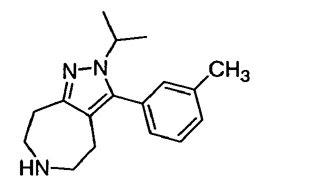

2-Isopropyl-3-m-tolyl-2,4,5,6,7,8-hexahydro-1,2,6-triaza-azulene.
The title compound (24 mg) was prepared according to Example 189 using 219 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 209 mg of 3-methylphenylboronic acid. MS (ESI): exact mass calculated for C₁₇H₂₃N₃, 269.19; found, *m*/*z* 270.5 [M+H]⁺. ¹H NMR (500 MHz, CD₃OD): 7.44-7.41 (m, 1 H), 7.34-7.33 (m, 1 H), 7.13-7.10 (m, 2H), 4.37 (m, 1 H), 3.42-3.40 (m, 2H), 3.19-3.17 (m, 2H), 2.78-2.76 (m, 2H), 2.42 (s, 3H), 1.38 (d, *J* = 6.7 Hz, 6H).

### Example 247

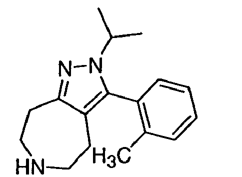

2-Isopropyl-3-o-tolyl-2,4,5,6,7,8-hexahydro-1,2,6-triaza-azulene.
The title compound (80 mg) was prepared according to Example 189 using 207 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 198 mg of 2-methylphenylboronic acid. MS (ESI): exact mass calculated for C₁₇H₂₃N₃, 269.19; found, *m*/*z* 270.5 [M+H]⁺. ¹H NMR (500 MHz, CD₃OD):
7.45-7.40 (m, 2H), 7.36-7.33 (m, 1 H), 7.19-7.18 (m, 1 H), 4.66 (br s, 2H), 4.10 (m, 1H), 4.00-3.66 (m, 2H), 2.76-2.61 (m, 2H), 2.13 (s, 3H), 1.45-1.29 (m, 6H).

### Example 248

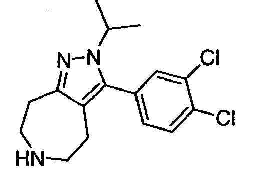

3-(3,4-Dichloro-phenyl)-2-isopropyl-2,4,5,6,7,8-hexahydro-1,2,6-triaza-azulene.
The title compound (60 mg) was prepared according to Example 189 using 200 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 268 mg of 3,4-dichlorophenylboronic acid. MS (ESI): exact mass calculated for C₁₆H₁₉Cl₂N₃, 323.10; found, *m*/*z* 324.4 [M+H]⁺, 326.4 [M+H]⁺. ¹H NMR (500 MHz, CD₃OD): 7.72-7.71 (m, 1H), 7.53-7.52 (m, 1H), 7.29-7.27 (m, 1H), 4.64 (br s, 2H), 4.32 (m, 1 H), 3.86-3.57 (m, 2H), 3.31-3.08 (m, 2H), 2.84-2.75 (m, 2H), 1.39 (d, J = 6.6 Hz, 6H).

### Example 249

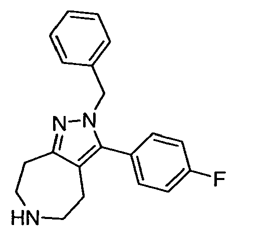

2-Benzyl-3-(4-fluoro-phenyl)-2,4,5,6,7,8-hexahydro-1,2,6-triaza-azulene.
Step A. 2-Benzyl-3-trifluoromethanesulfonyloxy-4,5,7,8-tetrahydro-2H-1,2,6-triaza-azulene-6-carboxylic acid *tert*-butyl ester. The desired triflate was prepared according to Example 189, Step A, using benzylhydrazine hydrochloride in place of isopropylhydrazine hydrochloride.
Step B. The title compound (29 mg) was prepared as in Example 177, Steps C and D, using 230 mg of the triflate from Step A and 234 mg of 4-fluorophenylboronic acid. MS (ESI): exact mass calculated for C₂₀H₂₀FN₃, 321.39; found, *m*/*z* 322.4 [M+H]⁺. ¹H NMR (500 MHz, CD₃OD): 7.31-7.19 (m, 7H), 6.97-6.93 (m, 2H), 5.20 (s, 2H), 3.45-3.40 (m, 2H), 3.35-3.30, (m, 2H), 3.20-3.15 (m, 2H), 2.83-2.78 (m, 2H).

### Example 250

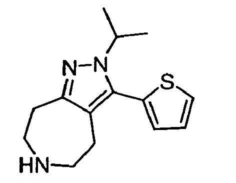

2-Isopropyl-3-thiophen-2-yl-2,4,5,6,7,8-hexahydro-1,2,6-triaza-azulene.
The title compound (46 mg) was prepared according to Example 189 using 208 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 187 mg of 2-thiopheneboronic acid. MS (ESI): exact mass calculated for C₁₄H₁₉N₃S, 261.13; found, *m*/*z* 262.4 [M+H]⁺. ¹H NMR (500 MHz, CD₃OD):
7.70-7.69 (m, 1 H), 7.25-7.23 (m, 1 H), 7.15-7.14 (m, 1 H), 4.65 (br s, 2H), 4.55-4.49 (m, 1 H), 3.8-3.6 (m, 2H), 3.23-3.10 (m, 2H), 2.93-2.83 (m, 2H), 1.44-1.36 (m, 6H).

### Example 251

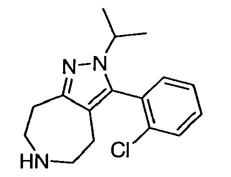

3-(2-Chloro-phenyl)-2-isopropyl-2,4,5,6,7,8-hexahydro-1,2,6-triaza-azulene.
The title compound (90 mg) was prepared according to Example 189 using 266 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 292 mg of 2-chlorophenylboronic acid. MS (ESI): exact mass calculated for C₁₆H₂₀ClN₃, 289.13; found, *m*/*z* 290.4 [M+H]⁺, 292.4 [M+H]⁺. ¹H NMR (500 MHz, CD₃OD): 7.65-7.63 (m, 1 H), 7.58-7.49 (m, 2H), 7.41-7.39 (m, 1 H), 4.66 (br s, 2H), 4.16 (m, 1 H), 4.00-3.44 (m, 2H), 3.0-2.6 (m, 2H), 1.47-1.38 (m, 6H).

### Example 252

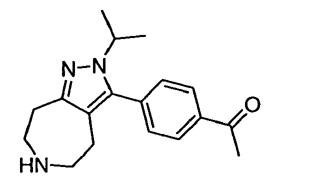

1-[4-(2-lsopropyl-2,4,5,6,7,8-hexahydro-1,2,6-triaza-azulen-3-yl)-phenyl]-ethanone.
The title compound (168 mg) was prepared according to Example 189 using 255 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 341 mg of 4-acetylphenylboronic acid. MS (ESI): exact mass calculated for C₁₈H₂₃N₃O, 297.18; found, *m*/*z* 298.5 [M+H]⁺. ¹H NMR (500 MHz, CD₃OD): 8.17-8.15 (m, 1H), 7.69-7.67 (m, 1H), 7.51-7.49 (m, 1H), 7.37-7.35 (m, 1H), 4.65 (br s, 1H), 4.46-4.38 (m, 1H), 4.00-3.50 (m, 2H), 3.48-3.42 (m, 1H), 3.25-3.17 (m, 1H), 3.13-2.81 (m, 2H), 2.67 (s, 1.5H), 1.55 (s, 1.5H), 1.44-1.40 (m, 6H).

### Example 253

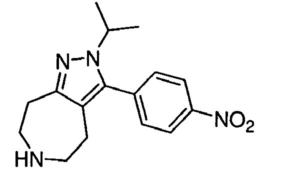

2-Isopropyl-3-(4-nitro-phenyl)-2,4,5,6,7,8-hexahydro-1,2,6-triaza-azulene.
The title compound (34 mg) was prepared according to Example 189 using 274 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 321 mg of 4-nitrophenylboronic acid. MS (ESI): exact mass calculated for C₁₆H₂₀N₄O₂, 300.16; found, *m*/*z* 301.4 [M+H]⁺. ¹H NMR (500 MHz, CD₃OD): 8.42-8.40 (m, 2H), 7.62-7.60 (m, 2H), 4.37 (m, 1 H), 3.43-3.41 (m, 2H), 3.21-3.18 (m, 2H), 2.82-2.79 (m, 2H), 1.41 (d, J = 8.2 Hz, 6H).

### Example 254

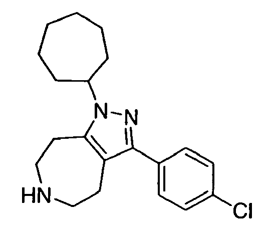

3-(4-Chloro-phenyl)-1-cycloheptyl-1,4,5,6,7,8-hexahydro-1,2,6-triaza-azutene.
The title compound (22 mg) was prepared from 3-(4-chloro-phenyl)-4,5,7,8-tetrahydro-1*H*-1,2,6-triaza-azulene-6-carboxylic acid *tert*-butyl ester (Example 103, Step B; 0.30 mmol) using chloro-cycloheptane (1.0 mmol) in place of chloro-cyclobutane according to Example 238. The reaction sequence also yielded 3-(4-chloro-phenyl)-2-cycloheptyl-1,4,5,6,7,8-hexahydro-1,2,6-triaza-azulene-6-carboxylic acid *tert*-butyl ester in the alkylation step. MS (ESI): exact mass calculated for C₂₀H₂₆ClN₃, 343.18; found, *m*/*z* 344.4 [M+H]⁺. ¹H NMR (500 MHz, CD₃OD): 7.40-7.34 (m, 4H), 4.31-4.27 (m, 1 H), 3.39-3.37 (m, 2H), 3.28-3.26 (m, 2H), 3.17-3.16 (m, 2H), 2.95-2.92 (m, 2H), 2.04-2.01 (m, 2H), 1.92-1.90 (m, 2H), 1.77-1.75 (m, 2H), 1.63-1.53 (m, 6H).

### Example 255

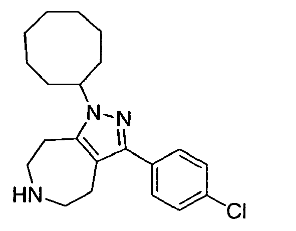

3-(4-Chloro-phenyl)-1-cyclooctyl-1,4,5,6,7,8-hexahydro-1,2,6-triaza-azulene.
The title compound (47 mg) was prepared from 3-(4-chloro-phenyl)-4,5,7,8-tetrahydro-1*H*-1,2,6-triaza-azulene-6-carboxylic acid *tert*-butyl ester (Example 103, Step B; 0.30 mmol) using chloro-cyclooctane (1.0 mmol) in place of chloro-cyclobutane according to Example 238. The reaction sequence also yielded 3-(4-chloro-phenyl)-2- cyclooctyl-1,4,5,6,7,8-hexahydro-1,2,6-triaza-azulene-6-carboxylic acid *tert*-butyl ester in the alkylation step. MS (ESI): exact mass calculated for C₂₁H₂₈ClN₃, 357.20; found, *m*/*z* 358.5 [M+H]⁺. ¹H NMR (500 MHz, CD₃OD): 7.50-7.44 (m, 4H), 4.49-4.45 (m, 1 H), 3.51-3.48 (m, 2H), 3.38-3.36 (m, 2H), 3.33-3.32 (m, 2H), 3.05-3.04 (m, 2H), 2.21-2.18 (m, 2H), 1.97-1.88 (m, 4H), 1.72-1.64 (m, 8H).

### Example 256

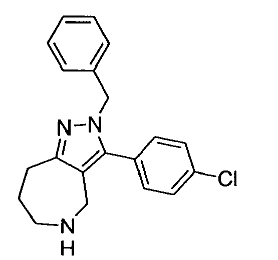

2-Benzyl-3-(4-chloro-phenyl)-2,4,5,6,7,8-hexahydro-1,2,5-triaza-azulene.
The title compound (0.023 g) was prepared from 3-(4-chloro-phenyl)-4,6,7,8-tetrahydro-1 *H*-1,2,5-triaza-azulene-5-carboxylic acid *tert*-butyl ester (Example 59, Step C; 0.1 g), as described in Example 60. MS (ESI): exact mass calculated for C₂₀H₂₀ClN₃, 337.13; found, *m*/*z* 338.4 [M+H]⁺. ¹H NMR (500 MHz, CD₃OD): 7.47-7.44 (m, 2H), 7.25-7.19 (m, 5H), 6.94-6.92 (m, 2H), 5.17 (s, 2H), 3.66 (s, 2H), 3.21-3.19 (m, 2H), 2.93-2.90 (m, 2H), 1.93-1.84 (s, 2H).

### Example 257

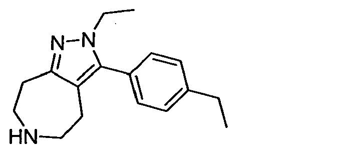

2-Ethyl-3-(4-ethyl-phenyl)-2,4,5,6,7,8-hexahydro-1,2,6-triaza-azulene.
The title compound (140 mg) was prepared according to Example 193 using 213 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A) and 232 mg of 4-ethylphenylboronic acid. MS (ESI): exact mass calculated for C₁₇H₂₃N₃, 269.19; found, *m*/*z* 270.5 [M+H]⁺. ¹H NMR (500 MHz, CD₃OD): 7.40-7.39 (m, 2H), 7.27-7.26 (m, 2H), 4.65 (br s, 2H), 4.05-4.00 (m, 2H), 3.8-3.6 (m, 2H), 3.18-3.00 (m, 2H), 2.88-2.81 (m, 2H), 2.76-2.71 (m, 2H), 1.32-1.24 (m, 6H).

### Example 258

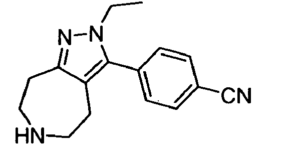

4-(2-Ethyl-2,4,5,6,7,8-hexahydro-1,2,6-triaza-azulen-3-yl)-benzonitrile.
The title compound (47 mg) was prepared according to Example 193 using 205 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A) and 218 mg of 4-cyanophenylboronic acid. MS (ESI): exact mass calculated for C₁₆H₁₈N₄, 266.15; found, *m*/*z* 267.5 [M+H]⁺. ¹H NMR (500 MHz, CD₃OD): 7.93-7.91 (m, 2H), 7.58-7.56 (m, 2H), 4.03 (q, J = 7.2 Hz, 2H), 3.43-3.40 (m, 2H), 3.18-3.16 (m, 2H), 2.82-2.80 (m, 2H), 1.29 (t, J = 7.2 Hz, 3H).

### Example 259

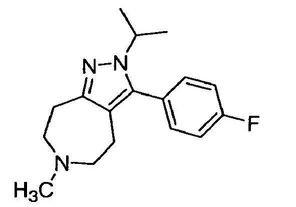

3-(4-Fluoro-phenyl)-2-isopropyl-6-methyl-2,4,5,6,7,8-hexahydro-1,2,6-triaza-azulene.
The title compound (113 mg) was prepared from 3-(4-fluoro-phenyl)-2-isopropyl-2,4,5,6,7,8-hexahydro-1,2,6-triaza-azulene (Example 190) and paraformaldehyde as in Example 35. MS (ESI): exact mass calculated for C₁₇H₂₂FN₃, 287.18; found, *m*/*z* 288.5 [M+H]⁺. ¹H NMR (500 MHz, CD₃OD): 7.42-7.40 (m, 2H), 7.34-7.30 (m, 2H), 4.42 (m, 1 H), 3.77-3.74 (m, 1 H), 3.67-3.62 (m, 2H), 3.36-3.34 (m, 1 H), 3.27-3.21 (m, 3H), 3.03 (s, 3H), 2.92-2.89 (m, 1 H), 2.80-2.76 (m, 1 H), 1.49-1.29 (m, 6H).

### Example 260

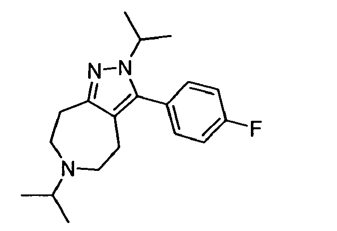

3-(4-Fluoro-phenyl)-2,6-diisopropyl-2,4,5,6,7,8-hexahydro-1,2,6-triaza-azulene.
The title compound (92 mg) was prepared from 3-(4-fluoro-phenyl)-2-isopropyl-2,4,5,6,7,8-hexahydro-1,2,6-triaza-azulene (Example 190) and acetone as in Example 35. MS (ESI): exact mass calculated for C₁₉H₂₆FN₃, 315.21; found, *m*/*z*316.5 [M+H]⁺. ¹H NMR (500 MHz, CD₃OD): 7.42-7.39 (m, 2H), 7.33-7.30 (m, 2H), 4.40 (m, 1 H), 3.78-3.74 (m, 2H), 3.68-3.63 (m, 1 H), 3.36-3.21 (m, 4H), 2.99-2.94 (m, 1 H), 2.80-2.76 (m, 1 H), 1.54-1.37 (m, 12H).

### Example 261

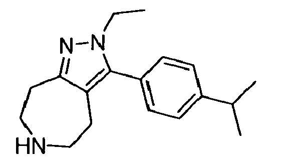

2-Ethyl-3-(4-isopropyl-phenyl)-2,4,5,6,7,8-hexahydro-1,2,6-triaza-azulene.
The title compound (131 mg) was prepared according to Example 193 using 205 mg of 2-ethyl-3-trifluoromethanesulfonyloxy-4,5,7,8-tetrahydro-2*H*-1,2,6-triaza-azulene-6-carboxylic acid *tert*-butyl ester (Example 193, Step A) and 245 mg of 4-isopropylphenylboronic acid. MS (ESI): exact mass calculated for C₁₈H₂₅N₃, 283.20; found, *m*/*z* 284.5 [M+H]⁺. ¹H NMR (500 MHz, CD₃OD): 7.48-7.46 (m, 2H), 7.34-7.33 (m, 2H), 4.12 (q, J = 7.3 Hz, 2H), 3.50-3.45 (m, 2H), 3.36-3.33 (m, 2H), 3.27-3.25 (m, 2H), 3.01 (m, 1 H), 2.87-2.85 (m, 2H), 1.34 (t, J = 7.3 Hz, 3H), 1.31 (d, J = 6.9 Hz, 6H).

### Example 262

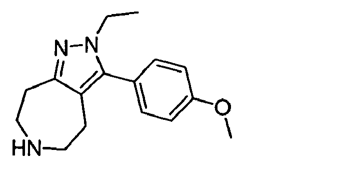

2-Ethyl-3-(4-methoxy-phenyl)-2,4,5,6,7,8-hexahydro-1,2,6-triaza-azulene.
The title compound (134 mg) was prepared according to Example 193 using 219 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A) and 241 mg of 4-methoxyphenylboronic acid. MS (ESI): exact mass calculated for C₁₆H₂₁N₃O, 271.17; found, *m*/*z* 272.5 [M+H]⁺. ¹H NMR (500 MHz, CD₃OD): 7.36-7.33 (m, 2H), 7.15-7.12 (m, 2H), 4.12 (q, J = 7.3 Hz, 2H), 3.88 (s, 3H), 3.49-3.47 (m, 2H), 3.36-3.34 (m, 2H), 3.28-3.25 (m, 2H), 2.88-2.85 (m, 2H), 1.35 (t, J = 7.3 Hz, 3H).

### Example 263

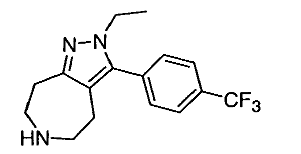

2-Ethyl-3-(4-trifluoromethyl-phenyl)-2,4,5,6,7,8-hexahydro-1,2,6-triaza-azulene.
Step A. 2-Ethyl-3-(4-trifluoromethyl-phenyl)-4,5,7,8-tetrahydro-2H-1,2,6-triaza-azulene-6-carboxylic acid *tert-*butyl ester. To a 25 mL round bottom flask was added 216 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A), 139 mg of 4-trifluoromethylphenylboronic acid, 17 mg of Bu₄N⁺Br⁻, 6 mg of dppf and 17 mg of PdCl₂(dppf). Toluene (5 mL) was added, followed by 0.8 mL of 2 M aq. Na₂CO₃, and the mixture was heated at 120 °C for 12 h under N₂. The mixture was filtered through diatomaceous earth and the filtrate was concentrated *in vacuo* to afford 294 mg of dark brown viscous oil. Chromatography on SiO₂ (0 to 25% EtOAc/hexanes) provided 177 mg of the desired product. MS (ESI): exact mass calculated for C₂₁H₂₆F₃N₃O₂, 409.20; found, *m*/*z* 410.5 [M+H]⁺.
Step B. The title compound (149 mg) was prepared according to Example 43, Step E. MS (ESI): exact mass calculated for C₁₆H₁₈F₃N₃, 309.15; found, *m*/*z* 310.5 [M+H]⁺. ¹H NMR (500 MHz, CD₃OD): 7.90-7.89 (m, 2H), 7.65-7.63 (m, 2H), 4.67 (br s, 2H), 4.10 (q, J = 7.2 Hz, 2H), 4.00-3.56 (m, 2H), 3.36-3.24 (m, 2H), 2.95-2.85 (m, 2H), 1.33 (t, J = 7.2 Hz, 3H).

### Example 264

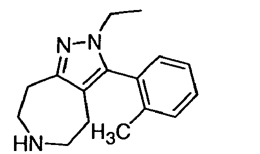

2-Ethyl-3-o-tolyl-2,4,5,6,7,8-hexahydro-1,2,6-triaza-azulene.
The title compound (138 mg) was prepared according to Example 263 using 206 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A) and 95 mg of 2-methylphenylboronic acid. MS (ESI): exact mass calculated for C₁₆H₂₁N₃, 255.17; found, *m*/*z* 256.5 [M+H]⁺. ¹H NMR (500 MHz, CD₃OD): 7.49-7.42 (m, 2H), 7.38-7.35 (m, 1 H), 7.25-7.24 (m, 1 H), 4.69 (br s, 2H), 4.03-3.17 (m, 5H), 2.76-2.68 (m, 2H), 2.15 (s, 3H), 1.28 (t, J = 7.2 Hz, 3H).

### Example 265

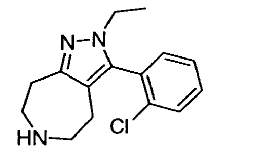

3-(2-Chloro-phenyl)-2-ethyl-2,4,5,6,7,8-hexahydro-1,2,6-triaza-azulene.
The title compound (73 mg) was prepared according to Example 263 using 227 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A) and 120 mg of 2-chlorophenylboronic acid. MS (ESI): exact mass calculated for C₁₅H₁₈ClN₃, 275.12; found, *m*/*z* 276.4 [M+H]⁺, 278.4 [M+H]⁺. ¹H NMR (500 MHz, CD₃OD): 7.64-7.62 (m, 1 H), 7.58-7.48 (m, 2H), 7.41-7.39 (m, 1 H), 3.97-3.86 (m, 2H), 3.44-3.42 (m, 2H), 3.20-3.17 (m, 2H), 2.70-2.63 (m, 2H), 1.26 (t, J = 7.2 Hz, 3H).

### Example 266

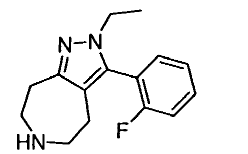

2-Ethyl-3-(2-fluoro-phenyl)-2,4,5,6,7,8-hexahydro-1,2,6-triaza-azulene.
The title compound (121 mg) was prepared according to Example 263 using 205 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A) and 97 mg of 2-fluorophenylboronic acid. MS (ESI): exact mass calculated for C₁₅H₁₈FN₃, 259.15; found, *m*/*z* 260.4 [M+H]⁺. ¹H NMR (500 MHz, CD₃OD): 7.61-7.55 (m, 1 H), 7.39-7.30 (m, 3H), 4.01-3.91 (m, 2H), 3.43-3.41 (m, 2H), 3.18-3.16 (m, 2H), 2.79-2.73 (m, 2H), 1.28 (t, J = 9.0 Hz, 3H).

### Example 267

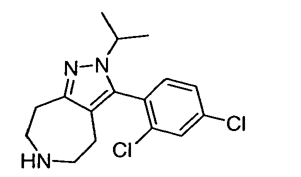

3-(2,4-Dichloro-phenyl)-2-isopropyl-2,4,5,6,7,8-hexahydro-1,2,6-triaza-azulene.
The title compound (37 mg) was prepared according to Example 189 using 230 mg of 2-isopropyl-3-trifluoromethanesulfonyloxy-4,5,7,8-tetrahydro-2H-1,2,6-triaza-azulene-6-carboxylic acid *tert*-butyl ester (Example 189, Step A) and 308 mg of 2,4-dichlorophenylboronic acid. MS (ESI): exact mass calculated for C₁₆H₁₉Cl₂N₃, 323.10; found, *m*/*z* 324.4 [M+H]⁺, 326.4 [M+H]⁺. ¹H NMR (500 MHz, CD₃OD): 7.73-7.72 (m, 1 H), 7.54-7.51 (m, 1 H), 7.37-7.35 (m, 1 H), 4.65 (br s, 1 H), 4.11-4.05 (m, 1 H), 3.43-3.40 (m, 2H), 3.29-3.16 (m, 3H), 2.70-2.63 (m, 2H), 1.39-1.32 (m, 6H).

### Example 268

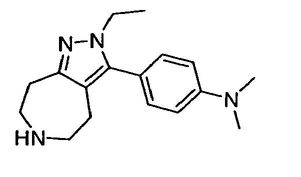

[4-(2-Ethyl-2,4,5,6,7,8-hexahydro-1,2,6-triaza-azulen-3-yl)-phenyl]-dimethylamine.
The title compound (57 mg) was prepared according to Example 263 using 205 mg of 2-ethyl-3-trifluoromethanesulfonyloxy-4,5,7,8-tetrahydro-2H-1,2,6-triaza-azulene-6-carboxylic acid *tert*-butyl ester (Example 193, Step A) and 115 mg of 4-dimethylaminophenylboronic acid. MS (ESI): exact mass calculated for C₁₇H₂₄N₄, 284.20; found, *m*/*z* 285.5 [M+H]⁺. ¹H NMR (500 MHz, CD₃OD): 7.87-7.85 (m, 2H), 7.65-7.63 (m, 2H), 4.67 (br s, 2H), 4.06 (q, J = 9.0 Hz, 2H), 4.00-3.62 (m, 2H), 3.36 (s, 6H), 3.32-3.29 (m, 2H), 3.18-2.81 (m, 2H), 1.31 (t, J = 9.0 Hz, 3H).

### Example 269

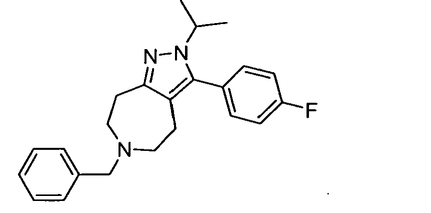

6-Benzyl-3-(4-fluoro-phenyl)-2-isopropyl-2,4,5,6,7,8-hexahydro-1,2,6-triaza-azulene.
The title compound (115 mg) was prepared from 3-(4-fluoro-phenyl)-2-isopropyl-2,4,5,6,7,8-hexahydro-1,2,6-triaza-azulene (Example 190) and benzaldehyde as in Example 35. MS (ESI): exact mass calculated for C₂₃H₂₆FN₃, 363.21; found, *m*/*z* 364.5 [M+H]⁺. ¹H NMR (500 MHz, CD₃OD): 7.58-7.55 (m, 2H), 7.53-7.51 (m, 3H), 7.37-7.33 (m, 2H), 7.31-7.27 (m, 2H), 4.52 (s, 2H), 4.34 (m, 1 H), 3.80-3.75 (m, 1 H), 3.68-3.64 (m, 1 H), 3.35-3.16 (m, 4H), 2.83-2.80 (m, 2H), 1.38 (t, J = 6.7 Hz, 6H).

### Example 270

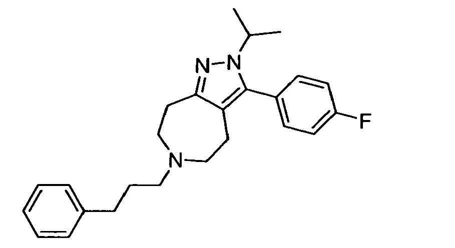

3-(4-Fluoro-phenyl)-2-isopropyl-6-(3-phenyl-propyl)-2,4,5,6,7,8-hexahydro-1,2,6-triaza-azulene.
The title compound (142 mg) was prepared from 3-(4-fluoro-phenyl)-2-isopropyl-2,4,5,6,7,8-hexahydro-1,2,6-triaza-azulene (Example 190) and 3-phenyl-propionaldehyde as in Example 35. MS (ESI): exact mass calculated for C₂₅H₃₀FN₃, 391.24; found, *m*/*z* 392.5 [M+H]⁺. ¹H NMR (500 MHz, CD₃OD): 7.45-7.41 (m, 2H), 7.35-7.26 (m, 6H), 7.22-7.19 (m, 1 H), 4.46 (m, 1 H), 3.79-3.76 (m, 1 H), 3.67-3.63 (m, 1 H), 3.46-3.43 (m, 1 H), 3.35-3.29 (m, 5H), 2.90-2.87 (m, 1 H), 2.83-2.73 (m, 3H), 2.19-2.12 (m, 2H), 1.44 (t, J = 6.7 Hz, 6H).

### Example 271

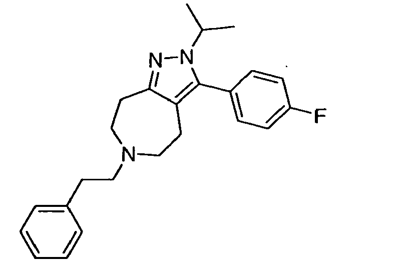

3-(4-Fluoro-phenyl)-2-isopropyl-6-phenethyl-2,4,5,6,7,8-hexahydro-1,2,6-triaza-azulene.
The title compound (104 mg) was prepared from 3-(4-fluoro-phenyl)-2-isopropyl-2,4,5,6,7,8-hexahydro-1,2,6-triaza-azulene (Example 190) and phenylacetaldehyde as in Example 35. MS (ESI): exact mass calculated for C₂₄H₂₈FN₃, 377.23; found, *m*/*z* 378.5 [M+H]⁺. ¹H NMR (500 MHz, CD₃OD): 7.41-7.27 (m, 9H), 4.39 (m, 1 H), 3.88-3.84 (m, 1 H), 3.73-3.71 (m, 1 H), 3.56-3.52 (m, 3H), 3.45-3.20 (m, 3H), 3.17-3.14 (m, 2H), 2.89-2.84 (m, 2H), 1.41 (t, J= 6.6 Hz, 6H).

### Example 272

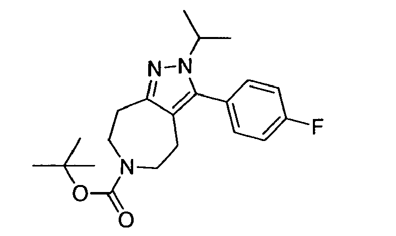

3-(4-Fluoro-phenyl)-2-isopropyl-4,5,7,8-tetrahydro-2H-1,2,6-triaza-azulene-6-carboxylic acid tert-butyl ester.
This compound was obtained as an intermediate in the sequence described for Example 190. MS (ESI): exact mass calculated for C₂₁H₂₈FN₃O₂, 373.46; found, *m*/*z* 374.5 [M+H]⁺. ¹H NMR (500 MHz, CDCl₃): 7.24-7.13 (m, 4H), 4.24 (m, 1H), 3.62-3.55 (m, 2H), 3.49-3.42 (m, 2H), 3.01-2.93 (m, 2H), 2.52-2.44 (m, 2H),1.50-1.45 (m, 9H), 1.39 (d. J = 6.9 Hz, 6H).

### Example 273

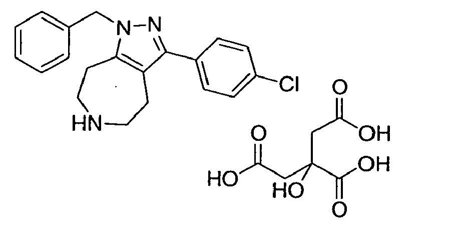

1 -Benzyl-3-(4-chloro-phenyl)-1,4,5,6,7,8-hexahydro-1,2,6-triaza-azulene citrate salt.
Step A. 5-Oxo-azepane-1,4-dicarboxylic acid 1-tert-butyl ester 4-ethyl ester. A dried, N₂-flushed, 500-mL, three-necked, round-bottomed flask equipped with a magnetic stir bar, was charged with 4-oxo-piperidine-1-carboxylic acid tert-butyl ester (20 g, 0.10 mol) and BF₃•Et₂O (14 mL, 0.11 mol) in Et₂O (200 mL) and the mixture was chilled to -5°C. Slowly, ethyl diazoacetate (13.7 mL, 0.13 mol) was added over a period of 1 h causing vigorous gas evolution. The internal temperature was maintained between 0 °C and -5 °C during the addition. The reaction was stirred for 1 h at 0 °C, then slowly quenched with 30% aq. Na₂CO₃ at 0 °C. The pH was adjusted to between 7 and 8 and then H₂O (30 mL) was added to the mixture. The organic layer was extracted with EtOAc (2 x 75 mL), dried with Na₂SO₄, filtered, and concentrated to an orange oil. The crude oil was purified by filtration chromatography (SiO₂: 14 cm OD, 8 cm in height; 10 to 30% EtOAc/hexanes) to recover the title compound as light yellow oil (85%). MS (ESI): exact mass calculated for C₁₄H₂₃NO₅; found, *m*/*z* none, unstable. HPLC (Method B): Rₜ = 8.53 min. ¹H NMR (400 MHz, CDCl₃): 4.25-2.03 (m, 11 H), 1.47-1.45 (d, J = 7.8 Hz, 9H), 1.31-1.24 (m, 3H).
Step B. 3-Oxo-2,3,4,5,7,8-hexahydro-1H-1,2,6-triaza-azulene-6-carboxylic acid tert-butyl ester. In a 1-L, one-necked, round-bottomed flask equipped with a magnetic stir bar was combined 5-oxo-azepane-1,4-dicarboxylic acid 1-tert-butyl ester 4-ethyl ester (24.42 g, 85.0 mmol) and hydrazine (3.0 mL, 0.095 mol) in EtOH (250 mL). The resulting reaction mixture was heated at reflux for 4 h, then was concentrated to provide the desired pyrazole as a white solid in 95% crude yield. The crude pyrazole was used in the next step without further purification. MS (ESI): exact mass calculated for C₁₂H₁₉N₃O₃, 253.14; found, m/z254.1 [M+H]⁺. HPLC (Method B): Rₜ = 6.48 min. ¹H NMR (400 MHz, CDCl₃): 3.64-3.54 (m, 4H), 2.91-2.86 (m, 2H), 2.70-2.65 (m, 2H), 1.49 (s, 9H).
Step C. 3-Trifluoromethanesulfonyloxy-4,5,7,8-tetrahydro-1 H-1,2,6-triaza-azulene-6-carboxylic acid tert-butyl ester. In a 250-mL, one-necked, round-bottomed flask equipped with a magnetic stirring bar, N-phenyltrifluoromethanesulfonimide (50 g, 0.14 mol) was suspended in 100 mL pyridine and then 3-oxo-2,3,4,5,7,8-hexahydro-1H-1,2,6-triaza-azulene-6-carboxylic acid tert-butyl ester (35.4 g, 0.14 mol) was added as a solid at rt. The reaction mixture formed a homogeneous solution after 1 h and stirring was continued at rt overnight (15 h). The solvent was evaporated under vacuum and then the residue was partitioned between Et₂O (500 mL) and 1 M aq. K₂CO₃ (300 mL). The organic layer was separated and washed with aq. K₂CO₃ (1 mol/L, 300 mL) three times and then with brine (200 mL) once, dried over MgSO₄, and evaporated to afford the product as a white solid (50.2 g, 0.13 mol, 93%), which was used on next reaction without further purification. MS (ESI): exact mass calculated for C₁₃H₁₈F₃N₃O₅S, 385.09; *m*/*z* found, 384.0 [M-H]⁻. HPLC (Method B): Rₜ = 9.55 min. ¹H NMR (500 MHz, CDCl₃): 9.52 (s, 1 H), 3.70-3.50 (m, 4H), 3.00-2.85 (m, 2H), 2.70-2.60 (m, 2H), 1.49 (s, 9H).
Step D. 1-Benzyl-3-trifluoromethanesulfonyloxy-4,5,7,8-tetrahydro-1H-1.2.6-triaza-azulene-6-carboxylic acid tert-butyl ester. In a 1-L, three-necked, round-bottomed flask containing a magnetic stirring bar, 3-trifluoromethanesulfonyloxy-4,5,7,8-tetrahydro-1 H-1,2,6-triaza-azulene-6-carboxylic acid tert-butyl ester (48 g, 0.125 mol) was dissolved in 500 mL of dry THF under N₂. The solution was cooled to 0 °C and potassium t-butoxide (15.4 g, 0.137 mol) was added portion-wise as solid. The reaction mixture was stirred for 10 min to form a clear, homogeneous solution. Benzyl bromide (23.4 g, 0.137 mol) was added through an addition funnel over 10 min. The resulting mixture was stirred at rt overnight (15 h). The solvent was evaporated and the residue was re-dissolved in EtOAc (300 mL). The organic layer was washed with H₂O (2x200 mL) and then with brine (200 mL), dried over MgSO₄, filtered, and concentrated. The crude product was purified by pad-filtration through a plug of SiO₂ to afford the pure product as a white solid (44.5 g, 94 mmol, 75%). MS (ESI): exact mass calculated for C₂₀H₂₄F₃N₃O₅S, 475.14; found, *m*/*z* 476.2 [M+H]⁺. HPLC (Method B): Rₜ = 10.90 min. ¹H NMR (500 MHz, CDCl₃): 7.40-7.25 (m, 5H), 7.10-7.05 (m, 2H), 5.22-5.15 (m, 2H), 3.60-3.50 (m, 4H), 2.80-2.60 (m, 4H), 1.47-1.42 (m, 9H).
Step E. 2-(4-Chloro-phenyl)-benzo[1,3,2]dioxaborole. In a 250-mL, one-necked, round-bottomed flask equipped with a Dean-Stark trap and a condenser, the reaction solution of 4-chlorophenylboronic acid (17.5 g, 0.112 mol) and catechol (12.3 g, 0.112 mol) in toluene (150 mL) was heated at reflux for 4 h. The solution was cooled to rt and a white solid precipitated. The solvent was evaporated and the crude product (25.8 g, 0.112 mol, 100%) was used as such in the next reaction without further purification. HPLC (Method B): Rₜ = 6.00 and 7.50 min. ¹H NMR (500 MHz, CDCl₃): 8.01 (d, J = 8.1 Hz, 2H), 7.47 (d, J = 8.1 Hz, 2H), 7.34-7.29 (m, 2H), 7.15-7.11 (m, 2H).
Step F. 1-Benzyl-3-(4-chloro-phenyl)-4,5,7,8-tetrahydro-1H-1,2,6-triaza-azulene-6-carboxylic acid tert-butvl ester. To a 1-L, three-necked, round-bottomed flask was added Pd(dppf)Cl₂ (2.8 g, 3.4 mmol), 1,1'-bis(diphenylphosphino)ferrocene (0.96 g, 1.73 mmol), Bu₄N⁺Br⁻ (2.78 g, 8.6 mmol), Na₂CO₃ (36.5 g, 344 mmol) and 2-(4-chloro-phenyl)-benzo[1,3,2]dioxaborole (23.8 g, 103 mmol), under N₂. A solution of 1-benzyl-3-trifluoromethanesulfonyloxy-4,5,7,8-tetrahydro-1H-1,2,6-triaza-azulene-6-carboxylic acid tert-butyl ester (41 g, 86 mmol) in toluene (250 mL) was added, followed by the addition of H₂O (250 mL) via syringe. The reaction mixture was stirred at reflux for 3 h and then was cooled to rt. The organic layer was diluted with EtOAc (200 mL) and then was washed with 1 M aq. K₂CO₃ until the color of the aqueous layer stabilized. The organic layer was washed with brine (200 mL), dried over MgSO₄, filtered, and concentrated. The crude product thus obtained was pad-filtered through a short plug of SiO₂ to afford the title compound (34.5, 79 mmol, 92%) as a white solid. MS (ESI): exact mass calculated for C₂₅H₂₈ClN₃O₂, 437.19; found, *m*/*z* 438.1, [M+H]⁺. HPLC (Method B): Rₜ = 10.89 min. ¹H NMR (400 MHz, CDCl₃): 7.50-7.45 (m, 2H), 7.40-7.36 (m, 2H), 7.36-7.25 (m, 3H), 7.13-7.10 (m, 2H), 5.35-5.33 (m, 2H), 3.56-3.50 (m, 4H), 2.83-2.75 (m, 4H), 1.28-1.25 (m, 9H).
Step G. 1-Benzyl-3-(4-chloro-phenyl)-1,4,5,6,7,8-hexahydro-1,2,6-triaza-azulene. In a 500-mL, one-necked, round-bottomed flask, 1-benzyl-3-(4-chloro-phenyl)-4,5,7,8-tetrahydro-1H-1,2,6-triaza-azulene-6-carboxylic acid tert-butyl ester (34 g, 77 mmol) was dissolved in CH₂Cl₂ (100 mL). Trifluoroacetic acid (70 mL) was added carefully. The reaction mixture was stirred at rt for 2 h. The solvent was evaporated and the residue was re-dissolved in CH₂Cl₂ (200 mL). Sat. aq. NaHCO₃ solution was added slowly until CO₂ evolution ceased. The aqueous layer was extracted with CH₂Cl₂ (2x200 mL). The organic layers were combined, dried over MgSO₄, filtered, and concentrated. The crude product was recrystallized from hot EtOAc to afford the pure product as a white solid (24 g, 71 mmol, 91%). MS (ESI): exact mass calculated for C₂₀H₂₀ClN₃, 337.13; found, *m*/*z* 338.3 [M+H]⁺. HPLC (Method B): Rₜ = 7.53 min. ¹H NMR (400 MHz, CDCl₃): 7.48-7.44 (m, 2H), 7.44-7.38 (m, 3H), 7.38-7.27 (m, 3H), 7.14-7.06 (m, 2H), 5.36 (s, 2H), 3.30-3.16 (m, 4H), 3.10-2.98 (m, 4H).
Step H. 1-Benzyl-3-(4-chloro-phenyl)-1,4,5,6,7,8-hexahydro-1,2,6-triaza-azulene citrate salt. In a 500-mL, one-necked, round-bottomed flask, 1-benzyl-3-(4-chloro-phenyl)-1,4,5,6,7,8-hexahydro-1,2,6-triaza-azulene 7 (10 g, 30 mmol) was suspended in MeOH (70 mL), and the mixture was heated until a homogeneous solution formed. A solution of citric acid monohydrate (7.5 g, 36 mmol) in MeOH (10 mL) was added dropwise. The resulting homogeneous solution was heated at reflux for 20 min and then was cooled to rt. The solvent was evaporated to form an oil. The oil was diluted with EtOAc (200 mL) and the mixture was heated to reflux. To this hot solution MeOH was slowly added to form a slurry. The slurry was cooled to rt and the precipitated solids were collected by filtration, washed with EtOAc, and dried under vacuum to afford the citrate salt (1:1 ratio based on ¹HNMR analysis, 9.1 g). The filtrate was concentrated and the above procedure to form the citrate salt was repeated by adding another 0.5 equivalents of citric acid to afford another 2 g of product. The combined yield was 71%. ¹H NMR (500 MHz, D₂O): 7.35-7.22 (m, 4H), 7.22-7.15 (m, 3H), 7.0-6.92 (m, 2H), 5.22 (s, 2H), 3.22-3.14 (m, 4H), 3.0-2.92 (m, 2H), 2.88-2.80 (m, 2H), 2.69 (d, J = 15 Hz, 2H), 2.57 (d, J = 15 Hz, 2H).

### Example 274

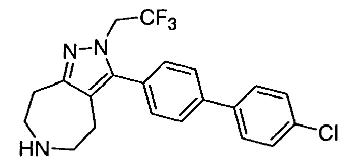

3-(4'-Chloro-biphenyl-4-yl)-2-(2,2,2-trifluoro-ethyl)-2,4,5,6,7,8-hexahydro-1,2,6-triaza-azulene.
The title compound (18 mg) was also obtained from Example 187, Step B. MS (ESI): exact mass calculated for C₂₁H₁₉ClF₃N₃, 405.12; found, *m*/*z* 406.1 [M+H]⁺, 408.0 [M+H]⁺. ¹H NMR (500 MHz, CD₃OD): 7.74-7.72 (m, 2H), 7.61-7.59 (m, 2H), 7.41-7.35 (m, 4H), 4.70-4.55 (m, 3H), 3.85-3.30 (m, 3H), 3.25-3.05 (m, 2H), 2.82-2.74 (m, 2H).

### Example 275

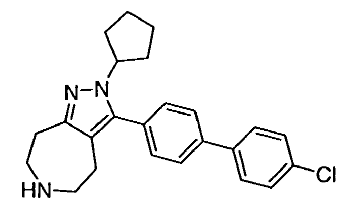

3-(4'-Chloro-biphenyl-4-yl)-2-cyclopentyl-2,4,5,6,7,8-hexahydro-1,2,6-triaza-azulene.
The title compound (33 mg) was also obtained from Example 181. MS (ESI): exact mass calculated for C₂₄H₂₆CIN₃, 391.18; found, *m*/*z* 392.1 [M+H]⁺, 394.1 [M+H]⁺. ¹H NMR (500 MHz, CD₃OD): 7.81-7.80 (m, 2H), 7.70-7.68 (m, 2H), 7.50-7.41 (m, 4H), 4.65-4.53 (m, 3H), 3.85-3.67 (m, 2H), 3.30-3.10 (m, 2H), 2.88 (br s, 2H), 2.01-1.91 (m, 6H), 1.63-1.60 (m, 2H).

### Example 276

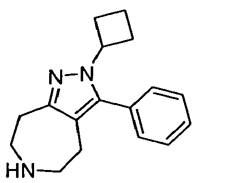

2-Cyclobutyl-3-phenyl-2,4,5,6,7,8-hexahydro-1,2,6-triaza-azulene.
Step A. 2-Cyclobutyl-3-trifluoromethanesulfonyloxy-4,5,7,8-tetrahydro-2H-1,2,6-triaza-azulene-6-carboxylic acid *tert*-butyl ester. The desired triflate was prepared as in Steps A and B of Example 176, using cyclobutylhydrazine hydrochloride (made from cyclobutanone as shown in Example 177, Step A) in place of phenylhydrazine and *t*-butanol in place of EtOH, with the addition of 3 equiv of triethylamine.
Step B. The title compound (118 mg) was prepared according to Example 263 using 189 mg of the product from Step A and 73 mg of phenylboronic acid. MS (ESI): exact mass calculated for C₁₇H₂₁N₃, 267.17; found, *m*/*z* 268.5 [M+H]⁺. ¹H NMR (500 MHz, CD₃OD): 7.60-7.50 (m, 3H), 7.34-7.30 (m, 2H), 4.71-4.63 (m, 2H), 4.00-3.40 (m, 2H), 3.24-3.22 (m, 3H), 3.00-2.80 (m, 2H), 2.68-2.59 (m, 2H), 2.30-2.24 (m, 2H), 2.30-2.24 (m, 2H), 1.84-1.71 (m, 2H).

### Example 277

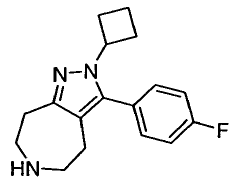

2-Cyclobutyl-3-(4-fluoro-phenyl)-2,4,5,6,7,8-hexahydro-1,2,6-triaza-azulene.
The title compound (122 mg) was prepared according to Example 263 using 198 mg of 2-cyclobutyl-3-trifluoromethanesulfonyloxy-4,5,7,8-tetrahydro-2H-1,2,6-triaza-azulene-6-carboxylic acid *tert*-butyl ester (Example 276, Step A) and 88 mg of 4-fluorophenylboronic acid. MS (ESI): exact mass calculated for C₁₇H₂₀FN₃, 285.16; found, *m*/*z* 286.4 [M+H]⁺. ¹H NMR (500 MHz, CD₃OD): 7.35-7.27 (m, 4H), 4.65-4.59 (m, 2H), 3.95-3.3.40 (m, 2H), 3.32-3.05 (m, 3H), 3.00-2.75 (m, 2H), 2.67-2.59 (m, 2H), 2.29-2.23 (m, 2H), 1.84-1.73 (m, 2H).

### Example 278

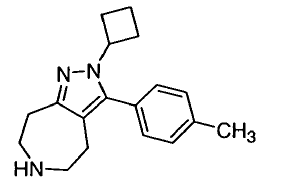

2-Cyclobutyl-3-p-tolyl-2,4,5,6,7,8-hexahydro-1,2,6-triaza-azulene.
The title compound (117 mg) was prepared according to Example 263 using 192 mg of 2-cyclobutyl-3-trifluoromethanesulfonyloxy-4,5,7,8-tetrahydro-2H-1,2,6-triaza-azulene-6-carboxylic acid *tert*-butyl ester (Example 276, Step A) and 83 mg of 4-methylphenylboronic acid. MS (ESI): exact mass calculated for C₁₈H₂₃N₃, 281.19; found, *m*/*z* 282.5 [M+H]⁺. ¹H NMR (500 MHz, CD₃OD): 7.41-7.32 (m, 2H), 7.23-7.13 (m, 2H), 4.71-4.65 (m, 2H), 4.00-3.41 (m, 2H), 3.32-3.05 (m, 3H), 2.95-2.79 (m, 2H), 2.67-2.58 (m, 2H), 2.43 (s, 3H), 2.28-2.23 (m, 2H), 1.84-1.71 (m, 2H).

### Example 279

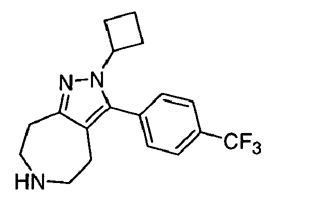

2-Cyclobutyl-3-(4-trifluoromethyl-phenyl)-2,4,5,6,7,8-hexahydro-1,2,6-triaza-azulene.
The title compound (73 mg) was prepared according to Example 263 using 201 mg of 2-cyclobutyl-3-trifluoromethanesulfonyloxy-4,5,7,8-tetrahydro-2H-1,2,6-triaza-azulene-6-carboxylic acid *tert*-butyl ester (Example 276, Step A) and 122 mg of 4-trifluoromethylphenylboronic acid. MS (ESI): exact mass calculated for C₁₈H₂₀F₃N₃, 335.16; found, *m*/*z* 336.4 [M+H]⁺. ¹H NMR (500 MHz, CD₃OD): 7.86-7.85 (m, 2H), 7.52-7.50 (m, 2H), 4.65-4.58 (m, 1 H), 3.45-3.41 (m, 2H), 3.22-3.20 (m, 2H), 2.81-2.79 (m, 2H), 2.67-2.62 (m, 2H), 2.30-2.24 (m, 2H), 1.84-1.74 (m, 2H).

### Example 280

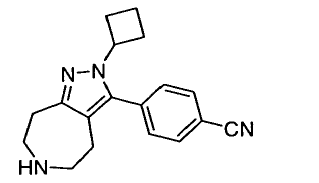

4-(2-Cyclobutyl-2,4,5,6,7,8-hexahydro-1,2,6-triaza-azulen-3-yl)-benzonitrile
The title compound (28 mg) was prepared according to Example 263 using 172 mg of 2-cyclobutyl-3-trifluoromethanesulfonyloxy-4,5,7,8-tetrahydro-2H-1,2,6-triaza-azulene-6-carboxylic acid *tert*-butyl ester (Example 276, Step A) and 172 mg of 4-cyanophenylboronic acid. MS (ESI): exact mass calculated for C₁₈H₂₀N₄, 292.17; found, *m*/*z* 293.5 [M+H]⁺. ¹H NMR (500 MHz, CD₃OD): 7.92-7.90 (m, 2H), 7.50-7.48 (m, 2H), 4.65-4.58 (m, 1 H), 3.42-3.40 (m, 2H), 3.21-3.19 (m, 2H), 2.81-2.78 (m, 2H), 2.66-2.62 (m, 2H), 2.30-2.25 (m, 2H), 1.85-1.74 (m, 2H).

### Example 281

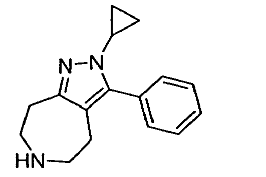

2-Cyclopropyl-3-phenyl-2,4,5,6,7,8-hexahydro-1,2,6-triaza-azulene.
Step A. *N*-cyclopropyl-hydrazinecarboxylic acid *tert-*butyl ester. To a solution of 1.37 g of 3-(4-cyano-phenyl)-oxaziridine-2-carboxylic acid *tert*-butyl ester in Et₂O (8 mL) was added 1.2 mL of cyclopropylamine. The mixture was aged for 2 h and then concentrated *in vacuo.* Chromatography on SiO₂ (0 to 25% EtOAc/hexanes) provided an impure pale yellow solid that was sublimed under high vacuum in a 50 °C oil bath to afford 641 mg of the desired compound. ¹H NMR (500 MHz, CDCl₃): 6.31 (br s, 1 H), 3.49 (br s, 1 H), 2.74 (br s, 1 H), 1.48 (s, 9H), 0.52-0.48 (m, 4H).
Step B. Cyclopropyl-hydrazine hydrochloride. To a solution of the product from Step A (636 mg) in CH₂Cl₂ (10 mL) was added 9 mL of 4.0 M HCI in 1,4-dioxane. The mixture was aged for 12 h and then concentrated *in vacuo* to provide 507 mg of the title compound. ¹H NMR (500 MHz, CD₃OD): 2.61-2.57 (m, 1 H), 0.71-0.59 (m, 4H).
Step C. 2-Cyclopropyl-3-trifluoromethanesulfonyloxy-4,5,7,8-tetrahydro-2H-1,2,6-triaza-azulene-6-carboxylic acid *tert*-butyl ester. The desired triflate was prepared as in Steps A and B of Example 176, using cyclopropyl-hydrazine hydrochloride in place of phenylhydrazine and *t*-butanol in place of EtOH, with the addition of 3 equiv. of triethylamine.
Step D. The title compound (128 mg) was prepared according to Example 263 using 208 mg of 2-cyclopropyl-3-trifluoromethanesulfonyloxy-4,5,7,8-tetrahydro-2H-1,2,6-triaza-azulene-6-carboxylic acid *tert*-butyl ester and 84 mg of phenylboronic acid. MS (ESI): exact mass calculated for C₁₆Hₗ₉N₃, 253.16; found, *m*/*z* 254.4 [M+H]⁺. ¹H NMR (500 MHz, CD₃OD): 7.57-7.44 (m, 5H), 3.57-3.54 (m, 1 H), 3.42-3.40 (m, 2H), 3.17-3.14 (m, 2H), 2.93-2.84 (m, 2H), 0.91-0.85 (m, 4H).

### Example 282

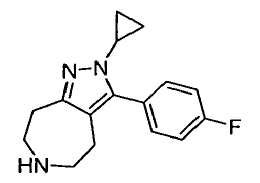

2-Cyclopropyl-3-(4-fluoro-phenyl)-2,4,5,6,7,8-hexahydro-1,2,6-triaza-azulene.
The title compound (134 mg) was prepared according to Example 281 using 200 mg of 2-cyclopropyl-3-trifluoromethanesulfonyloxy-4,5,7,8-tetrahydro-2H-1,2,6-triaza-azulene-6-carboxylic acid *tert*-butyl ester (Example 281, Step C) and 92 mg of 4-fluorophenylboronic acid. MS (ESI): exact mass calculated for C₁₆H₁₈FN₃, 271.15; found, *m*/*z* 272.5 [M+H]⁺. ¹H NMR (500 MHz, CD₃OD): 7.51-7.47 (m, 2H), 7.31-7.27 (m, 2H), 3.55-3.51 (m, 1 H), 3.41-3.39 (m, 2H), 3.23-3.14 (m, 2H), 3.00-2.83 (m, 2H), 0.93-.084 (m, 4H).

### Example 283

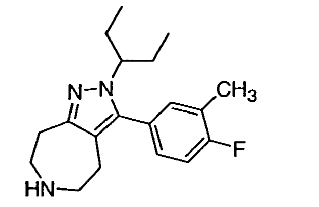

2-(1-Ethyl-propyl)-3-(4-fluoro-3-methyl-phenyl)-2,4,5,6,7,8-hexahydro-1,2,6-triaza-azulene.
The title compound (34 mg) was prepared according to Example 183 using 59 mg of 2-(1-ethyl-propyl)-3-trifluoromethanesulfonyloxy-4,5,7,8-tetrahydro-2H-1,2,6-triaza-azulene-6-carboxylic acid *tert*-butyl ester (Example 183, Step A) and 19 mg of 4-fluoro-3-methylphenylboronic acid. MS (ESI): exact mass calculated for C₁₉H₂₆FN₃, 315.21; found, *m*/*z* 316.5 [M+H]⁺. ¹H NMR (500 MHz, CD₃OD): 7.27-7.18 (m, 3H), 4.69-4.65 (m, 1 H), 3.93-3.89 (m, 1 H), 3.50-3.27 (m, 5H), 3.00-2.80 (m, 2H), 2.35 (s, 3H), 1.95-1.87 (m, 2H), 1.83-1.76 (m, 2H), 0.81-0.68 (m, 6H).

### Example 284

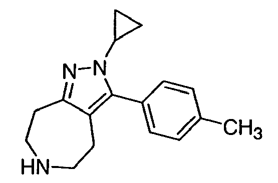

2-Cyclopropyl-3-p-tolyl-2,4,5,6,7,8-hexahydro-1,2,6-triaza-azulene.
The title compound (133 mg) was prepared according to Example 281 using 200 mg of 2-cyclopropyl-3-trifluoromethanesulfonyloxy-4,5,7,8-tetrahydro-2H-1,2,6-triaza-azulene-6-carboxylic acid *tert*-butyl ester (Example 281, Step C) and 90 mg of 4-methylphenylboronic acid. MS (ESI): exact mass calculated for C₁₇H₂₁N₃, 267.17; found, *m*/*z* 268.5 [M+H]⁺. ¹H NMR (500 MHz, CD₃OD): 7.42-7.34 (m, 4H), 4.68-4.65 (m, 2H), 3.80-3.30 (m, 6H), 2.97 (br s, 2H), 2.44 (s, 3H), 0.94-0.91 (m, 4H).

### Example 285

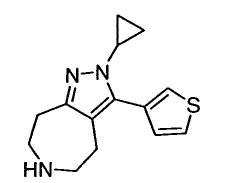

2-Cyclopropyl-3-thiophen-3-yl-2,4,5,6,7,8-hexahydro-1,2,6-triaza-azulene.
The title compound (134 mg) was prepared according to Example 281 using 200 mg of 2-cyclopropyl-3-trifluoromethanesulfonyloxy-4,5,7,8-tetrahydro-2H-1,2,6-triaza-azulene-6-carboxylic acid *tert*-butyl ester (Example 281, Step C) and 84 mg of 3-thiopheneboronic acid. MS (ESI): exact mass calculated for C₁₄H₁₇N₃S, 259.11; found, *m*/*z* 260.4 [M+H]⁺. ¹H NMR (500 MHz, CD₃OD): 7.64-7.63 (m, 2H), 7.31-7.29 (m, 1 H), 4.65 (br s, 1 H), 3.70-3.60 (br s, 1 H), 3.57-3.52 (m, 1 H), 3.40-3.38 (m, 1 H), 3.19 (br s, 1 H), 3.13-3.11 (m, 1H), 2.99-2.96 (m, 1 H), 2.91-2.89 (m, 1 H), 0.93-0.88 (m, 4H).

### Example 286

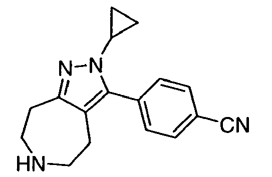

4-(2-Cyclopropyl-2,4,5,6,7,8-hexahydro-1,2,6-triaza-azulen-3-yl)-benzonitrile.
The title compound (91 mg) was prepared according to Example 281 using 200 mg of 2-cyclopropyl-3-trifluoromethanesulfonyloxy-4,5,7,8-tetrahydro-2H-1,2,6-triaza-azulene-6-carboxylic acid *tert*-butyl ester (Example 281, Step C) and 97 mg of 4-cyanophenylboronic acid. MS (ESI): exact mass calculated for C₁₇H₁₈N₄, 278.15; found, *m*/*z* 279.4 [M+H]⁺. ¹H NMR (500 MHz, CD₃OD): 7.94-7.92 (m, 2H), 7.71-7.70 (m, 2H), 4.66 (br s, 1 H), 3.71-3.68 (m, 1 H), 3.47 (br s, 1 H), 3.39-3.19 (m, 4H), 3.01-2.88 (m, 2H), 0.96-0.90 (m, 4H).

### Example 287

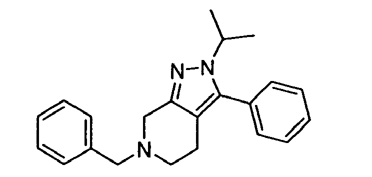

6-Benzyl-2-isopropyl-3-phenyl-4,5,6,7-tetrahydro-2H-pyrazolo[3,4-c]pyridine.
Step A. Trifluoro-methanesulfonic acid 6-benzyl-2-isopropyl-4,5,6,7-tetrahydro-2H-pyrazolo[3,4-c]pyridin-3-yl ester. The desired triflate was prepared as in Step A of Example 189, using 1-benzyl-3-oxo-piperidine-4-carboxylic acid ethyl ester in place of the product from Example 59, Step A.
Step B. The title compound (54 mg) was prepared as in Example 263 using 200 mg of trifluoro-methanesulfonic acid 6-benzyl-2-isopropyl-4,5,6,7-tetrahydro-2H-pyrazolo[3,4-c]pyridin-3-yl ester and 85 mg of phenylboronic acid. MS (ESI): exact mass calculated for C₂₂H₂₅N₃, 331.20; found, *m*/*z* 332.5 [M+H]⁺. ¹H NMR (500 MHz, CD₃OD): 7.60-7.48 (m, 7H), 7.39-7.37 (m, 2H), 4.59-4.48 (m, 3H), 4.44-4.34 (m, 2H), 3.81-3.79 (m, 1 H), 3.46-3.40 (m, 1 H), 2.94-2.84 (m, 2H), 1.46-1.29 (m, 6H).

### Example 288

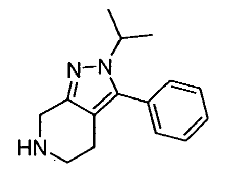

2-Isopropyl-3-phenyl-4,5,6,7-tetrahydro-2H-pyrazolo[3,4-c]pyridine.
To a solution of the compound (98 mg) from Example 287, Step B in 5 mL of EtOH was added 98 mg of 10% Pd/C followed by 0.14 mL of 1,4-cyclohexadiene. The mixture was placed under N₂ and heated in an 80 °C oil bath for 5h. The mixture was filtered and the filtrate was concentrated *in vacuo* to provide 67 mg of viscous colorless oil. Chromatography on SiO₂ (0 to 8% 2 M NH₃ in MeOH/EtOAc) afforded 59 mg of the title compound. The product (59 mg) was dissolved in Et₂O and treated with excess 1.0 M HCl in Et₂O for 30 min. The solvent was removed *in vacuo* to afford 68 mg of the corresponding HCl salt. MS (ESI): exact mass calculated for C₁₅H₁₉N₃, 241.16; found, *m*/*z* 242.4 [M+H]⁺. ¹H NMR (500 MHz, CD₃OD): 7.57-7.48 (m, 3H), 7.38-7.36 (m, 2H), 4.53 (m, 1 H), 4.40-4.32 (m, 2H), 3.47 (t, J = 6.2 Hz, 2H), 2.82 (t, J = 6.2 Hz, 2H), 1.41 (d, J = 6.7 Hz, 6H).

### Example 289

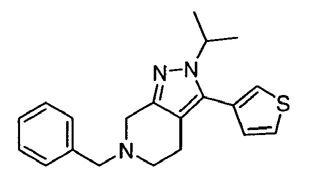

6-Benzyl-2-isopropyl-3-thiophen-3-yl-4,5,6,7-tetrahydro-2H-pyrazolo[3,4-c]pyridine.
The title compound (69 mg) was prepared according to Example 287 using 300 mg of trifluoro-methanesulfonic acid 6-benzyl-2-isopropyl-4,5,6,7-tetrahydro-2H-pyrazolo[3,4-c]pyridin-3-yl ester and 133 mg of 3-thiopheneboronic acid. MS (ESI): exact mass calculated for C₂₀H₂₃N₃S, 337.16; found, *m*/*z* 338.5 [M+H]⁺. ¹H NMR (500 MHz, CD₃OD): 7.66-7.65 (m, 1 H), 7.60-7.57 (m, 3H), 7.55-7.53 (m, 3H), 7.21-7.20 (m, 1 H), 4.65-4.52 (m, 3H), 4.42-4.33 (m, 2H), 3.82-3.79 (m, 1 H), 3.44-3.40 (m, 1 H), 2.94-2.91 (m, 2H), 1.46-1.35 (m, 6H).

### Example 290

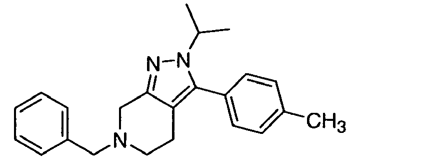

6-Benzyl-2-isopropyl-3-p-tolyl-4,5,6,7-tetrahydro-2H-pyrazolo[3,4-c]pyridine.
The title compound (67 mg) was prepared according to Example 287 using 300 mg of trifluoro-methanesulfonic acid 6-benzyl-2-isopropyl-4,5,6,7-tetrahydro-2H-pyrazolo[3,4-c]pyridin-3-yl ester and 141 mg of 4-methylphenylboronic acid. MS (ESI): exact mass calculated for C₂₃H₂₇N₃, 345.22; found, *m*/*z* 346.5 [M+H]⁺. ¹H NMR (500 MHz, CD₃OD): 7.60-7.58 (m, 2H), 7.54-7.53 (m, 3H), 7.37-7.35 (m, 2H), 7.28-7.24 (m, 2H), 4.58-4.49 (m, 3H), 4.42-4.33 (m, 2H), 3.81-3.78 (m, 1 H), 3.45-3.41 (m, 1 H), 2.90-2.82 (m, 2H), 2.41 (s, 3H), 1.42-1.29 (m, 6H).

### Example 291

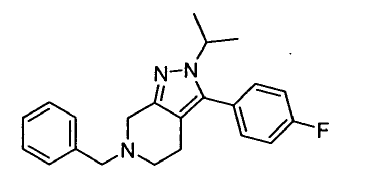

6-Benzyl-3-(4-fluoro-phenyl)-2-isopropyl-4,5,6,7-tetrahydro-2H-pyrazolo[3,4-c]pyridine.
The title compound (72 mg) was prepared according to Example 287 using 300 mg of trifluoro-methanesulfonic acid 6-benzyl-2-isopropyl-4,5,6,7-tetrahydro-2H-pyrazolo[3,4-c]pyridin-3-yl ester and 146 mg of 4-fluorophenylboronic acid. MS (ESI): exact mass calculated for C₂₂H₂₄FN₃, 349.20; found, *m*/*z* 350.5 [M+H]⁺. ¹H NMR (500 MHz, CD₃OD): 7.60-7.57 (m, 2H), 7.55-7.53 (m, 3H), 7.43-7.40 (m, 2H), 7.32-7.27 (m, 2H), 4.59-4.34 (m, 5H), 3.81-3.79 (m, 1 H), 3.46-3.40 (m, 1 H), 2.90-2.85 (m, 2H), 1.43-1.36 (m, 6H).

### Example 292

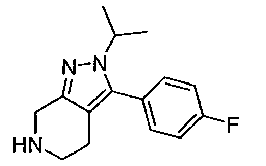

3-(4-Fluoro-phenyl)-2-isopropyl-4,5,6,7-tetrahydro-2H-pyrazolo[3,4-c]pyridine.
The title compound (101 mg) was prepared according to Example 288 using 153 mg of 6-benzyl-3-(4-fluoro-phenyl)-2-isopropyl-4,5,6,7-tetrahydro-2H-pyrazolo[3,4-c]pyridine in place of the product of Example 287, Step B. MS (ESI): exact mass calculated for C₁₅H₁₈FN₃, 259.15; found, *m*/*z* 260.4 [M+H]⁺. ¹H NMR (500 MHz, CD₃OD): 7.42-7.28 (m, 4H), 4.50-4.47 (m, 1 H), 4.35 (br s, 2H), 3.49-3.46 (m, 2H), 2.81-2.79 (m, 2H), 1.42-1.36 (m, 6H).

### Example 293

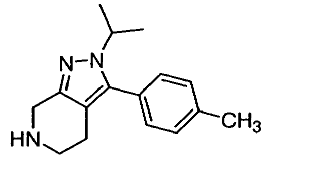

2-Isopropyl-3-p-tolyl-4,5,6,7-tetrahydro-2H-pyrazolo[3,4-c]pyridine.
The title compound (114 mg) was prepared according to Example 288 using 163 mg of 6-benzyl-2-isopropyl-3-p-tolyl-4,5,6,7-tetrahydro-2H-pyrazolo[3,4-c]pyridine in place of the product of Example 287, Step B. MS (ESI): exact mass calculated for C₁₆H₂₁N₃, 255.17; found, *m*/*z* 256.4 [M+H]⁺. ¹H NMR (500 MHz, CD₃OD): 7.43-7.32 (m, 2H), 7.30-7.20 (m, 2H), 4.52 (m, 1 H), 4.39-4.31 (m, 2H), 3.47 (t, *J* = 6.2 Hz, 2H), 2.80 (t, J = 6.2 Hz, 2H), 1.42 (d, *J* = 6.6 Hz, 6H.

### Example 294

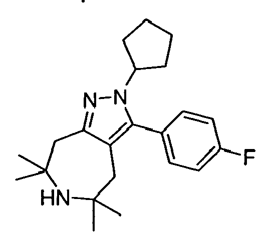

2-Cyclopentyl-3-(4-fluoro-phenyl)-5,5,7,7-tetramethyl-2,4,5,6,7,8-hexahydro-1,2,6-triaza-azulene.
Step A. Trifluoro-methanesulfonic acid 2-cyclopentyl-5,5,7,7-tetramethyl-2,4,5,6,7,8-hexahydro-1,2,6-triaza-azulen-3-yl ester. The desired triflate was prepared as in Step A of Example 180 using 2,2,7,7-tetramethyl-5-oxo-azepane-4-carboxylic acid ethyl ester (made from 2,2,6,6-tetramethyl-piperidin-4-one as shown in Step A of Example 59) in place of 5-oxo-azepane-1,4-dicarboxylic acid *tert*-butyl ester 4-ethyl ester.
Step B. The title compound was prepared as in Step A of Example 263, using 216 mg of trifluoro-methanesulfonic acid 2-cyclopentyl-5,5,7,7-tetramethyl-2,4,5,6,7,8-hexahydro-1,2,6-triaza-azulen-3-yl ester and 103 mg of 4-fluorophenylboronic acid. The product (134 mg) was dissolved in Et₂O and treated with excess 1.0 M HCl in Et₂O for 30 min. The solvent was removed *in vacuo* to afford 146 mg of the corresponding HCl salt. MS (ESI): exact mass calculated for C₂₂H₃₀FN₃, 355.24; found, *m*/*z* 356.5 [M+H]⁺. ¹H NMR (500 MHz, CD₃OD): 7.36-7.26 (m, 4H), 4.47-4.41 (m, 1 H), 3.15 (s, 2H), 2.71 (s, 2H), 2.03-1.88 (m, 6H), 1.61-1.57 (m, 2H), 1.44 (s, 6H), 1.35 (s, 6H).

### Example 295

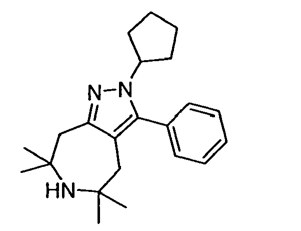

2-Cyclopentyl-5,5,7,7-tetramethyl-3-phenyl-2,4,5,6,7,8-hexahydro-1,2,6-triaza-azulene.
The title compound (129 mg) was prepared as in Example 294, using 263 mg of trifluoro-methanesulfonic acid 2-cyclopentyl-5,5,7,7-tetramethyl-2,4,5,6,7,8-hexahydro-1,2,6-triaza-azulen-3-yl ester and 110 mg of phenylboronic acid. MS (ESI): exact mass calculated for C₂₂H₃₁N₃, 337.25; found, *m*/*z* 338.5 [M+H]⁺. ¹H NMR (500 MHz, CD₃OD): 7.56-7.49 (m, 3H), 7.32-7.30 (m, 2H), 4.51-4.44 (m, 1 H), 3.12 (s, 2H), 2.72 (s, 2H), 2.03-1.88 (m, 6H), 1.61-1.57 (m, 2H), 1.45 (s, 6H), 1.35 (s, 6H).

### Example 296

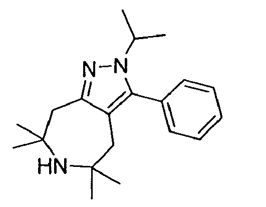

2-Isopropyl-5,5,7,7-tetramethyl-3-phenyl-2,4,5,6,7,8-hexahydro-1,2,6-triaza-azulene.
Step A. Trifluoro-methanesulfonic acid 2-isopropyl-5,5,7,7-tetramethyl-2,4,5,6,7,8-hexahydro-1,2,6-triaza-azulen-3-yl ester. The desired triflate was prepared as in Step A of Example 294 using isopropylhydrazine in place of cyclopentylhydrazine.
Step B. The title compound (98 mg) was prepared as in Step B of Example 294 using 196 mg trifluoro-methanesulfonic acid 2-isopropyl-5,5,7,7-tetramethyl-2,4,5,6,7,8-hexahydro-1,2,6-triaza-azulen-3-yl ester and 87 mg of phenylboronic acid. MS (ESI): exact mass calculated for C₂₀H₂₉N₃, 311.24; found, *m*/*z* 312.5 [M+H]⁺. ¹H NMR (500 MHz, CD₃OD): 7.57-7.52 (m, 3H), 7.32-7.31 (m, 2H), 4.34 (m, 1 H), 3.11 (s, 2H), 2.71 (s, 2H), 1.49-1.34 (m, 18H).

### Example 297

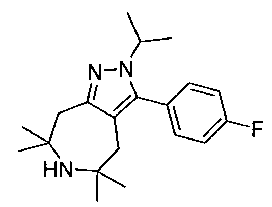

3-(4-Fluoro-phenyl)-2-isopropyl-5,5,7,7-tetramethyl-2,4,5,6,7,8-hexahydro-1,2,6-triaza-azulene.
The title compound (100 mg) was prepared as in Example 296 using 196 mg of trifluoro-methanesulfonic acid 2-isopropyl-5,5,7,7-tetramethyl-2,4,5,6,7,8-hexahydro-1,2,6-triaza-azulen-3-yl ester and 100 mg of 4-fluorophenylboronic acid. MS (ESI): exact mass calculated for C₂₀H₂₈FN₃, 329.23; found, *m*/*z* 330.5 [M+H]⁺. ¹H NMR (500 MHz, CD₃OD): 7.36-7.27 (m, 4H), 4.31 (m, 1H), 3.10 (s, 2H), 2.70 (s, 2H), 1.47-1.34 (m, 18H).

### Example 298

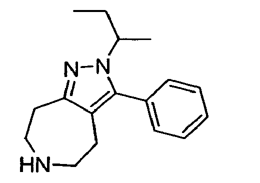

2-sec-Butyl-3-phenyl-2,4,5,6,7,8-hexahydro-1,2,6-triaza-azulene.
Step A. 2-sec-Butyl-3-trifluoromethanesulfonyloxy-4,5,7,8-tetrahydro-2H-1,2,6-triaza-azulene-6-carboxylic acid *tert*-butyl ester. The desired triflate was prepared according to Example 189, Step A, using sec-butylhydrazine hydrochloride (made from 2-butanone as shown in Example 177, Step A) in place of isopropylhydrazine hydrochloride.
Step B. The title compound (97 mg) was prepared as in Example 263 using 216 mg of the triflate from Step A and 106 mg of phenylboronic acid. MS (ESI): exact mass calculated for C₁₇H₂₃N₃, 269.38; found, *m*/*z* 270.5 [M+H]⁺. ¹H NMR (500 MHz, CD₃OD): 7.58-7.50 (m, 3H), 7.35-7.31 (m, 2H), 4.15-4.07 (m, 1 H), 3.50-3.18 (m, 6H), 2.88-2.73 (m, 2H), 1.97-1.86 (m, 1 H), 1:74-1.65 (m, 1 H), 1.43 (d, J = 6.8 Hz, 3H), 0.64 (t, J = 7.4 Hz, 3H).

### Example 299

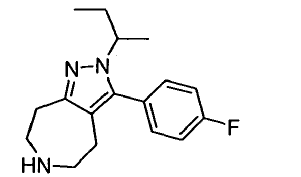

2-sec-Butyl-3-(4-fluoro-phenyl)-2,4,5,6,7,8-hexahydro-1,2,6-triaza-azulene.
The title compound (71 mg) was prepared as in Example 263 using 245 mg of the triflate from Example 298, Step A, and 153 mg of 4-fluorophenylboronic acid. MS (ESI): exact mass calculated for C₁₇H₂₂FN₃, 287.38; found, *m*/*z* 288.5 [M+H] ⁺. ¹H NMR (500 MHz, CD₃OD): 7.41-7.35 (m, 2H), 7.34-7.28 (m, 2H), 4.12-4.03 (m, 1 H), 3.51-3.20 (m, 6H), 2.90-2.73 (m, 2H), 1.97-1.87 (m, 1 H), 1.75-1.65 (m, 1 H), 1.44 (d, J = 6.6 Hz, 3H), 0.66 (t, J = 7.4 Hz, 3H).

### Example 300

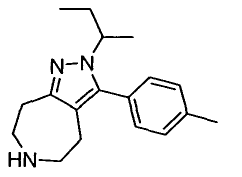

2-*sec-*Butyl-3-p-tolyl-2,4,5,6,7,8-hexahydro-1,2,6-triaza-azulene.
The title compound (116 mg) was prepared as in Example 263 using 249 mg of the triflate from Example 298, Step A, and 129 mg of 4-methylphenylboronic acid. MS (ESI): exact mass calculated for C₁₈H₂₅N₃, 283.41; found, *m*/*z* 284.5 [M+H]⁺. ¹H NMR (500 MHz, CD₃OD): 7.42-7.37 (m, 2H), 7.27-7.21 (m, 2H), 4.23-4.12 (m, 1 H), 3.55-3.23 (m, 6H), 2.92-2.75 (m, 2H), 2.43 (s, 3H), 1.98-1.88 (m, 1 H), 1.77-1.68 (m, 1 H), 1.46 (d, *J* = 6.6 Hz, 3H), 0.67 (t, *J* = 7.4 Hz, 3H).

### Example 301

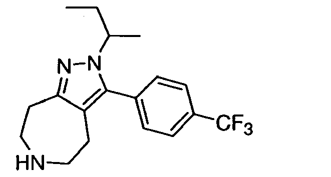

2-*sec*-Butyl-3-(4-trifluoromethyl-phenyl)-2,4,5,6,7,8-hexahydro-1,2,6-triaza-azulene.
The title compound (71 mg) was prepared as in Example 263 using 257 mg of the triflate from Example 298, Step A, and 175 mg of 4-trifluoromethylphenylboronic acid. MS (ESI): exact mass calculated for C₁₈H₂₂F₃N₃, 337.38; found, *m*/*z* 338.5 [M+H]⁺. ¹H NMR (500 MHz, CD₃OD): 7.91-7.85 (m, 2H), 7.61-7.54 (m, 2H), 4.11-4.03 (m, 1 H), 3.52-3.20 (m, 6H), 2.93-2.75 (m, 2H), 1.99-1.88 (m, 1 H), 1.75-1.65 (m, 1 H), 1.45 (d, J = 6.6 Hz, 3H), 0.65 (t, J= 7.4 Hz, 3H).

### Example 302

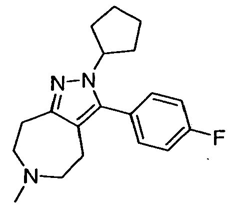

2-Cyclopentyl-3-(4-fluoro-phenyl)-6-methyl-2,4,5,6,7,8-hexahydro-1,2,6-triaza-azulene.
The title compound (186 mg) was prepared from 216 mg of the product of Example 182 according to Example 208. The product was dissolved in Et₂O and treated with excess 1.0 M HCl in Et₂O to afford the corresponding HCl salt. MS (ESI): exact mass calculated for C₁₉H₂₄FN₃, 313.41; found, *m*/*z* 314.4 [M+H]⁺. ¹H NMR (500 MHz, CD₃OD): 7.38-7.34 (m, 2H), 7.31-7.26 (m, 2H), 4.47 (m, 1 H), 3.75-3.69 (m, 1 H), 3.64-3.57 (m, 1 H), 3.33-3.15 (m, 4H), 3.02 (s, 3H), 2.91-2.83 (m, 1 H), 2.78-2.71 (m, 1 H), 2.04-1.84 (m, 6H), 1.65-1.54 (m, 2H).

### Example 303

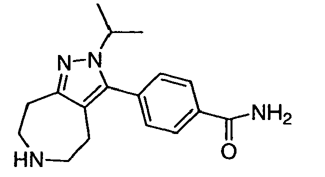

4-(2-lsopropyl-2,4,5,6,7,8-hexahydro-1,2,6-triaza-azulen-3-yl)-benzamide.
The title compound (26 mg) was prepared as in Example 263 using 206 mg of the triflate from Example 189, Step A, and 135 mg of 4-benzamide boronic acid. MS (ESI): exact mass calculated for C₁₇H₂₂N₄O, 298.38; found, *m*/*z* 299.5 [M+H]⁺. ¹H NMR (500 MHz, CDCl₃): 7.93-7.89 (m, 2H), 7.37-7.34 (m, 2H), 6.16 (br s, 1 H), 5.81 (br s, 1 H), 4.27 (m, 1 H), 3.05-3.00 (m, 2H), 2.97-2.88 (m, 4H), 2.51-2.46 (m, 2H), 1.41 (d, J = 6.6 Hz, 6H).

### Example 304

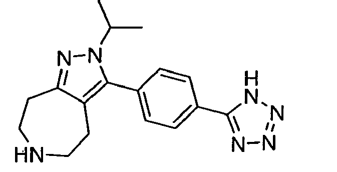

2-lsopropyl-3-[4-(1H-tetrazol-5-yl)-phenyl]-2,4,5,6,7,8-hexahydro-1,2,6-triaza-azulene.
Step A. 2-lsopropyl-3-[4-(1H-tetrazol-5-yl)-phenyl]-4,5,7,8-tetrahydro-2H-1,2,6-triaza-azulene-6-carboxylic acid *tert*-butyl ester. A toluene solution of 3-(4-cyano-phenyl)-2-isopropyl-4,5,7,8-tetrahydro-2H-1,2,6-triaza-azulene-6-carboxylic acid *tert*-butyl ester (intermediate in Example 212) and tributyltin azide was heated at reflux for 48 h. The mixture was concentrated and the residue was purified on SiO₂ (0 to 75% EtOAc/hexanes) to afford 89 mg of desired tetrazole as a glass.
Step B. The product from Step A was dissolved in dioxane and treated with HCI (4 M in dioxane, 1 mL) and mixture was stirred at RT. After 48 h, the liqiud was decanted and the solids washed with dioxane and dried under vacuum to afford 60 mg of the title compound. MS (ESI): exact mass calculated for C₁₇H₂₁N₇, 323.40; found, *m*/*z* 324.4 [M+H]⁺. ¹H NMR (500 MHz, CD₃OD): 8.24-8.20 (m, 2H), 7.58-7.55 (m, 2H), 4.42 (m, 1H), 3.44-3.40 (m, 2H), 3.34-3.30 (m, 2H), 3.21-3.17 (m, 2H), 2.84-2.80 (m, 2H), 1.42 (d, *J* = 6.8, 6H).

### Example 305

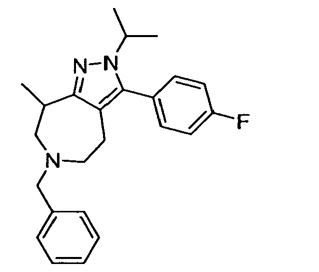

6-Benzyl-3-(4-fluoro-phenyl)-2-isopropyl-8-methyl-2,4,5,6,7,8-hexahydro-1,2,6-triaza-azulene.
Step A. Trifluoro-methanesulfonic acid 6-benzyl-2-isopropyl-8-methyl-2,4,5,6,7,8-hexahydro-1,2,6-triaza-azulen-3-yl ester. The desired triflate was prepared as in Step A of Example 189 using 1-benzyl-6-methyl-5-oxo-azepane-4-carboxylic acid ethyl ester (made from 1-benzyl-3-methyl-piperidin-4-one as shown in Step A of Example 59) in place of 5-oxo-azepane-1,4-dicarboxylic acid *tert*-butyl ester 4-ethyl ester.
Step B. The title compound (29 mg) was prepared as in Example 287, Step B, from 151 mg of the triflate from Step A and 110 mg of 4-fluorophenylboronic acid. MS (ESI): exact mass calculated for C₂₄H₂₈FN₃, 377.50; found, *m*/*z* 378.5 [M+H]⁺. ¹H NMR (500 MHz, CDCl₃): 7.41-7.37 (m, 2H), 7.33-7.29 (m, 2H), 7.27-7.18 (m, 3H), 7.16-7.10 (m, 2H), 4.24 (m, 1H), 3.78 (d, J= 13.4 Hz, 1 H), 3.70 (d, J = 13.4 Hz, 1 H), 3.19-3.12 (m, 1 H), 2.78-2.68 (m, 3H), 2.55-2.43 (m, 3H), 1.40 (d, J = 6.6, 3H), 1.37 (d, J = 6.6, 3H), 1.33 (d, J = 7.1, 3H).

### Example 306

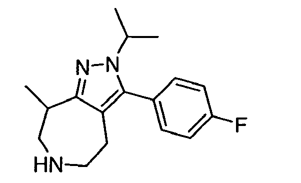

3-(4-Fluoro-phenyl)-2-isopropyl-8-methyl-2,4,5,6,7,8-hexahydro-1,2,6-triaza-azulene.
Step A. 3-Methyl-4-oxo-piperidine-1-carboxylic acid *tert*-butyl ester. To a -78 °C solution of of 4-oxo-piperidine-1-carboxylic acid *tert*-butyl ester in THF (100 mL) was added LDA (50 mL, 1.8 M in THF) with stirring over 1 h. Methyl iodide was then added (5 mL) and the mixture was allowed to warm slowly to RT and was stirred for 24 h. The reaction was quenched by the addition of satd. aq. NH₄Cl (20 mL). The mixture was poured into H₂O (800 mL), extracted with EtOAc, and concentrated. Purification on SiO₂ (120 g, 0 to 10% EtOAc/hexanes) gave 3.83 g of the desired product as an off-white solid. ¹H NMR (500 MHz, CDCl₃): 4.23-4.14 (m, 2H), 3.31-3.21 (m, 1 H), 2.61-2.37 (m, 4H), 1.50 (s, 9H), 1.05 (d, J = 6.6 Hz, 3H).
Step B. 2-Isopropyl-8-methyl-3-trifluoromethanesulfonyloxy-4,5,7,8-tetrahydro-2H-1,2,6-triaza-azulene-6-carboxylic acid *tert*-butyl ester. The product from Step A (2.01 g) was treated with ethyl diazoacetate (1.5 mL) as in Step A of Example 59. The resulting material (2.90 g) was then transformed to the desired triflate (2.68g) as shown in Step A of Example 189. The reaction sequence also produced 0.60 g of 2-isopropyl-4-methyl-3-trifluoromethanesulfonyloxy-4,5,7,8-tetrahydro-2H-1,2,6-triaza-azulene-6-carboxylic acid *tert*-butyl ester.
Step C. The title compound (1.62 g) was prepared as in Step A of Example 263 from 2.68 g of the triflate of Step B and 1.36 g of 4-fluorophenylboronic acid. The coupling product was treated with TFA (20 mL) in 50 mL of CH₂Cl₂ for 16 h. The mixture was concentrated and the residue was diluted with 1 M NaOH (50 mL) and extracted with CH₂Cl₂ (50 mL, 3x). The combined organic layers were dried over Na₂SO₄ and concentrated to provide the desired material. MS (ESI): exact mass calculated for C₁₇H₂₂FN₃, 287.18; found, *m*/*z* 288.4 [M+H]⁺. ¹H NMR (500 MHz, CDCl₃): 7.18-7.05 (m, 4H), 4.16 (m, 1H), 3.08-2.97 (m, 2H), 2.96-2.83 (m, 2H), 2.78-2.71 (m, 1 H), 2.49-2.31 (m, 2H), 1.34 (d, J = 6.6 Hz, 3H), 1.31 (d, J = 6.6 Hz, 3H), 1.29 (d, J = 7.3 Hz, 3H).

### Example 307

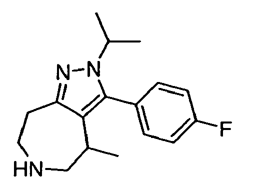

3-(4-Fluoro-phenyl)-2-isopropyl-4-methyl-2,4,5,6,7,8-hexahydro-1,2,6-triaza-azulene.
The title compound (154 mg) was prepared as in Example 177, Steps C and D, from 0.60 g of the triflate of Example 306, Step B, and 0.57 g of 4-fluorophenylboronic acid. MS (ESI): exact mass calculated for C₁₇H₂₂FN₃, 287.18; found, *m*/*z* 288.4 [M+H]⁺. ¹H NMR (500 MHz, CDCl₃): 7.25-7.20 (m, 2H), 7.17-7.12 (m, 2H), 4.15 (m, 1 H), 3.35-3.30 (m, 1 H), 3.08-3.03 (m, 1 H), 3.00-2.85 (m, 3H), 2.77-2.71 (m, 1 H), 2.57-2.51 (m, 1 H), 1.39 (d, J = 6.6 Hz, 3H), 1.36 (d, J = 6.6 Hz, 3H), 1.15 (d, J = 7.3 Hz, 3H).

### Example 308

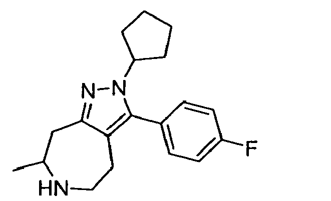

2-Cyclopentyl-3-(4-fluoro-phenyl)-7-methyl-2,4,5,6,7,8-hexahydro-1,2,6-triaza-azulene.
Step A. 2-Methyl-4-oxo-piperidine-1-carboxylic acid *tert*-butyl ester. A solution of 1,4-dioxa-8-aza-spiro[4.5]decane-8-carboxylic acid *tert*-butyl ester (2.97 g) in TMEDA (2.2 mL) was cooled to -78°C and *sec*-BuLi (1.8 M in THF, 13 mL) was added dropwise. The resulting yellow solution was aged at -78°C for 1 h. Methyl iodide (1.5 mL) was added and the mixture was warmed from -78°C to RT over 16 h. The reaction mixture was poured into water (800 mL) and extracted with EtOAc. The combined organic extracts were washed with H₂O, brine, and dried over Na₂SO₄. Purification on SiO₂ (330 g, 5 to 20% EtOAc/hexanes) provided 1.65 g of 7-methyl-1,4-dioxa-8-aza-spiro[4.5]decane-8-carboxylic acid *tert*-butyl ester. Multiple aliquots of this ester were combined (2.29 g), treated with 5 mL of conc. HCl in 10 mL of dioxane, and heated at 65 °C for 6 h. The solvent was removed and the residue was dissolved in CH₂Cl₂ and treated with di-*tert*-butyldicarbonate (1.0 g). After 5 d, the mixture was diluted with satd. aq. NaHCO₃ and H₂O, and extracted with CH₂Cl₂. Purification on SiO₂ (120 g, 5 to 15% EtOAc/hexanes) provided 1.40 g of the desired product as a white solid. ¹H NMR (500 MHz, CDCl₃): 4.69-4.59 (m, 1 H), 4.21-4.12 (m, 1 H), 3.30-3.20 (m, 1 H), 2.66-2.57 (m, 1 H), 2.47-2.36 (m, 1 H), 2.32-2.23 (m, 1 H), 2.22-2.15 (m, 1 H), 1.42 (s, 9H), 1.11 (d, J = 7.1 Hz, 3H).
Step B. 2-Cyclopentyl-7-methyl-3-trifluoromethanesulfonyloxy-4,5,7,8-tetrahydro-2H-1,2,6-triaza-azulene-6-carboxylic acid *tert*-butyl ester. The product from Step A (1.40 g) was treated with ethyl diazoacetate as in Step A of Example 59. The resulting material (1.0 g) was transformed to the desired triflate (0.78 g) as outlined in Step A of Example 180. The reaction sequence also produced 2-cyclopentyl-5-methyl-3-trifluoromethanesulfonyloxy-4,5,7,8-tetrahydro-2H-1,2,6-triaza-azulene-6-carboxylic acid *tert*-butyl ester.
Step C. The title compound (160.4 mg) was prepared as in Example 263 from 301 mg of the triflate from Step B and 185 mg of 4-fluorophenylboronic acid. The sequence also produced 2-cyclopentyl-3-(4-fluoro-phenyl)-5-methyl-2,4,5,6,7,8-hexahydro-1,2,6-triaza-azulene. The isomers were separated by SFC chromotagraphy. MS (ESI): exact mass calculated for C₁₉H₂₄FN₃, 313.41; found, *m*/*z* 314.4 [M+H]⁺. ¹H NMR (500 MHz, CD₃OD): 7.25-7.21 (m, 2H), 7.18-7.12 (m, 2H), 4.22 (m, 1 H), 3.24-3.18 (m, 1 H), 3.03-2.92 (m, 2H), 2.76-2.67 (m, 2H), 2.60-2.52 (m, 1H), 2.45-2.39 (m, 1H), 2.16-1.82 (m 6H), 1.59-1.47 (m, 2H), 1.25 (d, J = 6.3 Hz, 3H).

### Example 309

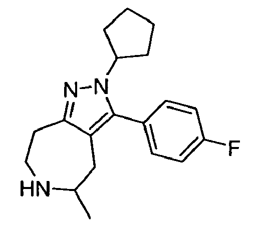

2-Cyclopentyl-3-(4-fluoro-phenyl)-5-methyl-2,4,5,6,7,8-hexahydro-1,2,6-triaza-azulene.
The title compound (3.8 mg) was prepared as outlined in Example 308. MS (ESI): exact mass calculated for C₁₉H₂₄FN₃, 313.41; found, *m*/*z* 314.4 [M+H]⁺. ¹H NMR (500 MHz, CDCl₃): 7.24-7.19 (m, 2H), 7.18-7.13 (m, 2H), 4.31 (m, 1 H), 3.42-3.35 (m, 1 H), 3.09-2.90 (m, 4H), 2.57-2.45 (m, 2H), 2.14-1.80 (m 6H), 1.58-1.48 (m, 2H), 1.22 (d, J = 6.3 Hz, 3H).

### Example 310

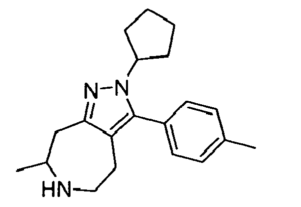

2-Cyclopentyl-7-methyl-3-p-tolyl-2,4,5,6,7,8-hexahydro-1,2,6-triaza-azulene.
The title compound (64 mg) was prepared from 193 mg of the triflate from Example 308, Step B, and 117 mg of 4-methylphenylboronic acid. MS (ESI): exact mass calculated for C₂₀H₂₇N₃, 309.45; found, *m*/*z* 310.4 [M+H]⁺. ¹H NMR (500 MHz, CDCl₃): 7.28-7.25 (m, 2H), 7.17-7.13 (m, 2H), 4.39 (m, 1 H), 3.21-3.16 (m, 1H), 3.03-2.92 (m, 2H), 2.74-2.67 (m, 2H), 2.59-2.52 (m, 1 H), 2.49-2.43 (m, 1H), 2.40 (s, 3H), 2.17-1.82 (m, 6H), 1.59-1.47 (m, 2H), 1.24 (d, J = 6.3 Hz, 3H).

### Example 311

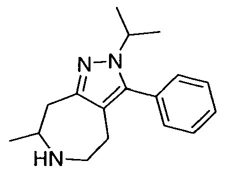

2-Isopropyl-7-methyl-3-phenyl-2,4,5,6,7,8-hexahydro-1,2,6-triaza-azulene.
The title compound (102 mg) was prepared as in Example 263 using 260 mg of 2-isopropyl-7-methyl-3-trifluoromethanesulfonyloxy-4,5,7,8-tetrahydro-2H-1,2,6-triaza-azulene-6-carboxylic acid *tert*-butyl ester (as outlined in Example 308 replacing cyclopentyl hydrazine with isopropyl hydrazine) and 101 mg of phenylboronic acid. The reaction sequence also yielded 2-isopropyl-5-methyl-3-phenyl-2,4,5,6,7,8-hexahydro-1,2,6-triaza-azulene. MS (ESI): exact mass calculated for C₁₇H₂₃N₃, 269.19; found, *m*/*z* 270.5 [M+H]⁺. ¹H NMR (600 MHz, CD₃OD): 7.58-7.52 (m, 3H), 7.36-7.35 (m, 2H), 4.43 (m, 1H), 3.65-3.57 (m, 1 H), 3.51-3.48 (m, 1 H), 3.23-3.11 (m, 3H), 2.87-2.82 (m, 2H), 2.76-2.73 (m, 1 H), 1.48 (d, J = 6.4 Hz, 3H), 1.43-1.40 (m, 6H).

### Example 312

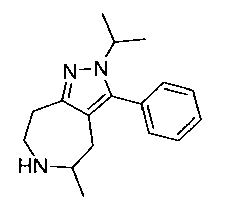

2-Isopropyl-5-methyl-3-phenyl-2,4,5,6,7,8-hexahydro-1,2,6-triaza-azulene.

The title compound (28 mg) was prepared as in Example 311 and purified by SFC chromatography. MS (ESI): exact mass calculated for C₁₇H₂₃N₃, 269.19; found, *m*/*z* 270.4 [M+H]⁺. ¹H NMR (600 MHz, CD₃OD): 7.58-7.52 (m, 3H), 7.35-7.33 (m, 2H), 4.40 (m, 1 H), 3.63-3.59 (m, 1 H), 3.5-3.47 (m, 1 H), 3.31-3.19 (m, 3H), 2.80-2.68 (m, 2H), 1.43-1.39 (m, 6H), 1.34 (d, J = 6.6 Hz, 3H).

### Example 313

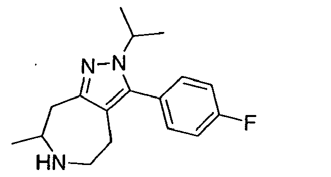

3-(4-Fluoro-phenyl)-2-isopropyl-7-methyl-2,4,5,6,7,8-hexahydro-1,2,6-triaza-azulene.
The title compound (127 mg) was prepared as in Example 311 using 260 mg of 2-isopropyl-7-methyl-3-trifluoromethanesulfonyloxy-4,5,7,8-tetrahydro-2H-1,2,6-triaza-azulene-6-carboxylic acid *tert*-butyl ester and 115 of 4-fluorophenylboronic acid. The reaction sequence also yielded 3-(4-fluorophenyl)-2-isopropyl-5-methyl-2,4,5,6,7,8-hexahydro-1,2,6-triaza-azulene. MS (ESI): exact mass calculated for C₁₇H₂₂FN₃, 287.18; found, *m*/*z* 288.5 [M+H]⁺. ¹H NMR (600 MHz, CD₃OD): 7.39-7.37 (m, 2H), 7.32-7.28 (m, 2H), 4.36 (m, 1 H), 3.61-3.56 (m, 1 H), 3.50-3.47 (m, 1 H), 3.20-3.08 (m, 3H), 2.85-2.80 (m, 1 H), 2.73-2.69 (m, 1 H), 1.46 (d, J = 6.6 Hz, 3H), 1.41-1.38 (m, 6H).

### Example 314

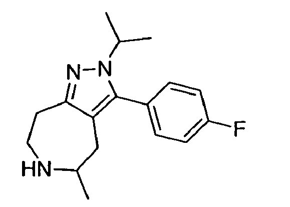

3-(4-Fluoro-phenyl)-2-isopropyl-5-methyl-2,4,5,6,7,8-hexahydro-1,2,6-triaza-azulene.
The title compound (36 mg) was prepared as in Example 311 and purified by chromatography on SFC. MS (ESI): exact mass calculated for C₁₇H₂₂FN₃, 287.18; found, *m*/*z* 288.5 [M+H]⁺. ¹H NMR (600 MHz, CD₃OD): 7.37-7.35 (m, 2H), 7.31-7.28 (m, 2H), 4.33 (m, 1 H), 3.61-3.58 (m, 1 H), 3.48-3.45 (m, 1 H), 3.27-3.13 (m, 3H), 2.77-2.65 (m, 2H), 1.41-1.37 (m, 6H), 1.34 (d, J= 6.6 Hz, 3H).

### Example 315

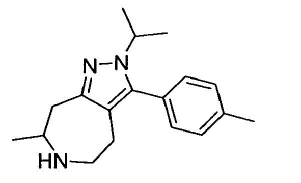

2-Isopropyl-7-methyl-3-p-tolyl-2,4,5,6,7,8-hexahydro-1,2,6-triaza-azulene.
The title compound (127 mg) was prepared as in Example 311 using 260 mg of 2-isopropyl-7-methyl-3-trifluoromethanesulfonyloxy-4,5,7,8-tetrahydro-2H-1,2,6-triaza-azulene-6-carboxylic acid *tert*-butyl ester and 112 mg of 4-methylphenylboronic acid. The reaction sequence also yielded 2-isopropyl-5-methyl-3-p-tolyl-2,4,5,6,7,8-hexahydro-1,2,6-triaza-azulene. MS (ESI): exact mass calculated for C₁₈H₂₅N₃, 283.20; found, *m*/*z* 284.5 [M+H]⁺. ¹H NMR (600 MHz, CD₃OD): 7.38-7.36 (m, 2H), 7.22-7.20 (m, 2H), 4.41 (m, 1 H), 3.60-3.56 (m, 1 H), 3.50-3.45 (m, 1 H), 3.21-3.06 (m, 3H), 2.84-2.70 (m, 2H), 1.46 (d, J = 6.6 Hz, 3H), 1.42-1.37 (m, 6H).

Examples 316 through 323 were prepared as described in Example 238, with adjustments as noted.

### Example 316

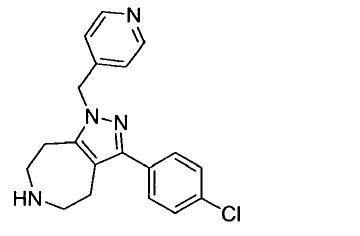

3-(4-Chloro-phenyl)-1-pyridin-4-ylmethyl-1,4,5,6,7,8-hexahydro-1,2,6-triaza-azulene.
The title compound (0.02 g) was prepared from 3-(4-chloro-phenyl)-4,5,7,8-tetrahydro-1 *H*-1,2,6-triaza-azulene-6-carboxylic acid *tert*-butyl ester (Example 103, Step B; 0.3 mmol) using 4-chloromethyl-pyridine hydrogen chloride (0.5 mmol) in place of 2-chloromethyl-thiophene. MS (ESI): exact mass calculated for C₁₉H₁₉ClN₄, 338.13; found, *m*/*z* 339.3 [M+H]⁺. ¹H NMR (500 MHz, CDCl₃): 8.49-8.48 (m, 2H), 7.43-7.41 (m, 2H), 7.33-7.31 (m, 2H), 6.90-6.89 (m, 2H), 5.28 (s, 2H), 2.92-2.87 (m, 2H), 2.75-2.73 (m, 1 H), 2.66-2.64 (m, 1H).

### Example 317

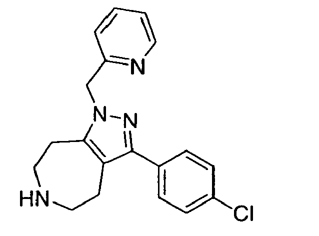

3-(4-Chloro-phenyl)-1-pyridin-2-ylmethyl-1,4,5,6,7,8-hexahydro-1,2,6-triaza-azulene.
The title compound (0.01 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.4 mmol) using 2-chloromethyl-pyridine hydrogen chloride (0.5 mmol) in place of 2-chloromethyl-thiophene. The reaction sequence also yielded 3-(4-chloro-phenyl)-2-pyridin-2-ylmethyl-4,5,7,8-tetrahydro-2*H*-1,2,6-triaza-azulene-6-carboxylic acid *tert*-butyl ester in the alkylation step. MS (ESI): exact mass calculated for C₁₉H₁₉ClN₄, 338.13; found, *m*/*z* 339.3 [M+H]⁺. ¹H NMR (500 MHz, CDCl₃): 8.49-8.48 (m, 1 H), 7.56-7.54 (m, 1 H), 7.44-7.42 (m, 2H), 7.32-7.30 (m, 2H), 7.19-7.11 (m, 1 H), 6.84-6.82 (m, 1 H), 5.39 (s, 2H), 2.93-2.87 (m, 4H), 2.76-2.74 (m, 4H).

### Example 318

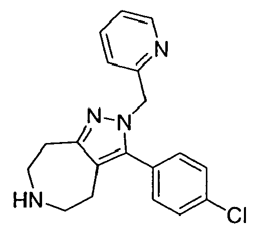

3-(4-Chloro-phenyl)-2-pyridin-2-ylmethyl-1,4,5,6,7,8-hexahydro-1,2,6-triaza-azulene.
The title compound (0.011 g) was prepared from 3-(4-chloro-phenyl)-2-pyridin-2-ylmethyl -4,5,7,8-tetrahydro-2*H*-1,2,6-triaza-azulene-6-carboxylic acid *tert-*butyl ester (Example 317) according to Example 103, Step C. MS (ESI): exact mass calculated for C₁₉H₁₉ClN₄, 338.13; found, *m*/*z* 339.3 [M+H]⁺. ¹H NMR (500 MHz, CDCl₃): 8.44-8.43 (m, 1 H), 7.56-7.55 (m, 1 H), 7.32-7.27 (m, 2H), 7.11-7.07 (m, 3H), 6.81-6.77 (m, 1 H), 5.20 (s, 2H), 2.98-2.96 (m, 2H), 2.89-2.86 (m, 4H), 2.48-2.46 (m, 2H).

### Example 319

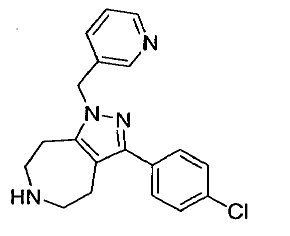

3-(4-Chloro-phenyl)-1-pyridin-3-ylmethyl-1,4,5,6,7,8-hexahydro-1,2,6-triaza-azulene.
The title compound was prepared from 3-(4-chloro-phenyl)-4,5,7,8-tetrahydro-1*H*-1,2,6-triaza-azulene-6-carboxylic acid *tert*-butyl ester (Example 103, Step B; 0.3 mmol) using 3-chloromethyl-pyridine hydrogen chloride (0.5 mmol) in place of 2-chloromethyl-thiophene. The title compound was obtained as a 2:1 mixture (25 mg) with 3-(4-chloro-phenyl)-2-pyridin-3-ylmethyl-2,4,5,6,7,8-hexahydro-1,2,6-triaza-azulene. Data for the mixture: MS (ESI): exact mass calculated for C₁₉H₁₉ClN₄, 338.13; found, *m*/*z* 339.4 [M+H]⁺. ¹H NMR (500 MHz, CDCl₃): 8.47-8.14 (m, 2H), 7.42-7.03 (m, 6H), 5.39-5.07 (two s, 2H), 2.97-2.84 (m, 2H), 2.72-2.43 (m, 2H).

### Example 320

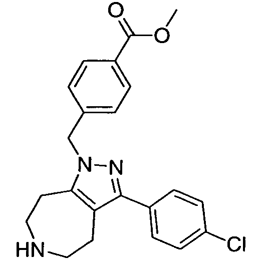

4-[3-(4-Chloro-phenyl)-5,6,7,8-tetrahydro-4H-1,2,6-triaza-azulen-1-ylmethyl]-benzoic acid methyl ester.
The title compound (0.03 g) was prepared from 3-(4-chloro-phenyl)-4,5,7,8-tetrahydro-1*H*-1,2,6-triaza-azulene-6-carboxylic acid *tert*-butyl ester (Example 103, Step B; 0.3 mmol) using 4-bromomethyl-benzoic acid methyl ester (0.5 mmol) in place of 2-chloromethyl-thiophene. MS (ESI): exact mass calculated for C₂₂H₂₂CIN₃O₂, 395.14; found, *m*/*z* 396.4 [M+H]⁺. ¹H NMR (500 MHz, CDCl₃): 7.63-7.19 (m, 7H), 7.03-7.02 (m, 2H), 5.27 (s, 2H), 3.15 (s, 2H), 2.79-2.67 (m, 8H).

### Example 321

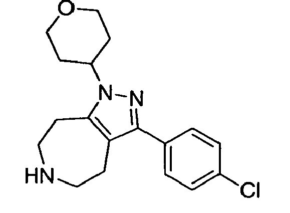

3-(4-Chloro-phenyl)-1-(tetrahydro-pyran-4-yl)-1,4,5,6,7,8-hexahydro-1,2,6-triaza-azulene.
The title compound was prepared from 3-(4-chloro-phenyl)-4,5,7,8-tetrahydro-1 *H*-1,2,6-triaza-azulene-6-carboxylic acid *tert*-butyl ester (Example 103, Step B; 0.40 mmol) using 4-chloro-tetrahydro-pyran (1.5 mmol) in place of chloro-cyclobutane. The title compound was obtained as a 2:1 mixture (10 mg) with 3-(4-chloro-phenyl)-2-(tetrahydro-pyran-4-yl)-1,4,5,6,7,8-hexahydro-1,2,6-triaza-azulene. Data for the mixture: MS (ESI): exact mass calculated for C₁₈H₂₂ClN₃O, 331.15; found, *m*/*z* 332.4 [M+H]⁺. ¹H NMR (500 MHz, CD₃OD): 7.44-7.42 (m, 1 H), 7.36-7.30 (m, 4H), 7.20-7.18 (m, 1 H), 4.36-4.33 (m, 1 H), 3.97-3.94 (m, 3H), 3.87-3.86 (m, 1 H), 3.51-3.49 (m, 3H), 3.28-3.25 (m, 1 H), 2.90-2.75 (m, 8H), 2.66-2.64 (m, 2H), 2.39-2.37 (m, 1 H), 2.19-2.10 (m, 3H), 1.74-1.71 (m, 2H), 1.65-1.62 (m, 1H).

### Example 322

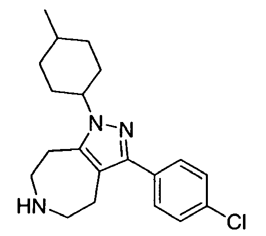

3-(4-Chloro-phenyl)-1-(4-methyl-cyclohexyl)-1,4,5,6,7,8-hexahydro-1,2,6-triaza-azulene.
The title compound (11 mg) was prepared from 3-(4-chloro-phenyl)-4,5,7,8-tetrahydro-1 *H*-1,2,6-triaza-azulene-6-carboxylic acid *tert*-butyl ester (Example 103, Step B; 0.30 mmol) using 1-bromo-4-methyl-cyclohexane (1.0 mmol) in place of chloro-cyclobutane. The reaction sequence also yielded 3-(4-chlorophenyl)-2-(4-methyl-cyclohexyl)-1,4,5,6,7,8-hexahydro-1,2,6-triaza-azulene-6-carboxylic acid *tert*-butyl ester in the alkylation step. MS (ESI): exact mass calculated for C₂₀H₂₆ClN₃, 343.18; found, *m*/*z 344.4* [M+H]⁺. ¹H NMR (500 MHz, CD₃OD): 7.40-7.34 (m, 4H), 4.10-4.06 (m, 1 H), 3.39-3.37 (m, 2H), 3.28-3.26 (m, 2H), 3.17-3.15 (m, 2H), 2.94-2.91 (m, 2H), 1.96-1.89 (m, 2H), 1.83-1.76 (m, 4H), 1.52-1.10 (m, 2H), 0.91-0.87 (m, 4H).

### Example 323

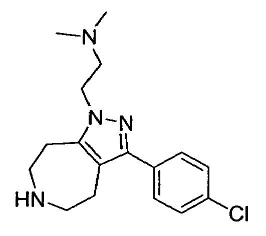

{2-[3-(4-Chloro-phenyl)-5,6,7,8-tetrahydro-4H-1,2,6-triaza-azulen-1-yl]-ethyl}-dimethyl-amine.
The title compound was prepared from 3-(4-chloro-phenyl)-4,5,7,8-tetrahydro-1 *H*-1,2,6-triaza-azulene-6-carboxylic acid *tert*-butyl ester (Example 103, Step B; 0.33 mmol) using (2-chloro-ethyl)-dimethyl-amine hydrogen chloride (0.66 mmol) in place of chloro-cyclobutane. The title compound was obtained as a 2:1 mixture (10 mg) with {2-[3-(4-chloro-phenyl)-5,6,7,8-tetrahydro-4H-1,2,6-triaza-azulen-2-yl]-ethyl}-dimethyl-amine. Data for the mixture: MS (ESI): exact mass calculated for C₁₇H₂₃ClN₄, 318.16; found, *m*/*z* 319.4 [M+H]⁺. ¹H NMR (500 MHz, CD₃OD): 7.50-7.45 (m, 2H), 7.37-7.33 (m, 2H), 4.51-4.49 (m, 1.3H), 4.27-4.26 (m, 0.7H), 3.63-3.61 (m, 1.3H), 3.48-3.42 (m, 2H), 3.33-3.29 (m, 2H), 3.24-3.23 (m, 2H), 3.14-3.12 (m, 0.7H), 3.00-2.98 (m, 1.3H), 2.91 (s, 4H), 2.84 (s, 2H), 2.75-2.73 (m, 0.7H).

### Example 324

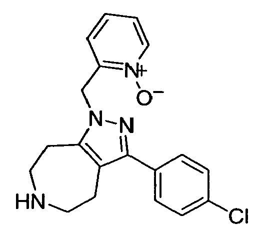

3-(4-Chloro-phenyl)-1-(1-oxy-pyridin-2-ylmethyl)-1,4,5,6,7,8-hexahydro-1,2,6-triaza-azulene.
A mixture of 3-(4-chloro-phenyl)-2-pyridin-2-ylmethyl-1,4,5,6,7,8-hexahydro-1,2,6-triaza-azulene-6-carboxylic acid *tert*-butyl ester (Example 317; 0.1 mmol) and mCPBA (0.1 g) in dichloroethane (10 mL) was heated at 80 °C for 1 h. Satd. aq. NaHCO₃ (20 mL) was added, and the layers were separated. The organic layer was concentrated, and the residue was diluted with MeOH (5 mL). Hydrogen chloride (1 M, 2 mL) was added and the mixture was stirred at 25 °C for 16 h. After concentration, purification by flash chromatography (2 M NH₃/MeOH in CH₂Cl₂) provided the desired compound (34 mg). MS (ESI): exact mass calculated for C₁₉H₁₉ClN₄O, 354.12; found, *m*/*z* 355.2 [M+H]⁺. ¹H NMR (500 MHz, CDCl₃): 8.20-8.19 (m, 1 H), 7.41-7.39 (m, 2H), 7.33-7.31 (m, 2H), 7.18-7.15 (m, 2H), 6.69-6.67 (m, 1 H), 5.50 (s, 2H), 3.10-3.03 (m, 2H), 2.93-2.86 (m, 2H).

### Example 325

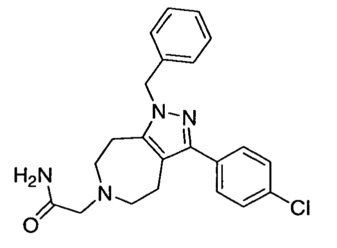

2-[1-Benzyl-3-(4-chloro-phenyl)-4,5,7,8-tetrahydro-1H-1,2,6-triaza-azulen-6-yl]-acetamide.
A mixture of 1-benzyl-3-(4-chloro-phenyl)-1-pyridin-3-ylmethyl-1,4,5,6,7,8-hexahydro-1,2,6-triaza-azulene (Example 59, Step E; 0.05 mmol), 2-bromo-acetamide (8 mg), and Na₂CO₃ (15 mg) in acetone (2 mL) was stirred at 25 °C for 16 h. After concentration, purification by flash chromatography (2 M NH₃/MeOH in CH₂Cl₂) provided the desired compound (6 mg). MS (ESI): exact mass calculated for C₂₂H₂₃ClN₄O, 394.16; found, *m*/*z* 395.3 [M+H]⁺. ¹H NMR (500 MHz, CDCl₃): 8.49-8.48 (m, 1 H), 7.56-7.54 (m, 1 H), 7.44-7.42 (m, 2H), 7.32-7.30 (m, 2H), 7.19-7.11 (m, 1 H), 6.84-6.82 (m, 1 H), 5.39 (s, 2H), 2.93-2.87 (m, 2H), 2.76-2.74 (m, 2H).

### Example 326

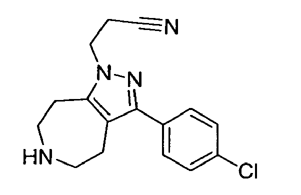

3-[3-(4-Chloro-phenyl)-5,6,7,8-tetrahydro-4H-1,2,6-triaza-azulen-1-yl]-propionitrile.
To a mixture of 3-(4-chloro-phenyl)-4,5,7,8-tetrahydro-1*H*-1,2,6-triaza-azulene-6-carboxylic acid *tert*-butyl ester (Example 103, Step B; 0.05 mmol), NaOH (50% aq., 0.2 mL), and Bu₄NHSO₄ (0.005 mmol) in dichloroethane (5 mL) was added 3-bromo-propionitrile (0.1 mmol). The mixture was stirred at 25 °C for 16 h and then was heated at 80 °C for 1 h. After concentration, purification by flash chromatography (EtOAc/hexanes) provided 3-[3-(4-chloro-phenyl)-5,6,7,8-tetrahydro-4H-1,2,6-triaza-azuten-1-yl]-propionitrite-6-carboxylic acid *tert*-butyl ester. Deprotection of this ester according to the deprotection method in Example 103, Step C, gave the title compound (9 mg). The reaction sequence also yielded 3-[3-(4-chloro-phenyl)-5,6,7,8-tetrahydro-4H-1,2,6-triaza-azulen-2-yl]-propionitrile-6-carboxylic acid *tert*-butyl ester in the alkylation step. MS (ESI): exact mass calculated for C₁₆H₁₇ClN₄, 300.11; found, *m*/*z* 301.4 [M+H]⁺. ¹H NMR (500 MHz, CD₃OD): 7.44-7.37 (m, 4H), 4.39-4.37 (t, *J* = 6.1 Hz, 2H), 3.41-3.39 (m, 2H), 3.29-3.27 (m, 2H), 3.24-3.22 (m, 2H), 2.99-2.96 (m, 2H), 2.95-2.92 (t, *J* = 6.1 Hz, 2H).

### Example 327

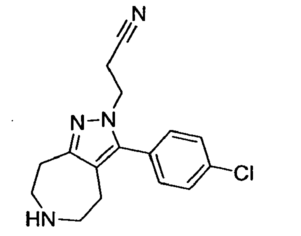

3-[3-(4-Chloro-phenyl)-5,6,7,8-tetrahydro-4H-1,2,6-triaza-azulen-2-yl]-propionitrile.
The title compound (0.004 g) was prepared from 3-[3-(4-chloro-phenyl)-5,6,7,8-tetrahydro-4H-1,2,6-triaza-azulen-2-yl]-propionitrile-6-carboxylic acid *tert*-butyl ester (Example 326) according to the deprotection method in Example 103, Step C. MS (ESI): exact mass calculated for C₁₆H₁₇ClN₄, 300.11; found, *m*/*z* 301.4 [M+H]⁺. ¹H NMR (500 MHz, CD₃OD): 7.50-7.48 (m, 2H), 7.31-7.30 (m, 2H), 4.14-4.11 (t, J = 6.1 Hz, 2H), 3.32-3.31 (m, 2H), 3.23-3.21 (m, 2H), 3.09-3.07 (m, 2H), 2.86-2.84 (t, J = 6.1 Hz, 2H), 2.72-2.70 (m, 2H).

### Example 328

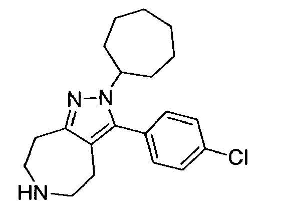

3-(4-Chloro-phenyl)-2-cycloheptyl-2,4,5,6,7,8-hexahydro-1,2,6-triaza-azulene.
The title compound (0.010 g) was prepared from 3-(4-chloro-phenyl)-2-cycloheptyl-2,4,5,6,7,8-hexahydro-1,2,6-triaza-azulene-6-carboxylic acid *tert-*butyl ester (Example 254, Step C) according to the deprotection method in Example 103, Step C. MS (ESI): exact mass calculated for C₂₀H₂₆CIN₃, 343.18; found, *m*/*z* 344.5 [M+H]⁺. ¹H NMR (500 MHz, CD₃OD): 7.60-7.58 (m, 2H), 7.33-7.31 (m, 2H), 4.10-4.06 (m, 1H), 3.41-3.39 (m, 2H), 3.31-3.29 (m, 2H), 3.18-3.16 (m, 2H), 2.78-2.75 (m, 2H), 2.10-2.05 (m, 2H), 1.91-1.87 (m, 2H), 1.80-1.76 (m, 2H), 1.60-1.58 (m, 4H), 1.40-1.39 (m, 2H).

### Example 329

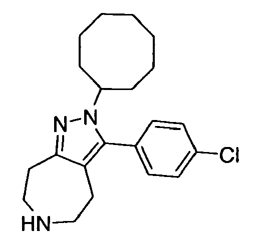

3-(4-Chloro-phenyl)-2-cyclooctyl-2,4,5,6,7,8-hexahydro-1,2,6-triaza-azulene.
The title compound (0.017 g) was prepared from 3-(4-chloro-phenyl)-2-cyclooctyl -2,4,5,6,7,8-hexahydro-1,2,6-triaza-azulene-6-carboxylic acid *tert-*butyl ester (Example 255, Step C) according to the deprotection method in Example 103, Step C. MS (ESI): exact mass calculated for C₂₁H₂₈ClN₃, 357.20; found, *m*/*z* 358.5 [M+H]⁺. ¹H NMR (500 MHz, CD₃OD): 7.61-7.58 (m, 2H), 7.34-7.32 (m, 2H), 4.24-4.21 (m, 1 H), 3.41-3.39 (m, 2H), 3.31-3.29 (m, 2H), 3.18-3.16 (m, 2H), 2.78-2.76 (m, 2H), 2.15-2.11 (m, 2H), 1.81-1.77 (m, 4H), 1.57-1.46 (m, 6H), 1.31-1.29 (m, 2H).

### Example 330

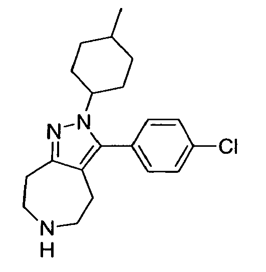

3-(4-Chloro-phenyl)-2-(4-methyl-cyclohexyl)-2,4,5,6,7,8-hexahydro-1,2,6-triaza-azulene.
The title compound (0.007 g) was prepared from 3-(4-chloro-phenyl)-2-(4-methyl-cyclohexyl)-2,4,5,6,7,8-hexahydro-1,2,6-triaza-azulene-6-carboxylic acid *tert*-butyl ester (Example 322, Step C) according to the deprotection method in Example 103, Step C. MS (ESI): exact mass calculated for C₂₀H₂₆ClN₃, 343.18; found, *m*/*z* 344.4 [M+H]⁺. ¹H NMR (500 MHz, CD₃OD): 7.59-7.57 (m, 2H), 7.33-7.31 (m, 2H), 3.95-3.92 (m, 1 H), 3.37-3.35 (m, 2H), 3.31-3.29 (m, 2H), 3.18-3.16 (m, 2H), 2.78-2.75 (m, 2H), 2.10-1.95 (m, 2H), 1.90-1.77 (m, 4H), 1.05-0.91 (m, 6H),

### Example 331

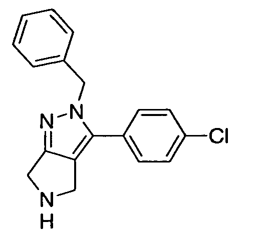

2-Benzyl-3-(4-chloro-phenyl)-2,4,5,6-tetrahydro-pyrrolo[3,4-c]pyrazole.
To a solution of LDA (1.80 M in THF, 20 mmol) in THF (100 mL) at -78 °C, was added a solution of 3-oxo-pyrrolidine-1-carboxylic acid *tert*-butyl ester (10 mmol) in THF (10 mL) dropwise. After 20 min, a solution of 4-chlorobenzyl chloride (15 mmol) in THF (10 mL) was added. Then the mixture was warmed to 25 °C and stirred for 16 h. Satd. aq. NaHCO₃ (100 mL) was added, and the organic layer was separated and concentrated to give 3-(4-chloro-benzyl)-4-oxo-pyrrolidine-1-carboxylic acid *tert*-butyl ester. This residue (1/8 portion, approx. 1.25 mmol) was diluted with EtOH (10 mL) and treated with benzyl hydrazine hydrogen chloride (1.5 mmol) and K₂CO₃ (5 mmol). The mixture was stirred at 25 °C for 16 h. Concentration and purification by flash chromatography (EtOAc/CH₂Cl₂) provided 2-benzyl-3-(4-chloro-phenyl)-2,6-dihydro-4H-pyrrolo[3,4-c]pyrazole-5-carboxylic acid *tert*-butyl ester. A solution of the ester and TFA (2 mL) in CH₂Cl₂ (10 mL) was stirred at 25 °C for 4 h. Concentration and purification by flash chromatography (2 M NH₃ in MeOH/CH₂Cl₂) provided the desired compound (10 mg). MS (ESI): exact mass calculated for C₁₈H₁₆CIN₃, 309.10; found, *m*/*z* 310.4 [M+H]⁺. ¹H NMR (500 MHz, CDCl₃): 7.37-7.21 (m, 7H), 7.08-7.05 (m, 2H), 5.29 (s, 2H), 4.09 (s, 2H), 4.04 (s, 2H).

### Example 332

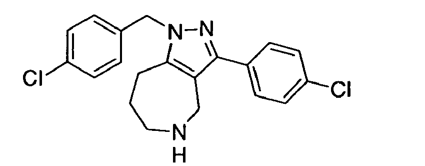

1-(4-Chloro-benzyl)-3-(4-chloro-phenyl)-1,4,5,6,7,8-hexahydro-1,2,5-triaza-azulene.
The title compound (0.017 g), as a hydrochloride salt, was prepared from 3-(4-chloro-phenyl)-4,6,7,8-tetrahydro-1 *H*-1,2,5-triaza-azulene-5-carboxylic acid *tert-*butyl ester (Example 59, Step C; 0.15 g) using 4-chlorobenzyl bromide (0.1 g) in place of benzyl chloride in Example 59, Step D. MS (ESI): exact mass calculated for C₂₀H₁₉Cl₂N₃, 371.10; found, *m*/*z* 372.1 [M+H]⁺. ¹H NMR (500 MHz, CD₃OD): 7.57-7.50 (m, 4H), 7.41-7.33 (m, 2H), 7.27-7.19 (m, 2H), 5.45 (s, 2H), 4.34 (s, 2H), 3.57-3.53 (m, 2H), 3.08-3.03 (m, 2H), 2.08-2.02 (m, 2H).

### Example 333

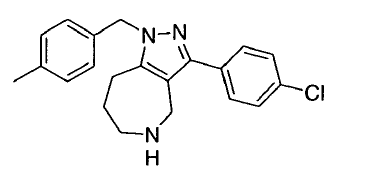

3-(4-Chloro-phenyl)-1-(4-methyl-benzyl)-1,4,5,6,7,8-hexahydro-1,2,5-triaza-azulene.
The title compound (0.011 g), as a hydrochloride salt, was prepared from 3-(4-chloro-phenyl)-4,6,7,8-tetrahydro-1*H*-1,2,5-triaza-azulene-5-carboxylic acid *tert-*butyl ester (Example 59, Step C; 0.15 g) using 4-methylbenzyl bromide (0.09 g) in place of benzyl chloride in Example 59, Step D. MS (ESI): exact mass calculated for C₂₁H₂₂CIN₃, 351.15; found, *m*/*z* 352.2 [M+H]⁺. ¹H NMR (500 MHz, CD₃OD): 7.46-7.39 (m, 2H), 7.21-7.18 (m, 2H), 6.98-6.95 (m, 2H), 6.77-6.74 (m, 2H), 5.08 (s, 2H), 3.97 (s, 2H), 3.46-3.43 (m, 2H), 2.96-2.92 (m, 2H), 2.17 (s, 3H), 2.01-1.97 (m, 2H).

### Example 334

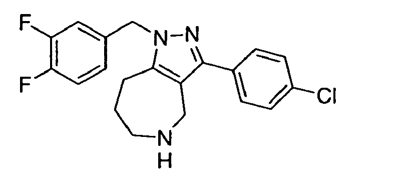

3-(4-Chloro-phenyl)-1-(3,4-difluoro-benzyl)-1,4,5,6,7,8-hexahydro-1,2,5-triaza-azulene.
The title compound (0.005 g) was prepared from 3-(4-chloro-phenyl)-4,6,7,8-tetrahydro-1*H*-1,2,5-triaza-azulene-5-carboxylic acid *tert*-butyl ester (Example 59, Step C; 0.07 g) using 3,4-difluorobenzyl bromide (0.06 g) in place of benzyl chloride in Example 59, Step D. MS (ESI): exact mass calculated for C₂₀H₁₈ClF₂N₃, 373.12; found, *m*/*z*374.1 [M+H]⁺. ¹H NMR (500 MHz, CD₃OD): 7.39-7.34 (m, 4H), 7.16-7.10 (m, 1 H), 6.98-6.97 (m, 1 H), 6.89-6.88 (m, 1 H), 5.27 (s, 2H), 3.81 (s, 2H), 3.09-3.06 (m, 2H), 2.80-2.76 (m, 2H), 1.75-1.70 (m, 2H).

### Example 335

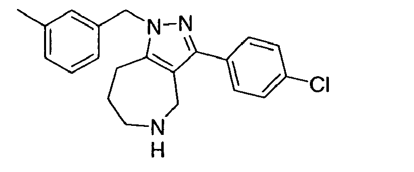

3-(4-Chloro-phenyl)-1-(3-methyl-benzyl)-1,4,5,6,7,8-hexahydro-1,2,5-triaza-azulene.
The title compound (0.012 g) was prepared from 3-(4-chloro-phenyl)-4,6,7,8-tetrahydro-1 *H*-1,2,5-triaza-azulene-5-carboxylic acid *tert*-butyl ester (Example 59, Step C; 0.1 g) using 3-methylbenzyl bromide (0.06 g) in place of benzyl chloride in Example 59, Step D. MS (ESI): exact mass calculated for C₂₁H₂₂ClN₃, 351.15; found, *m*/*z* 352.2 [M+H]⁺. ¹H NMR (500 MHz, CD₃OD): 7.49-7.43 (m, 4H), 7.19 (t, J = 7.6 Hz, 1 H), 7.08 (d, J = 7.6 Hz, 1 H), 7.00 (s, 1 H), 6.92 (d, *J* = 7.3 Hz, 1H), 5.34 (s, 2H), 3.88 (s, 2H), 3.16-3.13 (m, 2H), 2.88-2.85 (m, 2H), 2.30 (s, 3H), 1.80-1.77 (m, 2H).

### Example 336

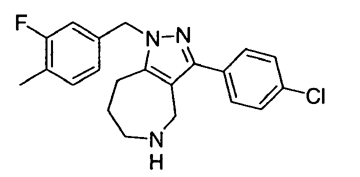

3-(4-Chloro-phenyl)-1-(3-fluoro-4-methyl-benzyl)-1,4,5,6,7,8-hexahydro-1,2,5-triaza-azulene.
The title compound (0.002 g) was prepared from 3-(4-chloro-phenyl)-4,6,7,8-tetrahydro-1*H*-1,2,5-triaza-azulene-5-carboxylic acid *tert*-butyl ester (Example 59, Step C; 0.1 g) using 3-fluoro-4-methylbenzyl bromide (0.09 g) in place of benzyl chloride in Example 59, Step D. MS (ESI): exact mass calculated for C₂₁H₂₁ClFN₃, 369.14; found, *m*/*z* 370.1 [M+H]⁺. ¹H NMR (500 MHz, CD₃OD): 7.49-7.43 (m, 4H), 7.19 (t, J = 7.9 Hz, 1 H), 6.88-6.80 (m, 2H), 5.34 (s, 2H), 3.88 (s, 2H), 3.16-3.13 (m, 2H), 2.87-2.85 (m, 2H), 2.23 (d, J = 1.5 Hz, 3H), 1.81-1.78 (m, 2H).

### Example 337

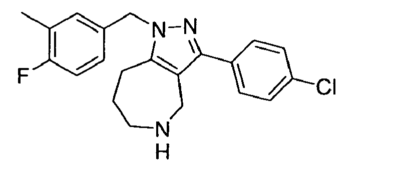

3-(4-Chloro-phenyl)-1-(4-fluoro-3-methyl-benzyl)-1,4,5,6,7,8-hexahydro-1,2,5-triaza-azulene.
The title compound (0.001 g) was prepared from 3-(4-chloro-phenyl)-4,6,7,8-tetrahydro-1*H*-1,2,5-triaza-azulene-5-carboxylic acid *tert*-butyl ester (Example 59, Step C; 0.1 g) using 4-fluoro-3-methylbenzyl bromide (0.09 g) in place of benzyl chloride in Example 59, Step D. MS (ESI): exact mass calculated for C₂₁H₂₁ClFN₃, 369.14; found, *m*/*z* 370.1 [M+H]⁺. ¹H NMR (500 MHz, CD₃OD): 7.48-7.43 (m, 4H), 7.08-7.06 (m, 1 H), 6.99-6.97 (m, 2H), 5.32 (s, 2H), 3.88 (s, 2H), 3.16-3.14 (m, 2H), 2.89-2.86 (m, 2H), 2.22 (d, J= 1.6 Hz, 3H), 1.80 (m, 2H).

### Example 338

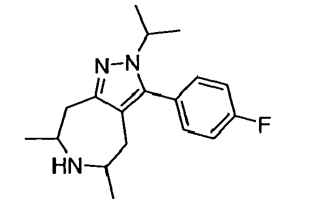

3-(4-Fluoro-phenyl)-2-isopropyl-5,7-dimethyl-2,4,5,6,7,8-hexahydro-1,2,6-triaza-azulene.
The title compound was prepared in a manner analogous to those described above.

### Assay Methods

### In vitro pharmacology

### 1. Affinity for 5-HT₇ receptor binding sites

The affinity of the compounds described in this invention for the 5-HT₇ receptor binding site was evaluated by single competition radioligand binding assay. The assay was performed on membranes prepared from HEK-293 cells that had been subjected to stable transfection with the rat 5-HT₇ₐ receptor (GB: NM022938). Cells were scraped from the culture plates, suspended in Tris-HCl 50 mM pH 7.5 and collected through centrifugation (1000 rpm for 5 min). The cell pellets were homogenized (Polytron, 15 s, setting 5) in 50 mM Tris-HCl (pH 7.5), 5 mM EDTA. Following centrifugation (15,000 rpm for 25 min), membranes (135 µg protein/mL) were resuspended in the same buffer and incubated for 60 min at RT with 1 nM [³H]5-CT in the presence of increasing concentration of test compounds. Nonspecific binding was defined in the presence of 10 µM 5-HT. Incubation was stopped by rapid filtration using the cell harvester (Packard). Radioactivity was counted in a TopCount-NXT (Packard).

Sigmoidal inhibition curves were generated and fitted by nonlinear regression analysis (GraphPad Prism). IC₅₀ values (concentration producing 50% inhibition of specific radioligand binding) were calculated. Kᵢ values were derived according to Cheng and Prussoff (Biochem. Pharmacol. (1973) 22: 3099-3108). Experiments were conducted in triplicate.

Stock drug solutions (10 mM) were prepared in DMSO (the final assay concentration of DMSO not exceeding 0.4%). Drug dilutions were prepared in assay buffer. Data are shown in Table 1 below.

### 2. Effect on adenylyl cyclase activity

In vitro functional properties of the compounds described in this invention were evaluated in an adenylyl cyclase assay. HEK-293 cells stably tranfected with the rat 5-HT₇ₐ receptor were plated into 96-well plates. Cells were washed with 200 µL DNEM/F12 and incubated for 10 min with 80 µL of 2 mM 3-isobutyl-1-methylxanthine. Compounds (10 µL) were added for another 10 min. Subsequently, 5-CT (10 µL) was added. After 20 min the incubation was stopped by the addition of 20 µL of 0.5 N HCl. Plates were incubated at 4 °C for 30 min. Twenty µL of the supernatant were assayed for cAMP content with a commercially available kit (Perkin Elmer) using ¹²⁵I-cAMP. Sigmoidal curves of best fit were calculated by nonlinear regression analysis using GrapPad Prism.

5-CT-stimulated adenylyl cyclase activity in r5-HT₇ₐ/HEK-293 cells was inhibited by Example 59 with an estimated pK_{B} ~ 8 in good agreement with the Kᵢ value determined from [³H]5-CT binding studies.

### 3. Affinity for 5-HT_{2A} receptor binding sites

The affinity of the compounds for the rat 5-HT_{2A} receptor was evaluated by competitive radioligand binding assay using [³H]ketanserine as the radioligand. The assay was performed on membranes from rat cortex as previously described (Schotte, A. et al., Psychopharmacology (1996) 124: 57-73). Briefly, brain tissue (rat cortex) was homogenized in 20 volumes per wet weight tissue of Tris-HCl buffer (50 mM, pH 7.4). The total membrane fraction was collected by centrifugation and washed by subsequent centrifugation runs (25 min at 25,000 g at 4 °C). Membranes were re-suspended in Tris-HCl buffer (50 mM, pH 7.4) containing 1 nM [³H]ketanserin. Non-specific binding was estimated in the presence of 10 µM risperidone. The incubation was terminated by rapid filtration over Whatman GF/B filters pre-soaked in 0.1 % polyethylenimine, and one washing step with 1 mL ice-cold Tris-HCl buffer, pH 7.4. pKᵢ values for all compounds were calculated by pKᵢ = -log Kᵢ where Kᵢ was calculated according to the method of Cheng and Prusoff (Biochem Pharmacol. (1973) 22: 3099-3108) (IC₅₀/(1+[S]/K_{d}) were [S] = 1 nM; K_{d} = 0.42 nM). All values in Table 1 are listed in nM units. Data are shown in Table 1 below.

### 4. Affinity for 5HT2 receptor binding sites

Receptor binding was performed using the human recombinant 5-HT_{2A} (GB: X57830), 5-HT_{2B} (GB: Z36748) and 5-HT_{2C} (GB: M81778) receptors. The affinity of the compounds for the 3 different human 5-HT₂ receptor subtypes was evaluated by competitive radioligand binding assays using [³H]ketanserin (h5-HT_{2A}) or [³H]mesulergine (h5-HT_{2B} and h5-HT_{2C}). The assays were performed on membranes prepared from NIH3T3 stably transfected with h5-HT_{2A} or CHO stably transfected with h5-HT_{2B} and h5-HT_{2C}. Kᵢ values for all compounds were calculated according to Cheng and Prusoff equation (Cheng and Prusoff, Biochem. Pharmacol. (1973)22:3099-3108) (IC₅₀/(1+[S]/K_{d}) where [S] = 1 nM (5-HT_{2A}), 4 nM (5-HT_{2B}) and 3 nM (5-HT_{2C}); K_{d} = 0.4 nM (5-HT_{2A}), 3.5 nM (5-HT_{2B}) and 3 nM (5-HT_{2C}). Data are shown in Table 1 below.

### 5. In Vitro Functional Assay for 5-HT2 Receptor (Intracellular Calcium)

In vitro functional properties of these compounds on the different 5-HT₂ receptor subtypes were determined using fluorometric imaging plate reader (FLIPR) based calcium assay as previously described (Porter et al., 1999, Jerman, J.C. et al. Eur. J. Pharmacol. (2001)414:23-30). The 5-HT₂ receptors are linked to the Gq family of G proteins and to subsequent activation of phospholipase C, induction of phosphoinositide metabolism and to an increase in intracellular calcium concentration. The same cell lines as described in the previous section (receptor binding) were used for the FLIPR experiments.

**Table 1. Binding Affinities (nM)**

| EX | Kᵢ | Kᵢ | Kᵢ | Kᵢ |
|---|---|---|---|---|
| | 5-HT₇ | 5-HT_{2A} | 5-HT_{2B} | 5-HT_{2C} |
| 1 | 120 | NT | NT | NT |
| 17 | 70 | NT | NT | NT |
| 18 | 25 | NT | NT | NT |
| 22 | 45 | NT | NT | NT |
| 26 | 18 | NT | NT | NT |
| 38 | pK_{b} 7.8 | NT | NT | NT |
| 47 | 7 | 9 | 64 | 24 |
| 57 | 15 | NT | NT | NT |
| 59 | 6 | 280 | 160 | 74 |
| 64 | 19 | 18 | NT | NT |
| 74 | 5 | 100 | 94 | 180 |
| 75 | 7 | 200 | 100 | 320 |
| 76 | 8 | 210 | 350 | 690 |
| 87 | 33 | NT | NT | NT |
| 98 | 40 | NT | NT | NT |
| 100 | 30 | NT | NT | NT |
| 103 | 7.7 | 60 | 44 | 150 |
| 104 | 9 | 80 | 52 | 360 |
| 108 | 9 | NT | 100 | 800 |
| 111 | 17 | NT | NT | NT |
| 114 | 32 | NT | 90 | 400 |
| 117 | 20 | NT | NT | NT |
| 118 | 8 | 20 | NT | NT |
| 119 | 39 | NT | NT | NT |
| 120 | 40 | NT | NT | NT |
| 131 | 120 | 7 | 4.2 | 50 |
| 133 | 125 | 2.3 | 3.5 | 10 |
| 160 | 7 | 300 | 350 | 3500 |
| 165 | 4 | 100 | 310 | 180 |
| 166 | 8 | 80 | 560 | 590 |
| 167 | 75 | NT | 350 | 10000 |
| 172 | 37 | NT | NT | NT |
| 174 | 40 | NT | NT | NT |
| 177 | 80 | 7 | 7 | 110 |
| 178 | 85 | 3 | 3 | NT |
| 180 | 10 | 1.5 | 1.4 | 12 |
| 181 | 37 | 1.5 | 1.8 | 11 |
| 182 | 90 | 0.74 | 1.4 | 18 |
| 183 | 240 | 7 | 54 | 70 |
| 184 | 120 | 1 | 17 | 15 |
| 186 | 61 | 1 | 24 | 20 |
| 190 | 16 | 10 | 22 | 51 |
| 191 | 30 | NT | NT | NT |
| 192 | 20 | 2.5 | 0.9 | 15 |
| 209 | 6 | 1.1 | 1.4 | 12 |
| 210 | 7 | 2 | 0.75 | 20 |
| 211 | 8.5 | 5 | 0.5 | 18 |
| 212 | 93 | 25 | 12 | 425 |
| 213 | 12 | 7.5 | 4.7 | 80 |
| 214 | 5 | NT | 2 | 170 |
| 215 | 30 | 8 | 95 | NT |
| 216 | 70 | 6 | 20 | 17 |
| 217 | 25 | 1 | 0.65 | 10 |
| 218 | 75 | 1.7 | 1.8 | 15 |
| 220 | 55 | 1.3 | 0.55 | 6.8 |
| 232 | 20 | 25 | 0.50 | 66 |
| 233 | 15 | 4 | 25 | 16 |
| 236 | 950 | 7 | 4.5 | 32 |
| 238 | 40 | 1 | 0.50 | 13 |
| 241 | 310 | 9 | 9.5 | 38 |
| 242 | 21 | 8 | 1.3 | 45 |
| 253 | 75 | NT | 25 | 625 |
| 255 | 60 | NT | NT | NT |
| 257 | 9 | NT | 2 | 110 |
| 273 | 5 | 400 | NT | NT |
| 276 | 90 | 3 | 2.5 | 90 |
| 277 | 150 | 0.9 | 3 | 40 |
| 278 | 35 | 0.1 | 0.2 | 10 |
| 279 | 80 | 0.8 | 1 | 45 |
| 280 | 3300 | 1.5 | 6 | 120 |
| 282 | 100 | 6 | 20 | 200 |
| 283 | 5000 | 10 | 60 | 150 |
| 284 | 10 | 1 | 2 | 60 |
| 285 | 29 | 60 | 6 | 500 |
| 286 | 335 | 50 | 80 | 5000 |
| 298 | 50 | 5 | 6.5 | 60 |
| 299 | 35 | 1.7 | 9 | 26 |
| 300 | 10 | 0.3 | 0.8 | 12 |
| 301 | 40 | 1.6 | 3 | 100 |
| 302 | 100 | 0.2 | 1.3 | 21.5 |
| 305 | 600 | 20 | 60 | 2200 |
| 306 | 120 | 1 | 22 | 39 |
| 308 | 5200 | 2 | 3 | 162 |
| 309 | 130 | 30 | 15 | 130 |
| 310 | 475 | 0.2 | 0.4 | 30 |
| 311 | 80 | 140 | 100 | 3000 |
| 313 | 30 | 100 | 40 | 1000 |
| 315 | 12 | 9 | 3 | 300 |
| 316 | 9.1 | 60 | 530 | 5000 |

| | | | | |
|---|---|---|---|---|
| NT = not tested | | | | |

## Claims

**1.** A compound having serotonin receptor modulating activity of formula (I): 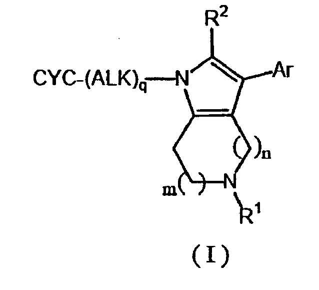 wherein
m is 0, 1 or 2;
n is 1, 2 or 3;
m+n is less than or equal to 4;
q is 0 or 1 ;
Ar is an aryl or heteroaryl ring selected from the group consisting of:
a) phenyl, optionally mono-, di- or tri-substituted with R^{r} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)- or -(CH₂)₁₋₂N(C₁₋₄alkyl)(CH₂)-;
R^{r} is selected from the group consisting of -OH, -C₁₋₆alkyl, -OC₁₋₆alkyl, -C₂₋₆alkenyl, -OC₃₋₆alkenyl, -C₂₋₆alkynyl, -OC₃₋₆alkynyl, -CN, -NO₂, -N(R^{y})R^{z} (wherein R^{y} and R^{z} are independently selected from H or C₁₋₆alkyl), -(C=O)N(R^{y})R^{z}, -(N-R^{t})COR^{t}, -(N-R^{t})SO₂C₁₋₆alkyl (wherein R^{t} is H or C₁₋₆alkyl), -(C=O)C₁₋₆alkyl, -(S=(O)ₙ)-C₁₋₆alkyl (wherein n is selected from 0, 1 or 2), -SO₂N(R^{y})R^{z}, -SCF₃, halo, -CF₃, -OCF₃, -COOH and -COOC₁₋₆alkyl;
b) phenyl or pyridyl fused at two adjacent carbon ring members to a three membered hydrocarbon moiety to form a fused five membered aromatic ring, which moiety has one carbon atom replaced by >O, >S, >NH or >N(C₁₋₄alkyl) and which moiety has up to one additional carbon atom optionally replaced by -N=, the fused rings optionally mono-, di- or tri-substituted with R^{r};
c) phenyl fused at two adjacent ring members to a four membered hydrocarbon moiety to form a fused six membered aromatic ring, which moiety has one or two carbon atoms replaced by -N=, the fused rings optionally mono-, di- or tri-substituted with R^{r};
d) naphthyl, optionally mono-, di- or tri-substituted with R^{r};
e) a monocyclic aromatic hydrocarbon group having five ring atoms, having a carbon atom which is the point of attachment, having one carbon atom replaced by >O, >S, >NH or >N(C₁₋₄alkyl), having up to one additional carbon atoms optionally replaced by -N=, optionally mono- or di-substituted with R^{r} and optionally benzofused or pyridofused at two adjacent carbon atoms, where the benzofused or pyridofused moiety is optionally mono-, di-, or tri-substituted with R^{r}; and
f) a monocyclic aromatic hydrocarbon group having six ring atoms, having a carbon atom which is the point of attachment, having one or two carbon atoms replaced by -N=, optionally mono- or di-substituted with R^{r} and optionally benzofused or pyridofused at two adjacent carbon atoms, where the benzofused or pyridofused moiety is optionally mono- or di-substituted with R^{r};
g) phenyl or pyridyl, substituted with a substituent selected from the group consisting of phenyl, pyridyl, thiophenyl, oxazolyl and tetrazolyl, where the resultant substituted moiety is optionally further mono-, di- or tri-substituted with R^{r};
ALK is a branched or unbranched C₁₋₈alkylene, C₂₋₈alkenylene, C₂₋₈alkynylene or C₃₋₈cycloalkenylene, optionally mono-, di-, or tri-substituted with a substituent independently selected from the group consisting of: -OH, -OC₁₋₆alkyl, -OC₃₋₆cycloalkyl, -CN, -NO₂, -N(R^{a})R^{b} (wherein R^{a} and R^{b} are independently selected from H, C₁₋₆alkyl or C₂₋₆alkenyl), -(C=O)N(R^{a})R^{b}, -(N-R^{c})COR^{c}, -(N-R^{c})SO₂C₁₋₆alkyl (wherein R^{c} is H or C₁₋₆alkyl), -(C=O)C₁₋₆alkyl, -(S=(O)_{d})-C₁₋₆alkyl (wherein d is selected from 0, 1 or 2), -SO₂N(R^{a})R^{b}, -SCF₃, halo, -CF₃, -OCF₃, -COOH and -COOC₁₋₆alkyl;
CYC is hydrogen or a carbocyclic, heterocyclic, aryl or heteroaryl ring selected from the group consisting of:
i) phenyl, optionally mono-, di- or tri-substituted with R^{q} or di-substituted on adjacent carbons with -OC₁₋₄alkyleneO-, -(CH₂)₂₋₃NH-, -(CH₂)₁₋₂NH(CH₂)-, -(CH₂)₂₋₃N(C₁₋₄alkyl)- or -(CH₂)₁₋₂N(C₁₋₄alkyl)(CH₂)-;
R^{q} is selected from the group consisting of -OH, -C₁₋₆alkyl, -OC₁₋₆alkyl, -C₃₋₆cycloalkyl, -OC₃₋₆cycloalkyl, phenyl, -Ophenyl, benzyl, -Obenzyl, -CN, -NO₂, -N(R^{a})R^{b} (wherein R^{a} and R^{b} are independently selected from H, C₁₋₆alkyl or C₂₋₆alkenyl, or R^{a} and R^{b} may be taken together with the nitrogen of attachment to form an otherwise aliphatic hydrocarbon ring, said ring having 5 to 7 members, optionally having one carbon replaced with >O, =N-, >NH or >N(C₁₋₄alkyl), optionally having one carbon substituted with -OH, and optionally having one or two unsaturated bonds in the ring), -(C=O)N(R^{a})R^{b}, -(N-R^{c})COR^{c}, -(N-R^{c})SO₂C₁₋₆alkyl (wherein R^{c} is H or C₁₋₆alkyl or two R^{c} in the same substituent may be taken together with the amide of attachment to form an otherwise aliphatic hydrocarbon ring, said ring having 4 to 6 members), -N-(SO₂C₁₋₆alkyl)₂, -(C=O)C₁₋₆alkyl, -(S=(O)_{d})-C₁₋₆alkyl (wherein d is selected from 0, 1 or 2), -SO₂N(R^{a})R^{b}, -SCF₃, halo, -CF₃, -OCF₃, -COOH and -COOC₁₋₆alkyl;
ii) phenyl or pyridyl fused at two adjacent carbon ring members to a three membered hydrocarbon moiety to form a fused five membered aromatic ring, which moiety has one carbon atom replaced by >O, >S, >NH or >N(C₁₋₄alkyl) and which moiety has up to one additional carbon atom optionally replaced by -N=, the fused rings optionally mono-, di- or tri-substituted with R^{q};
iii) phenyl fused at two adjacent carbon ring members to a four membered hydrocarbon moiety to form a fused six membered aromatic ring, which moiety has one or two carbon atoms replaced by -N=, the fused rings optionally mono-, di- or tri-substituted with R^{q};
iv) naphthyl, optionally mono-, di- or tri-substituted with R^{q};
v) a monocyclic aromatic hydrocarbon group having five ring atoms, having a carbon atom which is the point of attachment, having one carbon atom replaced by >O, >S, >NH or >N(C₁₋₄alkyl), having up to one additional carbon atoms optionally replaced by -N=, optionally mono- or di-substituted with R^{q} and optionally benzofused or pyridofused at two adjacent carbon atoms, where the benzofused or pyridofused moiety is optionally mono-, di-, or tri-substituted with R^{q};
vi) a monocyclic aromatic hydrocarbon group having six ring atoms, having a carbon atom which is the point of attachment, having one or two carbon atoms replaced by -N=, optionally mono- or di-substituted with R^{q} and optionally benzofused or pyridofused at two adjacent carbon atoms, where the benzofused or pyridofused moiety is optionally mono- or di-substituted with R^{q};
vii) a 3-8 membered non-aromatic carbocyclic or heterocyclic ring said ring having 0, 1 or 2 non-adjacent heteroatom members selected from O, S, -N=, >NH or >NR^{q}, having 0, 1 or 2 unsaturated bonds, having 0, 1 or 2 carbon members which is a carbonyl, optionally having one carbon member which forms a bridge, having 0 to 5 substituents R^{q} and optionally benzofused or pyridofused at two adjacent carbon atoms where the benzofused or pyridofused moiety has 0, 1, 2 or 3 substituents R^{q}; and
viii) a 4-7 membered non-aromatic carbocyclic or heterocyclic ring said ring having 0, 1 or 2 non-adjacent heteroatom members selected from O, S, -N=, >NH or >NR^{q}, having 0, 1 or 2 unsaturated bonds, having 0, 1 or 2 carbon members which is a carbonyl and optionally having one carbon member which forms a bridge, the heterocyclic ring fused at two adjacent carbon atoms forming a saturated bond or an adjacent carbon and nitrogen atom forming a saturated bond to a 4-7 membered carbocyclic or heterocyclic ring, having 0 or 1 possibly additional heteroatom member, not at the ring junction, selected from O, S, -N=, >NH or >NR^{q}, having 0, 1 or 2 unsaturated bonds, having 0, 1 or 2 carbon members which is a carbonyl and the fused rings having 0 to 5 substituents R^{q};
R¹ is selected from the group consisting of H, C₁₋₇alkyl, C₂₋₇alkenyl, C₂₋₇alkynyl, C₃₋₇cycloalkyl, C₃₋₇cycloalkylC₁₋₇alkyl, C₃₋₇cycloalkenyl, C₃₋₇cycloalkenylC₁₋₇alkyl and benzo-fusedC₄₋₇cycloalkyl, each optionally mono-, di-, or tri-substituted with R^{p};
R^{p} is selected from the group consisting of -OH, -OC₁₋₆alkyl, -C₃₋₆cycloalkyl, -OC₃₋₆cycloalkyl, -CN, -NO₂, phenyl, pyridyl, thienyl, furanyl, pyrrolyl, -N(R^{s})R^{u} (wherein R^{s} and R^{u} are independently selected from H or C₁₋₆alkyl, or may be taken together with the nitrogen of attachment to form an otherwise aliphatic hydrocarbon ring, said ring having 5 to 7 members, optionally having one carbon replaced with >O, =N-, >NH or >N(C₁₋₄alkyl) and optionally having one or two unsaturated bonds in the ring), -(C=O)N(R^{s})R^{u}, -(N-R^{v})COR^{v}, -(N-R^{v})SO₂C₁₋₆alkyl (wherein R^{v} is H or C₁₋₆alkyl or two R^{v} in the same substituent may be taken together with the amide of attachment to form an otherwise aliphatic hydrocarbon ring, said ring having 4 to 6 members), -(C=O)C₁₋₆alkyl, -(S=(O)ₙ)-C₁₋₆alkyl (wherein n is selected from 0, 1 or 2), -SO₂N(R^{s})R^{u}, -SCF₃, halo, -CF₃, -OCF₃, -COOH and -COOC₁₋₆alkyl, wherein the foregoing phenyl, pyridyl, thienyl, furanyl and pyrrolyl substituents are optionally mono-, di-, or tri-substituted with a substituent independently selected from the group consisting of: -OH, -C₁₋₆alkyl, -OC₁₋₆alkyl, -CN, -NO₂, -N(R^{a})R^{b} (wherein R^{a} and R^{b} are independently selected from H, C₁₋₆alkyl or C₂₋₆alkenyl), -(C=O)N(R^{a})R^{b}, -(N-R^{c})COR^{c}, -(N-R^{c})SO₂C₁₋₆alkyl (wherein R^{c} is H or C₁₋₆alkyl), -(C=O)C₁₋₆alkyl, -(S=(O)_{d})-C₁₋₆alkyl (wherein d is selected from 0, 1 or 2), -SO₂N(R^{a})R^{b}, -SCF₃, halo, -CF₃, -OCF₃, -COOH and -COOC₁₋₆alkyl;
R² is selected from the group consisting of H, C₁₋₇alkyl, C₂₋₇alkenyl, C₂₋₇alkynyl and C₃₋₇cycloalkyl;
and enantiomers, diastereomers, hydrates, solvates and pharmaceutically acceptable salts, esters and amides thereof.

**2.** The compound of claim 1 wherein m is 1 or 2.

**3.** The compound of claim 1 wherein m is 1.

**4.** The compound of claim 1 wherein n is 1 or 2.

**5.** The compound of claim 1 wherein m+n is 2 or 3.

**6.** The compound of claim 1 wherein q is 1.

**7.** The compound of claim 1 wherein Ar, optionally substituted, is selected from the group consisting of:
a) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
b) 4-, 5-, 6- or 7-benzoxazolyl, 4-, 5-, 6- or 7-benzothiophenyl, 4-, 5-, 6-or 7-benzofuranyl, 4-, 5-, 6- or 7-indolyl, 4-, 5-, 6- or 7-benzthiazolyl, 4-, 5-, 6-or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1H-pyrrolo[2,3-b]pyridin-4, 5 or 6-yl, 1 H-pyrrolo[3,2-c]pyridin-4, 6 or 7-yl, 1 H-pyrrolo[2,3-c]pyridin-4, 5 or 7-yl, 1H-pyrrolo[3,2-b]pyridin-5, 6 or 7-yl,
c) 5-, 6-, 7- or 8-isoquinolinyl, 5-, 6-, 7- or 8-quinolinyl, 5-, 6-, 7- or 8-quinoxalinyl, 5-, 6-, 7- or 8-quinazolinyl,
d) naphthyl,
e) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1 ,2,4-triazolyl, 3-indoxazinyl, 2-benzoxazolyl, 2- or 3-benzothiophenyl, 2- or 3-benzofuranyl, 2- or 3-indolyl, 2-benzthiazolyl, 2-benzimidazolyl, 3-indazolyl,
f) pyridinyl, pyridinyl-N-oxide, pyrazinyl, pyrimidinyl, pyridazinyl, 1-, 3- or 4-isoquinolinyl, 2-, 3- or 4-quinolinyl, 2- or 3-quinoxalinyl, 2- or 4-quinazolinyl, [1,5], [1,6], [1,7], or [1,8]naphthyridin-2-, 3-, or 4-yl, [2,5], [2,6], [2,7], [2,8]naphthyridin-1-, 3-, or 4-yl, and
g) biphenyl, 4-tetrazolylphenyl.

**8.** The compound of claim 1 wherein Ar, optionally substituted, is selected from the group consisting of phenyl, pyridyl, thiophen-2-yl and thiophen-3-yl.

**9.** The compound of claim 1 wherein Ar is selected from the group consisting of phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 3-nitrophenyl, 4-nitrophenyl, 3-chloro-4-fluorophenyl, 3-fluoro-4-chlorophenyl, benzo[1,3]dioxol-4 or 5-yl, 3-hydroxyphenyl, 4-hydroxyphenyl, 4-hydroxy-2-methylphenyl, 4-hydroxy-3-fluorophenyl, 3,4-dihydroxyphenyl,4-dimethylaminophenyl, 4-carbamoylphenyl, 4-fluoro-3-methylphenyl, furan-2-yl, furan-3-yl, thiophen-2-yl, thiophen-3-yl, 5-chlorothiophen-2-yl, 5-methylthiophen-2-yl, 5-chlorothiophen-3-yl, 5-methylthiophen-3-yl, 4'-chlorobiphenyl, and 4-tetrazolylphenyl.

**10.** The compound of claim 1 wherein ALK, optionally substituted, is selected from the group consisting of methylene, ethylene, propylene, butylene, tert-butylene, pentylene, 1-ethylpropylene, 2-ethylpropylene, 2-ethylbutylene, isopropylene, but-3-enylene, isobutylene, 3-methylbutylene, allylene, and prop-2-ynylene.

**11.** The compound of claim 1 wherein ALK is selected from the group consisting of methylene, trifluoromethylmethylene, methoxycarbonylmethyl, methylcarbamoylmethyl, ethylene, propylene, 3-methoxycarbonyl propylene, 3-carboxy propylene, butylene, tert-butylene, 4-hydroxybutylene, 4-methoxycarbonyl butylene, 4-carboxy butylene, pentylene, 5-hydroxypentylene, 1-ethylpropylene, 2-ethylpropylene, 2-ethylbutylene, isopropylene, but-3-enylene, isobutylene, 3-methylbutylene, prop-2-ynylene, 2-dimethylaminoethylene, and 2-cyanoethylene.

**12.** The compound of claim 1 wherein CYC, optionally substituted, is hydrogen or is selected from the group consisting of:
i) phenyl, 5-, 6-, 7-, 8-benzo-1,4-dioxanyl, 4-, 5-, 6-, 7-benzo-1,3-dioxolyl, 4-, 5-, 6-, 7-indolinyl, 4-, 5-, 6-, 7-isoindolinyl, 1,2,3,4-tetrahydro-quinolin-4, 5, 6 or 7-yl, 1,2,3,4-tetrahydro-isoquinolin-4, 5, 6 or 7-yl,
ii) 4-, 5-, 6- or 7-benzoxazolyl, 4-, 5-, 6- or 7-benzothiophenyl, 4-, 5-, 6-or 7-benzofuranyl, 4-, 5-, 6- or 7-indolyl, 4-, 5-, 6- or 7-benzthiazolyl, 4-, 5-, 6-or 7-benzimidazolyl, 4-, 5-, 6- or 7-indazolyl, imidazo[1,2-a]pyridin-5, 6, 7 or 8-yl, pyrazolo[1,5-a]pyridin-4, 5, 6 or 7-yl, 1 H-pyrrolo[2,3-b]pyridin-4, 5 or 6-yl, 1 H-pyrrolo[3,2-c]pyridin-4, 6 or 7-yl, 1 H-pyrrolo[2,3-c]pyridin-4, 5 or 7-yl, 1 H-pyrrolo[3,2-b]pyridin-5, 6 or 7-yl,
iii) 5-, 6-, 7- or 8-isoquinolinyl, 5-, 6-, 7- or 8-quinolinyl, 5-, 6-, 7- or 8-quinoxalinyl, 5-, 6-, 7- or 8-quinazolinyl,
iv) naphthyl,
v) furanyl, oxazolyl, isoxazolyl, 1,2,3-oxadiazolyl, 1,2,4-oxadiazolyl, 1,2,5-oxadiazolyl, 1,3,4-oxadiazolyl, thiophenyl, thiazolyl, isothiazolyl, pyrrolyl, imidazolyl, pyrazolyl, 1,2,3-triazolyl, 1,2,4-triazolyl, 3-indoxazinyl, 2-benzoxazolyl, 2- or 3-benzothiophenyl, 2- or 3-benzofuranyl, 2- or 3-indolyl, 2-benzthiazolyl, 2-benzimidazolyl, 3-indazolyl,
vi) pyridinyl, pyridinyl-N-oxide, pyrazinyl, pyrimidinyl, pyridazinyl, 1-, 3- or 4-isoquinolinyl, 2-, 3- or 4-quinolinyl, 2- or 3-quinoxalinyl, 2- or 4-quinazolinyl, [1,5], [1,6], [1,7], or [1,8]naphthyridin-2-, 3-, or 4-yl, [2,5], [2,6], [2,7], [2,8]naphthyridin-1-, 3-, or 4-yl,
vii) cyclopropyl, cyclobutyl, cyclopentyl, cyclohexyl, cyclohexenyl, cycloheptyl, cyclooctyl, adamantyl, pyrrolinyl, pyrrolidinyl, pyrazolinyl, piperidinyl, homopiperidinyl, azepanyl, tetrahydrofuranyl, tetrahydropyranyl, piperazinyl, morpholinyl, thiomorpholinyl, piperidinonyl, indanyl, dihydroindolyl, oxindolyl, dihydropyrrolopyridinyl, and
viii) bicyclo[4.1.0]heptane, octahydroindolyl, octahydroisoindolinyl, decahydroquinolinyl, decahydroisoquinolinyl, octahydropyrrolopyridinyl, and octahydropyrrolopyrrolidinyl.

**13.** The compound of claim 1 wherein CYC, optionally substituted, is selected from the group consisting of hydrogen, phenyl, indolyl, benzthiazolyl, isoquinolyl, quinazolinyl, naphthalen-1 or 2-yl, thiophen-2-yl, thiophen-3-yl, furan-2-yl, furan-3-yl, pyridinyl, cyclobutyl, cyclopentyl, cyclohexyl, cycloheptyl, cyclooctyl, piperidin-2,3 or 4-yl, 2-pyrrolin-2, 3, 4 or 5-yl, 3-pyrrolin-2 or 3-yl, 2-pyrazolin-3, 4 or 5-yl, morpholin-2, 3, 5 or 6-yl, thiomorpholin-2, 3, 5 or 6-yl, piperazin-2, 3, 5 or 6-yl, pyrrolidin-2 or 3-yl, homopiperidinyl, adamantanyl, and octahydroindolyl.

**14.** The compound of claim 1 wherein CYC, optionally substituted, is selected from the group consisting of hydrogen, phenyl, pyridyl, cyclobutyl, cyclopentyl, cyclohexyl, thiophen-2-yl, thiophen-3-yl, tetrahydropyranyl, furan-2-yl, furan-3-yl and naphthalen-1 or 2-yl.

**15.** The compound of claim 1 wherein CYC is selected from the group consisting of hydrogen, phenyl, 2-methoxyphenyl, 3-methoxyphenyl, 4-methoxyphenyl, 2-methylphenyl, 3-methylphenyl, 4-methylphenyl, 4-ethylphenyl, 2-chlorophenyl, 3-chlorophenyl, 4-chlorophenyl, 2-fluorophenyl, 3-fluorophenyl, 4-fluorophenyl, 2-bromophenyl, 3-bromophenyl, 4-bromophenyl, 2-trifluoromethylphenyl, 3-trifluoromethylphenyl, 4-trifluoromethylphenyl, 3-trifluoromethoxyphenyl, 4-trifluoromethoxyphenyl, 2-cyanophenyl, 3-cyanophenyl, 4-cyanophenyl, 3-acetylphenyl, 4-acetylphenyl, 3,4-difluorophenyl, 3,4-dichlorophenyl, 2,3-difluorophenyl, 2,3-dichlorophenyl, 2,4-difluorophenyl, 2,4-dichlorophenyl, 2,6-difluorophenyl, 2,6-dichlorophenyl, 2,6-dimethylphenyl, 2,4,6-trifluorophenyl, 2,4,6-trichlorophenyl, 3,4,5-trimethoxyphenyl, cyclobutyl, cyclohexyl, cyclopentyl, 4-fluoro-3-methylphenyl, 3-nitrophenyl, 4-nitrophenyl, 4-methyl-3-fluorophenyl, 3,4-dimethylphenyl, 4-methoxy-3-fluorophenyl, 4-methoxy-2-methylphenyl, 3-aminophenyl, 4-aminophenyl, 4-carbomethoxyphenyl, 3-methanesulfonylamino-phenyl, 4-methanesulfonylamino-phenyl, 3-dimethanesulfonylamino-phenyl, 4-dimethanesulfonylamino-phenyl, thiophen-2-yl, thiophen-3-yl, 5-chlorothiophen-2-yl, benzo[1,3]dioxol-4 or 5-yl, tetrahydropyran-2,3 or 4-yl, furan-2-yl, furan-3-yl, 5-carboxyethyl-furan-2-yl, naphthalen-1 or 2-yl, 3,4-bisbenzyloxyphenyl, 2-hydroxyphenyl, 3-hydroxyphenyl, 4-hydroxyphenyl, 4-hydroxy-2-methylphenyl, 4-hydroxy-3-fluorophenyl and 3,4-dihydroxyphenyl.

**16.** The compounds of claim 1 wherein R¹ is selected from the group consisting of hydrogen, C₁₋₃alkyl, C₂₋₄alkenyl, C₂₋₄alkynyl, C₃₋₆cycloalkyl, C₃₋₆ cycloalkylC₁₋₃alkyl, C₅₋₆cycloalkenyl, benzo-fusedC₅₋₆cycloalkyl, each optionally mono-, di-, or tri-substituted with R^{P}.

**17.** The compound of claim 1 wherein R¹, optionally R^{P} substituted, is selected from the group consisting of hydrogen, methyl, ethyl, propyl, and isopropyl.

**18.** The compound of claim 1 wherein R¹ is selected from the group consisting of hydrogen, methyl, ethyl, propyl, isopropyl, 3-hydroxypropyl, benzyl, 3,4-dimethoxybenzyl, methoxycarbonylmethyl, carbamoylmethyl, phenethyl, phenpropyl, and hydroxyethyl.

**19.** The compound of claim 1 wherein R² is hydrogen, C₁₋₃alkyl, C₂₋₄alkenyl, C₂₋₄alkynyl, or C₃₋₆cycloalkyl.

**20.** The compound of claim 1 wherein R² is hydrogen or methyl.

**21.** The compound of claim 1 selected from the group consisting of:
| EX | CHEMICAL NAME |
|---|---|
| 1 | 1-Benzyl-3-(4-nitro-phenyl)-4,5,6,7-tetrahydro-1*H*-pyrrolo[3,2-c]pyridine; |
| 2 | 1-Benzyl-3-(3-chloro-4-fluoro-phenyl)-4,5,6,7-tetrahydro-1*H* pyrrolo[3,2-c]pyridine; |
| 3 | 4-(1-Benzyl-4,5,6,7-tetrahydro-1*H*-pyrrolo[3,2-*c*]pyridin-3-yl)-phenol; |
| 4 | 1-Benzyl-3-(4-trifluoromethoxy-phenyl)-4,5,6,7-tetrahydro-1*H*-pyrrolo[3,2-*c*]pyridine; |
| 5 | 1-Benzyl-3-(5-chloro-thiophen-2-yl)-4,5,6,7-tetrahydro-1*H*-pyrrolo[3,2-*c*]pyridine; |
| 6 | 1-Benzyl-3-thiophen-2-yl-4,5,6,7-tetrahydro-1*H*-pyrrolo[3,2-*c*]pyridine; |
| 7 | 1-(3-Chloro-benzyl)-3-phenyl-4,5,6,7-tetrahydro-1*H*-pyrrolo[3,2-*c*]pyridine; |
| 8 | 1-Benzyl-3-(3-fluoro-phenyl)-4,5,6,7-tetrahydro-1*H*-pyrrolo[3,2-*c*]pyridine; |
| 9 | 3-(4-Chloro-phenyl)-1-(2-fluoro-benzyl)-4,5,6,7-tetrahydro-1*H*-pyrrolo[3,2-*c*]pyridine; |
| 10 | 1-(3-Chloro-benzyl)-3-(4-chloro-phenyl)-4,5,6,7-tetrahydro-1 H-pyrrolo[3,2-*c*]pyridine; |
| 11 | 1-(2-Chloro-benzyl)-3-phenyl-4,5,6,7-tetrahydro-1*H*-pyrrolo[3,2-*c*]pyridine; |
| 12 | 1-(4-Chloro-benzyl)-3-(4-chloro-phenyl)-4,5,6,7-tetrahydro-1 H-pyrrolo[3,2-*c*]pyridine; |
| 13 | 1-Benzyl-3-(2,4-dichloro-phenyl)-4,5,6,7-tetrahydro-1*H*-pyrrolo[3,2-*c*]pyridine; |
| 14 | 1-(4-Methoxy-benzyl)-3-phenyl-4,5,6,7-tetrahydro-1 *H*-pyrrolo[3,2-*c*]pyridine; |
| 15 | 1-(2-Chloro-benzyl)-3-(4-chloro-phenyl)-4,5,6,7-tetrahydro-1*H*-pyrrolo[3,2-c]pyridine; |
| 16 | 1-(2,4-Dichloro-benzyl)-3-phenyl-4,5,6,7-tetrahydro-1*H*-pyrrolo[3,2-*c*]pyridine; |
| 17 | 1-Benzyl-2-methyl-3-phenyl-4,5,6,7-tetrahydro-1*H*-pyrrolo[3,2-*c*]pyridine; |
| 18 | 1-Benzyl-3-*p*-tolyl-4,5,6,7-tetrahydro-1*H*-pyrrolo[3,2-*c*]pyridine; |
| 19 | 1-Benzyl-3-(3,4-dichloro-phenyl)-4,5,6,7-tetrahydro-1*H*-pyrrolo[3,2-*c*]pyridine; |
| 20 | 3-Benzo[1,3]dioxol-5-yl-1-benzyl-4,5,6,7-tetrahydro-1*H*-pyrrolo[3,2-c]pyridine; |
| 21 | 1-Benzyl-3-(4-fluoro-phenyl)-4,5,6,7-tetrahydro-1 *H*-pyrrolo[3,2-c]pyridine; |
| 22 | 1-Butyl-3-*p*-tolyl-4,5,6,7-tetrahydro-1*H*-pyrrolo[3,2-c]pyridine; |
| 23 | 1-Benzyl-3-(4-bromo-phenyl)-4,5,6,7-tetrahydro-1*H*-pyrrolo[3,2-c]pyridine; |
| 24 | 1-Benzyl-3-(4-trifluoromethyl-phenyl)-4,5,6,7-tetrahydro-1*H*-pyrrolo[3,2-*c*]pyridine; |
| 25 | 1-Benzyl-3-(4-chloro-phenyl)-4,5,6,7-tetrahydro-1*H*-pyrrolo[3,2-c]pyridine; |
| 26 | 1-Benzyl-3-phenyl-1,4,5,6,7,8-hexahydro-pyrrolo[2,3-*d*]azepine; |
| 27 | 1-Benzyl-3-(5-methyl-thiophen-2-yl)-4,5,6,7-tetrahydro-1*H*-pyrrolo[3,2-c]pyridine; |
| 28 | 1-Benzyl-3-(4-chloro-phenyl)-1,4,5,6,7,8-hexahydro-pyrrolo[2,3-*d*]azepine; |
| 29 | 1-Benzyl-3-(5-chloro-thiophen-2-yl)-1,4,5,6,7,8-hexahydro-pyrrolo[2,3-*d*]azepine; |
| 30 | 1-(4-Chloro-benzyl)-3-phenyl-4,5,6,7-tetrahydro-1*H*-pyrrolo[3,2-c]pyridine; |
| 31 | 1-Benzyl-3-phenyl-4,5,6,7-tetrahydro-1*H*-pyrrolo[3,2-c]pyridine; |
| 32 | 1-Benzyl-3-(3-chloro-phenyl)-1,4,5,6,7,8-hexahydro-pyrrolo[2,3-*d*]azepine; |
| 33 | 1-Benzyl-3-(3-chloro-phenyl)-4,5,6,7-tetrahydro-1*H*-pyrrolo[3,2-c]pyridine; |
| 34 | 1-Benzyl-3-(4-methoxy-phenyl)-4,5,6,7-tetrahydro-1*H*-pyrrolo[3,2-c]pyridine; |
| 35 | 1-Benzyl-3-(4-chloro-phenyl)-5-ethyl-4,5,6,7-tetrahydro-1*H*-pyrrolo[3,2-c]pyridine; |
| 36 | 1-Benzyl-3-(4-chloro-phenyl)-5-isopropyl-4,5,6,7-tetrahydro-*H*-pyrrolo[3,2-c]pyridine; |
| 37 | 3-[1-Benzyl-3-(4-chloro-phenyl)-1,4,6,7-tetrahydro-pyrrolo[3,2-*c*]pyridin-5-yl]-propan-1-ol; |
| 38 | 1-Benzyl-3-(4-chloro-phenyl)-5-methyl-4,5,6,7-tetrahydro-1*H*-pyrrolo[3,2-*c*]pyridine; |
| 39 | 1-Benzyl-3-(3-chloro-phenyl)-5-methyl-4,5,6,7-tetrahydro-1*H*-pyrrolo[3,2-*c*]pyridine; |
| 40 | 1-Benzyl-3-(3-chloro-4-fluoro-phenyl)-5-methyt-4,5,6,7-tetrahydro-1*H*-pyrrolo[3,2-*c*]pyridine; |
| 41 | 1,5-Dibenzyl-3-phenyl-4,5,6,7-tetrahydro-1*H*-pyrrolo[3,2-*c*]pyridine; and |
| 42 | 1-Benzyl-5-isopropyl-3-phenyl-4,5,6,7-tetrahydro-1*H*-pyrrolo[3,2-*c*]pyridine. |

**33.** The compound of claim 1 wherein said pharmaceutically acceptable salt is an effective amino addition salt.

**22.** The compound of claim 1 wherein said pharmaceutically acceptable salt is selected from the group consisting of hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate.

**23.** A pharmaceutical composition comprising a pharmaceutically acceptable carrier and a therapeutically effective amount of a compound according to claim 1.

**24.** A compound according to claim 1 for the treatment or prevention of a CNS disorder selected from the group consisting of: sleep disorders, depression/anxiety, generalized anxiety disorder, schizophrenia, bipolar disorders, psychotic disorders, obsessive-compulsive disorder, mood disorders, post-traumatic stress and other stress-related disorders, migraine, pain, eating disorders, obesity, sexual dysfunction, metabolic disturbances, hormonal imbalance, alcohol abuse, addictive disorders, nausea, inflammation, centrally mediated hypertension, sleep/wake disturbances, jetlag, and circadian rhythm abnormalities in mammals.

**25.** A compound according to claim 24 wherein said CNS disorder is selected from the group consisting of: depression/anxiety, sleep disorders, and circadian rhythm abnormalities.

**26.** A compound according to claim 1 for the treatment or prevention of a disease or condition selected from the group consisting of: hypotension, peripheral vascular disorders, cardiovascular shock, renal disorders, gastric motility, diarrhea, spastic colon, irritable bowel disorders, ischemias, septic shock, urinary incontinence, and other disorders related to the gastrointestinal and vascular systems in mammals.

**27.** A compound according to claim 1 for the treatment or prevention of an ocular disorder selected from the group consisting of: glaucoma, optic neuritis, diabetic retinopathy, retinal edema, and age-related macular degeneration in mammals.

**28.** A compound according to claim 1 for the treatment or prevention of a disease or condition selected from the group consisting of: depression/anxiety, sleep/wake disturbances, jetlag, migraine, urinary incontinence, gastric motility, and irritable bowel disorders in mammals.

**29.** A compound according to claim 1 for the treatment or prevention of a disease or condition selected from the group consisting of: depression/anxiety, generalized anxiety disorder, schizophrenia, bipolar disorders, psychotic disorders, obsessive-compulsive disorder, mood disorders, post-traumatic stress disorders, sleep disturbances, sexual dysfunction, eating disorders, migraine, addictive disorders, and peripheral vascular disorders in mammals.

**30.** A compound of claim 1 isotopically-labelled to be detectable by PET or SPECT.

**31.** A method for studying serotonin-mediated disorders comprising the step of using an ¹⁸F-labeled or ¹¹C-labelled compound of claim 1 as a positron emission tomography (PET) molecular probe.
